(19) Europäisches Patentamt / European Patent Office / Office européen des brevets

(11) EP 2 858 976 B1

(12) **EUROPEAN PATENT SPECIFICATION**

(45) Date of publication and mention of the grant of the patent:
**14.03.2018 Bulletin 2018/11**

(21) Application number: **13727342.1**

(22) Date of filing: **10.05.2013**

(51) Int Cl.:
***C07D 221/26*** *(2006.01)* ***A61K 31/439*** *(2006.01)*
***A61P 1/10*** *(2006.01)* ***A61P 29/00*** *(2006.01)*

(86) International application number:
**PCT/IB2013/000948**

(87) International publication number:
**WO 2013/167963 (14.11.2013 Gazette 2013/46)**

(54) **BENZOMORPHAN COMPOUNDS AS OPIOID RECEPTORS MODULATORS**

BENZOMORPHANVERBINDUNGEN ALS OPIOIDREZEPTORMODULATOREN

COMPOSÉS DE TYPE BENZOMORPHANE UTILISÉS EN TANT QUE MODULATEURS DES RÉCEPTEURS AUX OPIACÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.05.2012 US 201261646068 P**
**12.03.2013 US 201361778091 P**

(43) Date of publication of application:
**15.04.2015 Bulletin 2015/16**

(73) Proprietor: **Purdue Pharma LP**
**Stamford, CT 06901-3431 (US)**

(72) Inventors:
- **TAFESSE, Laykea**
**Robbinsvill,**
**NJ 08690 (US)**
- **PARK, Jae, Hyun**
**Princeton,**
**NJ 08540 (US)**
- **YU, Jianming**
**Plainsboro,**
**NJ 08536 (US)**
- **ROSEN, David**
**Kendall,**
**NJ 08824 (US)**

(74) Representative: **Vos, Derk**
**Maiwald Patentanwalts GmbH**
**Elisenhof**
**Elisenstraße 3**
**80335 München (DE)**

(56) References cited:
**WO-A1-99/65485 WO-A1-2009/068989**
**US-A- 4 425 353**

- **MARTA FILIZOLA ET AL: "Molecular determinants of non-specific recognition of [delta], [mu], and [kappa] opioid receptors", BIOORGANIC & MEDICINAL CHEMISTRY, vol. 9, no. 1, 1 January 2001 (2001-01-01) , pages 69-76, XP055067756, ISSN: 0968-0896, DOI: 10.1016/S0968-0896(00)00223-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 858 976 B1

Printed by Jouve, 75001 PARIS (FR)

## Description

### FIELD OF THE INVENTION

The invention is in the field of medicinal chemistry. It relates to benzomorphan analogs having activity as opioid receptor agonists and/or antagonists. In certain embodiments compounds of the invention have dual activity as opioid agonists and ORL-1 receptor antagonists.

### BACKGROUND OF THE INVENTION

Pain is the most common symptom for which patients seek medical advice and treatment. While acute pain is usually self-limited, chronic pain can persist for 3 months or longer and lead to significant changes in a patient's personality, lifestyle, functional ability and overall quality of life (K.M. Foley, Pain, in Cecil Textbook of Medicine 100-107, J.C. Bennett and F. Plum eds., 20th ed. 1996).

Pain has traditionally been managed by administering either a non-opioid analgesic (such as acetylsalicylic acid, choline magnesium trisalicylate, acetaminophen, ibuprofen, fenoprofen, diflunisal or naproxen), or an opioid analgesic (such as morphine, hydromorphone, methadone, levorphanol, fentanyl, oxycodone or oxymorphone).

Although the term "narcotic" is often used to refer to opioids, the term is not specifically applicable to opioids. The term "narcotic", derived from the Greek word for "stupor", originally referred to any drug that induced sleep, only later being associated with opioids (Gutstein, Howard B., Akil, Huda, "Chapter 21. Opioid Analgesics" (Chapter 21), Brunton, LL, Lazo, JS, Parker, KI: Goodman & Gilman's The Pharmacological Basis of Therapeutics, 11th Edition:http://www.accessmedicine.com/content.aspx?aID=940653). In the legal context, the term "narcotic" refers to a variety of mechanistically unrelated substances with abuse or addictive potential (Gutstein, Howard B., Akil, Huda, "Chapter 21. Opioid Analgesics" (Chapter 21), Brunton LL, Lazo JS, Parker KI: Goodman & Gilman's The Pharmacological Basis of Therapeutics, 11th Edition:http://www.accessmedicine.com/content.aspx?aID=940653). Thus, the term "narcotic" not only refers to opioids, but also refers to such drugs as cocaine, methamphetamine, ecstasy, etc., which exert their pharmacological effects via different receptors than opioids. Furthermore, because the term "narcotic" refers to such a wide variety of unrelated drugs, many of which do not possess analgesic properties, it cannot be assumed that a drug that has "narcotic" properties is necessarily analgesic. For example, drugs such as ecstasy and methamphetamine are not analgesic, and are not used to treat pain.

Until recently, there was evidence of three major classes of opioid receptors in the central nervous system (CNS), with each class having subtype receptors. These receptor classes are known as $\mu$, $\delta$ and $\kappa$. As opiates have a high affinity to these receptors while not being endogenous to the body, research followed in order to identify and isolate the endogenous ligands to these receptors. These ligands were identified as endorphins, enkephalins, and dynorphins, respectively. Additional experimentation has led to the identification of the opioid receptor-like (ORL-1) receptor, which has a high degree of homology to the known opioid receptor classes. This newly discovered receptor was classified as an opioid receptor based only on structural grounds, as the receptor did not exhibit pharmacological homology. It was initially demonstrated that non-selective ligands having a high affinity for $\mu$, $\delta$ and $\kappa$ receptors had low affinity for the ORL-1 receptor. This characteristic, along with the fact that an endogenous ligand had not yet been discovered, led to the ORL-1 receptor being designated as an "orphan receptor".

Subsequent research led to the isolation and structure of the endogenous ligand of the ORL-1 receptor. This ligand, nociceptin (also known as orphanin FQ (OFQ)), is a seventeen amino acid peptide structurally similar to members of the opioid peptide family. (C. Altier et al., "ORL-1 receptor-mediated internalization of N-type calcium channels." Nature Neuroscience, 2005, 9:31).

The discovery of the ORL-1 receptor and its endogenous ligand, presents an opportunity for the discovery of compounds that can be administered for pain management or other syndromes influenced by this receptor.

Many publications in the ORL-1/nociceptin field provide evidence that activation of ORL-1 receptors in the brain can inhibit opioid-mediated analgesia (*e.g.*, D. Barlocco et al., "The opioid-receptor-like 1 (ORL-1) as a potential target for new analgesics." Eur. J. Med. Chem., 2000, 35:275; J.S. Mogil et al., "Orphanin FQ is a functional anti-opioid peptide." Neurosci., 1996, 75:333; K. Lutfy et al., "Tolerance develops to the inhibitory effect of orphanin FQ on morphine-induced antinociception in the rat." NeuroReport, 1999, 10:103; M.M. Morgan et al., "Antinociception mediated by the periaqueductal gray is attenuated by orphanin FQ." NeuroReport, 1997, 8:3431; and J. Tian et al., "Involvement of endogenous Orphanin FQ in electroacupuncture-induced analgesia." NeuroReport, 1997, 8:497).

A growing body of evidence supports a more generalized regulatory role for ORL-1 against the actions of the $\mu$ receptor, possibly contributing to the development of [$\mu$-agonist tolerance in patients being treated with classical opiates (*e.g.*, J. Tian et al., "Functional studies using antibodies against orphanin FQ/nociceptin." Peptides, 2000, 21:1047; and H. Ueda et al., "Enhanced Spinal Nociceptin Receptor Expression Develops Morphine Tolerance and Dependence." J. Neurosci., 2000, 20:7640). Moreover, ORL-1 activation appears to have an inhibitory effect on the

rewarding properties of several drugs of abuse, including μ agonists.

Use of opioid analgesics often leads to constipation as a side effect. Constipation associated with the use of opioid analgesics is presumed to occur primarily and mechanistically as a result of the action of mu opioid agonists directly upon mu opioid receptors located in the bowel (Wood & Galligan (2004), Function of opioids in the enteric nervous system. Neurogastroenterology & Motility 16(Suppl.2): 17-28.). Stimulation of the mu opioid receptors in the bowel causes inhibition of normal gastrointestinal (GI) motility, leading to constipation. The effect of μ opioid agonism on μ opioid receptors in the bowel can be observed via the action of loperamide (Imodium™) in treating diarrhea. Loperamide is a potent μ opioid agonist that is administered orally, but which has little to no absorption into the blood stream. As a result, loperamide exerts its action locally upon the μ opioid receptors in the bowel, and this results in inhibition of GI motility, which treats diarrhea.

There has been recent interest in developing combinations of μ receptor agonists and antagonists having defined biodistribution properties that might serve to limit opioid-induced constipation. For example, the co-administration of an orally bio-available μ opioid receptor agonist (such as morphine, codeine, oxycodone or hydormorphone) together with a potent μ opioid receptor antagonist (such as N-methylnaloxone or N-methylnaltrexone) that is not orally bio-available may serve to prevent or reduce the constipation otherwise associated with mu opioid receptor agonist therapy. The rationale is that the agonist component will be absorbed and distributed throughout the periphery and the central nervous system (CNS), resulting in the desired analgesia, while the antagonist component will remain in the bowel where it will prevent or reduce any agonist-induced constipation that might otherwise occur.

Benzomorphan analog compounds, such as 3,11,11-trimethyl-1,2,3,4,5,6-hexahydro-2,6-methanobenzo[*d*]azocine-6,8-diol and 8-methoxy-3,11,11-trimethyl-1,2,3,4,5,6-hexahydro-2,6-methanobenzo[*d*]azocin-6-ol, having analgesic activity have been described (*e.g.* US 4,425,353; US 4,406,904; and US 4,366,325).

M. Filizola et al. describe in Bioorganic and Medicinal Chemistry, Vol. 9, No. 1, 2001, pages 69 to 76 molecular determinants of non-specific recognition of δ, μ, and κ opioid receptors.

The use of benzomorphan derivatives as analgesics is also disclosed in WO 99/65485 A1.

Benzomorphan compounds having a quarternary amine useful for treating constipation, preferably constipation caused by mu-opioid agonist therapy, are disclosed in WO 2009/068989 A1.

## BRIEF SUMMARY OF THE INVENTION

The present invention provides benzomorphan analog compounds useful for treating a variety of conditions, including pain, in particular chronic pain, and constipation. More specifically, the present invention provides compounds of Formula I as defined in claim 1, and the pharmaceutically acceptable salts, and solvates thereof, that exhibit affinity for one or more of the ORL-1, μ, δ, and/or κ opioid receptors. Such compounds, salts, and solvates are collectively referred to hereinafter as "Compounds of the Invention" (each is individually referred to hereinafter as a "Compound of the Invention"). The present disclosure furthermore provides compounds of Formula I and Formula I', below, and the pharmaceutically acceptable salts, and solvates thereof, that exhibit affinity for one or more of the ORL-1, μ, δ, and/or κ opioid receptors.

The present disclosure provides compounds of Formula I:

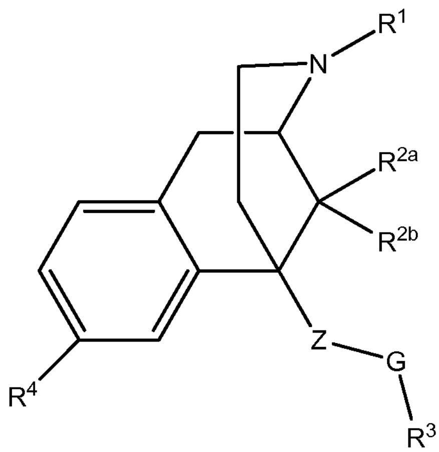

wherein

$R^1$ is selected from the group consisting of-$(C_1$-$C_{10})$alkyl, -$(C_2$-$C_{10})$alkenyl, -$(C_2$-$C_{10})$alkynyl, -$(C_3$-$C_{12})$cycloalkyl,

$(C_3-C_{12})$cycloalkyl-$(C_1-C_6)$alkyl-, -$(C_3-C_{12})$cycloalkenyl, $(C_3-C_{12})$cycloalkenyl-$(C_1-C_6)$alkyl-, -(6- to 14-membered)aryl, ((6-to 14-membered)aryl)-$(C_1-C_6)$alkyl-, -$(OCH_2CH_2)_s$-O-$(C_1-C_6)$alkyl, -$(CH_2CH_2O)_s$-$(C_1-C_6)$alkyl, $(C_1-C_{10})$alkoxy, $C(halo)_3$, $CH(halo)_2$, $CH_2$(halo), C(O)$R^5$, -C(O)O-$(C_1-C_{10})$alkyl, and -$(CH_2)_n$-N$(R^6)_2$, each of which is optionally substituted by 1, 2 or 3 independently selected $R^9$ groups;

$R^{2a}$ and $R^{2b}$ are each independently selected from:

(a) -H; or
(b) -$(C_1-C_5)$alkyl, -$(C_2-C_5)$alkenyl, or -$(C_2-C_5)$alkynyl;

Z is absent or -$(CH_2)_m$-, optionally substituted with 1 or 2-$(C_1-C_6)$alkyl;

G is selected from the group consisting of:

a) a bond, -$(C_1-C_6)$alkylene, -$(C_2-C_6)$alkenylene;
b) O, -OCO-, -C(=O);
c) $NR^8$,;
d) S, SO, and $SO_2$;

$R^3$ is selected from the group consisting of hydrogen, -$(C_1-C_{10})$alkyl, -$(C_2-C_{12})$alkenyl, - C(=O), C(=O)-$(C_1-C_6)$alkyl-, -C(=O)-$(C_1-C_6)$alkyl, -C(=O)-(6- to 14-membered)aryl, - C(=O)-/5- to 12-membered)heteroaryl, -$(C_2-C_{12})$alkynyl, -$(C_1-C_{10})$alkoxy, - $(OCH_2CH_2)_s$-O$(C_1-C_6)$alkyl, -$(CH_2CH_2O)_s$-$(C_1-C_6)$alkyl, -$NH_2$, -NH$(C_1-C_6)$alkyl, CN, -CON$R^5R^6$, -$(C_1-C_6)$alkyl-CO-N$R^5R^6$, -COO$R^7$, -$(C_1-C_6)$alkyl-CO-O$R^7$, -$(C_1-C_6)$alkoxy-COO$R^7$, -CO-$(CH_2)_n$-COO$R^7$, -CO-$(CH_2)_n$-CO-N$R^5R^6$, -$(C_3-C_{12})$cycloalkyl, (($C_3-C_{12}$)cycloalkyl)-$(C_1-C_6)$alkyl-, -$(C_4-C_{12})$cycloalkenyl, (($C_4-C_{12}$)cycloalkenyl)-$(C_1-C_6)$alkyl-, -$(C_6-C_{14})$bicycloalkyl, (($C_6-C_{14}$)bicycloalkyl)-$(C_1-C_6)$alkyl-, -$(C_8-C_{20})$tricycloalkyl, (($C_8-C_{20}$)tricycloalkyl)-$(C_1-C_6)$alkyl-, -$(C_7-C_{14})$bicycloalkenyl, (($C_7-C_{14}$)bicycloalkenyl)-$(C_1-C_6)$alkyl-, -$(C_8-C_{20})$tricycloalkenyl, (($C_8-C_{20}$)tricycloalkenyl)-$(C_1-C_6)$alkyl-, -(6- to 14-membered)aryl, ((6-to 14-membered)aryl)-$(C_1-C_6)$alkyl-, -(7-to 12-membered)bicyclic ring system, ((7- to 12-membered)bicyclic ring system)-$(C_1-C_6)$alkyl-, -(7- to 12-membered)bicyclic aryl, ((7- to 12-membered)bicyclic aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12 membered)heterocycle)-(C1-$C_6$)alkyl-, -(7- to 12-membered)bicycloheterocycle, ((7- to 12-membered)bicycloheterocycle)-$(C_1-C_6)$alkyl-, phenyl, benzyl and naphthyl; each of which is optionally substituted with one, two, or three substituents independently selected from the group consisting of -OH, (=O), halo, -$C(halo)_3$, -$CH(halo)_2$, -$CH_2$(halo), -$(C_1-C_6)$alkyl, halo$(C_1-C_6)$alkyl-, -$(C_2-C_6)$alkenyl, -$(C_2-C_6)$alkynyl, hydroxy$(C_1-C_6)$alkyl-, dihydroxy$(C_1-C_6)$alkyl-, -$(C_1-C_6)$alkoxy, (($C_1-C_6$)alkoxy)CO$(C_1-C_6)$alkoxy-, phenyl, benzyl, -$NH_2$, -N$R^5R^6$, -NH$(C_1-C_6)$alkyl, -$(C_1-C_6)$alkyl-NH$(C_1-C_6)$alkyl $R^{14}$, -CN, -SH, -O$R^4$, -CON$R^5R^6$, -($C_1$-$C_6$alkyl)-CO-N$R^5R^6$, -COO$R^7$, -$(C_1-C_6)$alkyl-CO-O$R^7$, -$(C_1-C_6)$alkoxy-COO$R^7$, -$(OCH_2CH_2)_s$-O$(C_1-C_6)$alkyl, - $(CH_2CH_2O)_s$-$(C_1-C_6)$alkyl, (($C_1-C_6$)alkyl)sulfonyl$(C_1-C_6)$alkyl-, -NH-$SO_2(C_1-C_6)$alkyl, -N$(SO_2(C_1-C_6)alkyl)_2$, -C(=NH)$NH_2$, -NH-CO-$(C_1-C_6)$alkyl, -NH-CO-$NH_2$, -NH-C(=O)-NH-$(C_1-C_6)$alkyl, -NH-C(=O)-(6- to 14- membered)aryl, -NH-C(=O)-$(C_1-C_6)$alkyl-(6- to 14- membered)aryl, -NH-$(C_1-C_6)$alkyl-CO-O$R^7$, -NH-C(=O)-$(C_1-C_6)$alkyl-CO-O$R^7$, -NH-C(=O)-CH($NH_2$)-$(C_1-C_6)$alkyl-CO-O$R^7$, -$(C_3-C_{12})$cycloalkyl, (($C_3-C_{12}$)cycloalkyl)-$(C_1-C_6)$alkyl-, -(6- to 14-membered)aryl, -(6- to 14-membered)aryloxy, -$(C_1-C_6)$alkoxyC(O)N$R^5R^6$, -NH-$(C_1-C_6)$alkylC(O)-N$R^5R^6$, - C(O)NH-$(C_1-C_6)$alkyl-COO$R^7$, ((6- to 14-membered)aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12-membered)heterocycle)-$(C_1-C_6)$alkyl-, -(7- to 12-membered)bicycloheterocycle, and ((7- to 12-membered)bicycloheterocycle)-$(C_1-C_6)$alkyl-;

$R^4$ is selected from

(a) -H, -OH, halo, -$C(halo)_3$, -$CH(halo)_2$, -$CH_2$(halo), COOH, or $CONH_2$; or
(b) -$(C_1-C_5)$alkyl, -$(C_2-C_5)$alkenyl, -$(C_2-C_5)$alkynyl, -$(CH_2)_n$-O-$(CH_2)_n$-$CH_3$, or -$(C_1-C_5)$alkoxy, each of which is optionally substituted with 1, 2, or 3 independently selected $R^9$ groups;

$R^5$ and $R^6$ are each independently selected from

(a) hydrogen, -OH, halo, -$C(halo)_3$, -$CH(halo)_2$, -$CH_2$(halo); or
(b) -$(C_1-C_6)$alkyl, -$(C_2-C_5)$alkenyl, -$(C_2-C_5)$alkynyl, -$(CH_2)_n$-O-$(CH_2)_n$-$CH_3$, -$(C_1-C_6)$alkoxy, each of which is optionally substituted with 1, 2, or 3 independently selected $R^9$ groups; or
(c) -$(C_3-C_8)$cycloalkyl, (($C_3-C_8$)cycloalkyl)-$(C_1-C_6)$alkyl-, -COO$R^7$, -$(C_1-C_6)$alkyl-COO$R^7$, -$CONH_2$, or

$(C_1-C_6)$alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl, - $(C_2-C_6)$alkynyl, $-(C_3-C_{12})$cycloalkyl, $-(C_4-C_{12})$cycloalkenyl, $((C_3-C_{12})$cycloalkyl)-$(C_1-C_6)$alkyl-, and $((C_4-C_{12})$cycloalkenyl)-$(C_1-C_6)$alkyl-;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)-$(C_1-C_6)$alkyl-, $((C_3-C_{12})$cycloalkenyl)-$(C_1-C_6)$alkyl-, -C(=O)$(C_1-C_6)$alkyl or $SO_2(C_1-C_6)$alkyl;

each $R^9$ is independently selected from -OH, halo, $-(C_1-C_{10})$alkyl, $-(C_2-C_{10})$alkenyl, $-(C_2-C_{10})$alkynyl, $-(C_1-C_{10})$alkoxy, $-(C_3-C_{12})$cycloalkyl , -CHO, -C(O)OH, $-C(halo)_3$, - $CH(halo)_2$, $CH_2(halo)$, or $-(CH_2)_n-O-(CH_2)_n-CH_3$;

each $R^{14}$ is independently selected from the group consisting of $-COOR^7$, $-(C_1-C_6)$alkyl-$COOR^7$, -C(=O)-$(C_1-C_6)$alkyl-$COOR^7$, $-(C_1-C_6)$alkyl-C(=O)-$(C_1-C_6)$alkyl-$COOR^7$, $CONH_2$, and $-(C_1-C_6)$alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts, and solvates thereof;

provided that when $R^1$, $R^{2a}$, and $R^{2b}$ are all methyl, and $R^4$ is OH or methoxy, then Z-G-$R^3$ is not OH.

In one embodiment, the present disclosure is directed to compounds of Forumla I described above, wherein the following compounds are excluded

(ii) provided that when Z is absent and G is selected as -O, then $R^3$ is not H, $(C_1-C_{10})$alkyl, $CH_2CH_2O-(C_1-C_6)$alkyl), $(C_2-C_{12})$alkenyl, $(C_2-C_{12})$alkynyl, (6- to 14-membered)aryl-$(C_1-C_6)$alkyl, (7- to 12-membered)bicyclic ring system-$(C_1-C_6)$alkyl, or (7- to 12-membered)bicyclic aryl-$(C_1-C_6)$alkyl;
(iii) provided that when Z is absent and G is selected as a bond, then $R^3$ is not $-(C_1-C_{10})$alkoxy, or $OCH_2CH_2-O(C_1-C_6)$alkyl;
(iv) provided that when Z is absent and G is selected as -O, then $R^3$ is not C(=O), (C=O)-$(C_1-C_6)$alkyl, or (C=O)-(6- to 14-membered aryl);
(v) provided that when Z is absent and G is selected as a bond, then $R^3$ is not H or phenyl; and
(vi) provided that Z-G-$R^3$ is not unsubstituted $(C_1-C_6)$alkyl.

The present invention provides compounds of Formula I:

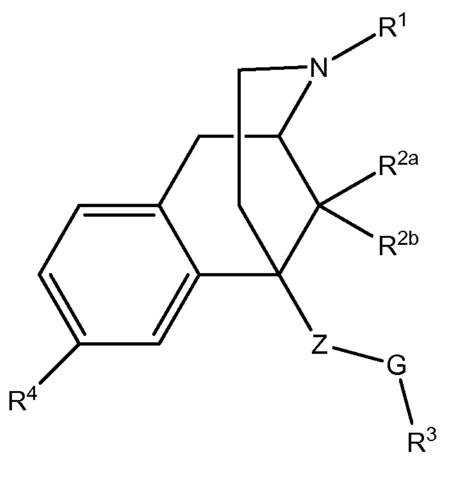

wherein

$R^1$ is selected from the group consisting of -($C_3$-$C_{12}$)cycloalkyl, ($C_3$-$C_{12}$)cycloalkyl-($C_1$-$C_6$)alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or
(b) -($C_1$-$C_5$)alkyl, -($C_2$-$C_5$)alkenyl, or -($C_2$-$C_5$)alkynyl;

Z is absent or -$(CH_2)_m$-, optionally substituted with 1 or 2 independently selected -($C_1$-$C_6$)alkyl;

G is selected from the group consisting of:

a) -($C_1$-$C_6$)alkylene, -($C_2$-$C_6$)alkenylene; or
b) -C(=O); or
c) $NR^8$;

$R^3$ is selected from the group consisting of hydrogen, -($C_1$-$C_{10}$)alkyl, -($C_2$-$C_{12}$)alkenyl, -C(=O)-($C_1$-$C_6$)alkyl, -C(=O)-(6- to 14-membered)aryl, -C(=O)-(5- to 12-membered)heteroaryl, -($C_2$-$C_{12}$)alkynyl, -($C_1$-$C_{10}$)alkoxy, -$(OCH_2CH_2)_s$-O($C_1$-$C_6$)alkyl, -$(CH_2CH_2O)_s$-($C_1$-$C_6$)alkyl, -$NH_2$, -NH($C_1$-$C_6$)alkyl, CN, -$CONR^5R^6$, -($C_1$-$C_6$)alkyl-$CONR^5R^6$, -$COOR^7$, -($C_1$-$C_6$)alkyl-$COOR^7$, -($C_1$-$C_6$)alkoxy-$COOR^7$, -C(=O)-$(CH_2)_n$-$COOR^7$, -C(=O)-$(CH_2)_n$-$CONR^5R^6$, -($C_3$-$C_{12}$)cycloalkyl, (($C_3$-$C_{12}$)cycloalkyl)-($C_1$-$C_6$)alkyl-, -($C_4$-$C_{12}$)cycloalkenyl, (($C_4$-$C_{12}$)cycloalkenyl)-($C_1$-$C_6$)alkyl-, -($C_6$-$C_{14}$)bicycloalkyl, (($C_6$-$C_{14}$)bicycloalkyl)-($C_1$-$C_6$)alkyl-, -($C_8$-$C_{20}$)tricycloalkyl, (($C_8$-$C_{20}$)tricycloalkyl)-($C_1$-$C_6$)alkyl-, -($C_7$-$C_{14}$)bicycloalkenyl, (($C_7$-$C_{14}$)bicycloalkenyl)-($C_1$-$C_6$)alkyl-, -($C_8$-$C_{20}$)tricycloalkenyl, (($C_8$-$C_{20}$)tricycloalkenyl)-($C_1$-$C_6$)alkyl-, -(6- to 14-membered)aryl, ((6- to 14-membered)aryl)-($C_1$-$C_6$)alkyl-, -(7- to 12-membered)bicyclic ring system, ((7- to 12-membered)bicyclic ring system)-($C_1$-$C_6$)alkyl-, -(7- to 12-membered)bicyclic aryl, ((7- to 12-membered)bicyclic aryl)-($C_1$-$C_6$)alkyl-, -(5- to 12-membered)heteroaryl, ((5- to 12-membered)heteroaryl)-($C_1$-$C_6$)alkyl-, -(3- to 12-membered)heterocycle, ((3- to 12 membered)heterocycle)-($C_1$-$C_6$)alkyl-, -(7- to 12-membered)bicycloheterocycle, and ((7- to 12-membered)bicycloheterocycle)-($C_1$-$C_6$)alkyl-; each of which is optionally substituted with one, two, or three substituents independently selected from the group consisting of -OH, (=O), halo, -$C(halo)_3$, -$CH(halo)_2$, -$CH_2$(halo), -($C_1$-$C_6$)alkyl, halo($C_1$-$C_6$)alkyl-, -($C_2$-$C_6$)alkenyl, -($C_2$-$C_6$)alkynyl, hydroxy($C_1$-$C_6$)alkyl-, dihydroxy($C_1$-$C_6$)alkyl-, -($C_1$-$C_6$)alkoxy, (($C_1$-$C_6$)alkoxy)-C(=O)-($C_1$-$C_6$)alkoxy-, -$NH_2$, -$NR^5R^6$, -NH($C_1$-$C_6$)alkyl, -($C_1$-$C_6$)alkyl-NH($C_1$-$C_6$)alkyl-$R^{14}$, -CN, -SH, -$OR^4$, -$CONR^5R^6$, -($C_1$-$C_6$alkyl)-C(=O)-$NR^5R^6$, -$COOR^7$, -($C_1$-$C_6$)alkyl-$COOR^7$, -($C_1$-$C_6$)alkoxy-$COOR^7$, -$(OCH_2CH_2)_s$-O($C_1$-$C_6$)alkyl, -$(CH_2CH_2O)_s$-($C_1$-$C_6$)alkyl, (($C_1$-$C_6$)alkyl)sulfonyl($C_1$-$C_6$)alkyl-, -NH-$SO_2$($C_1$-$C_6$)alkyl, -N-($SO_2$-($C_1$-$C_6$)alkyl$)_2$, -C(=NH)-$NH_2$, -NH-C(=O)-($C_1$-$C_6$)alkyl, -NH-C(=O)-$NH_2$, -NH-C(=O)-NH-($C_1$-$C_6$)alkyl, -NH-C(=O)-(6- to 14- membered)aryl, -NH-C(=O)-($C_1$-$C_6$)alkyl-(6- to 14- membered)aryl, -NH-($C_1$-$C_6$)alkyl-$COOR^7$, -NH-C(=O)-($C_1$-$C_6$)alkyl-$COOR^7$, -NH-C(=O)-CH($NH_2$)-($C_1$-$C_6$)alkyl-C(=O)-$OR^7$, -($C_3$-$C_{12}$)cycloalkyl, (($C_3$-$C_{12}$)cycloalkyl)-($C_1$-$C_6$)alkyl-, -(6- to 14-membered)aryl, -(6- to 14-membered)aryloxy, -($C_1$-$C_6$)alkoxyC(O)$NR^5R^6$, -NH-($C_1$-$C_6$)alkylC(O)-$NR^5R^6$, -C(O)NH-($C_1$-$C_6$)alkyl-$COOR^7$, ((6- to 14-membered)aryl)-($C_1$-$C_6$)alkyl-, -(5- to 12-membered)heteroaryl, ((5- to 12-membered)heteroaryl)-($C_1$-$C_6$)alkyl-, -(3- to 12-membered)heterocycle, ((3- to 12-membered)heterocycle)-($C_1$-$C_6$)alkyl-, -(7- to 12-membered)bicycloheterocycle, and ((7- to 12-membered)bicycloheterocycle)-($C_1$-$C_6$)alkyl-;

$R^4$ is selected from

(a) -H, -OH, halo, -$C(halo)_3$, -$CH(halo)_2$, -$CH_2$(halo), COOH, or $CONH_2$; or
(b) -($C_1$-$C_5$)alkyl, -($C_2$-$C_5$)alkenyl, -($C_2$-$C_5$)alkynyl, -$(CH_2)_n$-O-$(CH_2)_n$-$CH_3$, or -($C_1$-$C_5$)alkoxy, each of which is optionally substituted with 1, 2, or 3 independently selected $R^9$ groups;

$R^5$ and $R^6$ are each independently selected from

(a) hydrogen, -OH, halo, -$C(halo)_3$, -$CH(halo)_2$, -$CH_2$(halo); or
(b) -($C_1$-$C_6$)alkyl, -($C_2$-$C_5$)alkenyl, -($C_2$-$C_5$)alkynyl, -$(CH_2)_n$-O-$(CH_2)_n$-$CH_3$, -($C_1$-$C_6$)alkoxy, each of which is optionally substituted with 1, 2, or 3 independently selected $R^9$ groups; or
(c) -($C_3$-$C_8$)cycloalkyl, (($C_3$-$C_8$)cycloalkyl)-($C_1$-$C_6$)alkyl-, -$COOR^7$, -($C_1$-$C_6$)alkyl-$COOR^7$, -$CONH_2$, or ($C_1$-$C_6$)alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl, $-(C_2-C_6)$alkynyl, $-(C_3-C_{12})$cycloalkyl, $-(C_4-C_{12})$cycloalkenyl, $((C_3-C_{12})$cycloalkyl)$-(C_1-C_6)$alkyl-, and $((C_4-C_{12})$cycloalkenyl)$-(C_1-C_6)$alkyl-;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)$-(C_1-C_6)$alkyl-, $((C_3-C_{12})$cycloalkenyl)$-(C_1-C_6)$alkyl-, $-C(=O)(C_1-C_6)$alkyl or $SO_2(C_1-C_6)$alkyl;

each $R^9$ is independently selected from -OH, halo, $-(C_1-C_{10})$alkyl, $-(C_2-C_{10})$alkenyl, $-(C_2-C_{10})$alkynyl, $-(C_1-C_{10})$alkoxy, $-(C_3-C_{12})$cycloalkyl, -CHO, -C(O)OH, $-C(halo)_3$, - $CH(halo)_2$, $CH_2$(halo), or $-(CH_2)_n-O-(CH_2)_n-CH_3$;

each $R^{14}$ is independently selected from the group consisting of $-COOR^7$, $-(C_1-C_6)$alkyl-$COOR^7$, $-C(=O)-(C_1-C_6)$alkyl-$COOR^7$, $-(C_1-C_6)$alkyl-$C(=O)-(C_1-C_6)$alkyl-$COOR^7$, $CONH_2$, and $-(C_1-C_6)$alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof;

provided that Z-G-$R^3$ is not unsubstituted $(C_1-C_6)$alkyl.

In certain embodiments, the present invention provides compounds of Formula IA:

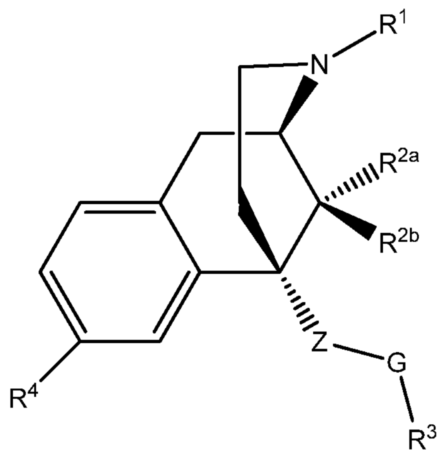

IA

wherein $R^1$, $R^{2a}$, $R^{2b}$, $R^3$, $R^4$, Z, and G are as defined above for Formula I.

In certain embodiments, the present invention provides compounds of Formula IB:

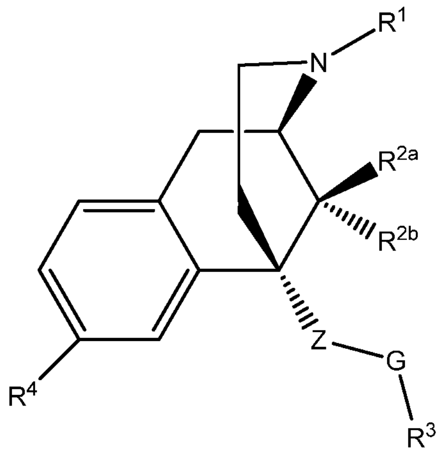

IB

wherein $R^1$, $R^{2a}$, $R^{2b}$, $R^3$, $R^4$, Z, and G are as defined above for Formula I.

In certain embodiments, the present invention provides compounds of Formula IC:

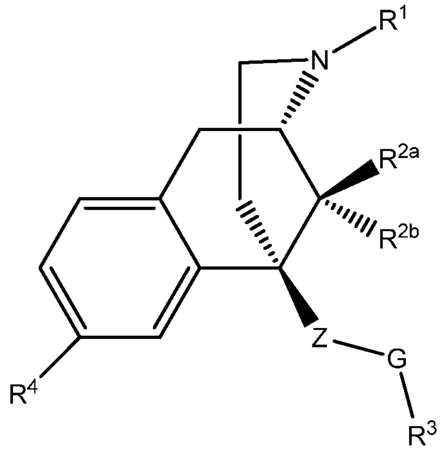

IC

wherein $R^1$, $R^{2a}$, $R^{2b}$, $R^3$, $R^4$, Z, and G are as defined above for Formula I.

In certain embodiments, the present invention provides compounds of Formula ID:

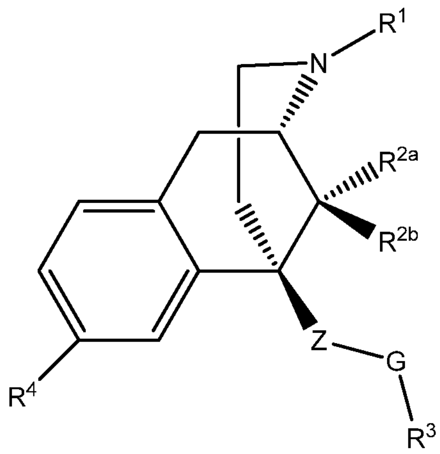

ID

wherein $R^1$, $R^{2a}$, $R^{2b}$, $R^3$, $R^4$, Z, and G are as defined above for Formula I.

The present disclosure further provides compounds of Formula I':

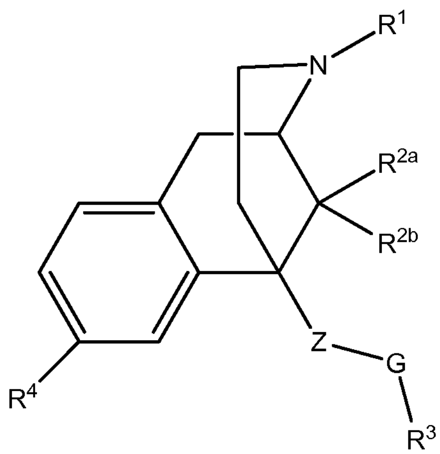

wherein

$R^1$ is selected from the group consisting of $-(C_1-C_{10})$alkyl, $-(C_2-C_{10})$alkenyl, $-(C_2-C_{10})$alkynyl, $-(C_3-C_{12})$cycloalkyl, $(C_3-C_{12})$cycloalkyl-$(C_1-C_6)$alkyl-, $-(C_3-C_{12})$cycloalkenyl, $(C_3-C_{12})$cycloalkenyl-$(C_1-C_6)$alkyl-, -(6- to 14-membered)aryl, ((6-to 14-membered)aryl)-$(C_1-C_6)$alkyl-, $-(OCH_2CH_2)_s$;-O-$(C_1-C_6)$alkyl, $-(CH_2CH_2O)_s-(C_1-C_6)$alkyl, $(C_1-C_{10})$alkoxy, $C(halo)_3$, $CH(halo)_2$, $CH_2(halo)$, $C(O)R^5$, $-C(O)O-(C_1-C_{10})$alkyl, and $-(CH_2)_n-N(R^6)_2$, each of which is optionally substituted by 1, 2 or 3 independently selected $R^9$ groups;

$R^{2a}$ and $R^{2b}$ are each independently selected from:

(a) -H; or
(b) $-(C_1-C_5)$alkyl, $-(C_2-C_5)$alkenyl, or $-(C_2-C_5)$alkynyl;

Z is absent or $-(CH_2)_m-$, optionally substituted with 1 or 2 independently selected $-(C_1-C_6)$alkyl;

G is selected from the group consisting of:

a) a bond, $-(C_1-C_6)$alkylene, $-(C_2-C_6)$alkenylene;
b) O, -OCO-, -C(=O);
c) $NR^8$;

d) S, SO, and $SO_2$;

$R^3$ is selected from the group consisting of hydrogen, -($C_1$-$C_{10}$)alkyl, -($C_2$-$C_{12}$)alkenyl, - C(=O), C(=O)-($C_1$-$C_6$)alkyl-, -C(=O)-(6- to 14-membered)aryl, -C(=O)-(5- to 12-membered)heteroaryl, -($C_2$-$C_{12}$)alkynyl, -($C_1$-$C_{10}$)alkoxy, -($OCH_2CH_2$)$_s$-O($C_1$-$C_6$)alkyl, -($CH_2CH_2O$)$_s$-($C_1$-$C_6$)alkyl, -$NH_2$, -NH($C_1$-$C_6$)alkyl, CN, -CONR$^5$R$^6$, -($C_1$-$C_6$)alkyl-CONR$^5$R$^6$, -COOR$^7$, -($C_1$-$C_6$)alkyl-COOR$^7$, -($C_1$-$C_6$)alkoxy-COOR$^7$, -C(=O)-($CH_2$)$_n$-COOR$^7$, -C(=O)-($CH_2$)$_n$-CONR$^5$R$^6$, -($C_3$-$C_{12}$)cycloalkyl, (($C_3$-$C_{12}$)cycloalkyl)-($C_1$-$C_6$)alkyl-, -($C_4$-$C_{12}$)cycloalkenyl, (($C_4$-$C_{12}$)cycloalkenyl)-($C_1$-$C_6$)alkyl-, -($C_6$-$C_{14}$)bicycloalkyl, (($C_6$-$C_{14}$)bicycloalkyl)-($C_1$-$C_6$)alkyl-, -($C_8$-$C_{20}$)tricycloalkyl, (($C_8$-$C_{20}$)tricycloalkyl)-($C_1$-$C_6$)alkyl-, -($C_7$-$C_{14}$)bicycloalkenyl, (($C_7$-$C_{14}$)bicycloalkenyl)-($C_1$-$C_6$)alkyl-, -($C_8$-$C_{20}$)tricycloalkenyl, (($C_8$-$C_{20}$)tricycloalkenyl)-($C_1$-$C_6$)alkyl-, -(6- to 14-membered)aryl, ((6-to 14-membered)aryl)-($C_1$-$C_6$)alkyl-, -(7- to 12-membered)bicyclic ring system, ((7- to 12-membered)bicyclic ring system)-($C_1$-$C_6$)alkyl-, -(7- to 12-membered)bicyclic aryl, ((7- to 12-membered)bicyclic aryl)-($C_1$-$C_6$)alkyl-, -(5- to 12-membered)heteroaryl, ((5- to 12-membered)heteroaryl)-($C_1$-$C_6$)alkyl-, -(3- to 12-membered)heterocycle, ((3- to 12 membered)heterocycle)-($C_1$-$C_6$)alkyl-, -(7- to 12-membered)bicycloheterocycle, ((7- to 12-membered)bicycloheterocycle)-($C_1$-$C_6$)alkyl-, phenyl, benzyl and naphthyl; each of which is optionally substituted with one, two, or three substituents independently selected from the group consisting of -OH, (=O), halo, -C(halo)$_3$, -CH(halo)$_2$, -$CH_2$(halo), -($C_1$-$C_6$)alkyl, halo($C_1$-$C_6$)alkyl-, -($C_2$-$C_6$)alkenyl, ($C_2$-$C_6$)alkynyl, hydroxy($C_1$-$C_6$)alkyl-, dihydroxy($C_1$-$C_6$)alkyl-, -($C_1$-$C_6$)alkoxy, (($C_1$-$C_6$)alkoxy)-C(=O)-($C_1$-$C_6$)alkoxy-, phenyl, benzyl, -$NH_2$, -NR$^5$R$^6$, -NH-($C_1$-$C_6$)alkyl, -($C_1$-$C_6$)alkyl-NH-($C_1$-$C_6$)alkyl-R$^{14}$, -CN, -SH, -OR$^4$, -CONR$^5$R$^6$, -($C_1$-$C_6$alkyl)-CONR$^5$R$^6$, -COOR$^7$, -($C_1$-$C_6$)alkyl-COOR$^7$, -($C_1$-$C_6$)alkoxy-COOR$^7$, -($OCH_2CH_2$)$_s$-O($C_1$-$C_6$)alkyl, -($CH_2CH_2O$)$_s$-($C_1$-$C_6$)alkyl, (($C_1$-$C_6$)alkyl)sulfonyl($C_1$-$C_6$)alkyl-, -NH-$SO_2$($C_1$-$C_6$)alkyl, -N-$SO_2$-($C_1$-$C_6$)alkyl)$_2$, -C(=NH)$NH_2$, -NH-C(=O)-($C_1$-$C_6$)alkyl, - NH-C(=O)-$NH_2$, -NH-C(=O)-NH-($C_1$-$C_6$)alkyl, -NH-C(=O)-(6- to 14- membered)aryl, - NH-C(=O)-($C_1$-$C_6$)alkyl-(6- to 14-membered)aryl, -NH-($C_1$-$C_6$)alkyl-COOR$^7$, -NH-C(=O)-($C_1$-$C_6$)alkyl-COOR$^7$, -NH-C(=O)-CH($NH_2$)-($C_1$-$C_6$)alkyl-C(=O)OR$^7$, -($C_3$-$C_{12}$)cycloalkyl, (($C_3$-$C_{12}$)cycloalkyl)-($C_1$-$C_6$)alkyl-, -(6- to 14-membered)aryl, -(6- to 14-membered)aryloxy, -($C_1$-$C_6$)alkoxy-C(=O)NR$^5$R$^6$, -NH-($C_1$-$C_6$)alkyl-C(=O)NR$^5$R$^6$, - C(=O)NH-($C_1$-$C_6$)alkyl-C(=O)OR$^7$, ((6- to 14-membered)aryl)-($C_1$-$C_6$)alkyl-, -(5- to 12-membered)heteroaryl, ((5- to 12-membered)heteroaryl)-($C_1$-$C_6$)alkyl-, -(3- to 12-membered)heterocycle, ((3- to 12-membered)heterocycle)-($C_1$-$C_6$)alkyl-, -(7- to 12-membered)bicycloheterocycle, and ((7- to 12-membered)bicycloheterocycle)-($C_1$-$C_6$)alkyl-;

$R^4$ is selected from

(a) -H, -OH, halo, -C(halo)$_3$, -CH(halo)$_2$, -$CH_2$(halo), C(=O)OH, or C(=O)$NH_2$; or
(b) -($C_1$-$C_5$)alkyl, -($C_2$-$C_5$)alkenyl, -($C_2$-$C_5$)alkynyl, -($CH_2$)$_n$-O-($CH_2$)$_n$-$CH_3$, or -($C_1$-$C_5$)alkoxy, each of which is optionally substituted with 1, 2, or 3 independently selected $R^9$ groups;

$R^5$ and $R^6$ are each independently selected from

(a) hydrogen, -OH, halo, -C(halo)$_3$, -CH(halo)$_2$, -$CH_2$(halo);
(b) -($C_1$-$C_6$)alkyl, -($C_2$-$C_5$)alkenyl, -($C_2$-$C_5$)alkynyl, -($CH_2$)$_n$-O-($CH_2$)$_n$-$CH_3$, -($C_1$-$C_6$)alkoxy, each of which is optionally substituted with 1, 2, or 3 independently selected $R^9$ groups;
(c) -($C_3$-$C_8$)cycloalkyl, (($C_3$-$C_8$)cycloalkyl)-($C_1$-$C_6$)alkyl-, -C(=O)OR$^7$, -($C_1$-$C_6$)alkyl-COOR$^7$, -CON$H_2$, or ($C_1$-$C_6$)alkyl-CONH-; or
(d) $R^5$ and $R^6$ together with the nitrogen atom to which they are attached form a (4-to 8-membered)heterocycle;

$R^7$ is selected from the group consisting of hydrogen, -($C_1$-$C_6$)alkyl, -($C_2$-$C_6$)alkenyl, - ($C_2$-$C_6$)alkynyl, -($C_3$-$C_{12}$)cycloalkyl, -($C_4$-$C_{12}$)cycloalkenyl, (($C_3$-$C_{12}$)cycloalkyl)-($C_1$-$C_6$)alkyl-, and (($C_4$-$C_{12}$)cycloalkenyl)-($C_1$-$C_6$)alkyl-;

$R^8$ is selected from the group consisting of H, -($C_1$-$C_6$)alkyl, -($C_2$-$C_6$)alkenyl, -($C_2$-$C_6$)alkynyl, -($C_1$-$C_{10}$)alkoxy, -($C_3$-$C_{12}$)cycloalkyl, -($C_3$-$C_{12}$)cycloalkenyl, (($C_3$-$C_{12}$)cycloalkyl)-($C_1$-$C_6$)alkyl-, (($C_3$-$C_{12}$)cycloalkenyl)-($C_1$-$C_6$)alkyl-, -C(=O)-($C_1$-$C_6$)alkyl and $SO_2$($C_1$-$C_6$)alkyl;

each $R^9$ is independently selected from -OH, halo, -($C_1$-$C_{10}$)alkyl, -($C_2$-$C_{10}$)alkenyl, -($C_2$-$C_{10}$)alkynyl, -($C_1$-$C_{10}$)alkoxy, -($C_3$-$C_{12}$)cycloalkyl, -CHO, -COOH, -C(halo)$_3$, - CH(halo)2, $CH_2$(halo), or -($CH_2$)$_n$-O-($CH_2$)$_n$-$CH_3$;

each $R^{14}$ is independently selected from the group consisting of -COOR$^7$, -($C_1$-$C_6$)alkyl-COOR$^7$, -C(=O)-($C_1$-$C_6$)alkyl-COOR$^7$, -($C_1$-$C_6$)alkyl-C(=O)-($C_1$-$C_6$)alkyl-COOR$^7$, CON$H_2$, and -($C_1$-$C_6$)alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof;

provided that when $R^1$, $R^{2a}$, $R^{2b}$ are all methyl, and $R^4$ is OH or methoxy, then Z-G-$R^3$ is not OH.

In certain embodiments, the present disclosure provides compounds of Formula IA:

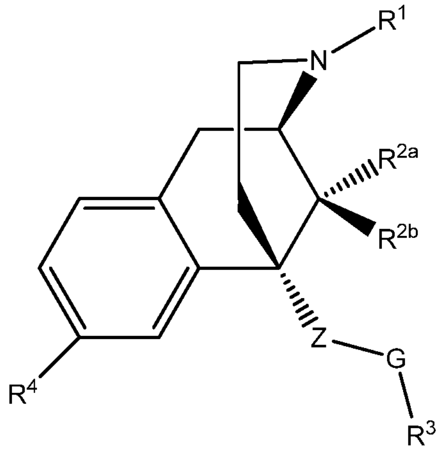

IA

wherein $R^1$, $R^{2a}$, $R^{2b}$, $R^3$, $R^4$, Z, and G are as defined above for Formula I'.

In certain embodiments, the present disclosure provides compounds of Formula IB:

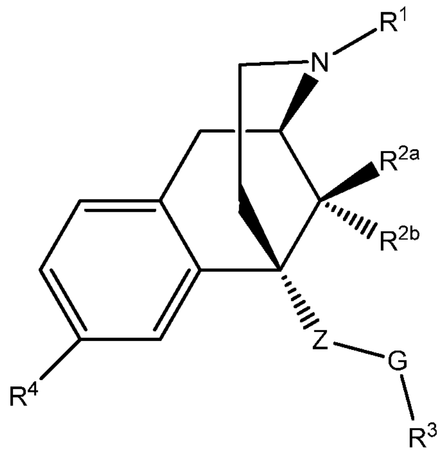

IB

wherein $R^1$, $R^{2a}$, $R^{2b}$, $R^3$, $R^4$, Z, and G are as defined above for Formula I'.

In certain embodiments, the present disclosure provides compounds of Formula IC:

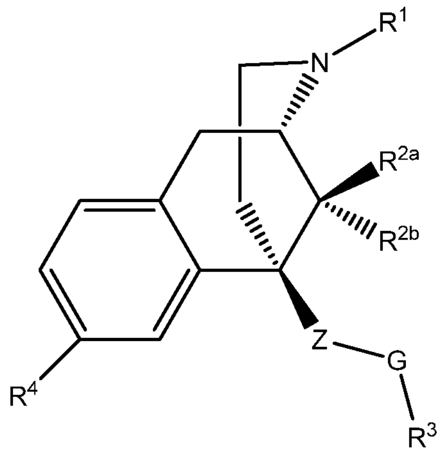

IC

wherein $R^1$, $R^{2a}$, $R^{2b}$, $R^3$, $R^4$, Z, and G are as defined above for Formula I'.

In certain embodiments, the present disclosure provides compounds of Formula ID:

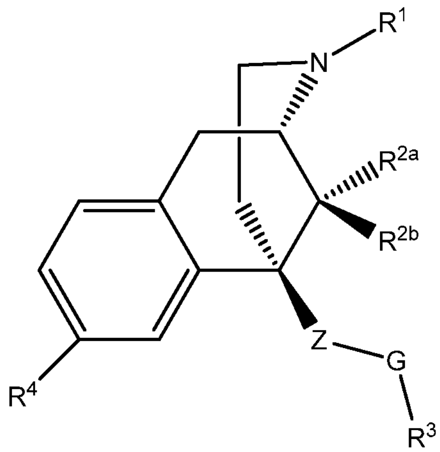

ID

wherein $R^1$, $R^{2a}$, $R^{2b}$, $R^3$, $R^4$, Z, and G are as defined above for Formula I'.

It is an object of certain embodiments of the present invention to provide new Compounds of the Invention that have antagonist activity at the ORL-1 receptor which is greater than compounds currently available, *e.g.*, JTC-801 (described in WO 99/48492; and Shinkai et al., "4-aminoquinolines: Novel nociceptin antagonists with analgesic activity", J. Med. Chem., 2000, 43:4667-4677) and J-113397 (described in WO 98/54168; and Kawamoto et al., "Discovery of the first potent and selective small molecule opioid receptor-like (ORL-1) antagonist: 1-[(3R,4R)-1-cyclooctylmethyl-3-hydroxymethyl-4-piperidyl]-3-ethyl-1,3-dihydro-2H-benzimidazol-2-one (J-113397)", J. Med. Chem., 1999, 42:5061-6063).

Certain Compounds of the Invention have agonist activity at the μ, δ and/or κ receptors which is greater than currently available compounds, *e.g.*, morphine.

Certain Compounds of the Invention have both: (i) antagonist activity at the ORL-1 receptor; and (ii) agonist activity at one or more of the μ, δ and/or κ receptors. Certain Compounds of the Invention have both: (i) antagonist activity at the ORL-1 receptor; and (ii) agonist activity at the μ receptor. Certain compounds of the invention will have both: (i) antagonist activity at the μ receptor; and (ii) agonist activity at the κ receptor. Certain compounds of the invention will have: (i) antagonist activity at the ORL-1 receptor; (ii) antagonist activity at the μ receptor; and (iii) agonist activity at the κ receptor. Certain compounds of the invention will have: (i) antagonist activity at the μ receptor; (ii) agonist activity at the κ receptor; and (iii) antagonist activity at the δ receptor.

Compounds of the Invention may be useful as analgesics; anti-inflammatories; diuretics; anesthetics; neuroprotective agents; anti-hypertensives; anxiolytics; agents for appetite control; hearing regulators; anti-tussives; anti-asthmatics; anti-epileptics; anticonvulsants; modulators of locomotor activity; modulators of learning and memory; regulators of neurotransmitter release; modulators of hormone release; kidney function modulators; anti-depressants; agents to treat memory loss due to Alzheimer's disease or other dementias; agents to treat withdrawal from alcohol and/or drugs of addiction; or agents to control water balance or sodium excretion; agents to treat arterial blood pressure disorders, UI, ulcers, IBD, IBS, diarrhea, constipation, addictive disorders, Parkinson's disease, parkinsonism, anxiety, epilepsy, stroke, a seizure, pruritic conditions, psychosis, cognitive disorders, memory deficits, restricted brain function, Huntington's chorea, ALS, dementia, retinopathy, muscle spasms, migraines, vomiting, dyskinesia, and/or depression (each being a "Condition").

The present invention further provides the Compounds of the Invention for use in treating a Condition, comprising administering to a subject in need thereof a therapeutically effective amount of a Compound of the Invention. In certain embodiments, the Condition is pain (chronic or acute pain). The Compounds of the Invention are particularly useful for treating chronic pain. In certain embodiments, the Compound of the Invention is an ORL-1 receptor antagonist. In other embodiments, the Compound of the Invention is an agonist at one or more of the μ, δ and/or κ receptor. In other embodiments, the Compound of the Invention is both an ORL-1 receptor antagonist and an agonist at one or more of the μ, δ and/or κ receptor. In other embodiments, the Compound of the Invention is both an ORL-1 receptor antagonist and an agonist at the μ receptor. In certain non-limiting embodiments, the Compound of the Invention produces fewer side effects and/or less severe side effects than currently available analgesic opioid compounds when administered at doses producing equivalent levels of analgesia and/or anti-hyperalgesia.

In certain non-limiting embodiments, the Compound of the Invention exhibits a substantially linear dose response curve, such that the bell-shaped dose response curve observed for most opioid analgesics (i.e. low and high doses do not produce significant analgesia, whereas mid-range doses produce analgesia) is not observed for the Compound of the Invention. It is expected, therefore, that it will be easier to titrate to an effective dose of the Compound of the Invention in a patient than it is for conventional opioid analgesics. It is further expected that the Compound of the Invention will produce effective analgesia and/or anti-hyperalgesia in a patient who has become tolerant to conventional opioids, and for whom a conventional opioid is no longer an effective treatment. It is further expected that a Compound of the Invention will produce effective analgesia and/or anti-hyperalgesia at doses that do not induce side effects such as respiratory depression in patients for whom a dose of a conventional opioid that is high enough to be an effective treatment also induces significant side effects such as respiratory depression.

The present invention further provides the Compounds of the Invention for use in preventing a Condition, comprising administering to an animal in need thereof a Condition-preventing effective amount of a Compound of the Invention.

Another object of the invention is to provide benzomorphan analog compounds useful for treating or preventing constipation, preferably μ opioid receptor-induced constipation. More specifically, the present invention provides compounds of Formula I and Formula I' below, and the pharmaceutically acceptable salts, and solvates thereof having activity as μ receptor antagonists. In certain embodiments, Compounds of the Invention are expected to have dual activity as both μ receptor antagonists and κ receptor agonists. In other embodiments, Compounds of the Invention are expected to have an activity wherein they are μ receptor antagonists, κ receptor agonists, and δ receptor antagonists, and inactive at ORL-1 receptors. In yet other embodiments, certain Compounds of the Invention are expected to have an activity wherein they are μ receptor antagonists, κ receptor agonists, and δ receptor antagonists, and ORL-1 receptor antagonists. In other embodiments, certain Compounds of the Invention are expected to have an activity wherein they are μ receptor antagonists, κ receptor agonists, and δ receptor antagonists, and ORL-1 receptor partial agonists. Certain Compounds of the Invention are expected to be substantially restricted to the GI tract.

Compounds of the Invention that have μ antagonist activity and are substantially restricted to the GI tract will significantly reduce or prevent constipation that would otherwise occur in a patient as a result of treatment with a μ agonist. In one embodiment, the reduction or prevention of constipation is obtained without reducing the desired analgesic effect of the μ agonist. Compounds of the Invention that also exhibit κ agonist activity should additionally stimulate GI motility via a non-μ receptor mediated mechanism.

The present invention provides a the Compounds of the Invention for use in treating a Condition in an animal. In certain embodiments, the Condition treated will be pain (acute or chronic pain). The present invention further provides the Compounds of the Invention for use in treating or preventing constipation, preferably constipation associated with μ-opioid agonist therapy, by administering an effective amount of a Compound of the Invention to a patient in need of such treatment or prevention. In one embodiment, the Compound of the Invention is a μ antagonist that is substantially restricted to the GI tract. In another embodiment, the Compound of the Invention is both a μ antagonist and a κ agonist, and is substantially restricted to the GI tract. In another embodiment, the method comprises co-administering to a patient both an effective amount of a Compound of the Invention that is a μ antagonist and is substantially restricted to the GI tract, and an analgesically effective amount of a μ agonist. In another embodiment, the method comprises co-administration to a patient of both an effective amount of a Compound of the Invention that is both a μ antagonist and a κ agonist, and which is substantially restricted to the GI tract, and an analgesically effective amount of a μ agonist.

The present invention further provides pharmaceutical compositions comprising a therapeutically effective amount of a Compound of the Invention admixed with a pharmaceutically acceptable carrier or excipient. Such compositions are useful for treating or preventing a Condition in an animal. The pharmaceutical compositions of the present invention may be formulated as immediate release formulations, or as controlled release formulations. Pharmaceutical compositions of the present invention may be formulated for administration by any of a number of different routes known in the art, including but not limited to, oral, intradermal, intramuscular, intraperitoneal, parenteral, intravenous, subcutaneous, intranasal, epidural, sublingual, intracerebral, intravaginal, transdermal, transmucosal, rectal, by inhalation, or topical (particularly to the ears, nose, eyes, or skin).

The present invention further provides methods for preparing a composition, comprising the step of admixing a Compound of the Invention and a pharmaceutically acceptable carrier or excipient to form a pharmaceutical composition. The invention still further relates to a kit comprising a container containing an effective amount of a Compound of the Invention.

## DETAILED DESCRIPTION OF THE INVENTION

The Compounds of the Invention are benzomorphan analogs. They are useful for treating one or more Conditions, such as pain or constipation. Compounds of the Invention may provide a reduced liability for developing analgesic tolerance and physical dependence.

The Compounds of the Invention are useful for modulating a pharmacodynamic response from ORL-1 receptors either centrally or peripherally, or both. The Compounds of the Invention may also be useful for modulating a pharmacodynamic response from one or more opioid receptors (μ, δ, κ) either centrally or peripherally, or both. The pharmacodynamic response may be attributed to the compound stimulating (agonizing) or inhibiting (antagonizing) the one or more receptors. Certain Compounds of the Invention may inhibit (or antagonize) the ORL-1 receptor, while also stimulating (or agonizing) one or more other receptors (e.g. as a μ, δ and/or κ agonist). Compounds of the Invention having agonist activity may be either full or partial agonists.

In certain embodiments, Compounds of the Invention can be used in combination with at least one other therapeutic agent. The other therapeutic agent can be, but is not limited to, a μ-opioid agonist, a non-opioid analgesic, a non-steroidal antiinflammatory agent, a Cox-II inhibitor, an anti-emetic, a β-adrenergic blocker, an anticonvulsant, an antidepressant, a $Ca^{2+}$-channel blocker, an anticancer agent, or a mixture thereof.

Various objects and advantages of the present invention will become apparent from the following detailed description.

The present invention provides compounds of Formula I:

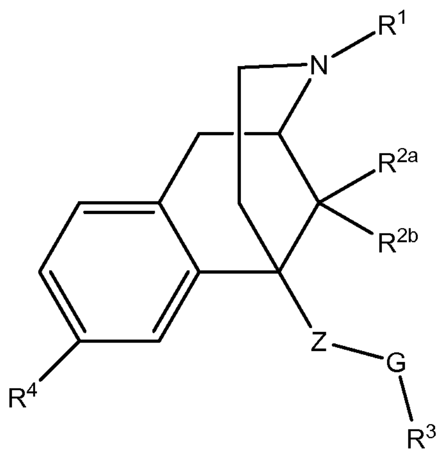

wherein

$R^1$ is selected from the group consisting of -($C_3$-$C_{12}$)cycloalkyl, ($C_3$-$C_{12}$)cycloalkyl-($C_1$-$C_6$)alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or
(b) $-(C_1-C_5)$alkyl, $-(C_2-C_5)$alkenyl, or $-(C_2-C_5)$alkynyl;

Z is absent or $-(CH_2)_m-$, optionally substituted with 1 or 2 independently selected $-(C_1-C_6)$alkyl;

G is selected from the group consisting of:

a) $-(C_1-C_6)$alkylene, $-(C_2-C_6)$alkenylene; or
b) -C(=O); or
c) $NR^8$;

$R^3$ is selected from the group consisting of hydrogen, $-(C_1-C_{10})$alkyl, $-(C_2-C_{12})$alkenyl, - C(=O)-$(C_1-C_6)$alkyl, -C(=O)-(6- to 14-membered)aryl, -C(=O)-(5- to 12-membered)heteroaryl, $-(C_2-C_{12})$alkynyl, $-(C_1-C_{10})$alkoxy, $-(OCH_2CH_2)_s-O(C_1-C_6)$alkyl, $-(CH_2CH_2O)_s-(C_1-C_6)$alkyl, $-NH_2$, $-NH(C_1-C_6)$alkyl, CN, $-CONR^5R^6$, $-(C_1-C_6)$alkyl-$CONR^5R^6$, $-COOR^7$, $-(C_1-C_6)$alkyl-$COOR^7$, $-(C_1-C_6)$alkoxy-$COOR^7$, $-C(=O)-(CH_2)_n-COOR^7$, $-C(=O)-(CH_2)_n-CONR^5R^6$, $-(C_3-C_{12})$cycloalkyl, $((C_3-C_{12})$cycloalkyl)-$(C_1-C_6)$alkyl-, $-(C_4-C_{12})$cycloalkenyl, $((C_4-C_{12})$cycloalkenyl)-$(C_1-C_6)$alkyl-, $-(C_6-C_{14})$bicycloalkyl, $((C_6-C_{14})$bicycloalkyl)-$(C_1C_6)$alkyl-, $-(C_8-C_{20})$tricycloalkyl, $((C_8-C_{20})$tricycloalkyl)-$(C_1-C_6)$alkyl-, $-(C_7-C_{14})$bicycloalkenyl, $((C_7-C_{14})$bicycloalkenyl)-$(C_1-C_6)$alkyl-, $-(C_8-C_{20})$tricycloalkenyl, $((C_8-C_{20})$tricycloalkenyl)-$(C_1-C_6)$alkyl-, -(6- to 14-membered)aryl, ((6-to 14-membered)aryl)-$(C_1-C_6)$alkyl-, -(7- to 12-membered)bicyclic ring system, ((7- to 12-membered)bicyclic ring system)-$(C_1-C_6)$alkyl-, -(7- to 12-membered)bicyclic aryl, ((7- to 12-membered)bicyclic aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12 membered)heterocycle)-$(C_1-C_6)$alkyl-, -(7- to 12-membered)bicycloheterocycle, and ((7- to 12-membered)bicycloheterocycle)-$(C_1-C_6)$alkyl-; each of which is optionally substituted with one, two, or three substituents independently selected from the group consisting of -OH, (=O), halo, $-C(halo)_3$, $-CH(halo)_2$, $-CH_2(halo)$, $-(C_1-C_6)$alkyl, halo$(C_1-C_6)$alkyl-, $-(C_2-C_6)$alkenyl, $-(C_2-C_6)$alkynyl, hydroxy$(C_1-C_6)$alkyl-, dihydroxy$(C_1-C_6)$alkyl-, $-(C_1-C_6)$alkoxy, $((C_1-C_6)$alkoxy)-C(=O)-$(C_1-C_6)$alkoxy-, $-NH_2$, $-NR^5R^6$, $-NH(C_1-C_6)$alkyl, $-(C_1-C_6)$alkyl-$NH(C_1-C_6)$alkyl-$R^{14}$, -CN, -SH, $-OR^4$, $-CONR^5R^6$, $-(C_1-C_6$alkyl)-C(=O)-$NR^5R^6$, $-COOR^7$, $-(C_1-C_6)$alkyl-$COOR^7$, $-(C_1-C_6)$alkoxy-$COOR^7$, $-(OCH_2CH_2)_s-O(C_1-C_6)$alkyl, $-(CH_2CH_2O)_s-(C_1-C_6)$alkyl, $((C_1-C_6)$alkyl)sulfonyl$(C_1-C_6)$alkyl-, $-NH-SO_2(C_1-C_6)$alkyl, $-N-(SO_2-(C_1-C_6)alkyl)_2$, $-C(=NH)-NH_2$, -NH-C(=O)-$(C_1-C_6)$alkyl, - NH-C(=O)-$NH_2$, -NH-C(=O)-NH-$(C_1-C_6)$alkyl, -NH-C(=O)-(6- to 14- membered)aryl, - NH-C(=O)-$(C_1-C_6)$alkyl-(6- to 14- membered)aryl, -NH-$(C_1-C_6)$alkyl-$COOR^7$, -NH-C(=O)-$(C_1-C_6)$alkyl-$COOR^7$, -NH-C(=O)-$CH(NH_2)$-$(C_1-C_6)$alkyl-C(=O)-$OR^7$, $-(C_3-C_{12})$cycloalkyl, $((C_3-C_{12})$cycloalkyl)-$(C_1-C_6)$alkyl-, -(6- to 14-membered)aryl, -(6- to 14-membered)aryloxy, $-(C_1-C_6)$alkoxyC(O)$NR^5R^6$, -NH-$(C_1-C_6)$alkylC(O)-$NR^5R^6$, - C(O)NH-$(C_1-C_6)$alkyl-$COOR^7$, ((6- to 14-membered)aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12-membered)heterocycle)-$(C_1-C_6)$alkyl-, -(7- to 12-membered)bicycloheterocycle, and ((7- to 12-membered)bicycloheterocycle)-$(C_1-C_6)$alkyl-;

$R^4$ is selected from

(a) -H, -OH, halo, $-C(halo)_3$, $-CH(halo)_2$, $-CH_2(halo)$, COOH, or $CONH_2$; or
(b) $-(C_1-C_5)$alkyl, $-(C_2-C_5)$alkenyl, $-(C_2-C_5)$alkynyl, $-(CH_2)_n-O-(CH_2)_n-CH_3$, or $-(C_1-C_5)$alkoxy, each of which is optionally substituted with 1, 2, or 3 independently selected $R^9$ groups;

$R^5$ and $R^6$ are each independently selected from

(a) hydrogen, -OH, halo, $-C(halo)_3$, $-CH(halo)_2$, $-CH_2(halo)$; or
(b) $-(C_1-C_6)$alkyl, $-(C_2-C_5)$alkenyl, $-(C_2-C_5)$alkynyl, $-(CH_2)_n-O-(CH_2)_n-CH_3$, $-(C_1-C_6)$alkoxy, each of which is optionally substituted with 1, 2, or 3 independently selected $R^9$ groups; or
(c) $-(C_3-C_8)$cycloalkyl, $((C_3-C_8)$cycloalkyl)-$(C_1-C_6)$alkyl-, $-COOR^7$, $-(C_1-C_6)$alkyl-$COOR^7$, $-CONH_2$, or $(C_1-C_6)$alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl,$-(C_2-C_6)$alkynyl, $-(C_3-C_{12})$cycloalkyl, $-(C_4-C_{12})$cycloalkenyl, $((C_3-C_{12})$cycloalkyl)-$(C_1-C_6)$alkyl-, and $((C_4-C_{12})$cycloalkenyl)-$(C_1-C_6)$alkyl-;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)-$(C_1-C_6)$alkyl-, $((C_3-C_{12})$cycloalkenyl)-$(C_1-C_6)$alkyl-, -C(=O)$(C_1-C_6)$alkyl

or $SO_2(C_1-C_6)$alkyl;

each $R^9$ is independently selected from -OH, halo, $-(C_1-C_{10})$alkyl, $-(C_2-C_{10})$alkenyl, $-(C_2-C_{10})$alkynyl, $-(C_1-C_{10})$alkoxy, $-(C_3-C_{12})$cycloalkyl , -CHO, -C(O)OH, $-C(halo)_3$, - CH(halo)2, $CH_2$(halo), or $-(CH_2)_n-O-(CH_2)_n-CH_3$;

each $R^{14}$ is independently selected from the group consisting of $-COOR^7$, $-(C_1-C_6)$alkyl-$COOR^7$, -C(=O)-$(C_1-C_6)$alkyl-$COOR^7$, $-(C_1-C_6)$alkyl-C(=O)-$(C_1-C_6)$alkyl-$COOR^7$, $CONH_2$, and $-(C_1-C_6)$alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof;

provided that Z-G-$R^3$ is not unsubstituted $(C_1-C_6)$alkyl.

In one embodiment, the present invention comprises compounds of Formula I:

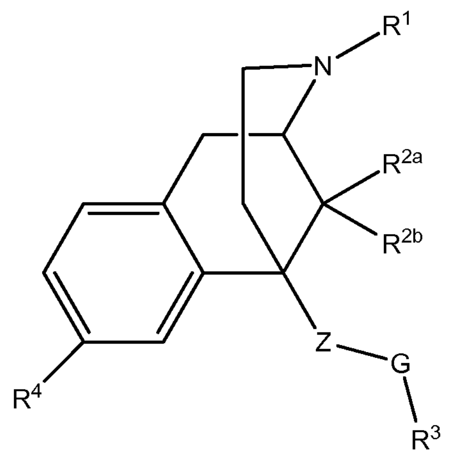

wherein

$R^1$ is selected from the group consisting of $-(C_3-C_{12})$cycloalkyl, $(C_3-C_{12})$cycloalkyl-$(C_1-C_6)$alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or
(b) $-(C_1-C_5)$alkyl, $-(C_2-C_5)$alkenyl, or $-(C_2-C_5)$alkynyl;

Z is absent or $-(CH_2)_m-$, optionally substituted with 1 or 2 $-(C_1-C_6)$alkyl;

G is selected from the group consisting of:

a) $-(C_1-C_6)$alkylene, $-(C_2-C_6)$alkenylene;
b) -C(=O); and
c) $NR^8$;

$R^3$ is selected from the group consisting of hydrogen, $-(C_1-C_{10})$alkyl, $-(C_2-C_{12})$alkenyl, - $(C_2-C_{12})$alkynyl, $-(C_1-C_{10})$alkoxy, $-(OCH_2CH_2)_s-O(C_1-C_6)$alkyl, $-(CH_2CH_2O)_s-(C_1-C_6)$alkyl, $-NH_2$, $-NH(C_1-C_6)$alkyl, CN, $-CONR^5R^6$, $-(C_1-C_6)$alkyl-CO-$NR^5R^6$, $-COOR^7$, $-(C_1-C_6)$alkyl-CO-$OR^7$, $-(C_1-C_6)$alkoxy-$COOR^7$, $-CO-(CH_2)_n-COOR^7$, $-CO-(CH_2)_n-CO-NR^5R^6$, $-(C_3-C_{12})$cycloalkyl, $((C_3-C_{12})$cycloalkyl$)-(C_1-C_6)$alkyl-, $-(C_4-C_{12})$cycloalkenyl, $((C_4-C_{12})$cy-

cloalkenyl)-($C_1$-$C_6$)alkyl-, -($C_6$-$C_{14}$)bicycloalkyl, (($C_6$-$C_{14}$)bicycloalkyl)-($C_1$-$C_6$)alkyl-, -($C_8$-$C_{20}$)tricycloalkyl, (($C_8$-$C_{20}$)tricycloalkyl)-($C_1$-$C_6$)alkyl-, -($C_7$-$C_{14}$)bicycloalkenyl, (($C_7$-$C_{14}$)bicycloalkenyl)-($C_1$-$C_6$)alkyl-, -($C_8$-$C_{20}$)tricycloalkenyl, (($C_8$-$C_{20}$)tricycloalkenyl)-($C_1$-$C_6$)alkyl-, -(6- to 14-membered)aryl, ((6- to 14-membered)aryl)-($C_1$-$C_6$)alkyl-, -(7- to 12-membered)bicyclic ring system, ((7-to 12-membered)bicyclic ring system)-($C_1$-$C_6$)alkyl-, -(7- to 12-membered)bicyclic aryl, ((7- to 12-membered)bicyclic aryl)-($C_1$-$C_6$)alkyl-, -(5- to 12-membered)heteroaryl, ((5-to 12-membered)heteroaryl)-($C_1$-$C_6$)alkyl-, -(3- to 12-membered)heterocycle, ((3- to 12 membered)heterocycle)-($C_1$-$C_6$)alkyl-, -(7- to 12-membered)bicycloheterocycle, and ((7- to 12-membered)bicycloheterocycle)-($C_1$-$C_6$)alkyl-; each of which is optionally substituted with one, two, or three substituents independently selected from the group consisting of -OH, (=O), halo, -C(halo)$_3$, -CH(halo)$_2$, -$CH_2$(halo), -($C_1$-$C_6$)alkyl, halo($C_1$-$C_6$)alkyl-, -($C_2$-$C_6$)alkenyl, -($C_2$-$C_6$)alkynyl, hydroxy($C_1$-$C_6$)alkyl-, dihydroxy($C_1$-$C_6$)alkyl-, -($C_1$-$C_6$)alkoxy, (($C_1$-$C_6$)alkoxy)CO($C_1$-$C_6$)alkoxy-, -$NH_2$, - NH($C_1$-$C_6$)alkyl, -($C_1$-$C_6$)alkyl-NH($C_1$-$C_6$)alkyl-$R^{14}$, -CN, -SH, -$OR^4$, -$CONR^5R^6$, -($C_1$-$C_6$alkyl)-CO-$NR^5R^6$, -$COOR^7$, -($C_1$-$C_6$)alkyl-CO-$OR^7$, -($C_1$-$C_6$)alkoxy-$COOR^7$, -($OCH_2CH_2$)$_s$-O($C_1$-$C_6$)alkyl, -($CH_2CH_2O$)$_s$-($C_1$-$C_6$)alkyl, (($C_1$-$C_6$)alkyl)sulfonyl($C_1$-$C_6$)alkyl-, -NH-$SO_2$($C_1$-$C_6$)alkyl, -N($SO_2$($C_1$-$C_6$)alkyl)$_2$, -C(=NH)$NH_2$, -NH-CO-($C_1$-$C_6$)alkyl, -NH-CO-$NH_2$, -NH-C(=O)-NH-($C_1$-$C_6$)alkyl, -NH-C(=O)-(6- to 14-membered)aryl, -NH-C(=O)-($C_1$-$C_6$)alkyl-(6- to 14- membered)aryl, -NH-($C_1$-$C_6$)alkyl-CO-$OR^7$,-NH-C(=O)-($C_1$-$C_6$)alkyl-CO-$OR^7$,-NH-C(=O)-CH($NH_2$)-($C_1$-$C_6$)alkyl-CO-$OR^7$, -($C_3$-$C_{12}$)cycloalkyl, (($C_3$-$C_{12}$)cycloalkyl)-($C_1$-$C_6$)alkyl-, -(6- to 14-membered)aryl, -(6- to 14-membered)aryloxy, -($C_1$-$C_6$)alkoxyC(O)$NR^5R^6$, -NH-($C_1$-$C_6$)alkylC(O)-$NR^5R^6$, -C(O)NH-($C_1$-$C_6$)alkyl-$COOR^7$, ((6- to 14-membered)aryl)-($C_1$-$C_6$)alkyl-, -(5-to 12-membered)heteroaryl, ((5- to 12-membered)heteroaryl)-($C_1$-$C_6$)alkyl-, -(3- to 12-membered)heterocycle, ((3- to 12-membered)heterocycle)-($C_1$-$C_6$)alkyl-, -(7- to 12-membered)bicycloheterocycle, and ((7- to 12-membered)bicycloheterocycle)-($C_1$-$C_6$)alkyl-;

$R^4$ is selected from

(a) -H, -OH, halo, -C(halo)$_3$, -CH(halo)$_2$, -$CH_2$(halo), COOH, or $CONH_2$; or
(b) -($C_1$-$C_5$)alkyl, -($C_2$-$C_5$)alkenyl, -($C_2$-$C_5$)alkynyl, -($CH_2$)$_n$-O-($CH_2$)$_n$-$CH_3$, or -($C_1$-$C_5$)alkoxy, each of which is optionally substituted with 1, 2, or 3 independently selected $R^9$ groups;

$R^5$ and $R^6$ are each independently selected from

(a) hydrogen, -OH, halo, -C(halo)$_3$, -CH(halo)$_2$, -$CH_2$(halo);
(b) -($C_1$-$C_6$)alkyl, -($C_2$-$C_5$)alkenyl, -($C_2$-$C_5$)alkynyl, -($CH_2$)$_n$-O-($CH_2$)$_n$-$CH_3$, -($C_1$-$C_6$)alkoxy, each of which is optionally substituted with 1, 2, or 3 independently selected $R^9$ groups;
(c) -($C_3$-$C_8$)cycloalkyl, (($C_3$-$C_8$)cycloalkyl)-($C_1$-$C_6$)alkyl-, -$COOR^7$, -($C_1$-$C_6$)alkyl-$COOR^7$, -$CONH_2$, or ($C_1$-$C_6$)alkyl-CONH-; or
(d) $R^5$ and $R^6$ together with the nitrogen atom to which they are attached form a (4-to 8-membered)heterocycle;

$R^7$ is selected from the group consisting of hydrogen, -($C_1$-$C_6$)alkyl, -($C_2$-$C_6$)alkenyl, - ($C_2$-$C_6$)alkynyl, -($C_3$-$C_{12}$)cycloalkyl, -($C_4$-$C_{12}$)cycloalkenyl, (($C_3$-$C_{12}$)cycloalkyl)-($C_1$-$C_6$)alkyl-, and (($C_4$-$C_{12}$)cycloalkenyl)-($C_1$-$C_6$)alkyl- ;

$R^8$ is selected from H, -($C_1$-$C_6$)alkyl, -($C_2$-$C_6$)alkenyl, -($C_2$-$C_6$)alkynyl, -($C_1$-$C_{10}$)alkoxy, -($C_3$-$C_{12}$)cycloalkyl, -($C_3$-$C_{12}$)cycloalkenyl, (($C_3$-$C_{12}$)cycloalkyl)-($C_1$-$C_6$)alkyl-, (($C_3$-$C_{12}$)cycloalkenyl)-($C_1$-$C_6$)alkyl-, -C(=O)($C_1$-$C_6$)alkyl or $SO_2$($C_1$-$C_6$)alkyl;

each $R^9$ is independently selected from -OH, halo, -($C_1$-$C_{10}$)alkyl, -($C_2$-$C_{10}$)alkenyl, -($C_2$-$C_{10}$)alkynyl, -($C_1$-$C_{10}$)alkoxy, -($C_3$-$C_{12}$)cycloalkyl, -CHO, -C(O)OH, -C(halo)$_3$, - CH(halo)2, $CH_2$(halo), or -($CH_2$)$_n$-O-($CH_2$)$_n$-$CH_3$;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts, and solvates thereof.

In one embodiment, the invention encompasses compounds of Formual I:

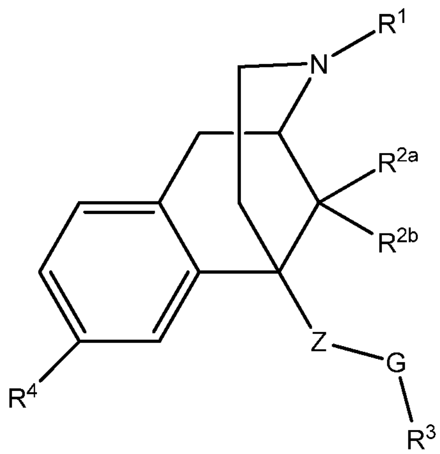

wherein

$R^1$ is selected from the group consisting of $-(C_3-C_{12})$cycloalkyl, $(C_3-C_{12})$cycloalkyl-$(C_1-C_6)$alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or

(b) $-(C_1-C_5)$alkyl, $-(C_2-C_5)$alkenyl, or $-(C_2-C_5)$alkynyl;

Z is selected from the group consisting of a bond and $-(CH_2)_m-$, optionally substituted with 1 or 2 $-(C_1-C_6)$alkyl;

G is selected from the group consisting of:

a) $-(C_1-C_6)$alkylene, $-(C_2-C_6)$alkenylene;
b) -C(=O); and
c) $NR^8$;

$R^3$ is selected from the group consisting of hydrogen, $-(C_1-C_{10})$alkyl, $-(C_2-C_{12})$alkenyl, - $(C_2-C_{12})$alkynyl, $-(C_1-C_{10})$alkoxy, $-(OCH_2CH_2)_s-O(C_1-C_6)$alkyl, $-(CH_2CH_2O)_s-(C_1-C_6)$alkyl, $-NH_2$, $-NH(C_1-C_6)$alkyl, CN, $-CONR^5R^6$, $-(C_1-C_6)$alkyl-CO-$NR^5R^6$, $-COOR^7$, $-(C_1-C_6)$alkyl-CO-$OR^7$, $-(C_1-C_6)$alkoxy-$COOR^7$, $-CO-(CH_2)_n-COOR^7$, $-CO-(CH_2)_n-CO-NR^5R^6$, $-(C_3-C_{12})$cycloalkyl, $((C_3-C_{12})$cycloalkyl)-$(C_1-C_6)$alkyl-, $-(C_4-C_{12})$cycloalkenyl, $((C_4-C_{12})$cycloalkenyl)-$(C_1-C_6)$alkyl-, $-(C_6-C_{14})$bicycloalkyl, $((C_6-C_{14})$bicycloalkyl)-$(C_1-C_6)$alkyl-, $-(C_8-C_{20})$tricycloalkyl, $((C_8-C_{20})$tricycloalkyl)-$(C_1-C_6)$alkyl-, $-(C_7-C_{14})$bicycloalkenyl, $((C_7-C_{14})$bicycloalkenyl)-$(C_1-C_6)$alkyl-, $-(C_8-C_{20})$tricycloalkenyl, $((C_8-C_{20})$tricycloalkenyl)-$(C_1-C_6)$alkyl-, -(6- to 14-membered)aryl, ((6- to 14-membered)aryl)-$(C_1-C_6)$alkyl-, -(7- to 12-membered)bicyclic ring system, ((7-to 12-membered)bicyclic ring system)-$(C_1-C_6)$alkyl-, -(7- to 12-membered)bicyclic aryl, ((7- to 12-membered)bicyclic aryl)-$(C_1-C_6)$alkyl-, -(5- to 12-membered)heteroaryl, ((5-to 12-membered)heteroaryl)-$(C_1-C_6)$alkyl-, -(3- to 12-membered)heterocycle, ((3- to 12 membered)heterocycle)-$(C_1-C_6)$alkyl-, -(7- to 12-membered)bicycloheterocycle, and ((7- to 12-membered)bicycloheterocycle)-$(C_1-C_6)$alkyl-; each of which is optionally substituted with one, two, or three substituents independently selected from the group consisting of -OH, (=O), halo, $-C(halo)_3$, $-CH(halo)_2$, $-CH_2(halo)$, $-(C_1-C_6)$alkyl, halo$(C_1-C_6)$alkyl-, $-(C_2-C_6)$alkenyl, $-(C_2-C_6)$alkynyl, hydroxy$(C_1-C_6)$alkyl-, dihydroxy$(C_1-C_6)$alkyl-, $-(C_1-C_6)$alkoxy, $((C_1-C_6)$alkoxy)CO$(C_1-C_6)$alkoxy-, $-NH_2$, - $NH(C_1-C_6)$alkyl, $-(C_1-C_6)$alkyl-$NH(C_1-C_6)$alkyl-$R^{14}$, -CN, -SH, $-OR^4$, $-CONR^5R^6$, $-(C_1-C_6$alkyl)-CO-$NR^5R^6$, $-COOR^7$, $-(C_1-C_6)$alkyl-CO-$OR^7$, $-(C_1-C_6)$alkoxy-$COOR^7$, $-(OCH_2CH_2)_s-O(C_1-C_6)$alkyl, $-(CH_2CH_2O)_s-(C_1-C_6)$alkyl, $((C_1-C_6)$alkyl)sulfonyl$(C_1-C_6)$alkyl-, $-NH-SO_2(C_1-C_6)$alkyl, $-N(SO_2(C_1-C_6)alkyl)_2$, $-C(=NH)NH_2$, -NH-CO-$(C_1-C_6)$alkyl, $-NH-CO-NH_2$, -NH-C(=O)-NH-$(C_1-C_6)$alkyl, -NH-C(=O)-(6- to 14-membered)aryl, -NH-C(=O)-$(C_1-C_6)$alkyl-(6- to 14- membered)aryl, -NH-$(C_1-C_6)$alkyl-CO-$OR^7$,-NH-C(=O)-$(C_1-C_6)$alkyl-CO-$OR^7$,-NH-C(=O)-$CH(NH_2)$-$(C_1-C_6)$alkyl-CO-$OR^7$, $-(C_3-C_{12})$cycloalkyl, $((C_3-C_{12})$cycloalkyl)-$(C_1-C_6)$alkyl-, -(6- to 14-membered)aryl, -(6- to 14-membered)aryloxy, $-(C_1-C_6)$alkoxyC(O)$NR^5R^6$, -NH-$(C_1-C_6)$alkylC(O)-$NR^5R^6$, -C(O)NH-$(C_1-C_6)$alkyl-$COOR^7$, ((6- to 14-membered)aryl)-$(C_1-C_6)$alkyl-, -(5-to 12-membered)heteroaryl, ((5- to 12-membered)heteroaryl)-$(C_1-C_6)$alkyl-, -(3- to 12-membered)heterocycle, ((3- to 12-

membered)heterocycle)-$(C_1-C_6)$alkyl-, -(7- to 12-membered)bicycloheterocycle, and ((7- to 12-membered)bicycloheterocycle)-$(C_1-C_6)$alkyl-;

$R^4$ is selected from

(a) -H, -OH, halo, $-C(halo)_3$, $-CH(halo)_2$, $-CH_2(halo)$, COOH, or $CONH_2$; or
(b) $-(C_1-C_5)$alkyl, $-(C_2-C_5)$alkenyl, $-(C_2-C_5)$alkynyl, $-(CH_2)_n-O-(CH_2)_n-CH_3$, or $-(C_1-C_5)$alkoxy, each of which is optionally substituted with 1, 2, or 3 independently selected $R^9$ groups;

$R^5$ and $R^6$ are each independently selected from

(a) hydrogen, -OH, halo, $-C(halo)_3$, $-CH(halo)_2$, $-CH_2(halo)$;
(b) $-(C_1-C_6)$alkyl, $-(C_2-C_5)$alkenyl, $-(C_2-C_5)$alkynyl, $-(CH_2)_n-O-(CH_2)_n-CH_3$, $-(C_1-C_6)$alkoxy, each of which is optionally substituted with 1, 2, or 3 independently selected $R^9$ groups;
(c) $-(C_3-C_8)$cycloalkyl, $((C_3-C_8)$cycloalkyl)-$(C_1-C_6)$alkyl-, $-COOR^7$, $-(C_1-C_6)$alkyl-$COOR^7$, $-CONH_2$, or $(C_1-C_6)$alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl, - $(C_2-C_6)$alkynyl, $-(C_3-C_{12})$cycloalkyl, $-(C_4-C_{12})$cycloalkenyl, $((C_3-C_{12})$cycloalkyl)-$(C_1-C_6)$alkyl-, and $((C_4-C_{12})$cycloalkenyl)-$(C_1-C_6)$alkyl- ;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)-$(C_1-C_6)$alkyl-, $((C_3-C_{12})$cycloalkenyl)-$(C_1-C_6)$alkyl-, $-C(=O)(C_1-C_6)$alkyl or $SO_2(C_1-C_6)$alkyl;

each $R^9$ is independently selected from -OH, halo, $-(C_1-C_{10})$alkyl, $-(C_2-C_{10})$alkenyl, $-(C_2-C_{10})$alkynyl, $-(C_1-C_{10})$alkoxy, $-(C_3-C_{12})$cycloalkyl, -CHO, -C(O)OH, $-C(halo)_3$, - CH(halo)2, $CH_2(halo)$, or $-(CH_2)_n-O-(CH_2)_n-CH_3$;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts, and solvates thereof.

In certain embodiments, the present invention provides compounds of Formula IA:

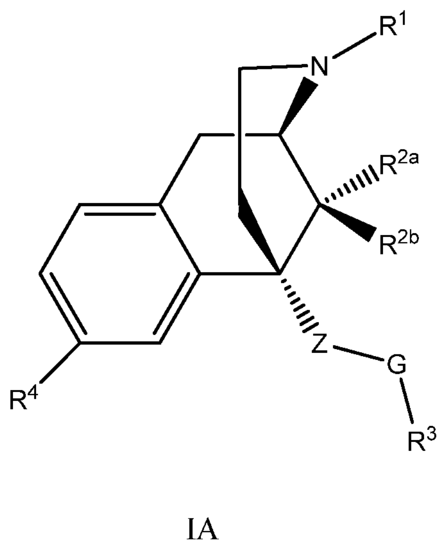

IA

wherein $R^1$, $R^{2a}$, $R^{2b}$, $R^3$, $R^4$, Z, and G are as defined above for Formula I.

In certain embodiments, the present invention provides compounds of Formula IB:

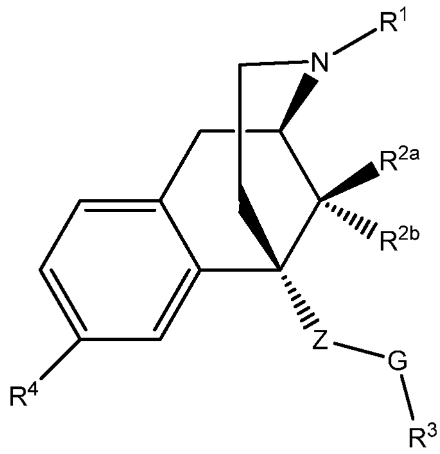

IB

wherein $R^1$, $R^{2a}$, $R^{2b}$, $R^3$, $R^4$, Z, and G are as defined above for Formula I.

In certain embodiments, the present invention provides compounds of Formula IC:

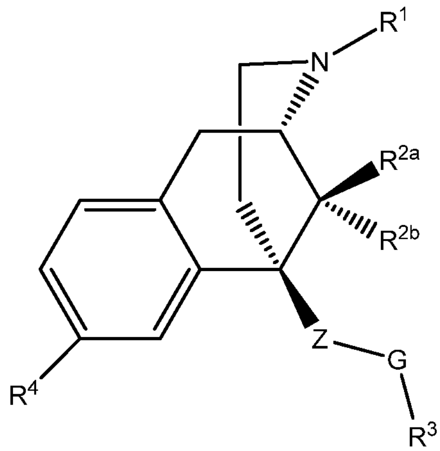

IC

wherein $R^1$, $R^{2a}$, $R^{2b}$, $R^3$, $R^4$, Z, and G are as defined above for Formula I.

In certain embodiments, the present invention provides compounds of Formula ID:

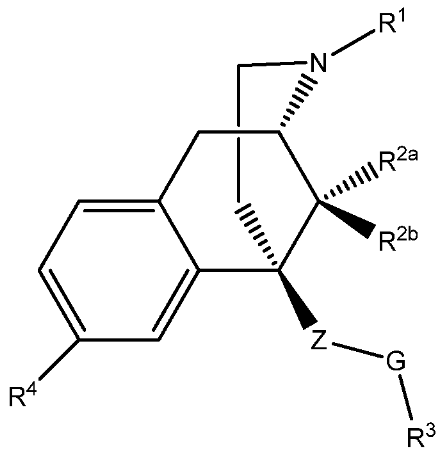

ID

wherein $R^1$, $R^{2a}$, $R^{2b}$, $R^3$, $R^4$, Z, and G are as defined above for Formula I.

The present disclosure provides compounds of Formula I':

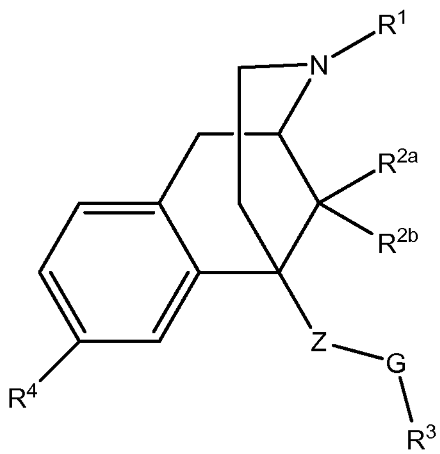

wherein

$R^1$ is selected from the group consisting of $-(C_1-C_{10})$alkyl, $-(C_2-C_{10})$alkenyl, $-(C_2-C_{10})$alkynyl, $-(C_3-C_{12})$cycloalkyl, $(C_3-C_{12})$cycloalkyl-$(C_1-C_6)$alkyl-, $-(C_3-C_{12})$cycloalkenyl, $(C_3-C_{12})$cycloalkenyl-$(C_1-C_6)$alkyl-, -(6- to 14-membered)aryl, ((6-to 14-membered)aryl)-$(C_1-C_6)$alkyl-, $-(OCH_2CH_2)_s-O-(C_1-C_6)$alkyl, $-(CH_2CH_2O)_s-(C_1-C_6)$alkyl, $(C_1-C_{10})$alkoxy, $C(halo)_3$, $CH(halo)_2$, $CH_2(halo)$, $C(O)R^5$, $-C(O)O-(C_1-C_{10})$alkyl, and $-(CH_2)_n-N(R^6)_2$, each of which is optionally substituted by 1, 2 or 3 independently selected $R^9$ groups;

$R^{2a}$ and $R^{2b}$ are each independently selected from:

(a) -H; or
(b) $-(C_1-C_5)$alkyl, $-(C_2-C_5)$alkenyl, or $-(C_2-C_5)$alkynyl;

Z is absent or $-(CH_2)_m-$, optionally substituted with 1 or 2 independently selected $-(C_1-C_6)$alkyl;

G is selected from the group consisting of:

a) a bond, $-(C_1-C_6)$alkylene, $-(C_2-C_6)$alkenylene;
b) O, -OCO-, -C(=O);
c) $NR^8$;

d) S, SO, and $SO_2$;

$R^3$ is selected from the group consisting of hydrogen, $-(C_1-C_{10})$alkyl, $-(C_2-C_{12})$alkenyl, - C(=O), C(=O)-$(C_1-C_6)$alkyl-, -C(=O)-(6- to 14-membered)aryl, -C(=O)-(5- to 12-membered)heteroaryl, $-(C_2-C_{12})$alkynyl, $-(C_1-C_{10})$alkoxy, $-(OCH_2CH_2)_s-O(C_1-C_6)$alkyl, $-(CH_2CH_2O)_s-(C_1-C_6)$alkyl, $-NH_2$, $-NH(C_1-C_6)$alkyl, CN, $-CONR^5R^6$, $-(C_1-C_6)$alkyl-$CONR^5R^6$, $-COOR^7$, $-(C_1-C_6)$alkyl-$COOR^7$, $-(C_1-C_6)$alkoxy-$COOR^7$, $-C(=O)-(CH_2)_n-COOR^7$, $-C(=O)-(CH_2)_n-CONR^5R^6$, $-(C_3-C_{12})$cycloalkyl, $((C_3-C_{12})$cycloalkyl)-$(C_1-C_6)$alkyl-, $-(C_4-C_{12})$cycloalkenyl, $((C_4-C_{12})$cycloalkenyl)-$(C_1-C_6)$alkyl-, $-(C_6-C_{14})$bicycloalkyl, $((C_6-C_{14})$bicycloalkyl)-$(C_1-C_6)$alkyl-, $-(C_8-C_{20})$tricycloalkyl, $((C_8-C_{20})$tricycloalkyl)-$(C_1-C_6)$alkyl-, $-(C_7-C_{14})$bicycloalkenyl, $((C_7-C_{14})$bicycloalkenyl)-$(C_1-C_6)$alkyl-, $-(C_8-C_{20})$tricycloalkenyl, $((C_8-C_{20})$tricycloalkenyl)-$(C_1-C_6)$alkyl-, -(6- to 14-membered)aryl, ((6- to 14-membered)aryl)-$(C_1-C_6)$alkyl-, -(7- to 12-membered)bicyclic ring system, ((7- to 12-membered)bicyclic ring system)-$(C_1-C_6)$alkyl-, -(7- to 12-membered)bicyclic aryl, ((7- to 12-membered)bicyclic aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12 membered)heterocycle)-$(C_1-C_6)$alkyl-, -(7- to 12-membered)bicycloheterocycle, ((7- to 12-membered)bicycloheterocycle)-$(C_1-C_6)$alkyl-, phenyl, benzyl and naphthyl; each of which is optionally substituted with one, two, or three substituents independently selected from the group consisting of -OH, (=O), halo, $-C(halo)_3$, $-CH(halo)_2$, $-CH_2(halo)$, $-(C_1-C_6)$alkyl, halo$(C_1-C_6)$alkyl-, $-(C_2-C_6)$alkenyl, - $(C_2-C_6)$alkynyl, hydroxy$(C_1-C_6)$alkyl-, dihydroxy$(C_1-C_6)$alkyl-, $-(C_1-C_6)$alkoxy, $((C_1-C_6)$alkoxy)-C(=O)-$(C_1-C_6)$alkoxy-, phenyl, benzyl, $-NH_2$, $-NR^5R^6$, $-NH(C_1-C_6)$alkyl, $-(C_1-C_6)$alkyl-NH-$(C_1-C_6)$alkyl-$R^{14}$, -CN, -SH, $-OR^4$, $-CONR^5R^6$, $-(C_1-C_6$alkyl)-$CONR^5R^6$, $-COOR^7$, $-(C_1-C_6)$alkyl-$COOR^7$, $-(C_1-C_6)$alkoxy-$COOR^7$, $-(OCH_2CH_2)_s-O(C_1-C_6)$alkyl, $-(CH_2CH_2O)_s-(C_1-C_6)$alkyl, $((C_1-C_6)$alkyl)sulfonyl$(C_1-C_6)$alkyl-, $-NH-SO_2(C_1-C_6)$alkyl, $-N-SO_2(C_1-C_6$alkyl$)_2$, $-C(=NH)NH_2$, -NH-C(=O)-$(C_1-C_6)$alkyl, -NH-C(=O)-$NH_2$, -NH-C(=O)-NH-$(C_1-C_6)$alkyl, -NH-C(=O)-(6- to 14- membered)aryl, -NH-C(=O)-$(C_1-C_6)$alkyl-(6- to 14-membered)aryl, -NH-$(C_1-C_6)$alkyl-$COOR^7$, -NH-C(=O)-$(C_1-C_6)$alkyl-$COOR^7$, -NH-C(=O)-$CH(NH_2)-(C_1-C_6)$alkyl-$COOR^7$, $-(C_3-C_{12})$cycloalkyl, $((C_3-C_{12})$cycloalkyl)-$(C_1-C_6)$alkyl-, -(6- to 14-membered)aryl, -(6- to 14-membered)aryloxy, $-(C_1-C_6)$alkoxy$CONR^5R^6$, -NH-$(C_1-C_6)$alkyl-$CONR^5R^6$, -CONH-$(C_1-C_6)$alkyl-$COOR^7$, ((6- to 14-membered)aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12-membered)heterocycle)-$(C_1-C_6)$alkyl-, -(7- to 12-membered)bicycloheterocycle, and ((7- to 12-membered)bicycloheterocycle)-$(C_1-C_6)$alkyl-;

$R^4$ is selected from

(a) -H, -OH, halo, $-C(halo)_3$, $-CH(halo)_2$, $-CH_2(halo)$, COOH, or $CONH_2$; or
(b) $-(C_1-C_5)$alkyl, $-(C_2-C_5)$alkenyl, $-(C_2-C_5)$alkynyl, $-(CH_2)_n-O-(CH_2)_n-CH_3$, or $-(C_1-C_5)$alkoxy, each of which is optionally substituted with 1, 2, or 3 independently selected $R^9$ groups;

$R^5$ and $R^6$ are each independently selected from

(a) hydrogen, -OH, halo, $-C(halo)_3$, $-CH(halo)_2$, $-CH_2(halo)$; or
(b) $-(C_1-C_6)$alkyl, $-(C_2-C_5)$alkenyl, $-(C_2-C_5)$alkynyl, $-(CH_2)_n-O-(CH_2)_n-CH_3$, $-(C_1-C_6)$alkoxy, each of which is optionally substituted with 1, 2, or 3 independently selected $R^9$ groups; or
(c) $-(C_3-C_8)$cycloalkyl, $((C_3-C_8)$cycloalkyl)-$(C_1-C_6)$alkyl-, $-COOR^7$, $-(C_1-C_6)$alkyl-$COOR^7$, $-CONH_2$, or $(C_1-C_6)$alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl, - $(C_2-C_6)$alkynyl, $-(C_3-C_{12})$cycloalkyl, $-(C_4-C_{12})$cycloalkenyl, $((C_3-C_{12})$cycloalkyl)-$(C_1-C_6)$alkyl-, and $((C_4-C_{12})$cycloalkenyl)-$(C_1-C_6)$alkyl- ;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)-$(C_1-C_6)$alkyl-, $((C_3-C_{12})$cycloalkenyl)-$(C_1-C_6)$alkyl-, -C(=O)-$(C_1-C_6)$alkyl or $SO_2-(C_1-C_6)$alkyl;

each $R^9$ is independently selected from -OH, halo, $-(C_1-C_{10})$alkyl, $-(C_2-C_{10})$alkenyl, $-(C_2-C_{10})$alkynyl, $-(C_1-C_{10})$alkoxy, $-(C_3-C_{12})$cycloalkyl, -CHO, -COOH, $-C(halo)_3$, - $CH(halo)_2$, $CH_2(halo)$, or $-(CH_2)_n-O-(CH_2)_n-CH_3$;

each $R^{14}$ is independently selected from the group consisting of $-COOR^7$, $-(C_1-C_6)$alkyl-$COOR^7$, -C(=O)-$(C_1-C_6)$alkyl-$COOR^7$, $-(C_1-C_6)$alkyl-C(=O)-$(C_1-C_6)$alkyl-$COOR^7$, $CONH_2$, and $-(C_1-C_6)$alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof;

provided that when $R^1$, $R^{2a}$, $R^{2b}$ are all methyl, and $R^4$ is OH or methoxy, then Z-G-$R^3$ is not OH.

In certain embodiments, the present disclosure provides compounds of Formula IA:

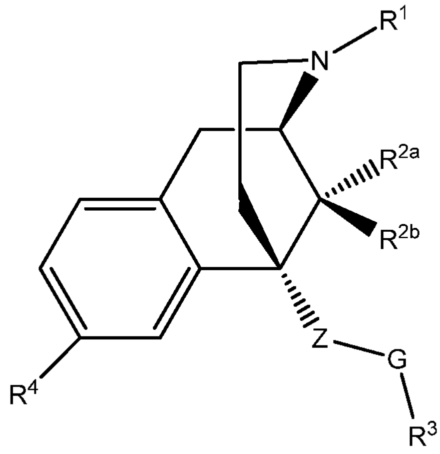

IA

wherein $R^1$, $R^{2a}$, $R^{2b}$, $R^3$, $R^4$, Z, and G are as defined above for Formula I'.

In certain embodiments, the present disclosure provides compounds of Formula IB:

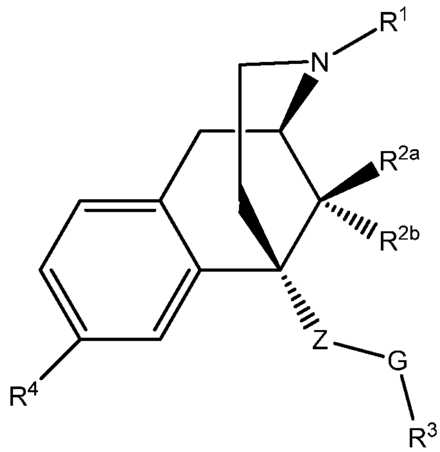

IB

wherein $R^1$, $R^{2a}$, $R^{2b}$, $R^3$, $R^4$, Z, and G are as defined above for Formula I'.

In certain embodiments, the present disclosure provides compounds of Formula IC:

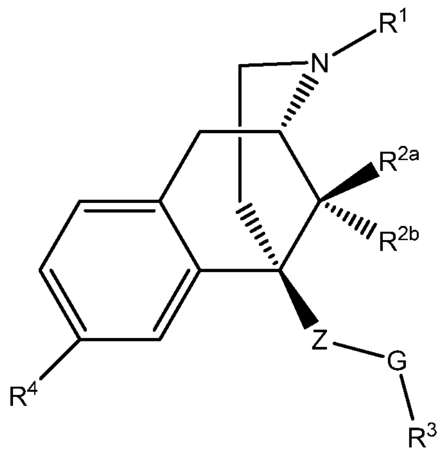

IC

wherein $R^1$, $R^{2a}$, $R^{2b}$, $R^3$, $R^4$, Z, and G are as defined above for Formula I'.

In certain embodiments, the present disclosure provides compounds of Formula ID:

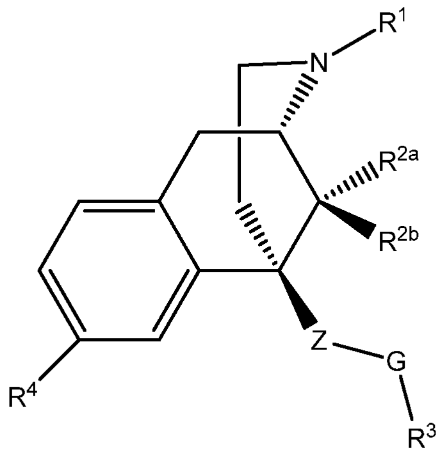

ID

wherein $R^1$, $R^{2a}$, $R^{2b}$, $R^3$, $R^4$, Z, and G are as defined above for Formula I'.

In one embodiment, the present invention provides compounds of Formula I.1:

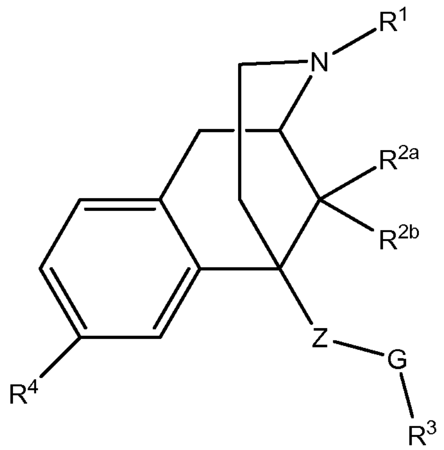

wherein

$R^1$ is selected from the group consisting of $-(C_3-C_{12})$cycloalkyl, $(C_3-C_{12})$cycloalkyl-$(C_1-C_6)$alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or
(b) $-(C_1-C_5)$alkyl, $-(C_2-C_5)$alkenyl, or $-(C_2-C_5)$alkynyl;

Z is absent or $-(CH_2)_m-$, optionally substituted with 1 or 2 independently selected $-(C_1-C_6)$alkyl;

G is selected from the group consisting of:

a) $-(C_1-C_6)$alkylene, $-(C_2-C_6)$alkenylene; or
b) -C(=O); or
c) $NR^8$;

$R^3$ is selected from the group consisting of hydrogen, $-(C_1-C_{10})$alkyl, $-(C_2-C_{12})$alkenyl, - C(=O)-$(C_1-C_6)$alkyl, -C(=O)-(6- to 14-membered)aryl, -C(=O)-(5- to 12-membered)heteroaryl, $-(C_2-C_{12})$alkynyl, $-(C_1-C_{10})$alkoxy, $-(OCH_2CH_2)_s-O(C_1-C_6)$alkyl, $-(CH_2CH_2O)_s-(C_1-C_6)$alkyl, $-NH_2$, $-NH(C_1-C_6)$alkyl, CN, $-CONR^5R^6$, $-(C_1-C_6)$alkyl-$CONR^5R^6$, $-COOR^7$, $-(C_1-C_6)$alkyl-$COOR^7$, $-(C_1-C_6)$alkoxy-$COOR^7$, $-C(=O)-(CH_2)_n-COOR^7$, $-C(=O)-(CH_2)_n-CONR^5R^6$, $-(C_3-C_{12})$cycloalkyl, $((C_3-C_{12})$cycloalkyl)-$(C_1-C_6)$alkyl-, $-(C_4-C_{12})$cycloalkenyl, $((C_4-C_{12})$cycloalkenyl)-$(C_1-C_6)$alkyl-, $-(C_6-C_{14})$bicycloalkyl, $((C_6-C_{14})$bicycloalkyl)-$(C_1-C_6)$alkyl-, $-(C_8-C_{20})$tricycloalkyl, $((C_8-C_{20})$tricycloalkyl)-$(C_1-C_6)$alkyl-, $-(C_7-C_{14})$bicycloalkenyl, $((C_7-C_{14})$bicycloalkenyl)-$(C_1-C_6)$alkyl-, $-(C_8-C_{20})$tricycloalkenyl, $((C_8-C_{20})$tricycloalkenyl)-$(C_1-C_6)$alkyl-, -(6- to 14-membered)aryl, ((6- to 14-membered)aryl)-$(C_1-C_6)$alkyl-, -(7- to 12-membered)bicyclic ring system, ((7- to 12-membered)bicyclic ring system)-$(C_1-C_6)$alkyl-, -(7- to 12-membered)bicyclic aryl, ((7- to 12-membered)bicyclic aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12 membered)heterocycle)-$(C_1-C_6)$alkyl-, -(7- to 12-membered)bicycloheterocycle, and ((7- to 12-membered)bicycloheterocycle)-$(C_1-C_6)$alkyl-; each of which is optionally substituted with one, two, or three substituents independently selected from the group consisting of -OH, (=O), halo, $-C(halo)_3$, $-CH(halo)_2$, $-CH_2(halo)$, $-(C_1-C_6)$alkyl, halo$(C_1-C_6)$alkyl-, $-(C_2-C_6)$alkenyl, $-(C_2-C_6)$alkynyl, hydroxy$(C_1-C_6)$alkyl-, dihydroxy$(C_1-C_6)$alkyl-, $-(C_1-C_6)$alkoxy, $((C_1-C_6)$alkoxy)-C(=O)-$(C_1-C_6)$alkoxy-, $-NH_2$, $-NR^5R^6$, $-NH(C_1-C_6)$alkyl, $-(C_1-C_6)$alkyl-$NH(C_1-C_6)$alkyl-$R^{14}$, -CN, -SH, $-OR^4$, $-CONR^5R^6$, $-(C_1-C_6$alkyl)-C(=O)-$NR^5R^6$, $-COOR^7$, $-(C_1-C_6)$alkyl-$COOR^7$, $-(C_1-C_6)$alkoxy-$COOR^7$, $-(OCH_2CH_2)_s-O(C_1-C_6)$alkyl, $-(CH_2CH_2O)_s-(C_1-C_6)$alkyl, $((C_1-C_6)$alkyl)sulfonyl$(C_1-C_6)$alkyl-, $-NH-SO_2(C_1-C_6)$alkyl, $-N-(SO_2-(C_1-C_6)alkyl)_2$, $-C(=NH)-NH_2$, -NH-C(=O)-$(C_1-C_6)$alkyl, - NH-C(=O)-$NH_2$, -NH-C(=O)-NH-$(C_1-C_6)$alkyl, -NH-C(=O)-(6- to 14- membered)aryl, - NH-C(=O)-$(C_1-C_6)$alkyl-(6- to 14- membered)aryl, -NH-$(C_1-C_6)$alkyl-$COOR^7$, -NH-C(=O)-$(C_1-C_6)$alkyl-$COOR^7$, -NH-C(=O)-$CH(NH_2)$-$(C_1-C_6)$alkyl-C(=O)-$OR^7$, $-(C_3-C_{12})$cycloalkyl, $((C_3-C_{12})$cycloalkyl)-$(C_1-C_6)$alkyl-, -(6- to 14-membered)aryl, -(6- to 14-membered)aryloxy, $-(C_1-C_6)$alkoxyC(O)$NR^5R^6$, -NH-$(C_1-C_6)$alkylC(O)-$NR^5R^6$, - C(O)NH-$(C_1-C_6)$alkyl-$COOR^7$, ((6- to 14-membered)aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12-

membered)heterocycle)-($C_1$-$C_6$)alkyl-, -(7- to 12-membered)bicycloheterocycle, and ((7- to 12-membered)bicycloheterocycle)-($C_1$-$C_6$)alkyl-;

$R^4$ is selected from

(a) -H, -OH, halo, -C(halo)$_3$, -CH(halo)$_2$, -$CH_2$(halo), COOH, or $CONH_2$; or
(b) -($C_1$-$C_5$)alkyl, -($C_2$-$C_5$)alkenyl, -($C_2$-$C_5$)alkynyl, -$(CH_2)_n$-O-$(CH_2)_n$-$CH_3$, or -($C_1$-$C_5$)alkoxy, each of which is optionally substituted with 1, 2, or 3 independently selected $R^9$ groups;

$R^5$ and $R^6$ are each independently selected from

(a) hydrogen, -OH, halo, -C(halo)$_3$, -CH(halo)$_2$, -$CH_2$(halo); or
(b) -($C_1$-$C_6$)alkyl, -($C_2$-$C_5$)alkenyl, -($C_2$-$C_5$)alkynyl, -$(CH_2)_n$-O-$(CH_2)_n$-$CH_3$, -($C_1$-$C_6$)alkoxy, each of which is optionally substituted with 1, 2, or 3 independently selected $R^9$ groups; or
(c) -($C_3$-$C_8$)cycloalkyl, (($C_3$-$C_8$)cycloalkyl)-($C_1$-$C_6$)alkyl-, -COOR$^7$, -($C_1$-$C_6$)alkyl-COOR$^7$, -$CONH_2$, or ($C_1$-$C_6$)alkyl-CONH-; or
(d) $R^5$ and $R^6$ together with the nitrogen atom to which they are attached form a (4-to 8-membered)heterocycle;

$R^7$ is selected from the group consisting of hydrogen, -($C_1$-$C_6$)alkyl, -($C_2$-$C_6$)alkenyl, - ($C_2$-$C_6$)alkynyl, -($C_3$-$C_{12}$)cycloalkyl, -($C_4$-$C_{12}$)cycloalkenyl, (($C_3$-$C_{12}$)cycloalkyl)-($C_1$-$C_6$)alkyl-, and (($C_4$-$C_{12}$)cycloalkenyl)-($C_1$-$C_6$)alkyl- ;

$R^8$ is selected from H, -($C_1$-$C_6$)alkyl, -($C_2$-$C_6$)alkenyl, -($C_2$-$C_6$)alkynyl, -($C_1$-$C_{10}$)alkoxy, -($C_3$-$C_{12}$)cycloalkyl, -($C_3$-$C_{12}$)cycloalkenyl, (($C_3$-$C_{12}$)cycloalkyl)-($C_1$-$C_6$)alkyl-, (($C_3$-$C_{12}$)cycloalkenyl)-($C_1$-$C_6$)alkyl-, -C(=O)($C_1$-$C_6$)alkyl or $SO_2$($C_1$-$C_6$)alkyl;

each $R^9$ is independently selected from -OH, halo, -($C_1$-$C_{10}$)alkyl, -($C_2$-$C_{10}$)alkenyl, -($C_2$-$C_{10}$)alkynyl, -($C_1$-$C_{10}$)alkoxy, -($C_3$-$C_{12}$)cycloalkyl, -CHO, -C(O)OH, -C(halo)$_3$, - CH(halo)2, $CH_2$(halo), or -$(CH_2)_n$-O-$(CH_2)_n$-$CH_3$;

each $R^{14}$ is independently selected from the group consisting of -COOR$^7$, -($C_1$-$C_6$)alkyl-COOR$^7$, -C(=O)-($C_1$-$C_6$)alkyl-COOR$^7$, -($C_1$-$C_6$)alkyl-C(=O)-($C_1$-$C_6$)alkyl-COOR$^7$, $CONH_2$, and -($C_1$-$C_6$)alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof.

In a further embodiment, the present invention provides compounds of Formula 1.2:

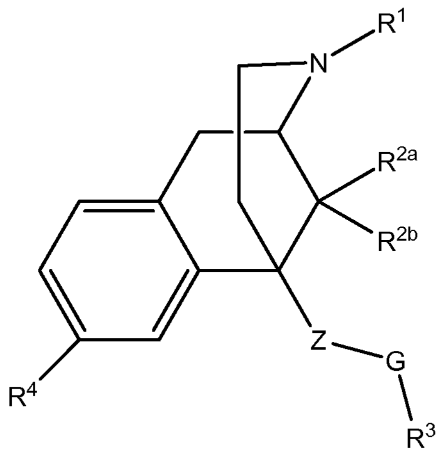

wherein

$R^1$ is selected from the group consisting of -($C_3$-$C_{12}$)cycloalkyl, ($C_3$-$C_{12}$)cycloalkyl-($C_1$-$C_6$)alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or
(b) -($C_1$-$C_5$)alkyl, -($C_2$-$C_5$)alkenyl, or -($C_2$-$C_5$)alkynyl;

Z is absent or -$(CH_2)_m$-, optionally substituted with 1 or 2 independently selected -($C_1$-$C_6$)alkyl;

G is selected from the group consisting of:

(a) -($C_1$-$C_6$)alkylene, -($C_2$-$C_6$)alkenylene;
(b) -C(=O); and
(c) $NR^8$;

$R^3$ is selected from the group consisting of hydrogen, -($C_1$-$C_{10}$)alkyl, -($C_2$-$C_{12}$)alkenyl, - ($C_2$-$C_{12}$)alkynyl, -($C_1$-$C_{10}$)alkoxy, -$(OCH_2CH_2)_s$-O($C_1$-$C_6$)alkyl, -$(CH_2CH_2O)_s$-($C_1$-$C_6$)alkyl, -$NH_2$, -NH($C_1$-$C_6$)alkyl, CN, -$CONR^5R^6$, -($C_1$-$C_6$)alkyl-CO-$NR^5R^6$, -$COOR^7$, -($C_1$-$C_6$)alkyl-CO-$OR^7$, -($C_1$-$C_6$)alkoxy-$COOR^7$, -CO-$(CH_2)_n$-$COOR^7$, -CO-$(CH_2)_n$-CO-$NR^5R^6$, -($C_3$-$C_{12}$)cycloalkyl, (($C_3$-$C_{12}$)cycloalkyl)-($C_1$-$C_6$)alkyl-, -($C_4$-$C_{12}$)cycloalkenyl, (($C_4$-$C_{12}$)cycloalkenyl)-($C_1$-$C_6$)alkyl-, -($C_6$-$C_{14}$)bicycloalkyl, (($C_6$-$C_{14}$)bicycloalkyl)-($C_1$-$C_6$)alkyl-, -($C_8$-$C_{20}$)tricycloalkyl, (($C_8$-$C_{20}$)tricycloalkyl)-($C_1$-$C_6$)alkyl-, -($C_7$-$C_{14}$)bicycloalkenyl, (($C_7$-$C_{14}$)bicycloalkenyl)-($C_1$-$C_6$)alkyl-, -($C_8$-$C_{20}$)tricycloalkenyl, (($C_8$-$C_{20}$)tricycloalkenyl)-($C_1$-$C_6$)alkyl-, -(6- to 14-membered)aryl, ((6- to 14-membered)aryl)-($C_1$-$C_6$)alkyl-, -(7- to 12-membered)bicyclic ring system, ((7-to 12-membered)bicyclic ring system)-($C_1$-$C_6$)alkyl-, -(7- to 12-membered)bicyclic aryl, ((7- to 12-membered)bicyclic aryl)-($C_1$-$C_6$)alkyl-, -(5- to 12-membered)heteroaryl, ((5-to 12-membered)heteroaryl)-($C_1$-$C_6$)alkyl-, -(3- to 12-membered)heterocycle, ((3- to 12 membered)heterocycle)-($C_1$-$C_6$)alkyl-, -(7- to 12-membered)bicycloheterocycle, and ((7- to 12-membered)bicycloheterocycle)-($C_1$-$C_6$)alkyl-; each of which is optionally substituted with one, two, or three substituents independently selected from the group consisting of -OH, (=O), halo, -$C(halo)_3$, -$CH(halo)_2$, -$CH_2$(halo), -($C_1$-$C_6$)alkyl, halo($C_1$-$C_6$)alkyl-, -($C_2$-$C_6$)alkenyl, -($C_2$-$C_6$)alkynyl, hydroxy($C_1$-$C_6$)alkyl-, dihydroxy($C_1$-$C_6$)alkyl-, -($C_1$-$C_6$)alkoxy, (($C_1$-$C_6$)alkoxy)CO($C_1$-$C_6$)alkoxy-, -$NH_2$, - NH($C_1$-$C_6$)alkyl, -($C_1$-$C_6$)alkyl-NH($C_1$-$C_6$)alkyl-$R^{14}$, -CN, -SH, -$OR^4$, -$CONR^5R^6$, -($C_1$-$C_6$alkyl)-CO-$NR^5R^6$, -$COOR^7$, -($C_1$-$C_6$)alkyl-CO-$OR^7$, -($C_1$-$C_6$)alkoxy-$COOR^7$, -$(OCH_2CH_2)_s$-O($C_1$-$C_6$)alkyl, -$(CH_2CH_2O)_s$-($C_1$-$C_6$)alkyl, (($C_1$-$C_6$)alkyl)sulfonyl($C_1$-$C_6$)alkyl-, -NH-$SO_2$($C_1$-$C_6$)alkyl, -N($SO_2$($C_1$-$C_6$)alkyl$)_2$, -C(=NH)$NH_2$, -NH-CO-($C_1$-$C_6$)alkyl, -NH-CO-$NH_2$, -NH-C(=O)-NH-($C_1$-$C_6$)alkyl, -NH-C(=O)-(6- to 14-membered)aryl, -NH-C(=O)-($C_1$-$C_6$)alkyl-(6- to 14- membered)aryl, -NH-($C_1$-$C_6$)alkyl-CO-$OR^7$, -NH-C(=O)-($C_1$-$C_6$)alkyl-CO-$OR^7$, -NH-C(=O)-CH($NH_2$)-($C_1$-$C_6$)alkyl-CO-$OR^7$, -($C_3$-$C_{12}$)cycloalkyl, (($C_3$-$C_{12}$)cycloalkyl)-($C_1$-$C_6$)alkyl-, -(6- to 14-membered)aryl, -(6- to 14-membered)aryloxy, -($C_1$-$C_6$)alkoxyC(O)$NR^5R^6$, -NH-($C_1$-$C_6$)alkylC(O)-$NR^5R^6$, -C(O)NH-($C_1$-$C_6$)alkyl-$COOR^7$, ((6- to 14-membered)aryl)-($C_1$-$C_6$)alkyl-, -(5-to 12-membered)heteroaryl, ((5- to 12-membered)heteroaryl)-($C_1$-$C_6$)alkyl-, -(3- to 12-membered)heterocycle, ((3- to 12-membered)heterocycle)-($C_1$-$C_6$)alkyl-, -(7- to 12-membered)bicycloheterocycle, and ((7- to 12-membered)bicycloheterocycle)-($C_1$-$C_6$)alkyl-;

$R^4$ is selected from

(a) -H, -OH, halo, -$C(halo)_3$, -$CH(halo)_2$, -$CH_2$(halo), COOH, or $CONH_2$; or
(b) -($C_1$-$C_5$)alkyl, -($C_2$-$C_5$)alkenyl, -($C_2$-$C_5$)alkynyl, -$(CH_2)_n$-O-$(CH_2)_n$-$CH_3$, or - ($C_1$-$C_5$)alkoxy, each of which is optionally substituted with 1, 2, or 3 independently selected $R^9$ groups;

$R^5$ and $R^6$ are each independently selected from

(a) hydrogen, -OH, halo, -$C(halo)_3$, -$CH(halo)_2$, -$CH_2$(halo);
(b) -($C_1$-$C_6$)alkyl, -($C_2$-$C_5$)alkenyl, -($C_2$-$C_5$)alkynyl, -$(CH_2)_n$-O-$(CH_2)_n$-$CH_3$, - ($C_1$-$C_6$)alkoxy, each of which is optionally substituted with 1, 2, or 3 independently selected $R^9$ groups;
(c) -($C_3$-$C_8$)cycloalkyl, (($C_3$-$C_8$)cycloalkyl)-($C_1$-$C_6$)alkyl-, -$COOR^7$, -($C_1$-$C_6$)alkyl-$COOR^7$, -$CONH_2$, or ($C_1$-$C_6$)alkyl-CONH-; or
(d) $R^5$ and $R^6$ together with the nitrogen atom to which they are attached form a (4- to 8-membered)heterocycle;

$R^7$ is selected from the group consisting of hydrogen, -($C_1$-$C_6$)alkyl, -($C_2$-$C_6$)alkenyl, - ($C_2$-$C_6$)alkynyl, -($C_3$-$C_{12}$)cy-

cloalkyl, -($C_4$-$C_{12}$)cycloalkenyl, (($C_3$-$C_{12}$)cycloalkyl)-($C_1$-$C_6$)alkyl-, and (($C_4$-$C_{12}$)cycloalkenyl)-($C_1$-$C_6$)alkyl- ;

$R^8$ is selected from H, -($C_1$-$C_6$)alkyl, -($C_2$-$C_6$)alkenyl, -($C_2$-$C_6$)alkynyl, -($C_1$-$C_{10}$)alkoxy, -($C_3$-$C_{12}$)cycloalkyl, -($C_3$-$C_{12}$)cycloalkenyl, (($C_3$-$C_{12}$)cycloalkyl)-($C_1$-$C_6$)alkyl-, (($C_3$-$C_{12}$)cycloalkenyl)-($C_1$-$C_6$)alkyl-, -C(=O)($C_1$-$C_6$)alkyl or $SO_2$($C_1$-$C_6$)alkyl;

each $R^9$ is independently selected from -OH, halo, -($C_1$-$C_{10}$)alkyl, -($C_2$-$C_{10}$)alkenyl, -($C_2$-$C_{10}$)alkynyl, -($C_1$-$C_{10}$)alkoxy, -($C_3$-$C_{12}$)cycloalkyl, -CHO, -C(O)OH, -C(halo)$_3$, - CH(halo)2, $CH_2$(halo), or -($CH_2$)$_n$-O-($CH_2$)$_n$-$CH_3$;

each $R^{14}$ is independently selected from the group consisting of -COOR$^7$, -($C_1$-$C_6$)alkyl-COOR$^7$, -C(=O)-($C_1$-$C_6$)alkyl-COOR$^7$, -($C_1$-$C_6$)alkyl-C(=O)-($C_1$-$C_6$)alkyl-COOR$^7$, $CONH_2$, and -($C_1$-$C_6$)alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts, and solvates thereof.

In another embodiment, the invention provides compounds of Formula 1.3:

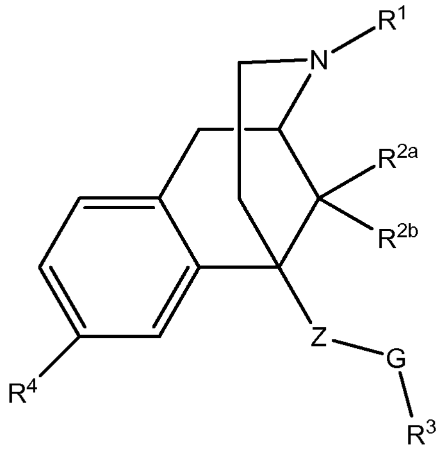

wherein

$R^1$ is selected from the group consisting of -($C_3$-$C_{12}$)cycloalkyl, ($C_3$-$C_{12}$)cycloalkyl-($C_1$-$C_6$)alkyl-, and -(6- to 14-membered)aryl, each of which is optionally substituted by 1, 2 or 3 independently selected $R^9$ groups;

$R^{2a}$ and $R^{2b}$ are each independently selected from:

(a) -H; or
(b) -($C_1$-$C_5$)alkyl,

Z is absent or -($CH_2$)$_m$-, optionally substituted with 1 or 2 independently selected -($C_1$-$C_6$)alkyl;

G is selected from the group consisting of:

a) -($C_1$-$C_6$)alkylene, -($C_2$-$C_6$)alkenylene; or
b) -C(=O); or
c) $NR^8$;

$R^3$ is selected from the group consisting of hydrogen, -($C_1$-$C_{10}$)alkyl, -($C_2$-$C_{12}$)alkenyl, - C(=O)-($C_1$-$C_6$)alkyl, -C(=O)-(6- to 14-membered)aryl, -C(=O)-(5- to 12-membered)heteroaryl, -($C_2$-$C_{12}$)alkynyl, -($C_1$-$C_{10}$)alkoxy,

$-(OCH_2CH_2)_s-O(C_1-C_6)$alkyl, $-(CH_2CH_2O)_s-(C_1-C_6)$alkyl, $-NH_2$, $-NH(C_1-C_6)$alkyl, CN, $-CONR^5R^6$, $-(C_1-C_6)$alkyl-$CONR^5R^6$, $-COOR^7$, $-(C_1-C_6)$alkyl-$COOR^7$, $-(C_1-C_6)$alkoxy-$COOR^7$, $-C(=O)-(CH_2)_n-COOR^7$, $-C(=O)-(CH_2)_n-CONR^5R^6$, $-(C_3-C_{12})$cycloalkyl, $((C_3-C_{12})$cycloalkyl)-$(C_1-C_6)$alkyl-, $-(C_4-C_{12})$cycloalkenyl, $((C_4-C_{12})$cycloalkenyl)-$(C_1-C_6)$alkyl-, $-(C_6-C_{14})$bicycloalkyl, $((C_6-C_{14})$bicycloalkyl)-$(C_1-C_6)$alkyl-, $-(C_8-C_{20})$tricycloalkyl, $((C_8-C_{20})$tricycloalkyl)-$(C_1-C_6)$alkyl-, $-(C_7-C_{14})$bicycloalkenyl, $((C_7-C_{14})$bicycloalkenyl)-$(C_1-C_6)$alkyl-, $-(C_8-C_{20})$tricycloalkenyl, $((C_8-C_{20})$tricycloalkenyl)-$(C_1-C_6)$alkyl-, -(6- to 14-membered)aryl, ((6- to 14-membered)aryl)-$(C_1-C_6)$alkyl-, -(7- to 12-membered)bicyclic ring system, ((7- to 12-membered)bicyclic ring system)-$(C_1-C_6)$alkyl-, -(7- to 12-membered)bicyclic aryl, ((7- to 12-membered)bicyclic aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12 membered)heterocycle)-$(C_1-C_6)$alkyl-, -(7- to 12-membered)bicycloheterocycle, and ((7- to 12-membered)bicycloheterocycle)-$(C_1-C_6)$alkyl-; each of which is optionally substituted with one, two, or three substituents independently selected from the group consisting of -OH, (=O), halo, $-C(halo)_3$, $-CH(halo)_2$, $-CH_2(halo)$, $-(C_1-C_6)$alkyl, halo$(C_1-C_6)$alkyl-, $-(C_2-C_6)$alkenyl, $-(C_2-C_6)$alkynyl, hydroxy$(C_1-C_6)$alkyl-, dihydroxy$(C_1-C_6)$alkyl-, $-(C_1-C_6)$alkoxy, $((C_1-C_6)$alkoxy)-C(=O)-$(C_1-C_6)$alkoxy-, $-NH_2$, $-NR^5R^6$, $-NH(C_1-C_6)$alkyl, $-(C_1-C_6)$alkyl-$NH(C_1-C_6)$alkyl-$R^{14}$, -CN, -SH, $-OR^4$, $-CONR^5R^6$, $-(C_1-C_6$alkyl)-C(=O)-$NR^5R^6$, $-COOR^7$, $-(C_1-C_6)$alkyl-$COOR^7$, $-(C_1-C_6)$alkoxy-$COOR^7$, $-(OCH_2CH_2)_s-O(C_1-C_6)$alkyl, $-(CH_2CH_2O)_s-(C_1-C_6)$alkyl, $((C_1-C_6)$alkyl)sulfonyl$(C_1-C_6)$alkyl-, $-NH-SO_2(C_1-C_6)$alkyl, $-N-(SO_2-(C_1-C_6)alkyl)_2$, $-C(=NH)-NH_2$, -NH-C(=O)-$(C_1-C_6)$alkyl,-NH-C(=O)-$NH_2$, -NH-C(=O)-NH-$(C_1-C_6)$alkyl, -NH-C(=O)-(6- to 14- membered)aryl,-NH-C(=O)-$(C_1-C_6)$alkyl-(6- to 14- membered)aryl, -NH-$(C_1-C_6)$alkyl-$COOR^7$, -NH-C(=O)-$(C_1-C_6)$alkyl-$COOR^7$, -NH-C(=O)-$CH(NH_2)$-$(C_1-C_6)$alkyl-C(=O)-$OR^7$, $-(C_3-C_{12})$cycloalkyl, $((C_3-C_{12})$cycloalkyl)-$(C_1-C_6)$alkyl-, -(6- to 14-membered)aryl, -(6- to 14-membered)aryloxy, $-(C_1-C_6)$alkoxyC(O)$NR^5R^6$, -NH-$(C_1-C_6)$alkylC(O)-$NR^5R^6$,-C(O)NH-$(C_1-C_6)$alkyl-$COOR^7$, ((6- to 14-membered)aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12-membered)heterocycle)-$(C_1-C_6)$alkyl-, -(7- to 12-membered)bicycloheterocycle, and ((7- to 12-membered)bicycloheterocycle)-$(C_1-C_6)$alkyl-;

$R^4$ is selected from

(a) -H, or
(b) $(C_1-C_5)$alkoxy, each of which is optionally substituted with 1, 2, or 3 independently selected $R^9$ groups;

$R^5$ and $R^6$ are each independently selected from

(a) hydrogen, -OH, halo, $-C(halo)_3$, $-CH(halo)_2$, $-CH_2(halo)$; or
(b) $-(C_1-C_6)$alkyl, $-(C_2-C_5)$alkenyl, $-(C_2-C_5)$alkynyl, $-(CH_2)_n-O-(CH_2)_n-CH_3$, $-(C_1-C_6)$alkoxy, each of which is optionally substituted with 1, 2, or 3 independently selected $R^9$ groups; or
(c) $-(C_3-C_8)$cycloalkyl, $((C_3-C_8)$cycloalkyl)-$(C_1-C_6)$alkyl-, $-COOR^7$, $-(C_1-C_6)$alkyl-$COOR^7$, $-CONH_2$, or $(C_1-C_6)$alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl,$-(C_2-C_6)$alkynyl, $-(C_3-C_{12})$cycloalkyl, $-(C_4-C_{12})$cycloalkenyl, $((C_3-C_{12})$cycloalkyl)-$(C_1-C_6)$alkyl-, and $((C_4-C_{12})$cycloalkenyl)-$(C_1-C_6)$alkyl-;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)-$(C_1-C_6)$alkyl-, $((C_3-C_{12})$cycloalkenyl)-$(C_1-C_6)$alkyl-, -C(=O)$(C_1-C_6)$alkyl or $SO_2(C_1-C_6)$alkyl;

each $R^9$ is independently selected from -OH, halo, $-(C_1-C_{10})$alkyl, $-(C_2-C_{10})$alkenyl, $-(C_2-C_{10})$alkynyl, $-(C_1-C_{10})$alkoxy, $-(C_3-C_{12})$cycloalkyl, -CHO, -C(O)OH, $-C(halo)_3$,-CH(halo)2, $CH_2$(halo), or $-(CH_2)_n-O-(CH_2)_n-CH_3$;

each $R^{14}$ is independently selected from the group consisting of $-COOR^7$, $-(C_1-C_6)$alkyl-$COOR^7$, -C(=O)-$(C_1-C_6)$alkyl-$COOR^7$, $-(C_1-C_6)$alkyl-C(=O)-$(C_1-C_6)$alkyl-$COOR^7$, $CONH_2$, and $-(C_1-C_6)$alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof.

In another embodiment, the invention provides compounds of Formula 1.4:

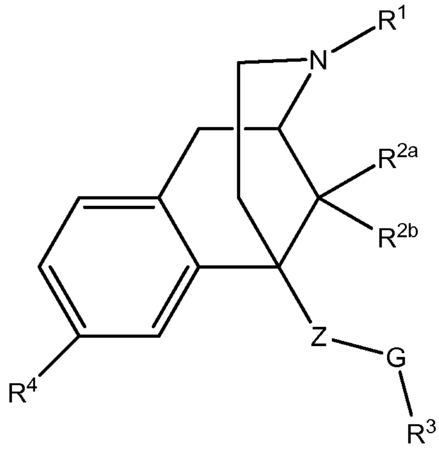

wherein

$R^1$ is $(C_3\text{-}C_{12})$cycloalkyl-$(C_1\text{-}C_6)$alkyl-, which is optionally substituted by 1, 2 or 3 independently selected $R^9$ groups;

$R^{2a}$ and $R^{2b}$ are each independently selected from:

-$(C_1\text{-}C_5)$alkyl;

Z is absent or -$(CH_2)_m$-, optionally substituted with 1 or 2 independently selected -$(C_1\text{-}C_6)$alkyl;

G is selected from the group consisting of:

a) -$(C_1\text{-}C_6)$alkylene, -$(C_2\text{-}C_6)$alkenylene; or
b) -C(=O); or
c) $NR^8$;

$R^3$ is selected from the group consisting of hydrogen, -$(C_1\text{-}C_{10})$alkyl, -$(C_2\text{-}C_{12})$alkenyl,-C(=O)-$(C_1\text{-}C_6)$alkyl, -C(=O)-(6- to 14-membered)aryl, -C(=O)-(5- to 12-membered)heteroaryl, -$(C_2\text{-}C_{12})$alkynyl, -$(C_1\text{-}C_{10})$alkoxy, -$(OCH_2CH_2)_s$-O$(C_1\text{-}C_6)$alkyl, -$(CH_2CH_2O)_s$-$(C_1\text{-}C_6)$alkyl, -$NH_2$, -NH$(C_1\text{-}C_6)$alkyl, CN, -$CONR^5R^6$, -$(C_1\text{-}C_6)$alkyl-$CONR^5R^6$, -$COOR^7$, -$(C_1\text{-}C_6)$alkyl-$COOR^7$, -$(C_1\text{-}C_6)$alkoxy-$COOR^7$, -C(=O)-$(CH_2)_n$-$COOR^7$, -C(=O)-$(CH_2)_n$-$CONR^5R^6$, -$(C_3\text{-}C_{12})$cycloalkyl, ($(C_3\text{-}C_{12})$cycloalkyl)-$(C_1\text{-}C_6)$alkyl-, -$(C_4\text{-}C_{12})$cycloalkenyl, ($(C_4\text{-}C_{12})$cycloalkenyl)-$(C_1\text{-}C_6)$alkyl-, -$(C_6\text{-}C_{14})$bicycloalkyl, ($(C_6\text{-}C_{14})$bicycloalkyl)-$(C_1\text{-}C_6)$alkyl-, -$(C_8\text{-}C_{20})$tricycloalkyl, ($(C_8\text{-}C_{20})$tricycloalkyl)-$(C_1\text{-}C_6)$alkyl-, -$(C_7\text{-}C_{14})$bicycloalkenyl, ($(C_7\text{-}C_{14})$bicycloalkenyl)-$(C_1\text{-}C_6)$alkyl-, -$(C_8\text{-}C_{20})$tricycloalkenyl, ($(C_8\text{-}C_{20})$tricycloalkenyl)-$(C_1\text{-}C_6)$alkyl-, -(6- to 14-membered)aryl, ((6- to 14-membered)aryl)-$(C_1\text{-}C_6)$alkyl-, -(7- to 12-membered)bicyclic ring system, ((7- to 12-membered)bicyclic ring system)-$(C_1\text{-}C_6)$alkyl-, -(7- to 12-membered)bicyclic aryl, ((7- to 12-membered)bicyclic aryl)-$(C_1\text{-}C_6)$alkyl-, -(5- to 12-membered)heteroaryl, ((5- to 12-membered)heteroaryl)-$(C_1\text{-}C_6)$alkyl-, -(3- to 12-membered)heterocycle, ((3- to 12 membered)heterocycle)-$(C_1\text{-}C_6)$alkyl-, -(7- to 12-membered)bicycloheterocycle, and ((7- to 12-membered)bicycloheterocycle)-$(C_1\text{-}C_6)$alkyl-; each of which is optionally substituted with one, two, or three substituents independently selected from the group consisting of -OH, (=O), halo, -C$(halo)_3$, -CH$(halo)_2$, -$CH_2$(halo), -$(C_1\text{-}C_6)$alkyl, halo$(C_1\text{-}C_6)$alkyl-, -$(C_2\text{-}C_6)$alkenyl, -$(C_2\text{-}C_6)$alkynyl, hydroxy$(C_1\text{-}C_6)$alkyl-, dihydroxy$(C_1\text{-}C_6)$alkyl-, -$(C_1\text{-}C_6)$alkoxy, ($(C_1\text{-}C_6)$alkoxy)-C(=O)-$(C_1\text{-}C_6)$alkoxy-, -$NH_2$, -$NR^5R^6$, -NH$(C_1\text{-}C_6)$alkyl, -$(C_1\text{-}C_6)$alkyl-NH$(C_1\text{-}C_6)$alkyl-$R^{14}$, -CN, -SH, -$OR^4$, -$CONR^5R^6$, -$(C_1\text{-}C_6$alkyl)-C(=O)-$NR^5R^6$, -$COOR^7$, -$(C_1\text{-}C_6)$alkyl-$COOR^7$, -$(C_1\text{-}C_6)$alkoxy-$COOR^7$, -$(OCH_2CH_2)_s$-O$(C_1\text{-}C_6)$alkyl, -$(CH_2CH_2O)_s$-$(C_1\text{-}C_6)$alkyl, ($(C_1\text{-}C_6)$alkyl)sulfonyl$(C_1\text{-}C_6)$alkyl-, -NH-$SO_2(C_1\text{-}C_6)$alkyl, -N-$(SO_2\text{-}(C_1\text{-}C_6)alkyl)_2$, -C(=NH)-$NH_2$, -NH-C(=O)-$(C_1\text{-}C_6)$alkyl,-NH-C(=O)-$NH_2$, -NH-C(=O)-NH-$(C_1\text{-}C_6)$alkyl, -NH-C(=O)-(6- to 14- membered)aryl,-NH-C(=O)-$(C_1\text{-}C_6)$alkyl-(6- to 14- membered)aryl, -NH-$(C_1\text{-}C_6)$alkyl-$COOR^7$, -NH-C(=O)-$(C_1\text{-}C_6)$alkyl-$COOR^7$, -NH-C(=O)-CH$(NH_2)$-$(C_1\text{-}C_6)$alkyl-C(=O)-$OR^7$, -$(C_3\text{-}C_{12})$cycloalkyl, ($(C_3\text{-}C_{12})$cycloalkyl)-$(C_1\text{-}C_6)$alkyl-, -(6- to 14-membered)aryl, -(6- to 14-membered)aryloxy, -$(C_1\text{-}C_6)$alkoxyC(O)$NR^5R^6$,

$-NH-(C_1-C_6)alkylC(O)-NR^5R^6$,$-C(O)NH-(C_1-C_6)alkyl-COOR^7$, ((6- to 14-membered)aryl)-$(C_1-C_6)$alkyl-, -(5- to 12-membered)heteroaryl, ((5- to 12-membered)heteroaryl)-$(C_1-C_6)$alkyl-, -(3- to 12-membered)heterocycle, ((3- to 12-membered)heterocycle)-$(C_1-C_6)$alkyl-, -(7- to 12-membered)bicycloheterocycle, and ((7- to 12-membered)bicycloheterocycle)-$(C_1-C_6)$alkyl-;

$R^4$ is selected from -OH or $-OCH_3$;

$R^5$ and $R^6$ are each independently selected from

(a) hydrogen, -OH, halo, $-C(halo)_3$, $-CH(halo)_2$, $-CH_2(halo)$; or
(b) $-(C_1-C_6)$alkyl, $-(C_2-C_5)$alkenyl, $-(C_2-C_5)$alkynyl, $-(CH_2)_n-O-(CH_2)_n-CH_3$, $-(C_1-C_6)$alkoxy, each of which is optionally substituted with 1, 2, or 3 independently selected $R^9$ groups; or
(c) $-(C_3-C_8)$cycloalkyl, $((C_{3-}C_8)$cycloalkyl)-$(C_1-C_6)$alkyl-, $-COOR^7$, $-(C_1-C_6)alkyl-COOR^7$, $-CONH_2$, or $(C_{1-}C_6)$alkyl-CONH-; or
(d) $R^5$ and $R^6$ together with the nitrogen atom to which they are attached form a (4-to 8-membered)heterocycle;

$R^7$ is selected from the group consisting of hydrogen, $-(C_1-C_6)$alkyl, $-(C_2-C_6)$alkenyl,$-(C_2-C_6)$alkynyl, $-(C_{3-}C_{12})$cycloalkyl, $-(C_4-C_{12})$cycloalkenyl, $((C_3-C_{12})$cycloalkyl)-$(C_1-C_6)$alkyl-, and $((C_{4-}C_{12})$cycloalkenyl)-$(C_1-C_6)$alkyl-;

$R^8$ is selected from H, $-(C_1-C_6)$alkyl, $-(C_2-C_6)$alkenyl, $-(C_2-C_6)$alkynyl, $-(C_1-C_{10})$alkoxy, $-(C_3-C_{12})$cycloalkyl, $-(C_3-C_{12})$cycloalkenyl, $((C_3-C_{12})$cycloalkyl)-$(C_1-C_6)$alkyl-, $((C_3-C_{12})$cycloalkenyl)-$(C_1-C_6)$alkyl-, $-C(=O)(C_1-C_6)$alkyl or $SO_2(C_1-C_6)$alkyl;

each $R^9$ is independently selected from -OH, halo, $-(C_{1-}C_{10})$alkyl, $-(C_{2-}C_{10})$alkenyl, $-(C_{2-}C_{10})$alkynyl, $-(C_{1-}C_{10})$alkoxy, $-(C_{3-}C_{12})$cycloalkyl, -CHO, -C(O)OH, $-C(halo)_3$,$-CH(halo)_2$, $CH_2(halo)$, or $-(CH_2)_n-O-(CH_2)_n-CH_3$;

each $R^{14}$ is independently selected from the group consisting of $-COOR^7$, $-(C_1-C_6)alkyl-COOR^7$, $-C(=O)-(C_1-C_6)alkyl-COOR^7$, $-(C_1-C_6)alkyl-C(=O)-(C_1-C_6)alkyl-COOR^7$, $CONH_2$, and $-(C_1-C_6)$alkyl-CONH;

m is an integer 1, 2, 3, 4, 5, or 6;

n is an integer 0, 1, 2, 3, 4, 5, or 6;

s in an integer 1, 2, 3, 4, 5, or 6;

and the pharmaceutically acceptable salts and solvates thereof.

In another embodiment, the present invention provides compounds of Formula I.1, I.2, I.3, and I.4, R3 provided that Z-G-$R^3$ is not unsubstituted $(C_1-C_6)$alkyl.
Regarding any of the Formulae presented above, in one embodiment, Z is absent.
In another embodiment, Z is methylene.
In another embodiment, Z is ethylene.
In another embodiment, Z is propylene.
In one embodiment, G is $-(C_1-C_6)$alkylene.
In another embodiment, G is methylene.
In another embodiment, G is ethylene.
In another embodiment, G is propylene.
In one embodiment, G is $-(C_2-C_6)$alkenylene
In another embodiment, G is ethenylene.
In another embodiment, G is propenylene.
In another embodiment, G is -C(=O).
In another embodiment, G is $NR^8$.
In another embodiment, $R^8$ is H.
In another embodiment, $R^8$ is $-(C_1-C_6)$alkyl.
In another embodiment, $R^8$ is $-(C_1-C_{10})$alkoxy.
In another embodiment, $R^8$ is $-(C_3-C_{12})$cycloalkyl.
In another embodiment, $R^8$ is $(C_3-C_{12})$cycloalkyl-$(C_1-C_6)$alkyl-.
In one embodiment, $R^3$ is $NH_2$.

In one embodiment, $R^3$ is $-CONR^5R^6$.

In another embodiment, $R^3$ is $-(C_1-C_6)alkyl-CONR^5R^6$.

In another embodiment, $R^3$ is -C(=O).

In another embodiment, $R^3$ is $-C(=O)-(C_1-C_6)alkyl$.

In another embodiment, $R^3$ is $COOR^7$.

In another embodiment $R^7$ is H.

In another embodiment $R^7$ is $-(C_1-C_6)alkyl$.

In another embodiment, $R^3$ is a -(6- to 14-membered)aryl.

In another embodiment, $R^3$ is ((6- to 14-membered)aryl)-$(C_1-C_6)$alkyl-.

In another embodiment, $R^3$ is benzyl.

In another embodiment, $R^3$ is phenyl.

In another embodiment, $R^3$ is -C(=O)-(6- to 14-membered)aryl.

In another embodiment, $R^3$ is a -(3- to 12-membered)heterocycle.

In another embodiment, $R^3$ is ((3- to 12 membered)heterocycle)-$(C_1-C_6)$alkyl-.

In another embodiment, $R^3$ is -(5- to 12-membered)heteroaryl.

In another embodiment, $R^3$ is ((5- to 12-membered)heteroaryl)-$(C_1-C_6)$alkyl-.

In another embodiment, $R^3$ is -C(=O)-(5- to 12-membered)heteroaryl.

In another embodiment, $R^3$ is $-(C_1-C_{10})alkoxy$.

In one embodiment, $R^3$ is substituted with $-COOR^7$.

In another embodiment, $R^3$ is substituted with $-NR^5R^6$.

In another embodiment, $R^3$ is substituted with phenyl.

In another embodiment, $R^3$ is substituted with benzyl.

In another embodiment, $R^3$ is substituted with -NH-C(=O)-(6- to 14-membered)aryl.

In another embodiment, $R^3$ is substituted with -NH-C(=O)-$(C_1-C_6)$alkyl-(6- to 14-membered)aryl.

In another embodiment, $R^3$ is substituted with -C(=O).

In another embodiment, $R^3$ is substituted with -OH.

In another embodiment, $R^3$ is substituted with hydroxy$(C_1-C_6)$alkyl-.

In another embodiment, $R^3$ is substituted with dihydroxy$(C_1-C_6)$alkyl-.

In another embodiment, R3 is substituted with $-NR^5R^6$.

In one embodiment, $R^1$ is $-(C_1-C_{10})alkyl$.

In another embodiment, $R^1$ is $-(C_3-C_{12})cycloalkyl$.

In another embodiment, $R^1$ is $((C_3-C_{12})cycloalkyl)-(C_1-C_6)alkyl-$.

In another embodiment, $R^1$ is -(6- to 14-membered)aryl.

In aother embodiment, $R^1$ is ((6- to 14-membered)aryl)-$(C_1-C_6)$alkyl-.

In one embodiment, $R^4$ is OH.

In another embodiment, $R^4$ is $-(C_1-C_5)alkoxy$.

In another embodiment, $R^4$ is $-(C_1-C_5)alkyl$.

In another embodiment, $R^4$ is COOH.

In one embodiment, Z-G-$R^3$ is $-(CH_2)_3-COOH$.

In another embodiment, Z-G-$R^3$ is $-(CH_2)_3-CONH_2$.

In another embodiment, Z-G-$R^3$ is $-(CH_2)_3$-O-(5- to 12-membered)heteroaryl-$COOR^7$.

In another embodiment, Z-G-$R^3$ is $-(CH_2)_3-CONR^5R^6$.

In another embodiment, Z-G-$R^3$ is $-(CH_2)_3-C(=O)-NH-(C_1-C_6)alkyl-COOR^7$.

In another embodiment, Z-G-$R^3$ is dihydroxy$(C_1-C_6)$alkyl.

In another embodiment, Z-G-$R^3$ is $-(CH_2)_3-C(=O)-NH-(C_1-C_6)alkyl-C(=O)$.

In another embodiment, Z-G-$R^3$ is $-(CH_2)_2$-((6- to 14-membered)aryl)-$COOR^7$.

In another embodiment, Z-G-$R^3$ is $-(CH_2)_2$-NH-C(=O)-((6- to 14-membered)aryl).

In another embodiment, Z-G-$R^3$ is $-(CH_2)_2$-NH-C(=O)-((5- to 12-membered)heteroaryl).

In another embodiment, Z-G-$R^3$ is $-(CH_2)_2$-NH-C(=O)-((6- to 14-membered)aryl)-$COOR^7$.

In another embodiment, Z-G-$R^3$ is $-(CH_2)_2$-NH-C(=O)-((5- to 12-membered)heteroaryl)-$COOR^7$.

In another embodiment, Z-G-$R^3$ is $-(CH_2)_2$-O-((6- to 14-membered)aryl)-$COOR^7$.

In another embodiment, at least one of $R^{2a}$ or $R^{2b}$ is methyl.

In another embodiment, one of $R^{2a}$ or $R^{2b}$ is methyl, and the other is absent.

In another embodiment, $R^4$ is OH, OMe, or F.

Specific compounds of the invention include:

(2*S*)-2-(2-((6*R*,11*R*)-3-(cyclopropylmethyl)-8-hydroxy-11-methyl-1,2,3,4,5,6-hexahydro-2,6-methanobenzo[*d*]azocin-6-yl)acetamido)propanamide **(Compound 1);**

4-((6*S*,11*R*)-3-(cyclopropylmethyl)-8-hydroxy-11-methyl-1,2,3,4,5,6-hexahydro-2,6-methanobenzo[*d*]azocin-6-yl)butanamide **(Compound 2);**
4-((6*S*,11*R*)-3-(cyclopropylmethyl)-8-methoxy-11-methyl-1,2,3,4,5,6-hexahydro-2,6-methanobenzo[*d*]azocin-6-yl)butanoic acid **(Compound 3);**
4-((6*S*,11*R*)-3-(cyclopropylmethyl)-8-hydroxy-11-methyl-1,2,3,4,5,6-hexahydro-2,6-methanobenzo[*d*]azocin-6-yl)butanoic acid **(Compound 4);**
2-((6*R*,11*R*)-3-(cyclopropylmethyl)-8-methoxy-11-methyl-1,2,3,4,5,6-hexahydro-2,6-methanobenzo[*d*]azocin-6-yl)acetic acid **(Compound 5);**
(2*S*)-3-((6*R*,11*R*)-3-(cyclopropylmethyl)-8-methoxy-11-methyl-1,2,3,4,5,6-hexahydro-2,6-methanobenzo[*d*]azocin-6-yl)propane-1,2-diol **(Compound 6);**
(2*S*)-2-(2-((6*R*,11*R*)-3-(cyclopropylmethyl)-8-methoxy-11-methyl-1,2,3,4,5,6-hexahydro-2,6-methanobenzo[*d*]azocin-6-yl)acetamido)propanamide **(Compound 7);**
(*E*)-methyl 4-((6*S*,11*R*)-3-(cyclopropylmethyl)-8-methoxy-11-methyl-1,2,3,4,5,6-hexahydro-2,6-methanobenzo[*d*]azocin-6-yl)but-2-enoate **(Compound 8);**
4-((6*S*,11*R*)-3-(cyclopropylmethyl)-8-methoxy-11-methyl-1,2,3,4,5,6-hexahydro-2,6-methanobenzo[*d*]azocin-6-yl)-N-isobutylbutan-1-amine **(Compound 9);**
(2*R*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol **(Compound 10);**
(2*S*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol **(Compound 11);**
(6*S*,11*R*)-6-(4-(benzyloxy)butyl)-3-(cyclopropylmethyl)-8-methoxy-11-methyl-1,2,3,4,5,6-hexahydro-2,6-methanobenzo[*d*]azocine **(Compound 12);**
4-((6*S*,11*R*)-3-(cyclopropylmethyl)-8-methoxy-11-methyl-1,2,3,4,5,6-hexahydro-2,6-methanobenzo[*d*]azocin-6-yl)butan-1-ol **(Compound 13 369);**
N-((*S*)-1-amino-1-oxopropan-2-yl)-4-((6*S*,11*R*)-3-(cyclopropylmethyl)-8-methoxy-11-methyl-1,2,3,4,5,6-hexahydro-2,6-methanobenzo[*d*]azocin-6-yl)butanamide **(Compound 14);**
(2*R*,6*S*,11*S*)-3-(cyclopropylmethyl)-6-(3-(furan-2-yl)propyl)-8-methoxy-11-methyl-1,2,3,4,5,6-hexahydro-2,6-methanobenzo[*d*]azocine **(Compound 15);** and the pharmaceutically acceptable salts, prodrugs, and solvates thereof.

Specific compounds of the invention also include:

5-(2-((2*R*,6*S*,11*R*)-3-(cyclopropylmethyl)-8-hydroxy-11-methyl-1,2,3,4,5,6-hexahydro-2,6-methanobenzo[*d*]azocin-6-yl)ethoxy)nicotinic acid **(Compound 16);**
4-((2*R*,6*R*,11*R*)-8-hydroxy-3-isopropyl-11-methyl-1,2,3,4,5,6-hexahydro-2,6-methanobenzo[*d*]azocin-6-yl)butanamide **(Compound 17)*;**
4-((2*R*,6*R*,11*R*)-8-hydroxy-3-isobutyl-11-methyl-1,2,3,4,5,6-hexahydro-2,6-methanobenzo[*d*]azocin-6-yl)butanamide **(Compound 18)*;**
4-((2*R*,6*R*,11*R*)-3-benzyl-8-hydroxy-11-methyl-1,2,3,4,5,6-hexahydro-2,6-methanobenzo[*d*]azocin-6-yl)butanamide **(Compound 19);**
*4*-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamide **(Compound 20);**
(*S*)-methyl 2-(4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamido)propanoate **(Compound 21);**
*N*-((*S*)-1-amino-1-oxopropan-2-yl)-4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamide **(Compound 22);**
methyl 4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanoate **(Compound 23);**
3-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoic acid **(Compound 24);**
4-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoic acid **(Compound 25);**
methyl 3-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoate **(Compound 26);**
4-(2-((2*R*,6*S*,11*R*)-3-(cyclopropylmethyl)-8-methoxy-11-methyl-1,2,3,4,5,6-hexahydro-2,6-methanobenzo[*d*]azocin-6-yl)ethoxy)benzoic acid **(Compound 27);**
4-(2-((2*R*,6*S*,11*R*)-3-(cyclopropylmethyl)-8-hydroxy-11-methyl-1,2,3,4,5,6-hexahydro-2,6-methanobenzo[*d*]azocin-6-yl)ethoxy)benzoic acid **(Compound 28);**
2-((2*S*,6*R*,11*S*)-3-(cyclopropylmethyl)-8-methoxy-11-methyl-1,2,3,4,5,6-hexahydro-2,6-methanobenzo[*d*]azocin-6-yl)acetic acid **(Compound 29);**

*N*-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)-2-(dimethylamino)acetamide **(Compound 30);**

2-amino-*N*-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)acetamide **(Compound 31);**

and the pharmaceutically acceptable salts and solvates thereof.
*: not claimed

As used herein, the term "-($C_1$-$C_{10}$)alkyl refers to straight-chain and branched non-cyclic saturated hydrocarbons having 1, 2, 3, 4, 5, 6, 7, 8, 9, or 10 carbon atoms. Representative straight chain -($C_1$-$C_{10}$) alkyl groups include methyl, -ethyl, -n-propyl,-n-butyl, -n-pentyl, -n-hexyl, n-heptyl, n-octyl, n-nonyl and n-decyl. Representative branched-($C_1$-$C_{10}$)alkyl groups include isopropyl, sec-butyl, isobutyl, tert-butyl, isopentyl, neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 5-methylhexyl, 6-methylheptyl, and the like.

As used herein, the term "-($C_1$-$C_6$)alkyl" refers to straight-chain and branched non-cyclic saturated hydrocarbons having from 1 to 6 carbon atoms. Representative straight chain -($C_1$-$C_6$)alkyl groups include methyl, -ethyl, -n-propyl, -n-butyl, -n-pentyl, and -n-hexyl. Representative branched-chain -($C_1$-$C_6$)alkyl groups include isopropyl, sec-butyl, isobutyl, tert-butyl, isopentyl, neopentyl, 1-methylbutyl, 2-methylbutyl, 3-methylbutyl, 1,1-dimethylpropyl, and 1,2-dimethylpropyl, methylpentyl, 2-methylpentyl, 3-methylpentyl, 4-mehtylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, and the like.

As used herein, the term "-($C_2$-$C_{12}$)alkenyl" refers to straight chain and branched non-cyclic hydrocarbons having 2, 3, 4, 5, 6, 7, 8, 9, 10, 11, or 12 carbon atoms and including at least one carbon-carbon double bond. Representative straight chain and branched -($C_2$-$C_{12}$)alkenyl groups include -vinyl, allyl, -1-butenyl, -2-butenyl, - isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, 3-hexenyl, and the like.

As used herein, the term "-($C_2$-$C_{10}$)alkenyl" refers to straight chain and branched non-cyclic hydrocarbons having 2, 3, 4, 5, 6, 7, 8, 9, or 10 carbon atoms and including at least one carbon-carbon double bond. Representative straight chain and branched - ($C_2$-$C_{10}$)alkenyl groups include -vinyl, allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, 3-hexenyl, and the like.

As used herein, the term "-($C_2$-$C_6$)alkenyl" refers to straight chain and branched non-cyclic hydrocarbons having from 2 to 6 carbon atoms and including at least one carbon-carbon double bond. Representative straight chain and branched -($C_2$-$C_6$)alkenyl groups include -vinyl, allyl, -1-butenyl, -2-butenyl, -isobutylenyl, -1-pentenyl, -2-pentenyl, -3-methyl-1-butenyl, -2-methyl-2-butenyl, and the like.

As used herein, the term "-($C_2$-$C_{12}$)alkynyl" refers to straight chain and branched non-cyclic hydrocarbons having 2, 3, 4, 5, 6, 7, 8, 9, 10, 11, or 12 carbon atoms and including at least one carbon-carbon triple bond. Representative straight chain and branched-($C_2$-$C_{12}$)alkynyl groups include -acetylenyl, -propynyl, -1 butynyl, -2-butynyl, -1-pentynyl, -2-pentynyl, -3-methyl-1-butynyl, -4-pentynyl, -1-hexynyl, -2-hexynyl, -5-hexynyl, and the like.

As used herein, the term "-($C_2$-$C_{10}$)alkynyl" refers to straight chain and branched non-cyclic hydrocarbons having 2, 3, 4, 5, 6, 7, 8, 9, or 10 carbon atoms and including at least one carbon-carbon triple bond. Representative straight chain and branched - ($C_2$-$C_{10}$)alkynyl groups include -acetylenyl, -propynyl, -1 butynyl, -2-butynyl, -1-pentynyl, -2-pentynyl, -3-methyl-1-butynyl, -4-pentynyl, -1-hexynyl, -2-hexynyl, -5-hexynyl, and the like.

As used herein, the term "-($C_2$-$C_6$)alkynyl" refers to straight chain and branched non-cyclic hydrocarbons having from 2 to 6 carbon atoms and including at least one carbon-carbon triple bond. Representative straight chain and branched -($C_2$-$C_6$)alkynyl groups include -acetylenyl, -propynyl, -1 butynyl, -2-butynyl, -1-pentynyl, -2-pentynyl, -3-methyl-1-butynyl, -4-pentynyl, and the like.

As used herein, "-($C_1$-$C_{10}$)alkoxy" means a straight chain or branched non-cyclic hydrocarbon having one or more ether groups and 1, 2, 3, 4, 5, 6, 7, 8, 9, or 10 carbon atoms. Representative straight chain and branched ($C_1$-$C_{10}$)alkoxys include -methoxy,-ethoxy, -propoxy, -butyloxy, -pentyloxy, -hexyloxy, -heptyloxy, -methoxymethyl, -2-methoxyethyl, -5-methoxypentyl, -3-ethoxybutyl and the like.

As used herein, "-($C_1$-$C_6$)alkoxy" means a straight chain or branched non-cyclic hydrocarbon having one or more ether groups and from 1 to 6 carbon atoms. Representative straight chain and branched ($C_1$-$C_5$)alkoxys include -methoxy, -ethoxy,-propoxy, -butyloxy, -pentyloxy, -hexyloxy, -methoxymethyl, -2-methoxyethyl, -5-methoxypentyl, -3-ethoxybutyl and the like.

As used herein, "-($C_1$-$C_5$)alkoxy" means a straight chain or branched non-cyclic hydrocarbon having one or more ether groups and from 1 to 5 carbon atoms. Representative straight chain and branched ($C_1$-$C_5$)alkoxys include -methoxy, -ethoxy,-propoxy, -butyloxy, -pentyloxy, -methoxymethyl, -2-methoxyethyl, -5-methoxypentyl,-3-ethoxybutyl

and the like.

As used herein, the term "-($C_3$-$C_{12}$)cycloalkyl" refers to a cyclic saturated hydrocarbon having 3, 4, 5, 6, 7, 8, 9, 10, 11, or 12 carbon atoms. Representative ($C_3$-$C_{12}$)cycloalkyls include cyclopropyl, cyclobutyl, cyclopentyl, cyclohexyl, cycloheptyl, cyclooctyl, and cyclononyl, cyclodecyl, cycloundecyl, cyclododecyl, and the like.

As used herein, "-($C_6$-$C_{14}$)bicycloalkyl" means a bicyclic hydrocarbon ring system having 6, 7, 8, 9, 10, 11, 12, 13, or 14 carbon atoms and at least one saturated cyclic alkyl ring. Representative -($C_6$-$C_{14}$)bicycloalkyls include -indanyl, -norbornyl,-1,2,3,4-tetrahydronaphthalenyl, -5,6,7,8-tetrahydronaphthalenyl, -perhydronaphthalenyl, and the like.

As used herein, "-($C_8$-$C_{20}$)tricycloalkyl" means a tricyclic hydrocarbon ring system having 8, 9, 10, 11, 12, 13, 14, 15, 16, 17, 18, 19, or 20 carbon atoms and at least one saturated cyclic alkyl ring. Representative -($C_8$-$C_{20}$)tricycloalkyls include-pyrenyl, -adamantyl, -1,2,3,4-tetrahydroanthracenyl, -perhydroanthracenyl-aceanthrenyl, -1,2,3,4-tetrahydropenanthrenyl, -5,6,7,8-tetrahydrophenanthrenyl, -perhydrophenanthrenyl, tetradecahydro-1*H*-cyclohepta[*a*]naphthalenyl, tetradecahydro-1*H*-cycloocta[*e*]indenyl, tetradecahydro-1*H*-cyclohepta[*e*]azulenyl, hexadecahydrocycloocta[*b*]naphthalenyl, hexadecahydrocyclohepta[*a*]heptalenyl, tricyclo-pentadecanyl, tricyclo-octadecanyl, tricyclo-nonadecanyl, tricyclo-icosanyl, and the like.

As used herein, the term "-($C_3$-$C_{12}$)cycloalkenyl" refers to a cyclic hydrocarbon having 3, 4, 5, 6, 7, 8, 9, 10, 11, or 12 carbon atoms, and including at least one carbon-carbon double bond. Representative -($C_3$-$C_{12}$)cycloalkenyls include -cyclopropenyl,-cyclobutenyl, -cyclopentenyl, -cyclopentadienyl, -cyclohexenyl, -cyclohexadienyl,-cycloheptenyl, -cycloheptadienyl, -cycloheptatrienyl, -cyclooctenyl, -cyclooctadienyl,-cyclooctatrienyl, -cyclooctatetraenyl, -cyclononenyl, -cyclononadienyl, -cyclodecenyl,-cyclodecadienyl, -norbornenyl, and the like.

As used herein, the term "-($C_4$-$C_{12}$)cycloalkenyl" refers to a cyclic hydrocarbon having from 4 to 12 carbon atoms, and including at least one carbon-carbon double bond. Representative -($C_4$-$C_{12}$)cycloalkenyls include -cyclobutenyl, -cyclopentenyl,-cyclopentadienyl, -cyclohexenyl, -cyclohexadienyl, -cycloheptenyl, -cycloheptadienyl, -cycloheptatrienyl, -cyclooctenyl, -cyclooctadienyl, -cyclooctatrienyl,-cyclooctatetraenyl, -cyclononenyl, -cyclononadienyl, -cyclodecenyl, -cyclodecadienyl,-norbornenyl, and the like.

As used herein, "-($C_7$-$C_{14}$)bicycloalkenyl" means a bi-cyclic hydrocarbon ring system having at least one carbon-carbon double bond in at least one of the rings and from 7 to 14 carbon atoms. Representative -($C_7$-$C_{14}$)bicycloalkenyls include-bicyclo[3.2.0]hept-2-enyl, -indenyl, -pentalenyl, -naphthalenyl, -azulenyl, -heptalenyl,-1,2,7,8-tetrahydronaphthalenyl, and the like.

As used herein, "-($C_8$-$C_{20}$)tricycloalkenyl" means a tri-cyclic hydrocarbon ring system having at least one carbon-carbon double bond in one of the rings and from 8 to 20 carbon atoms. Representative -($C_8$-$C_{20}$)tricycloalkenyls include -anthracenyl,-phenanthrenyl, -phenalenyl, -acenaphthalenyl, as-indacenyl, s-indacenyl, 2,3,6,7,8,9,10,11-octahydro-1*H*-cycloocta[*e*]indenyl, 2,3,4,7,8,9,10,11-octahydro-1*H*-cyclohepta[a]naphthalenyl, 8,9,10,11-tetrahydro-7*H*-cyclohepta[*a*]naphthalenyl, 2,3,4,5,6,7,8,9,10,11,12,13-dodecahydro-1*H*-cyclohepta[*a*]heptalenyl, 1,2,3,4,5,6,7,8,9,10,11,12,13,14-tetradecahydro-dicyclohepta[*a*,*c*]cyclooctenyl, 2,3,4,5,6,7,8,9,10,11,12,13-dodecahydro-1*H*-dibenzo[*a*,*d*]cyclononenyl, and the like.

As used herein, "-(3- to 12-membered)heterocycle" or "-(3- to 12-membered)heterocyclo" means a 3- to 12-membered monocyclic heterocyclic ring which is either saturated, or unsaturated, non-aromatic, or aromatic. A 3-membered heterocycle can contain up to 1 heteroatom; a 4-membered heterocycle can contain up to 2 heteroatoms; a 5-membered heterocycle can contain up to 4 heteroatoms; a 6-membered heterocycle can contain up to 4 heteroatoms; and a 7-membered heterocycle can contain up to 5 heteroatoms. Each heteroatom is independently selected from nitrogen (which can be quaternized), oxygen, and sulfur (including sulfoxide and sulfone). The -(3- to 12-membered)heterocycle can be attached via a nitrogen or carbon atom. Representative -(3- to 12-membered)heterocycles include aziridinyl, thiazolidinyl, morpholinyl, pyrrolidinonyl, pyrrolidinyl, piperidinyl, piperazinyl, 2,3-dihydrofuranyl, dihydropyranyl, hydantoinyl, valerolactamyl, oxiranyl, oxetanyl, tetrahydrofuranyl, tetrahydropyranyl, dihydropyridinyl, tetrahydropyridinyl, tetrahydropyrimidinyl, tetrahydrothiophenyl, tetrahydrothiopyranyl, oxepanyl, thiepinyl, 3,4,5,6-tetrahydro-2*H*-azepinyl, 1,4-thiazepinyl, azocinyl, thiocanyl, and the like.

As used herein, "-(5- to 12-membered)heterocycle" or "-(5- to 12-membered)heterocyclo" means a 5- to 12-membered monocyclic heterocyclic ring which is either saturated, or unsaturated, non-aromatic, or aromatic. A 5-membered heterocycle can contain up to 4 heteroatoms; a 6-membered heterocycle can contain up to 4 heteroatoms; and a 7-membered heterocycle can contain up to 5 heteroatoms. Representative (5- to 12-membered)heterocycles include thiazolidinyl, morpholinyl, pyrrolidinonyl, pyrrolidinyl, piperidinyl, piperazinyl, 2,3-dihydrofuranyl, dihydropyranyl, hydantoinyl, valerolactamyl, tetrahydrofuranyl, tetrahydropyranyl, dihydropyridinyl, tetrahydropyridinyl, tetrahydropyrimidinyl, tetrahydrothiophenyl, tetrahydrothiopyranyl, oxepanyl, thiepinyl, 3,4,5,6-tetrahydro-2*H*-azepinyl, 1,4-thiazepinyl, azocinyl, thiocanyl, and the like.

As used herein, "-(4- to 8-membered)heterocycle" or "-(4- to 8-membered)heterocyclo" means a 4- to 8-membered monocyclic heterocyclic ring which is either saturated or unsaturated, non-aromatic, or aromatic. A 4-membered heterocycle can contain up to 2 heteroatoms; a 5-membered heterocycle can contain up to 4 heteroatoms; a 6-membered heterocycle can contain up to 4 heteroatoms; and a 7-membered heterocycle can contain up to 5 heteroatoms. Each

heteroatom is independently selected from nitrogen (which can be quaternized), oxygen, and sulfur (including sulfoxide and sulfone). The -(4- to 8-membered)heterocycle can be attached via a nitrogen or carbon atom. Representative -(4- to 8-membered)heterocycles include morpholinyl, piperidinyl, piperazinyl, 2,3-dihydrofuranyl, dihydropyranyl, hydantoinyl, valerolactamyl, oxiranyl, oxetanyl, tetrahydrofuranyl, tetrahydropyranyl, dihydropyridinyl, tetrahydropyridinyl, tetrahydropyrimidinyl, tetrahydrothiophenyl, tetrahydrothiopyranyl, and the like.

As used herein, "-(7- to 12-membered)bicycloheterocycle" or "-(7- to 12-membered)bicycloheterocyclo" means a 7- to 12-membered bicyclic, heterocyclic ring which is either saturated, unsaturated, non-aromatic, or aromatic. At least one ring of the bicycloheterocycle contains at least one heteroatom. A -(7- to 12-membered)bicycloheterocycle contains from 1 to 4 heteroatoms independently selected from nitrogen (which can be quaternized), oxygen, and sulfur (including sulfoxide and sulfone). The -(7- to 12-membered)bicycloheterocycle can be attached via a nitrogen or carbon atom. Representative -(7- to 10-membered)bicycloheterocycles include -quinolinyl, -isoquinolinyl, -chromonyl, -coumarinyl, -indolyl, -indolizinyl, -benzo[b]furanyl, -benzo[b]thiophenyl, -benzo[*d*][1,3]dioxolyl, -indazolyl, -purinyl,-4H-quinolizinyl, -isoquinolyl, -quinolyl, -phthalazinyl, -naphthyridinyl, -carbazolyl, -β-carbolinyl, -indolinyl, isoindolinyl, -1,2,3,4-tetrahydroquinolinyl, -1,2,3,4-tetrahydroisoquinolinyl, pyrrolopyrrolyl and the like.

As used herein a "-(6- to 14- membered)aryl" means an aromatic carbocyclic ring containing 6 to 14 carbon atoms, including both mono- and bicyclic ring systems. Representative -(5- to 14-membered)aryl groups include -indenyl, -phenyl, -naphthyl, and the like.

As used herein a "-(7- to 12- membered)bicyclic aryl" means an bicyclic aromatic carbocyclic ring containing 7 to 12 carbon atoms. Representative -(7- to 12-membered) bicyclic aryl groups include -indenyl, -naphthyl, and the like.

As used herein a "-(6- to 14- membered)aryloxy" means an oxygen substituted by an aromatic carbocyclic ring containing 6 to 14 carbon atoms, including both mono- and bicyclic ring systems, e.g. such as defined for the -(6- to 14-membered)aryl group above. Representative -(6- to 14-membered)aryloxy groups include phenoxy and 4-fluorophenoxy, and the like.

As used herein a "hydroxy($C_1$-$C_6$)alkyl" means any of the above-mentioned $C_{1-6}$ alkyl groups substituted by one or more hydroxy groups. Representative hydroxy($C_1$-$C_6$)alkyl groups include hydroxymethyl, hydroxyethyl, hydroxypropyl and hydroxybutyl groups, and especially hydroxymethyl, 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

As used herein a "dihydroxy($C_1$-$C_6$)alkyl" means any of the above-mentioned $C_{1-6}$ alkyl groups substituted by two hydroxy groups. Representative dihydroxy($C_1$-$C_6$)alkyl groups include dihydroxyethyl, dihydroxypropyl and dihydroxybutyl groups, and especially 1,2-dihydroxyethyl, 1,3-dihydroxypropyl, 2,3-dihydroxypropyl, 1,3-dihydroxybutyl, 1,4-dihydroxybutyl, and 1,3-dihydroxyprop-2-yl.

As used herein a "-(5- to 12- membered)carbocyclic ring" means a mono- or bicyclic hydrocarbon ring system having from 5 to 12 carbon atoms, which is either saturated, unsaturated, non-aromatic or aromatic. Representative -(5- to 12-membered)carbocyclic rings include cyclopentyl, cyclohexyl, cycloheptyl, cyclooctyl, cyclononyl, cyclodecyl, cycloundecyl, cyclododecyl, -indanyl, -norbornyl, -1,2,3,4-tetrahydronaphthalenyl, -5,6,7,8-tetrahydronaphthalenyl, -perhydronaphthalenyl, adamantyl, cyclopentenyl, -cyclopentadienyl, -cyclohexenyl, -cyclohexadienyl, - cycloheptenyl, -cycloheptadienyl, -cycloheptatrienyl, -cyclooctenyl, -cyclooctadienyl, - cyclooctatrienyl, -cyclooctatetraenyl, -cyclononenyl, -cyclononadienyl, -cyclodecenyl, - cyclodecadienyl, -norbornenyl, heptalenyl, and the like.

As used herein a "-(7- to 12- membered)bicyclic ring system" means a 7- to 12-membered carbocyclic or heterocyclic ring, which may be either unsaturated, saturated, non-aromatic or aromatic. Representative -(7- to 12-membered)bicyclic ring systems include azulenyl, -norbornyl, -1,2,3,4-tetrahydronaphthalenyl, -5,6,7,8-tetrahydronaphthalenyl, -perhydronaphthalenyl, bicyclo[3.2.0]hept-2-enyl, - indenyl, naphthyl, -pentalenyl, -naphthalenyl, -azulenyl, -heptalenyl, -1,2,7,8-tetrahydronaphthalenyl, -quinolinyl, -isoquinolinyl, -chromonyl, -coumarinyl, -indolyl,-indolizinyl, -benzo[b]furanyl, -benzo[b]thiophenyl, -benzo[*d*][1,3]dioxolyl, -indazolyl,-purinyl, -4H-quinolizinyl, -isoquinolyl, -quinolyl, -phthalazinyl, -naphthyridinyl, -carbazolyl, -β-carbolinyl, -indolinyl, isoindolinyl, -1,2,3,4-tetrahydroquinolinyl,-1,2,3,4-tetrahydroisoquinolinyl, pyrrolopyrrolyl, and the like.

As used herein, "-(5- to 12-membered)heteroaryl" means an aromatic heterocycle ring of 5 to 12 members, including both mono- and bicyclic ring systems, where at least one carbon atom (of one or both of the rings) is replaced with a heteroatom independently selected from nitrogen, oxygen, and sulfur, or at least two carbon atoms of one or both of the rings are replaced with a heteroatom independently selected from nitrogen, oxygen, and sulfur. In one embodiment, one of the bicyclic -(5-to 12-membered)heteroaryl rings contains at least one carbon atom. In another embodiment, both of the bicyclic -(5- to 12-membered)heteroaryl rings contain at least one carbon atom. Representative -(5- to 12-membered)heteroaryls include pyridyl, furyl, benzofuranyl, thiophenyl, benzothiophenyl, quinolinyl, isoquinolinyl, pyrrolyl, indolyl, oxazolyl, benzoxazolyl, imidazolyl, benzimidazolyl, thiazolyl, benzothiazolyl, isoxazolyl, oxadiazolinyl, pyrazolyl, isothiazolyl, pyridazinyl, pyrimidyl, pyrimidinyl, pyrazinyl, thiadiazolyl, triazinyl, thienyl, thiadiazolyl, cinnolinyl, phthalazinyl, quinazolinyl, and the like.

As used herein, the terms "halo" and "halogen" refer to fluoro, chloro, bromo or iodo.

As used herein, "-$CH_2$(halo)" means a methyl group where one of the hydrogens of the methyl group has been

replaced with a halogen. Representative $-CH_2$(halo) groups include $-CH_2F$, $-CH_2Cl$, $-CH_2Br$, and $-CH_2I$.

As used herein, "-CH(halo)$_2$" means a methyl group where two of the hydrogens of the methyl group have been replaced with a halogen. Representative -CH(halo)$_2$ groups include $-CHF_2$, $-CHCl_2$, $-CHBr_2$, -CHBrCl, -CHClI, and $-CHI_2$.

As used herein, "-C(halo)$_3$" means a methyl group where each of the hydrogens of the methyl group has been replaced with a halogen. Representative -C(halo)$_3$ groups include $-CF_3$, $-CCl_3$, $-CBr_3$, and $-CI_3$.

As used herein, the term "sulfonyl" means $-SO_2-$.

As used herein, the term "-($C_1$-$C_6$)alkylene" refers to bridging straight-chain and branched non-cyclic saturated hydrocarbons having 1, 2, 3, 4, 5, or 6 carbon atoms. Representative "-($C_1$-$C_6$)alkylene" groups include methylene ($-CH_2$)-), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$) and hexylene ($-CH_2CH_2CH_2CH_2CH_2CH_2-$), and the like.

The term "-($C_2$-$C_6$)alkenylene" refers to bridging straight-chain and branched non-cyclic hydrocarbons having 2, 3, 4, 5, or 6 carbon atoms including at least one carbon-carbon double bond. Representative "-($C_2$-$C_6$)alkenylene" groups include ethenylene, propenylene, butenylene, pentenylene and hexenylene and the like

As used herein, the term "optionally substituted" refers to a group that is either unsubstituted or substituted.

Optional substituents on optionally substituted groups, when not otherwise indicated, include 1, 2, or 3 groups each independently selected from the group consisting of -($C_1$-$C_6$)alkyl, OH, halo, -C(halo)$_3$, -CH(halo)$_2$, $-CH_2$(halo), $NH_2$, -NH($C_1$-$C_6$)alkyl, CN, SH, -(5- to 12-membered)carbocyclic ring, -(5- to 12-membered)heterocycle, phenyl, benzyl, (=O), halo($C_1$-$C_6$)alkyl-, -($C_2$-$C_6$)alkenyl, -($C_2$-$C_6$)alkynyl, hydroxy($C_1$-$C_6$)alkyl-, $OR^{10}$ (such as -OC(halo)$_3$ and -O($C_1$-$C_6$)alkyl), $-CONR^{11}R^{12}$, and $-COOR^{13}$, where $R^{10}$ is selected from the group consisting of -($C_1$-$C_6$)alkyl, -($C_2$-$C_6$)alkenyl, -($C_2$-$C_6$)alkynyl, -C(halo)$_3$, hydroxy($C_1$-$C_6$)alkyl-, -($C_3$-$C_{12}$)cycloalkyl, -($C_6$-$C_{14}$)bicycloalkyl, -($C_8$-$C_{20}$)tricycloalkyl, -($C_4$-$C_{12}$)cycloalkenyl,-($C_7$-$C_{14}$)bicycloalkenyl, -($C_8$-$C_{20}$)tricycloalkenyl, -(6- to 14-membered)aryl, -(5- to 12-membered)heteroaryl, -(3- to 12-membered)heterocycle, and -(7- to 12-membered)bicycloheterocycle; $R^{11}$ and $R^{12}$ are each independently -($C_1$-$C_6$)alkyl, -($C_3$-$C_8$)cycloalkyl, (($C_3$-$C_8$)cycloalkyl)-($C_1$-$C_6$)alkyl-, or together with the nitrogen atom to which they are attached form a (4- to 8- membered)heterocycle; and $R^{13}$ is selected from the group consisting of hydrogen, -($C_1$-$C_6$)alkyl, -($C_2$-$C_6$)alkenyl, -($C_2$-$C_6$)alkynyl, -($C_3$-$C_{12}$)cycloalkyl, -($C_4$-$C_{12}$)cycloalkenyl, (($C_3$-$C_{12}$)cycloalkyl)-($C_1$-$C_6$)alkyl-, (($C_4$-$C_{12}$)cycloalkenyl)-($C_1$-$C_6$)alkyl-, -($C_1$-$C_6$)alkoxy-$COOR^7$, -NH-C(=O)-NH-($C_1$-$C_6$)alkyl, -NH-C(=O)-(6- to 14- membered)aryl, -NH-C(=O)-($C_1$-$C_6$)alkyl-(6- to 14-membered)aryl, -NH-($C_1$-$C_6$)alkyl-CO-$OR^7$, -NH-C(=O)-($C_1$-$C_6$)alkyl-CO-$OR^7$, -NH-C(=O)-CH($NH_2$)-($C_1$-$C_6$)alkyl-CO-$OR^7$, -($C_3$-$C_{12}$)cycloalkyl, -(6- to 14-membered)aryl, -(6- to 14-membered)aryloxy, -($C_1$-$C_6$)alkoxyC(O)$NR^5R^6$, -NH-($C_1$-$C_6$)alkylC(O)-$NR^5R^6$, -C(O)NH-($C_1$-$C_6$)alkyl-$COOR^7$, -($C_1$-$C_6$)alkyl-C(=O)-($C_1$-$C_6$)alkoxy, -($C_1$-$C_6$)alkoxy-C(=O)-($C_1$-$C_6$)alkyl, -($C_1$-$C_6$)alkyl-CN, -($C_1$-$C_6$)alkyl-$COOR^7$, -($C_1$-$C_6$)alkoxy-$COOR^7$, -($C_3$-$C_{12}$)cycloalkyl, (($C_3$-$C_{12}$)cycloalkyl)-($C_1$-$C_6$)alkyl-, (($C_3$-$C_{12}$)cycloalkyl)-($C_1$-$C_6$)alkoxy-, (($C_3$-$C_{12}$)cycloalkyl)-($C_1$-$C_6$)alkoxy-($C_1$-$C_6$)alkyl-,-($C_4$-$C_{12}$)cycloalkenyl, (($C_4$-$C_{12}$)cycloalkenyl)-($C_1$-$C_6$)alkyl-, (($C_4$-$C_{12}$)cycloalkenyl)-($C_1$-$C_6$)alkoxy-, (($C_4$-$C_{12}$)cycloalkenyl)-($C_1$-$C_6$)alkoxy-($C_1$-$C_6$)alkyl-, -(6- to 14-membered)aryl, ((6- to 14-membered)aryl)-($C_1$-$C_6$)alkyl-, ((6- to 14-membered)aryl)-($C_1$-$C_6$)alkoxy-, ((6- to 14-membered)aryl)-($C_1$-$C_6$)alkoxy-($C_1$-$C_6$)alkyl-, -(5- to 12-membered)heteroaryl, ((5- to 12-membered)heteroaryl)-($C_1$-$C_6$)alkyl-, ((5- to 12-membered)heteroaryl)-($C_1$-$C_6$)alkoxy-, ((5- to 12-membered)heteroaryl)-($C_1$-$C_6$)alkoxy-($C_1$-$C_6$)alkyl-, -(3- to 12-membered)heterocycle, ((3- to 12 membered)heterocycle)-($C_1$-$C_6$)alkyl-, ((3- to 12 membered)heterocycle)-($C_1$-$C_6$)alkoxy-, and ((3- to 12 membered)heterocycle)-($C_1$-$C_6$)alkoxy-($C_1$-$C_6$)alkyl-.

As used herein, the term "Z is unsubstituted" means that Z is "-($CH_2$)$_m$-" and m is selected from 1, 2, 3, 4, 5, or 6.

As used herein, the term "Z is substituted" means that Z is "-($CH_2$)$_m$-" and m is selected from 1, 2, 3, 4, 5, or 6 and at least one of the hydrogen atoms has been replaced by a -($C_1$-$C_6$)alkyl group.

As used herein, the terms "Z is absent" or "Z is a bond" means that the bridgehead carbon atom to which the Z-group is attached is directly attached to the G-group.

As used herein, compounds that bind to receptors and mimic the regulatory effects of endogenous ligands are defined as "agonists". Compounds that bind to receptors and are only partly effective as agonists are defined as "partial agonists". Compounds that bind to a receptor but produce no regulatory effect, but rather block the binding of ligands to the receptor are defined as "antagonists". (Ross and Kenakin, "Ch. 2: Pharmacodynamics: Mechanisms of Drug Action and the Relationship Between Drug Concentration and Effect", pp. 31-32, in Goodman & Gilman's the Pharmacological Basis of Therapeutics, 10th Ed. (J.G. Hardman, L.E. Limbird and A.Goodman-Gilman eds., 2001).

Compounds disclosed herein can be in the form of prodrugs of the compounds of Formula I, Formula IA, Formula IB, Formula IC, or Formula ID. Prodrugs are covalently bonded carrier molecules that release an active compound of Formula I, Formula IA, Formula IB, Formula IC, or Formula ID *in vivo.* Non-limiting examples of prodrugs will typically include esters of the Compounds disclosed herein that can be metabolized to the active compound by the action of enzymes in the body. Such prodrugs may be prepared by reacting a compound of Formula I, Formula IA, Formula IB, Formula IC, or Formula ID, with an anhydride such as succinic anhydride.

Compounds of the Invention can be isotopically-labeled (*i.e.,* radio-labeled). Examples of isotopes that can be incorporated into the disclosed compounds include isotopes of hydrogen, carbon, nitrogen, oxygen, phosphorous, fluorine and chlorine, such as $^2H$, $^3H$, $^{11}C$, $^{13}C$, $^{14}C$, $^{15}N$, $^{18}O$, $^{17}O$, $^{31}P$, $^{32}P$, $^{35}S$, $^{18}F$ and $^{36}Cl$, respectively, and preferably $^3H$,

$^{11}C$, and $^{14}C$. Isotopically-labeled Compounds of the Invention can be prepared by methods known in the art in view of this disclosure. For example, tritiated Compounds of the Invention can be prepared by introducing tritium into the particular compound by catalytic dehalogenation with tritium. This method may include reacting a suitable halogen-substituted precursor of a Compound of the Invention with tritium gas in the presence of an appropriate catalyst such as Pd/C in the presence of a base. Other suitable methods for preparing tritiated compounds are generally described in Filer, Isotopes in the Physical and Biomedical Sciences, Vol. 1, Labeled Compounds (Part A), Chapter 6 (1987). $^{14}C$-labeled compounds can be prepared by employing starting materials having a $^{14}C$ carbon.

Isotopically labeled Compounds of the Invention, as well as the pharmaceutically acceptable salts, and solvates thereof, can be used as radioligands to test for the binding of compounds to an opioid or ORL-1 receptor. For example, a radio-labeled Compound of the Invention can be used to characterize specific binding of a test or candidate compound to the receptor. Binding assays utilizing such radio-labeled compounds can provide an alternative to animal testing for the evaluation of chemical structure-activity relationships. In a non-limiting embodiment, the present invention provides a method for screening a candidate compound for the ability to bind to an opioid or ORL-1 receptor, comprising the steps of: a) introducing a fixed concentration of the radio-labeled compound to the receptor under conditions that permit binding of the radio-labeled compound to the receptor to form a complex; b) titrating the complex with a candidate compound; and c) determining the binding of the candidate compound to said receptor.

Compounds of the Invention disclosed herein may contain one or more asymmetric centers, thus giving rise to enantiomers, diastereomers, and other stereoisomeric forms. The present invention encompasses all such possible forms, as well as their racemic and resolved forms and mixtures thereof, and the uses thereof. The individual enantiomers may be separated according to methods known to those of ordinary skill in the art in view of the present disclosure. When the compounds described herein contain olefinic double bonds or other centers of geometric asymmetry, and unless specified otherwise, they include both E and Z geometric isomers. All tautomers are intended to be encompassed by the present invention as well.

As used herein, the term "stereoisomer" is a general term for all isomers of individual molecules that differ only in the orientation of their atoms in space. It includes enantiomers and isomers of compounds with more than one chiral center that are not mirror images of one another (diastereoisomers).

The term "chiral center" refers to a carbon atom to which four different groups are attached.

The terms "enantiomer" and "enantiomeric" refer to a molecule that cannot be superimposed on its mirror image and hence is optically active such that the enantiomer rotates the plane of polarized light in one direction and its mirror image compound rotates the plane of polarized light in the opposite direction.

The term "racemic" refers to a mixture of equal parts of enantiomers and which mixture is optically inactive. Racemic compounds can be separated into their enantiomers by chiral chromatography.

The term "resolution" refers to the separation or concentration or depletion of one of the two enantiomeric forms of a molecule.

The terms "a" and "an" refer to one or more.

Compounds of the Invention encompass all salts of the disclosed compounds of Formula I, Formula IA, Formula IB, Formula IC, or Formula ID. The present invention preferably includes any and all non-toxic, pharmaceutically acceptable salts of the disclosed compounds. Examples of pharmaceutically acceptable salts include inorganic and organic acid addition salts and basic salts. The pharmaceutically acceptable salts include, but are not limited to, metal salts such as sodium salt, potassium salt, cesium salt, and the like; alkaline earth metals such as calcium salt, magnesium salt and the like; organic amine salts such as triethylamine salt, pyridine salt, picoline salt, ethanolamine salt, triethanolamine salt, dicylohexylamine salt, N,N'-dibenzylethylenediamine salt and the like; inorganic acid salts such as hydrochloride, hydrobromide, phosphate, sulphate and the like; organic acid salts such as citrate, lactate, tartrate, maleate, fumarate, mandelate, acetate, dichloroacetate, trifluoroacetate, oxalate, formate and the like; sulfonates such as methanesulfonate, benzenesulfonate, p-toluenesulfonate and the like; and amino acid salts such as arginate, glutamate and the like.

Acid addition salts can be formed by mixing a solution of the particular compound of the present invention with a solution of a pharmaceutically acceptable non-toxic acid such as hydrochloric acid, fumaric acid, maleic acid, succinic acid, acetic acid, citric acid, tartaric acid, carbonic acid, phosphoric acid, oxalic acid, dichloroacetic acid, and the like. Basic salts can be formed by mixing a solution of the particular compound of the present invention and a pharmaceutically acceptable non-toxic base such as sodium hydroxide, potassium hydroxide, choline hydroxide, sodium carbonate and the like.

Compounds of the Invention also encompass solvates of the disclosed compounds of Formula I, Formula IA, Formula IB, Formula IC, or Formula ID. The term "solvate" as used herein is a combination, physical association and/or solvation of a compound of Formula I, Formula IA, Formula IB, Formula IC, or Formula ID with a solvent molecule such as, e.g. a disolvate, monosolvate or hemisolvate, where the ratio of solvent molecule to compound of Formula I, Formula IA, Formula IB, Formula IC, or Formula ID is 2:1, 1:1 or 1:2, respectively. This physical association involves varying degrees of ionic and covalent bonding, including hydrogen bonding. In certain instances, the solvate can be isolated, such as when one or more solvent molecules are incorporated into the crystal lattice of a crystalline solid. Thus, "solvate"

encompasses both solution-phase and isolatable solvates. A compound of Formula I, Formula IA, Formula IB, Formula IC, or Formula ID or may be present as a solvated form with a pharmaceutically acceptable solvent, such as water, methanol, ethanol, and the like, and it is intended that the invention include both solvated and unsolvated forms of Formula I, Formula IA, Formula IB, Formula IC, or Formula ID compounds. One type of solvate is a hydrate. A "hydrate" relates to a particular subgroup of solvates where the solvent molecule is water. Solvates typically can function as pharmacological equivalents. Preparation of solvates is known in the art. See, for example, M. Caira et al, J. Pharmaceut. Sci., 93(3):601-611 (2004), which describes the preparation of solvates of fluconazole with ethyl acetate and with water. Similar preparation of solvates, hemisolvates, hydrates, and the like are described by E.C. van Tonder et al., AAPS Pharm. Sci. Tech., 5(1):Article 12 (2004), and A.L. Bingham et al., Chem. Commun., 603-604 (2001). A typical, non-limiting, process of preparing a solvate would involve dissolving a compound of Formula I, Formula IA, Formula IB, Formula IC, or Formula ID in a desired solvent (organic, water, or a mixture thereof) at temperatures above about 20°C to about 25°C, then cooling the solution at a rate sufficient to form crystals, and isolating the crystals by known methods, e.g., filtration. Analytical techniques such as infrared spectroscopy can be used to confirm the presence of the solvent in a crystal of the solvate.

The present invention also provides the use of a Compound of the Invention in the manufacture of a medicament for treating or preventing a Condition. In one embodiment, the Condition is pain, such as acute or chronic pain. In one embodiment, a Compound of the Invention has agonist activity at the μ, δ and/or κ receptors. In another embodiment a Compound of the Invention has agonist activity at the μ receptor. In another embodiment, a Compound of the Invention has antagonist activity at the ORL-1 receptor. In another embodiment, certain Compounds of the invention can stimulate one receptor (*e.g.,* a μ, δ and/or κ agonist) and inhibit a different receptor (*e.g.,* an ORL-1 antagonist). In another embodiment, the Compound of the Invention is an agonist at the μ receptor, and an antagonist at the ORL-1 receptor. In another embodiment, the Compound of the Invention is an antagonist at the μ receptor, and an agonist at the κ receptor.

<u>List of abbreviations</u>;

| | |
|---|---|
| ACN | acetonitrile |
| AcOH | acetic acid |
| Boc | *tert*-butoxycarbonyl |
| °C | degrees Celcius |
| Cbz | benzyloxycarbonyl |
| d | day(s) |
| DCM | dichloromethane |
| DEAD | diethyl azodicarboxylate |
| DIPEA | diisopropylethylamine |
| DMF | dimethylformamide |
| DMSO | dimethylsulfoxide |
| EDCI | 1-ethyl-3-(3-dimethylaminopropyl)carbodiimide) |
| EtOAc | ethyl acetate |
| EtOH | ethanol |
| h | hour(s) |
| HATU | 2-(7-aza-1H-benzotriazole-1-yl)-1,1,3,3-tetramethyluronium hexafluorophosphate |
| HPLC | high pressure liquid chromatography |
| LAH | lithium aluminum hydride |
| LDA | lithium diisopropylamide |
| MeOH | methanol |
| min | minute(s) |
| MPLC | medium pressure liquid chromatography |
| $(Ph)_3P$ | triphenylphosphine |
| PTSA | p-toluenesulfonic acid |
| PyBOP | benzotriazol-1-yl-oxytripyrrolidinophosphonium hexafluorophosphate |
| RT | room temperature |
| s | second(s) |
| TEA | triethylamine |
| Tf | trifluoromethanesulfonyl |
| TFA | trifluoroacetic acid |
| THF | tetrahydrofuran |

TMEDA *N,N,N',N'*-tetramethylethylenediamine

***Synthesis of Compounds***

Compounds of Formula 1 can be made using conventional organic synthesis in view of this disclosure, or by the illustrative methods shown in the schemes below.

## Scheme A

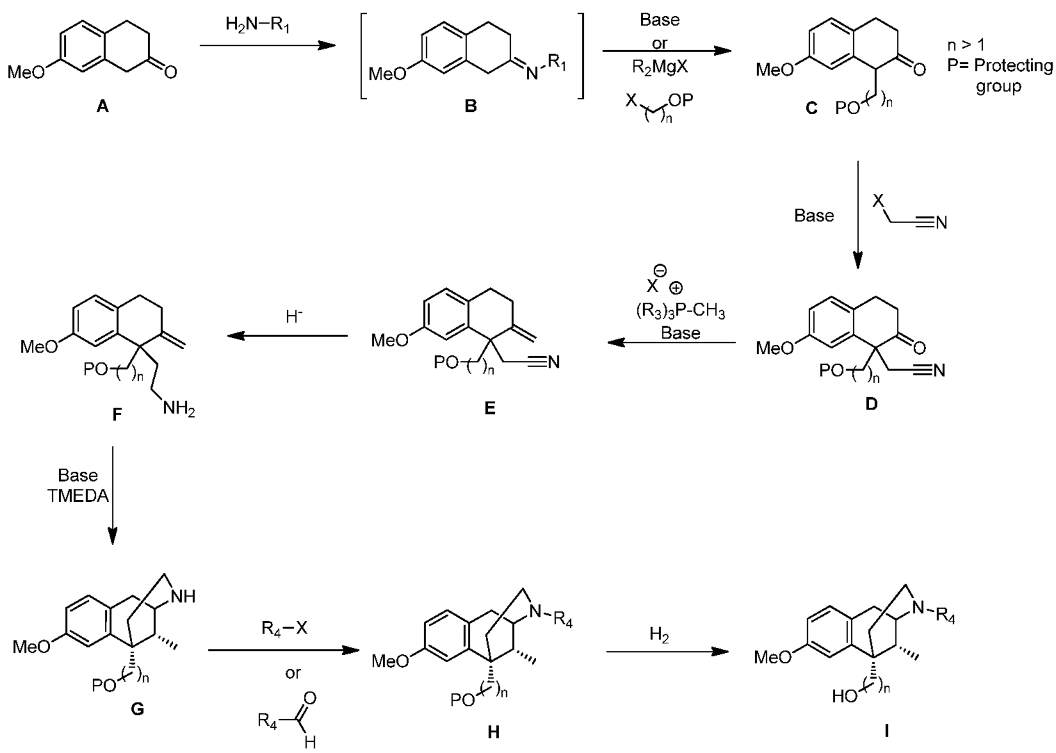


Tetralone **A** can be converted to intermediate **B** with suitable amine such as *N,N*-diethylenediamine in a suitable solvent such as toluene at room temperature to 130 °C (Hatakeyama, et al., J. Am. Chem. Soc., 2005, 127, 14192-14193). Compound **C** is prepared by alkylation of imine **B** with a suitable protected haloalcohol such as a benzyl bromoalkyl ether in a suitable solvent such as tetrahydrofuran (THF) in the presence of an Grignard reagent such as 2-mesityl magnesium bromide at room temperature to 70°C. Compound **C** can be alkylated again with a haloacetonitrile in the presence of inorganic base such as sodium hydride in a suitable solvent such as toluene at room temperature to 130°C to provide nitrile **D.** Olefin **E** can be provided by Wittig reaction on nitrile **D** with an yilde which can be prepared by reacting suitable phosphonium salt such as methyltriphenylphosphonium bromide with suitable organic base such as potassium *tert*-butoxide in suitable solvent such as THF at -78°C to 80°C. Olefin **E** can be reduced to amine **F** with a suitable reducing agent such as lithium aluminum hydride in suitable solvent such as diethylether at 0 °C to room temperature. Benzomorphan **G** is prepared by cyclizing amine **F** with suitable base such as lithium diisopropylamide in the presence of tetramethylethylenediamine in suitable solvent such as THF at -78°C to 80°C (Trost et al., J. Am. Chem. Soc., 2003, 125, 8744-8745). Benzomorphan **G** can be *N*-alkylated either by reaction with an alkyl halide in a suitable solvent such as acetonitrile with a suitable base such as potassium carbonate or by reductive amination with an aldehyde in an suitable solvent such as isopropyl acetate with a suitable reducing agent such as sodium triacetoxyborohydride . Alcohol **I** is prepared by removing the benzyl protecting group with suitable catalyst such as palladium on carbon in the presence of hydrogen gas in suitable solvent such as 20% acetic acid in methanol.

## Scheme B

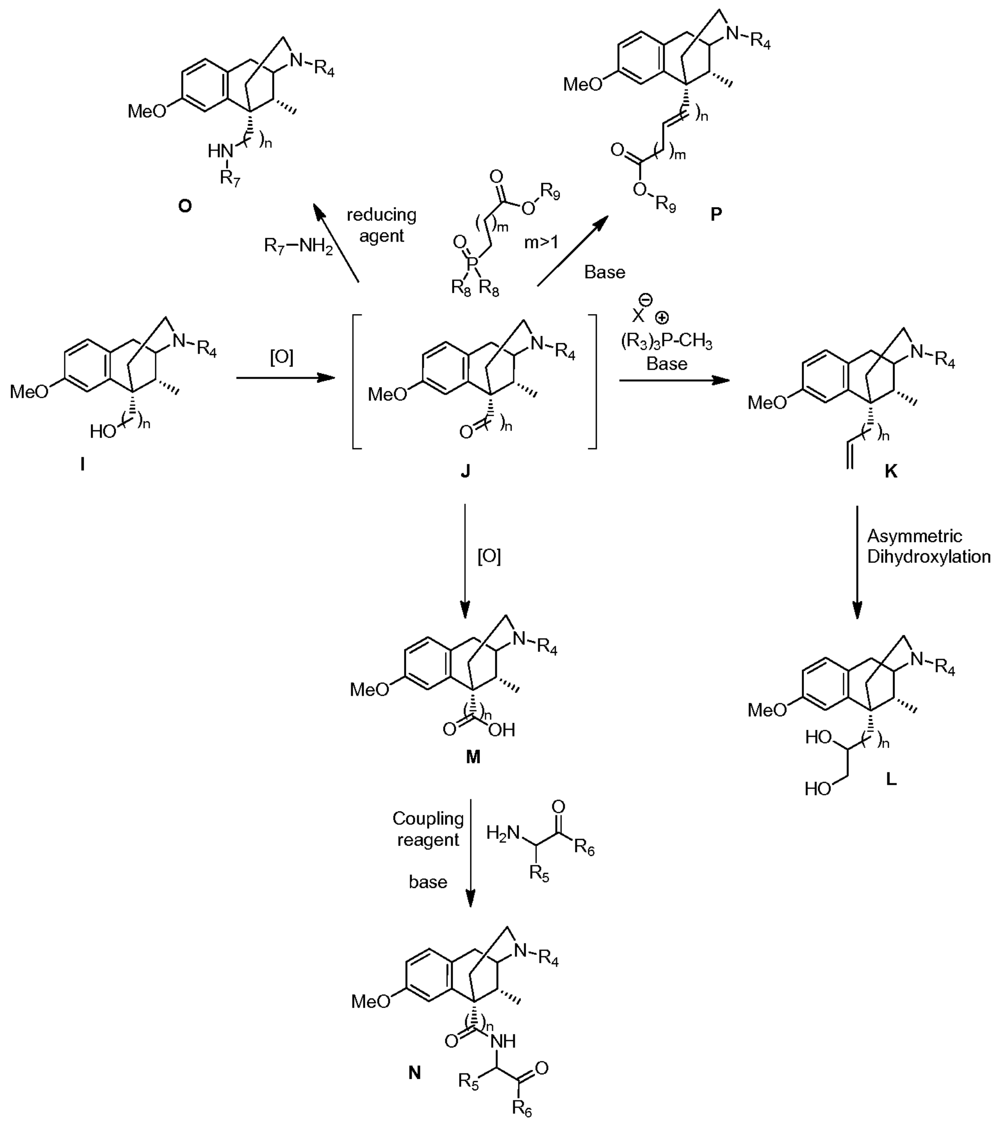


Alcohol **I** can be oxidized to aldehyde **J** with suitable oxidizing agent such as oxalyl chloride, dimethylsulfoxide (DMSO) and triethylamine in a suitable solvent such as dichloromethane (DCM) at -78 °C to room temperature.

Olefin **K** can be prepared by Wittig reaction of aldehyde **J** with an yilde which can be prepared by reacting suitable phosphonium salt such as methyltriphenylphosphonium bromide with suitable organic base such as potassium *tert*-butoxide in suitable solvent such as THF or toluene at -78 °C to 130 °C. Olefin **K** can be converted to diol **L** with suitable asymmetric dihydroxylating reagent such as AD-mix in suitable solvent such as a mixture of isopropyl alcohol and water at room temperature.

Acid **M** can be prepared by oxidizing aldehyde **J** with suitable oxidizing agent such as sodium chlorite and sodium bisulfate in suitable solvent such as a mixture of water and acetonitrile at room temperature. Amide **N** can be prepared by coupling acid **M** with suitable amino acid derivatives such as amidated alanine and suitable coupling reagent such as 2-(1H-7-azabenzotriazol-1-yl)-1,1,3,3-tetramethyl uronium hexafluorophosphate methanaminium (HATU) and suitable organic base such as *N,N*-diisopropylethylamine (DIPEA) in suitable solvent such as *N,N*-dimethylformamide (DMF) at room temperature.

Amine **O** can be prepared from aldehyde **J** by reductive amination with an amine in an suitable solvent such as isopropyl acetate with a suitable reducing agent such as sodium triacetoxyborohydride at room temperature to 100 °C.

C-6 substution chain can be extended to ester olefin **P** by Horner-Wadsworth-Emmons reaction of aldehyde **J** with suitable phosphonate such as methyl diethylphosphonoacetate in the presence of suitable organic base such as potassium *tert*-butoxide in suitable solvent such as THF at -78 °C to 80 °C.

## Scheme C

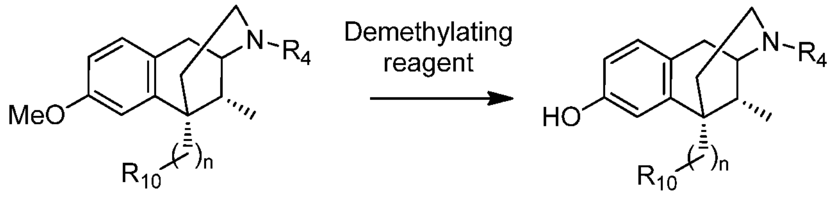


Q R

Phenol **R** can be prepared by demethylating methoxyphenol **Q** with a suitable demethylating reagent such as $BBr_3$ in suitable solvent such as dichloromethane at -78 °C to room temperature.

## Scheme D

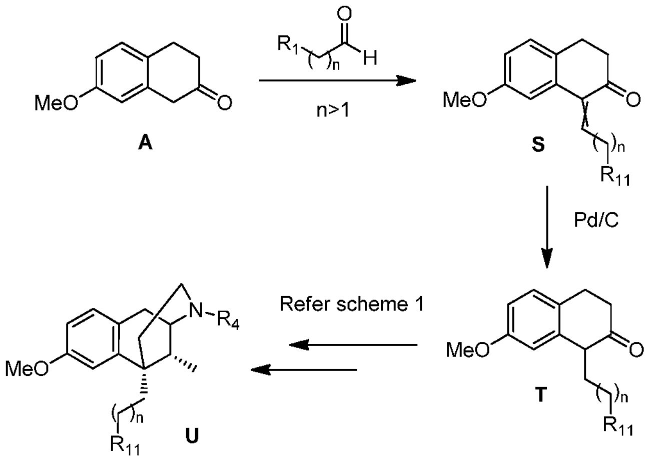


A S T U

Tetralone **A** can be converted to intermediate **S** with a suitable aldehyde such as (*E*)-3-(furan-2-yl)acrylaldehyde in a suitable solvent such as toluene at room temperature to 130 °C. Compound **T** is prepared by hydrogenation of olefin **S** with a suitable catalyst such as palladium on carbon in the presence of hydrogen gas in a suitable solvent such as a mixture of EtOAc and EtOH.

In a similar manner to preparation of compound **I** in Scheme 1, compound **T** was carried on to prepare compound **U.**

## Scheme E

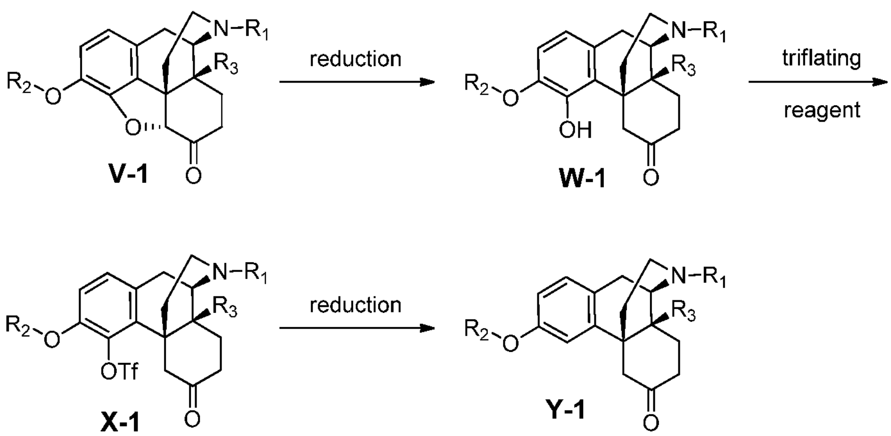


V-1 W-1 X-1 Y-1

The alpha substituted ketone in an opioid such as Compound **V-1** can be reduced to keto phenol **W-1** with a suitable reducing agent such as zinc metal in a suitable solvent such as aqueous acetone. The phenol in Compound **W-1** can be converted to a suitable leaving group such as a triflate by treatment with a suitable triflating reagent such as N-phenyl triflimide in the presence of a suitable base such as cesium carbonate in a suitable solvent such as THF. The triflate in Compound **X-1** can be reduced to Compound **Y-1** by treatment with a suitable reagent such as triethylsilane in the presence of a suitable catalyst such as palladium acetate in a suitable solvent such as DMF.

## Scheme F

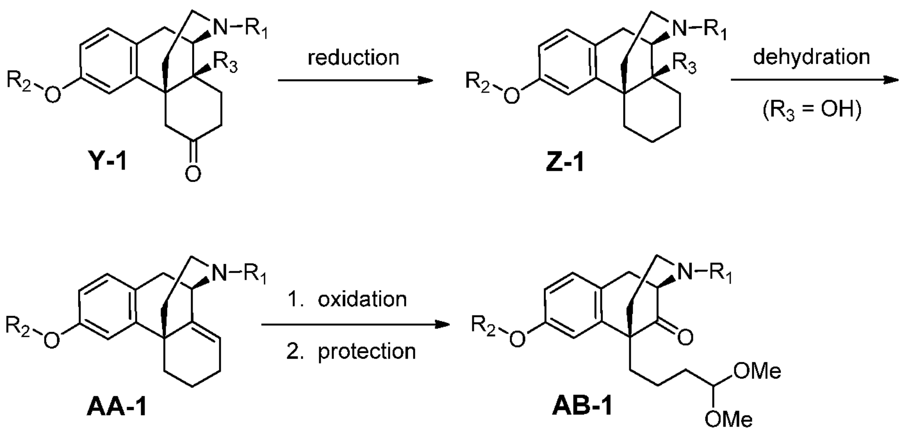


**Y-1** **Z-1** **AA-1** **AB-1**

The ketone group in compound **Y-1** can be reduced to a methylene group by a suitable reagent such as tosylhydrazine in a suitable solvent such as EtOH, followed a reducing agent such as catecholborane in a suitable solvent such as a mixture of THF and chloroform. Dehydration of the tertiary alcohol in Compound **Z-1** ($R_3$ = OH) to give alkene **AA-1** can be accomplished by treatment with a suitable dehydrating reagent such as thionyl chloride in a suitable base such as pyridine. Oxidative cleavage of the double bond in Compound **AA-1** can be accomplished by a suitable oxidizing reagent such as ozone in a suitable solvent such as DCM to give a keto aldehyde. The aldehyde can be protected by treatment with an alcohol such as MeOH in the presence of a suitable acid catalyst such as PTSA to give Compound **AB-1.**

## Scheme G

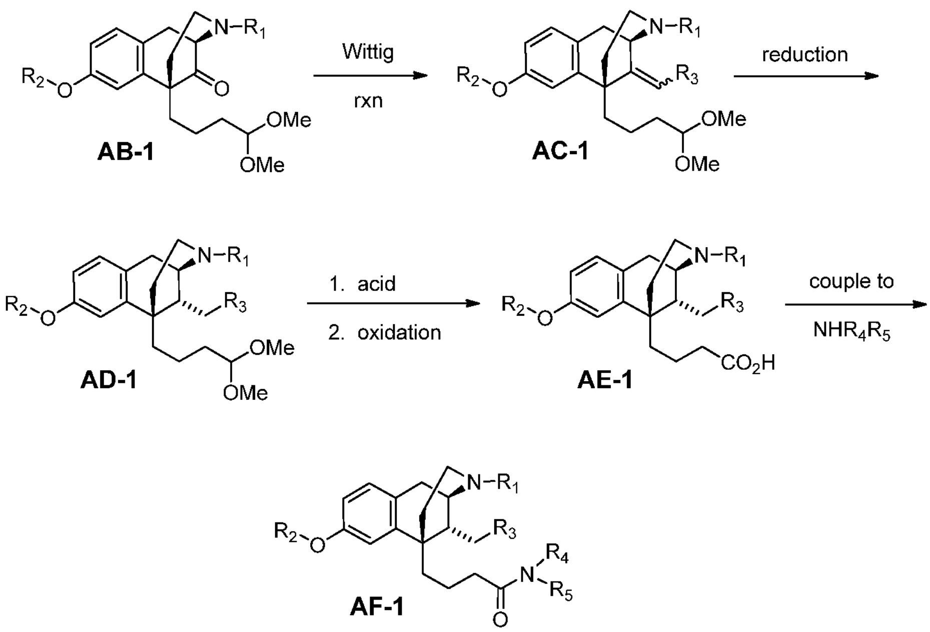


**AB-1** **AC-1** **AD-1** **AE-1** **AF-1**

The ketone in Compound **AB-1** can be reacted with a suitable reagent such as a Wittig reagent in the presence of a suitable base such as potassium tert-butoxide in a suitable solvent such as THF to give olefin **AC-1** which can be reduced to alkane **AD-1** by reduction with a suitable reagent such as hydrogen in the presence of a suitable catalyst such as palladium on carbon in an appropriate solvent such as MeOH. The protected aldehyde group in Compound **AD-1** can be deprotected by treatment with as suitable acid such as TFA in a suitable solvent or solvent mixture such as DCM, acetone and water. The resulting aldehyde can be oxidized to carboxylic acid **AE-1** by treatment with a suitable oxidizing agent such as a mixture of sodium chlorite and sodium dihydrogen phosphate in a suitable solvent such as aqueous ACN. Compound **AE-1** can be converted to amide **AF-1** by activation of the carboxylate group by a suitable reagent such as oxalyl chloride in a suitable solvent such as DCM followed by treatment with the appropriate amine.

## Scheme H

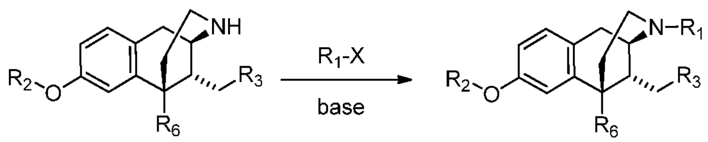


**AG-1** **AH-1**

An opioid with a secondary amine such as Compound **AG-1** can be converted to a tertiary amine such as Compound **AH-1** by treatment with a suitable alkyl halide in the presence of a suitable base such as potassium carbonate in a suitable solvent such as ACN.

***Testing of Compounds***

**μ-opioid Receptor Binding Assay Procedures:** Radioligand dose-displacement binding assays for μ-opioid receptors used 0.3 nM [$^3$H]-diprenorphine (Perkin Elmer, Shelton, CT), with 5 mg membrane protein/well in a final volume of 500 μl binding buffer (10 mM $MgCl_2$, 1 mM EDTA, 5% DMSO, 50 mM HEPES, pH 7.4). Reactions were carried out in the absence or presence of increasing concentrations of unlabeled naloxone. All reactions were conducted in 96-deep well polypropylene plates for 2 hr at room temperature. Binding reactions were terminated by rapid filtration onto 96-well Unifilter GF/C filter plates (Perkin Elmer, Shelton, CT), presoaked in 0.5% polyethylenimine using a 96-well tissue harvester (Perkin Elmer, Shelton, CT.) followed by performing three filtration washes with 500 μl of ice-cold binding buffer. Filter plates were subsequently dried at 50°C for 2-3 hours. BetaScint scintillation cocktail (Perkin Elmer, Shelton, CT) was added (50 μl/well), and plates were counted using a Packard Top-Count for 1 min/well. The data were analyzed using the one-site competition curve fitting functions in GraphPad PRISM™ v. 3.0 or higher (San Diego, Calif.), or an in-house function for one-site competition curve-fitting.

**μ-opioid Receptor Binding Data:** Generally, the lower the Ki value, the more effective the Compounds of the Invention will be at treating or preventing pain or another Condition. Typically, the Compounds of the Invention will have a Ki (nM) of about 1000 or less for binding to μ-opioid receptors. In one embodiment the Compounds of the Invention will have a Ki (nM) of about 300 or less for binding to μ-opioid receptors. In one embodiment, Compounds of the Invention will have a Ki (nM) of about 100 or less. In another embodiment, Compounds of the Invention will have a Ki (nM) of about 10 or less. In still another embodiment, Compounds of the Invention will have a Ki (nM) of about 1 or less. In still another embodiment, Compounds of the Invention will have a Ki (nM) of about 0.1 or less.

**μ-Opioid Receptor Functional Assay Procedures:** [$^{35}$S]GTPγS functional assays were conducted using freshly thawed μ-receptor membranes (Perkin Elmer, Shelton, CT). Assay reactions were prepared by sequentially adding the following reagents to binding buffer (100 mM NaCl, 10 mM $MgCl_2$, 20 mM HEPES, pH 7.4) on ice (final concentrations indicated): membrane protein (0.026 mg/mL), saponin (10 mg/mL), GDP (3mM) and [$^{35}$S]GTPγS (0.20 nM; Perkin Elmer, Shelton, CT). The prepared membrane solution (190 μl/well) was transferred to 96-shallow well polypropylene plates containing 10 μl of 20x concentrated stock solutions of the agonist [D-Ala$^2$, N-methyl-Phe$^4$ Gly-ol$^5$]-enkephalin (DAMGO) prepared in dimethyl sulfoxide (DMSO). Plates were incubated for 30 min at about 25°C with shaking. Reactions were terminated by rapid filtration onto 96-well Unifilter GF/B filter plates (Perkin Elmer, Shelton, CT) using a 96-well tissue harvester (Perkin Elmer, Shelton, CT.) followed by three filtration washes with 200 μl of ice-cold wash buffer (10 mM $NaH_2PO_4$, 10 mM $Na_2HPO_4$, pH 7.4). Filter plates were subsequently dried at 50°C for 2-3 hr. BetaScint scintillation cocktail (Perkin Elmer, Shelton, CT) was added (50 μl/well) and plates were counted using a Packard Top-Count for 1 min/well. Data were analyzed using the sigmoidal dose-response curve fitting functions in GraphPad PRISM v. 3.0, or an in-house function for non-linear, sigmoidal dose-response curve-fitting.

**μ-Opioid Receptor Functional Data:** μ GTP $EC_{50}$ is the concentration of a compound providing 50% of the

maximal response for the compound at a μ-opioid receptor. Compounds of the Invention will typically have a μ GTP $EC_{50}$ (nM) of about 5000 or less. In certain embodiments, Compounds of the Invention will have a μ GTP $EC_{50}$ (nM) of about 2000 or less; or about 1000 or less; or about 100 or less; or about 10 or less; or about 1 or less; or about 0.1 or less.

μ GTP $E_{max}$ (%) is the maximal effect elicited by a compound relative to the effect elicited by DAMGO, a standard μ agonist. Generally, the μ GTP $E_{max}$ (%) value measures the efficacy of a compound to treat or prevent pain or other Conditions. Typically, Compounds of the Invention will have a μ GTP $E_{max}$ (%) of greater than about 10%; or greater than about 20%. In certain embodiments, Compounds of the Invention will have a μ GTP $E_{max}$ (%) of greater than about 50%; or greater than about 65%; or greater than about 75%; or greater than about 85%; or greater than about 100%.

**κ-opioid Receptor Binding Assay Procedures:** Membranes from recombinant HEK-293 cells expressing the human κ opioid receptor (κ) (cloned in house) were prepared by lysing cells in ice cold hypotonic buffer (2.5 mM $MgCl_2$, 50 mM HEPES, pH 7.4) (10 mL/10 cm dish) followed by homogenization with a tissue grinder/Teflon pestle. Membranes were collected by centrifugation at 30,000 x g for 15 min at 4°C and pellets were resuspended in hypotonic buffer to a final concentration of 1-3mg/mL. Protein concentrations were determined using the BioRad protein assay reagent with bovine serum albumen as standard. Aliquots of κ receptor membranes were stored at -80 °C.

Radioligand dose displacement assays used 0.4 nM [$^3$H]-U69,593 (GE Healthcare, Piscataway, NJ; 40 Ci/mmole) with 15 μg membrane protein (recombinant κ opioid receptor expressed in HEK 293 cells; in-house prep) in a final volume of 200 μl binding buffer (5% DMSO, 50 mM Trizma base, pH 7.4). Non-specific binding was determined in the presence of 10 μM unlabeled naloxone or U69,593. All reactions were performed in 96-well polypropylene plates for 1 hr at a temperature of about 25°C. Binding reactions were terminated by rapid filtration onto 96-well Unifilter GF/C filter plates (Perkin Elmer, Shelton, CT) presoaked in 0.5% polyethylenimine (Sigma). Harvesting was performed using a 96-well tissue harvester (Perkin Elmer, Shelton, CT) followed by five filtration washes with 200 μl ice-cold binding buffer. Filter plates were subsequently dried at 50°C for 1-2 hours. Fifty μl/well scintillation cocktail (Perkin Elmer, Shelton, CT) was added and plates were counted in a Packard Top-Count for 1 min/well.

**κ-opioid Receptor Binding Data:** In certain embodiments, the Compounds of the Invention will have a Ki (nM) for κ receptors of about 10,000 or more (which, for purposes of this invention, is interpreted as having no binding to the κ receptors). Certain Compounds of the Invention will have a Ki (nM) of about 20,000 or less for κ receptors. In certain embodiments, Compounds of the Invention will have a Ki (nM) of about 10,000 or less; or about 5000 or less; or about 1000 or less; or about 500 or less; or about 450 or less; or about 350 or less; or about 200 or less; or about 100 or less; or about 50 or less; or about 10 or less; or about 1 or less; or about 0.1 or less.

**κ-Opioid Receptor Functional Assay Procedures:** Functional [$^{35}$S]GTPγS binding assays were conducted as follows. κ opioid receptor membrane solution was prepared by sequentially adding final concentrations of 0.026 μg/μl κ membrane protein (in-house), 10 μg/mL saponin, 3 μM GDP and 0.20 nM [$^{35}$S]GTPγS to binding buffer (100 mM NaCl, 10 mM $MgCl_2$, 20 mM HEPES, pH 7.4) on ice. The prepared membrane solution (190 μl/well) was transferred to 96-shallow well polypropylene plates containing 10 μl of 20x concentrated stock solutions of agonist prepared in DMSO. Plates were incubated for 30 min at a temperature of about 25°C with shaking. Reactions were terminated by rapid filtration onto 96-well Unifilter GF/B filter plates (Perkin Elmer, Shelton, CT) using a 96-well tissue harvester (Packard) and followed by three filtration washes with 200 μl ice-cold binding buffer (10 mM $NaH_2PO_4$, 10 mM $Na_2HPO_4$, pH 7.4). Filter plates were subsequently dried at 50°C for 2-3 hours. Fifty μl/well scintillation cocktail (Perkin Elmer, Shelton, CT) was added and plates were counted in a Packard Top-Count for 1 min/well.

**κ-Opioid Receptor Functional Data:** κ GTP $EC_{50}$ is the concentration of a compound providing 50% of the maximal response for the compound at a κ receptor. Certain Compounds of the Invention will have a κ GTP $EC_{50}$ (nM) of about 20,000 or less to stimulate κ opioid receptor function. In certain embodiments, Compounds of the Invention will have a κ GTP $EC_{50}$ (nM) of about 10, 000 or less; or about 5000 or less; or about 2000 or less; or about 1500 or less; or about 1000 or less; or about 600 or less; or about 100 or less; or about 50 or less; or about 25 or less; or about 10 or less; or about 1 or less; or about 0.1 or less.

κ GTP $E_{max}$ (%) is the maximal effect elicited by a compound relative to the effect elicited by U69,593. Certain Compounds of the Invention will have a κ GTP $E_{max}$ (%) of greater than about 1%; or greater than about 5%; or greater than about 10%; or greater than about 20%. In certain embodiments, Compounds of the Invention will have a κ GTP $E_{max}$ (%) of greater than about 50%; or greater than about 75%; or greater than about 90%; or greater than about 100%.

**δ-opioid Receptor Binding Assay Procedures:** δ-opioid Receptor Binding Assay Procedures were conducted as follows. Radioligand dose-displacement assays used 0.3 nM [$^3$H]-Naltrindole (Perkin Elmer, Shelton, CT; 33.0 Ci/mmole) with 5 μg membrane protein (Perkin Elmer, Shelton, CT) in a final volume of 500 μl binding buffer (5 mM $MgCl_2$, 5% DMSO, 50 mM Trizma base, pH 7.4). Non-specific binding was determined in the presence of 25 μM unlabeled naloxone. All reactions were performed in 96-deep well polypropylene plates for 1 hr at a temperature of about 25°C. Binding reactions were terminated by rapid filtration onto 96-well Unifilter GF/C filter plates (Perkin Elmer, Shelton, CT) presoaked in 0.5% polyethylenimine (Sigma). Harvesting was performed using a 96-well tissue harvester (Perkin Elmer, Shelton, CT) followed by five filtration washes with 500 μl ice-cold binding buffer. Filter plates were subsequently dried

at 50°C for 1-2 hours. Fifty μl/well scintillation cocktail (Perkin Elmer, Shelton, CT) was added and plates were counted in a Packard Top-Count for 1 min/well.

**δ-opioid Receptor Binding Data:** In certain embodiments, the Compounds of the Invention will have a Ki (nM) for δ receptors of about 10,000 or more (which, for the purposes of this invention, is interpreted as having no binding to the δ receptors). Certain Compounds of the Invention will have a Ki (nM) of about 20,000 or less for δ receptors. In one embodiment, the Compounds of the Invention will have a Ki (nM) of about 10,000 or less; or of about 9000 or less. In another embodiment, the Compounds of the Invention will have a Ki (nM) of about 7500 or less; or of about 6500 or less; or of about 5000 or less; or of about 3000 or less; or of about 2500 or less. In another embodiment, the Compounds of the Invention will have a Ki (nM) of about 1000 or less; or of about 500 or less; or of about 350 or less; or of about 250 or less; or of about 100 or less; or of about 10 or less.

**δ-Opioid Receptor Functional Assay Procedures:** Functional [$^{35}$S]GTPγS binding assays were conducted as follows. δ opioid receptor membrane solution was prepared by sequentially adding final concentrations of 0.026 μg/μl δ membrane protein (Perkin Elmer, Shelton, CT), 10 μg/mL saponin, 3 μM GDP and 0.20 nM [$^{35}$S]GTPγS to binding buffer (100mM NaCl, 10mM $MgCl_2$, 20mM HEPES, pH 7.4) on ice. The prepared membrane solution (190 μl/well) was transferred to 96-shallow well polypropylene plates containing 10 μl of 20x concentrated stock solutions of agonist prepared in DMSO. Plates were incubated for 30 min at a temperature of about 25°C with shaking. Reactions were terminated by rapid filtration onto 96-well Unifilter GF/B filter plates (Perkin Elmer, Shelton, CT) using a 96-well tissue harvester (Packard) and followed by three filtration washes with 200 μl ice-cold binding buffer (10 mM $NaH_2PO_4$, 10 mM $Na_2HPO_4$, pH 7.4). Filter plates were subsequently dried at 50°C for 1-2 hours. Fifty μl/well scintillation cocktail (Perkin Elmer, Shelton, CT) was added and plates were counted in a Packard Top-count for 1 min/well.

**δ-Opioid Receptor Functional Data:** δ GTP $EC_{50}$ is the concentration of a compound providing 50% of the maximal response for the compound at a δ receptor. Certain Compounds of the Invention will have a δ GTP $EC_{50}$ (nM) of about 20, 000 or less; or about 10,000 or less. In certain embodiments, the Compounds of the Invention will have a δ GTP $EC_{50}$ (nM) of about 3500 or less; or of about 1000 or less; or of about 500 or less; or of about 100 or less; or of about 90 or less; or of about 50 or less; or of about 25 or less; or of about 10 or less.

δ GTP $E_{max}$ (%) is the maximal effect elicited by a compound relative to the effect elicited by met-enkephalin. Certain Compounds of the Invention of the invention will have a δ GTP $E_{max}$ (%) of greater than about 1%; or of greater than about 5%; or of greater than about 10%. In one embodiment, the Compounds of the Invention will have a δ GTP $E_{max}$ (%) of greater than about 30%. In other embodiments, the Compounds of the Invention will have a δ GTP $E_{max}$ (%) of greater than about 50%; or of greater than about 75%; or of greater than about 90%. In another embodiment, the Compounds of the Invention will have a δ GTP $E_{max}$ (%) of about 100% or greater.

**ORL-1 Receptor Binding Assay Procedure:** Membranes from recombinant HEK-293 cells expressing the human opioid receptor-like receptor (ORL-1) (Perkin Elmer, Shelton, CT) were prepared by lysing cells in ice-cold hypotonic buffer (2.5 mM $MgCl_2$, 50 mM HEPES, pH 7.4) (10 ml/10 cm dish) followed by homogenization with a tissue grinder/Teflon pestle. Membranes were collected by centrifugation at 30,000 x g for 15 min at 4°C and pellets resuspended in hypotonic buffer to a final concentration of 1-3 mg/ml. Protein concentrations were determined using the BioRad protein assay reagent with bovine serum albumen as standard. Aliquots of the ORL-1 receptor membranes were stored at -80°C.

Radioligand binding assays (screening and dose-displacement) used 0.1 nM [$^{3}$H]-nociceptin (Perkin Elmer, Shelton, CT; 87.7 Ci/mmole) with 12 μg membrane protein in a final volume of 500 μl binding buffer (10 mM $MgCl_2$, 1 mM EDTA, 5% DMSO, 50 mM HEPES, pH 7.4). Non-specific binding was determined in the presence of 10 nM unlabeled nociceptin (American Peptide Company). All reactions were performed in 96-deep well polypropylene plates for 1 h at room temperature. Binding reactions were terminated by rapid filtration onto 96-well Unifilter GF/C filter plates (Perkin Elmer, Shelton, CT) presoaked in 0.5% polyethylenimine (Sigma). Harvesting was performed using a 96-well tissue harvester (Perkin Elmer, Shelton, CT) followed by three filtration washes with 500 μl ice-cold binding buffer. Filter plates were subsequently dried at 50°C for 2-3 hours. Fifty μl/well scintillation cocktail (Perkin Elmer, Shelton, CT) was added and plates were counted in a Packard Top-Count for 1 min/well. The data from screening and dose-displacement experiments were analyzed using Microsoft Excel and the curve fitting functions in GraphPad PRISM™, v. 3.0 or higher, respectively, or an in-house function for one-site competition curve-fitting.

**ORL-1 Receptor Binding Data:** Certain Compounds of the Invention will have a Ki (nM) of about 1000 or less. In one embodiment, the Compounds of the Invention will have a Ki (nM) of about 500 or less. In other embodiments, the Compounds of the Invention will have a Ki (nM) of about 300 or less; or of about 100 or less; or of about 50 or less; or of about 20 or less. In yet other embodiments, the Compounds of the Invention will have a Ki (nM) of about 10 or less; or of about 1 or less; or of about 0.1 or less.

**ORL-1 Receptor Functional Assay Procedure:** Membranes from recombinant HEK-293 cells expressing the human opioid receptor-like (ORL-1) (Perkin Elmer, Shelton, CT) were prepared by lysing cells in ice-cold hypotonic buffer (2.5 mM Mg $Cl_2$, 50 mM HEPES, pH 7.4) (10 ml/10 cm dish) followed by homogenization with a tissue grinder/Teflon pestle. Membranes were collected by centrifugation at 30,000 x g for 15 min at 4°C, and pellets resuspended in hypotonic

buffer to a final concentration of 1-3 mg/ml. Protein concentrations were determined using the BioRad protein assay reagent with bovine serum albumen as standard. Aliquots of the ORL-1 receptor membranes were stored at -80°C.

Functional [$^{35}$S]GTPγS binding assays were conducted as follows. ORL-1 membrane solution was prepared by sequentially adding final concentrations of 0.026 μg/μl ORL-1 membrane protein, 10 μg/ml saponin, 3 μM GDP and 0.20 nM [$^{35}$S]GTPγS to binding buffer (100 mM NaCl, 10 mM $MgCl_2$, 20 mM HEPES, pH 7.4) on ice. The prepared membrane solution (190 μl/well) was transferred to 96-shallow well polypropylene plates containing 10 μl of 20x concentrated stock solutions of agonist/nociceptin prepared in DMSO. Plates were incubated for 30 min at room temperature with shaking. Reactions were terminated by rapid filtration onto 96-well Unifilter GF/B filter plates (Perkin Elmer, Shelton, CT) using a 96-well tissue harvester (Packard) and followed by three filtration washes with 200 μl ice-cold binding buffer (10 mM $NaH_2PO_4$, 10 mM $Na_2HPO_4$, pH 7.4). Filter plates were subsequently dried at 50°C for 2-3 hours. Fifty μl/well scintillation cocktail (Perkin Elmer, Shelton, CT) was added and plates were counted in a Packard Top-Count for 1 min/well. Data were analyzed using the sigmoidal dose-response curve fitting functions in GraphPad PRISM v. 3.0 or higher, or an in-house function for non-linear, sigmoidal dose-response curve-fitting.

**ORL-1 Receptor Functional Data:** ORL-1 GTP $EC_{50}$ is the concentration of a compound providing 50% of the maximal response for the compound at an ORL-1 receptor. In certain embodiments, the Compounds of the Invention that have a high binding affinity (i.e. low $K_i$ value) will have an ORL-1 GTP $EC_{50}$ (nM) of greater than about 10,000 (i.e. will not stimulate at therapeutic concentrations) In certain embodiments Compounds of the Invention will have an ORL-1 GTP $EC_{50}$ (nM) of about 20,000 or less. In one embodiment, the Compounds of the Invention will have an ORL-1 GTP $EC_{50}$ (nM) of about 10,000 or less; or of about 5000 or less; or of about 1000 or less. In still other embodiments, the Compounds of the Invention will have an ORL-1 GTP $EC_{50}$ (nM) of about 100 or less; or of about 10 or less; or of about 1 or less; or of about 0.1 or less..

ORL-1 GTP $E_{max}$ % is the maximal effect elicited by a compound relative to the effect elicited by nociceptin, a standard ORL-1 agonist. In certain embodiments, Compounds of the Invention will have an ORL-1 GTP $E_{max}$ of less than 10% (which, for the purposes of this invention, is interpreted as having antagonist activity at ORL-1 receptors). Certain Compounds of the Invention will have an ORL-1 GTP $E_{max}$ (%) of greater than 1%; or of greater than 5%; or of greater than 10%. In other embodiments the Compounds of the Invention will have an ORL-1 GTP $E_{max}$ of greater than 20%; or of greater than 50%; or of greater than 75%; or of greater than 88%; or of greater than 100%.

***In Vivo Assays for Prevention or Treatment of Pain***

**Test Animals:** Each experiment uses rats weighing between 200-260 g at the start of the experiment. The rats are group-housed and have free access to food and water at all times, except prior to oral administration of a Compound of the Invention when food is removed for about 16 hours before dosing. A control group acts as a comparison to rats treated with a Compound of the Invention. The control group is administered the carrier for the Compound of the Invention. The volume of carrier administered to the control group is the same as the volume of carrier and Compound of the Invention administered to the test group.

**Acute Pain:** To assess the actions of a Compound of the Invention for the treatment or prevention of acute pain, the rat tail flick can be used. Rats are gently restrained by hand and the tail exposed to a focused beam of radiant heat at a point 5 cm from the tip using a tail flick unit (Model 7360, commercially available from Ugo Basile of Italy). Tail flick latencies are defined as the interval between the onset of the thermal stimulus and the flick of the tail. Animals not responding within 20 seconds are removed from the tail flick unit and assigned a withdrawal latency of 20 seconds. Tail flick latencies are measured immediately before (pre-treatment) and 1, 3, and 5 hours following administration of a Compound of the Invention. Data are expressed as tail flick latency(s) and the percentage of the maximal possible effect (% MPE), *i.e.,* 20 seconds, is calculated as follows:

$$\%\ MPE = \frac{[\ (\text{post administration latency}) - (\text{pre-administration latency})\ ]}{(20\ s -\ \text{pre-administration latency})} \times\ 100$$

The rat tail flick test is described in F.E. D'Amour et al., "A Method for Determining Loss of Pain Sensation," J. Pharmacol. Exp. Ther. 72:74-79 (1941).

To assess the actions of a Compound of the Invention for the treatment or prevention of acute pain, the rat hot plate test can also be used. Rats are tested using a hot plate apparatus consisting of a clear plexiglass cylinder with a heated metal floor maintained at a temperature of 48-52 °C (Model 7280, commercially available from Ugo Basile of Italy). Rats are placed into the cylinder on the hot plate apparatus for a maximum duration of 30 s, or until it exhibits a nocifensive behavior (behavioral endpoint), at which time it is removed from the hot plate, and the response latency recorded. Hot plate latencies are measured immediately before (pre-treatment) and 1, 3, and 5 hours following administration of a Compound of the Invention. The nocifensive behavioral endpoint is defined as any of the following: 1) paw withdrawal, either as a sustained lift or with shaking or licking; 2) alternating foot lifting; 3) excape or attempted escapre from the testing device; or 4) vocalization. Data are expressed as response latency(s) and the percentage of the maximal possible effect is calculated as described above for the tail flick test. The hot plate test is described in G. Woolfe and A.D. Macdonald, J. Pharmacol. Exp. Ther. 80:300-307 (1944).

**Inflammatory Pain:** To assess the actions of a Compound of the Invention for the treatment or prevention of inflammatory pain, the Freund's complete adjuvant ("FCA") model of inflammatory pain can be used. FCA-induced inflammation of the rat hind paw is associated with the development of persistent inflammatory mechanical hyperalgesia and provides reliable prediction of the anti-hyperalgesic action of clinically useful analgesic drugs (L. Bartho et al., "Involvement of Capsaicin-sensitive Neurones in Hyperalgesia and Enhanced Opioid Antinociception in Inflammation," Naunyn-Schmiedeberg's Archives of Pharmacol. 342:666-670 (1990)). The left hind paw of each animal is administered a 50 $\mu$L intraplantar injection of 50% FCA. Prior to injection of FCA (baseline) and 24 hour post injection, the animal is assessed for response to noxious mechanical stimuli by determining the PWT, as described below. Rats are then administered a single injection of 1, 3, or 10 mg/kg of either a Compound of the Invention; 30mg/kg of a control drug selected from Celebrex, indomethacin or naproxen; or carrier. Responses to noxious mechanical stimuli are determined 1, 3, 5 and 24 hours post administration. Percentage reversal of hyperalgesia for each animal is defined as:

$$\% \text{ Reversal} = \frac{[\,(\text{post administration PWT}) - (\text{pre-administration PWT})\,]}{[\,(\text{baseline PWT}) - (\text{pre-administration PWT})\,]} \times 100$$

**Neuropathic Pain:** To assess the actions of a Compound of the Invention for the treatment or prevention of neuropathic pain, either the Seltzer model or the Chung model can be used.

In the Seltzer model, the partial sciatic nerve ligation model of neuropathic pain is used to produce neuropathic hyperalgesia in rats (Z. Seltzer et al., "A Novel Behavioral Model of Neuropathic Pain Disorders Produced in Rats by Partial Sciatic Nerve Injury," Pain 43:205-218 (1990)). Partial ligation of the left sciatic nerve is performed under isoflurane/$O_2$ inhalation anaesthesia. Following induction of anesthesia, the left thigh of the rat is shaved and the sciatic nerve exposed at high thigh level through a small incision and is carefully cleared of surrounding connective tissues at a site near the trocanther just distal to the point at which the posterior biceps semitendinosus nerve branches off of the common sciatic nerve. A 7-0 silk suture is inserted into the nerve with a 3/8 curved, reversed-cutting mini-needle and tightly ligated so that the dorsal ⅓ to ½ of the nerve thickness is held within the ligature. The wound is closed with a single muscle suture (4-0 nylon (Vicryl)) and vetbond tissue glue. Following surgery, the wound area is dusted with antibiotic powder. Sham-treated rats undergo an identical surgical procedure except that the sciatic nerve is not manipulated. Following surgery, animals are weighed and placed on a warm pad until they recover from anesthesia. Animals are then returned to their home cages until behavioral testing begins. The animal is assessed for response to noxious mechanical stimuli by determining PWT, as described below, prior to surgery (baseline), then immediately prior to and 1, 3, and 5 hours after drug administration. Percentage reversal of neuropathic hyperalgesia is defined as:

$$\% \text{ Reversal} = \frac{[\,(\text{post administration PWT}) - (\text{pre-administration PWT})\,]}{[\,(\text{baseline PWT}) - (\text{pre-administration PWT})\,]} \times 100$$

In the Chung model, the spinal nerve ligation model of neuropathic pain is used to produce mechanical hyperalgesia, thermal hyperalgesia and tactile allodynia in rats. Surgery is performed under isoflurane/$O_2$ inhalation anaesthesia. Following induction of anaesthesia, a 3 cm incision is made and the left paraspinal muscles are separated from the spinous process at the $L_4$ - $S_2$ levels. The $L_6$ transverse process is carefully removed with a pair of small rongeurs to identify visually the $L_4$ - $L_6$ spinal nerves. The left $L_5$ (or $L_5$ and $L_6$) spinal nerve(s) is isolated and tightly ligated with silk thread. A complete hemostasis is confirmed and the wound is sutured using non-absorbable sutures, such as nylon sutures or stainless steel staples. Sham-treated rats undergo an identical surgical procedure except that the spinal nerve(s) is not manipulated. Following surgery animals are weighed, administered a subcutaneous (s.c.) injection of saline or ringers lactate, the wound area is dusted with antibiotic powder and they are kept on a warm pad until they recover from the anesthesia. Animals are then returned to their home cages until behavioral testing begins. The animals are assessed for response to noxious mechanical stimuli by determining PWT, as described below, prior to surgery (baseline), then immediately prior to and 1, 3, and 5 hours after being administered a Compound of the Invention. The

animal can also be assessed for response to noxious thermal stimuli or for tactile allodynia, as described below. The Chung model for neuropathic pain is described in S.H. Kim, "An Experimental Model for Peripheral Neuropathy Produced by Segmental Spinal Nerve Ligation in the Rat," Pain 50(3):355-363 (1992).

**Response to Mechanical Stimuli as an Assessment of Mechanical**

**Hyperalgesia:** The paw pressure assay can be used to assess mechanical hyperalgesia. For this assay, hind paw withdrawal thresholds (PWT) to a noxious mechanical stimulus are determined using an analgesymeter (Model 7200, commercially available from Ugo Basile of Italy) as described in C. Stein, "Unilateral Inflammation of the Hindpaw in Rats as a Model of Prolonged Noxious Stimulation: Alterations in Behavior and Nociceptive Thresholds," Pharmacol. Biochem. and Behavior 31:451-455 (1988). The maximum weight that is applied to the hind paw is set at 250 g and the end point is taken as complete withdrawal of the paw. PWT is determined once for each rat at each time point and either only the affected (ipsilateral; same side as the injury) rear paw is tested, or both the ipsilateral and contralateral (non-injured; opposite to the injury) rear paw are tested.

**Response to Thermal Stimuli as an Assessment of Thermal Hyperalgesia:** The plantar test can be used to assess thermal hyperalgesia. For this test, hind paw withdrawal latencies to a noxious thermal stimulus are determined using a plantar test apparatus (commercially available from Ugo Basile of Italy) following the technique described by K. Hargreaves et al., "A New and Sensitive Method for Measuring Thermal Nociception in Cutaneous Hyperalgesia," Pain 32(1):77-88 (1988). The maximum exposure time is set at 32 seconds to avoid tissue damage and any directed paw withdrawal from the heat source is taken as the end point. Three latencies are determined at each time point and averaged. Either only the affected (ipsilateral) paw is tested, or both the ipsilateral and contralateral (non-injured) paw are tested.

**Assessment of Tactile Allodynia:** To assess tactile allodynia, rats are placed in clear, plexiglass compartments with a wire mesh floor and allowed to habituate for a period of at least 15 minutes. After habituation, a series of von Frey monofilaments are presented to the plantar surface of the affected (ipsilateral) foot of each rat. The series of von Frey monofilaments consists of six monofilaments of increasing diameter, with the smallest diameter fiber presented first. Five trials are conducted with each filament with each trial separated by approximately 2 minutes. Each presentation lasts for a period of 4-8 seconds or until a nociceptive withdrawal behavior is observed. Flinching, paw withdrawal or licking of the paw are considered nociceptive behavioral responses.

**Assessment of Respiratory Depression:** To assess respiratory depression, rats can be prepared by implanting a femoral artery cannula via which blood samples are taken. Blood samples are taken prior to drug administration, then 1, 3, 5 and 24 hours post-treatment. Blood samples are processed using an arterial blood gas analyzer (e.g., IDEXX VetStat with Respiratory/Blood Gas test cartridges). Comparable devices are a standard tool for blood gas analysis (e.g., D. Torbati et al., 2000 Intensive Care Med. (26) 585-591).

**Assessment of Gastric Motility:** Animals are treated with vehicle, reference compound or test article by oral gavage at a volume of 10 mL/kg. At one hour post-dose, all animals are treated with charcoal meal solution (5% non-activated charcoal powder in a solution of 1 % carboxymethylcellulose in water) at a volume of 10 mL/kg. At two hours post-dose (one hour post-charcoal), animals are sacrificed by carbon dioxide inhalation or isoflurane overdose and the transit of charcoal meal identified. The stomach and small intestine are removed carefully and each placed on a saline-soaked absorbent surface. The distance between the pylorus and the furthest progression of charcoal meal is measured and compared to the distance between the pylorus and the ileocecal junction. The charcoal meal transit is expressed as a percentage of small intestinal length traveled.

***Pharmaceutical Compositions***

Due to their activity, the Compounds of the Invention are advantageously useful in human and veterinary medicine. As described above, the Compounds of the Invention are useful for treating or preventing a Condition in an animal in need thereof. The Compounds of the Invention can be administered to any animal requiring modulation of the opioid and/or ORL-1 receptors.

When administered to an animal, a Compound of the Invention can be administered as a component of a composition that comprises a pharmaceutically acceptable carrier or excipient. A Compound of the Invention can be administered by any appropriate route, as determined by the medical practitioner. Methods of administration may include intradermal, intramuscular, intraperitoneal, parenteral, intravenous, subcutaneous, intranasal, epidural, oral, sublingual, intracerebral, intravaginal, transdermal, transmucosal, rectal, by inhalation, or topical (particularly to the ears, nose, eyes, or skin). Delivery can be either local or systemic. In certain embodiments, administration will result in the release of a Compound of the Invention into the bloodstream.

Pharmaceutical compositions of the invention can take the form of solutions, suspensions, emulsions, tablets, pills, pellets, multi-particulates, capsules, capsules containing liquids, capsules containing powders, capsules containing

multi-particulates, lozenges, sustained-release formulations, suppositories, aerosols, sprays, or any other form suitable for use. In one embodiment, the composition is in the form of a capsule (*see, e.g.,* U.S. Patent No. 5,698,155). Other examples of suitable pharmaceutical excipients are described in Remington's Pharmaceutical Sciences 1447-1676 (Alfonso R. Gennaro ed., 19th ed. 1995).

Pharmaceutical compositions of the invention preferably comprise a suitable amount of a pharmaceutically acceptable excipient so as to provide the form for proper administration to the animal. Such a pharmaceutical excipient can be a diluent, suspending agent, solubilizer, binder, disintegrant, preservative, coloring agent, lubricant, and the like. The pharmaceutical excipient can be a liquid, such as water or an oil, including those of petroleum, animal, vegetable, or synthetic origin, such as peanut oil, soybean oil, mineral oil, sesame oil, and the like. The pharmaceutical excipient can be saline, gum acacia, gelatin, starch paste, talc, keratin, colloidal silica, urea, and the like. In addition, auxiliary, stabilizing, thickening, lubricating, and coloring agents can be used. In one embodiment, the pharmaceutically acceptable excipient is sterile when administered to an animal. Water is a particularly useful excipient when a Compound of the Invention is administered intravenously. Saline solutions and aqueous dextrose and glycerol solutions can also be employed as liquid excipients, particularly for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. The invention compositions, if desired, can also contain minor amounts of wetting or emulsifying agents, or pH buffering agents. Specific examples of pharmaceutically acceptable carriers and excipients that can be used to formulate oral dosage forms are described in the Handbook of Pharmaceutical Excipients, American Pharmaceutical Association (1986).

In certain embodiments, the Compounds of the Invention are formulated for oral administration. A Compound of the Invention to be orally delivered can be in the form of tablets, capsules, gelcaps, caplets, lozenges, aqueous or oily solutions, suspensions, granules, powders, emulsions, syrups, or elixirs, for example. When a Compound of the Invention is incorporated into oral tablets, such tablets can be compressed, tablet triturates, enteric-coated, sugar-coated, film-coated, multiply compressed or multiply layered.

An orally administered Compound of the Invention can contain one or more additional agents such as, for example, sweetening agents such as fructose, aspartame or saccharin; flavoring agents such as peppermint, oil of wintergreen, or cherry; coloring agents; and preserving agents, and stabilizers, to provide stable, pharmaceutically palatable dosage forms. Techniques and compositions for making solid oral dosage forms are described in Pharmaceutical Dosage Forms: Tablets (Lieberman, Lachman and Schwartz, eds., 2nd ed.) published by Marcel Dekker, Inc. Techniques and compositions for making tablets (compressed and molded), capsules (hard and soft gelatin) and pills are also described in Remington's Pharmaceutical Sciences 1553-1593 (Arthur Osol, ed., 16th ed., Mack Publishing, Easton, PA 1980). Liquid oral dosage forms include aqueous and nonaqueous solutions, emulsions, suspensions, and solutions and/or suspensions reconstituted from non-effervescent granules, optionally containing one or more suitable solvents, preservatives, emulsifying agents, suspending agents, diluents, sweeteners, coloring agents, flavoring agents, and the like. Techniques and compositions for making liquid oral dosage forms are described in Pharmaceutical Dosage Forms: Disperse Systems, (Lieberman, Rieger and Banker, eds.) published by Marcel Dekker, Inc.

When a Compound of the Invention is formulated for parenteral administration by injection (e.g., continuous infusion or bolus injection), the formulation can be in the form of a suspension, solution, or emulsion in an oily or aqueous vehicle, and such formulations can further comprise pharmaceutically necessary additives such as one or more stabilizing agents, suspending agents, dispersing agents, and the like. When a Compound of the Invention is to be injected parenterally, it can be, *e.g.,* in the form of an isotonic sterile solution. A Compound of the Invention can also be in the form of a powder for reconstitution as an injectable formulation.

In certain embodiments, a Compound of the Invention is formulated into a pharmaceutical composition for intravenous administration. Typically, such compositions comprise sterile isotonic aqueous buffer. Where necessary, the compositions can also include a solubilizing agent. A Compound of the Invention for intravenous administration can optionally include a local anesthetic such as benzocaine or prilocaine to lessen pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where a Compound of the Invention is to be administered by infusion, it can be dispensed, for example, with an infusion bottle containing sterile pharmaceutical grade water or saline. Where a Compound of the Invention is administered by injection, an ampule of sterile water for injection or saline can be provided so that the ingredients can be mixed prior to administration.

When a Compound of the Invention is to be administered by inhalation, it can be formulated into a dry aerosol, or an aqueous or partially aqueous solution.

In another embodiment, a Compound of the Invention can be delivered in a vesicle, in particular a liposome (*see* Langer, Science 249:1527-1533 (1990); and Treat et al., Liposomes in the Therapy of Infectious Disease and Cancer 317-327 and 353-365 (1989)).

In certain embodiments, a Compound of the Invention is administered locally. This can be achieved, for example,

by local infusion during surgery, topical application, *e.g.,* in conjunction with a wound dressing after surgery, by injection, by means of a catheter, by means of a suppository or enema, or by means of an implant, said implant being of a porous, non-porous, or gelatinous material, including membranes, such as sialastic membranes, or fibers.

In certain embodiments, a Compound of the Invention can be delivered in an immediate release form. In other embodiments, a Compound of the Invention can be delivered in a controlled-release system or sustained-release system. Controlled- or sustained-release pharmaceutical compositions can have a common goal of improving drug therapy over the results achieved by their non-controlled or non-sustained-release counterparts. In one embodiment, a controlled- or sustained-release composition comprises a minimal amount of a Compound of the Invention to treat or prevent the Condition (or a symptom thereof) in a minimum amount of time. Advantages of controlled- or sustained-release compositions include extended activity of the drug, reduced dosage frequency, and increased compliance. In addition, controlled- or sustained-release compositions can favorably affect the time of onset of action or other characteristics, such as blood levels of the Compound of the Invention, and can thus reduce the occurrence of adverse side effects.

Controlled- or sustained-release compositions can initially release an amount of a Compound of the Invention that promptly produces the desired therapeutic or prophylactic effect, and gradually and continually release other amounts of the Compound of the Invention to maintain a level of therapeutic or prophylactic effect over an extended period of time. To maintain a constant level of the Compound of the Invention in the body, the Compound of the Invention can be released from the dosage form at a rate that will replace the amount of Compound of the Invention being metabolized and excreted from the body. Controlled- or sustained-release of an active ingredient can be stimulated by various conditions, including but not limited to, changes in pH, changes in temperature, concentration or availability of enzymes, concentration or availability of water, or other physiological conditions or compounds.

Controlled-release and sustained-release means for use according to the present invention may be selected from those known in the art. Examples include, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Such dosage forms can be used to provide controlled- or sustained-release of one or more active ingredients using, for example, hydropropylmethyl cellulose, other polymer matrices, gels, permeable membranes, osmotic systems, multilayer coatings, microparticles, multiparticulates, liposomes, microspheres, or a combination thereof to provide the desired release profile in varying proportions. Suitable controlled- or sustained-release formulations known in the art, including those described herein, can be readily selected for use with the active ingredients of the invention in view of this disclosure. See also Goodson, "Dental Applications" (pp. 115-138) in Medical Applications of Controlled Release, Vol. 2*,* Applications and Evaluation, R.S. Langer and D.L. Wise eds., CRC Press (1984). Other controlled- or sustained-release systems that are discussed in the review by Langer, Science 249:1527-1533 (1990) can be selected for use according to the present invention. In one embodiment, a pump can be used (Langer, Science 249:1527-1533 (1990); Sefton, CRC Crit. Ref. Biomed. Eng. 14:201 (1987); Buchwald et al., Surgery 88:507 (1980); and Saudek et al., N. Engl. J. Med. 321:574 (1989)). In another embodiment, polymeric materials can be used (*see* Medical Applications of Controlled Release (Langer and Wise eds., 1974); Controlled Drug Bioavailability, Drug Product Design and Performance (Smolen and Ball eds., 1984); Ranger and Peppas, J. Macromol. Sci. Rev. Macromol. Chem. 23:61 (1983); Levy et al., Science 228:190 (1985); During et al., Ann. Neurol. 25:351 (1989); and Howard et al., J. Neurosurg. 71:105 (1989)). In yet another embodiment, a controlled- or sustained-release system can be placed in proximity of a target of a Compound of the Invention, *e.g.*, the spinal column, brain, or gastrointestinal tract, thus requiring only a fraction of the systemic dose.

When in tablet or pill form, a pharmaceutical composition of the invention can be coated to delay disintegration and absorption in the gastrointestinal tract thereby providing a sustained action over an extended period of time. Selectively permeable membranes surrounding an osmotically active driving compound are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be used. Oral compositions can include standard excipients such as mannitol, lactose, starch, magnesium stearate, sodium saccharin, cellulose, and magnesium carbonate. In one embodiment, the excipients are of pharmaceutical grade.

Pharmaceutical compositions of the invention include single unit dosage forms suitable for oral administration such as, but not limited to, tablets, capsules, gelcaps, and caplets that are adapted for controlled- or sustained-release.

The amount of the Compound of the Invention that is effective for the treatment or prevention of a condition can be determined by standard clinical techniques. In addition, *in vitro* and/or *in vivo* assays can optionally be employed to help identify optimal dosage ranges. The precise dose to be employed will also depend on, *e.g.*, the route of administration and the extent of the Condition to be treated, and can be decided according to the judgment of a practitioner and/or each animal's circumstances. Variations in dosing may occur depending upon typical factors such as the weight, age, gender and physical condition (*e.g.,* hepatic and renal function) of the animal being treated, the affliction to be treated, the severity of the symptoms, the frequency of the dosage interval, the presence of any deleterious side-effects,

and the particular compound utilized, among other things.

Suitable effective dosage amounts can range from about 0.01mg/kg of body weight to about 3000 mg/kg of body weight of the animal per day, although they are typically from about 0.01mg/kg of body weight to about 2500 mg/kg of body weight of the animal per day or from about 0.01mg/kg of body weight to about 1000 mg/kg of body weight of the animal per day. In one embodiment, the effective dosage amount is about 100 mg/kg of body weight of the animal per day or less. In another embodiment, the effective dosage amount ranges from about 0.01mg/kg of body weight to about 100 mg/kg of body weight of the animal per day of a Compound of the Invention, in another embodiment, about 0.02 mg/kg of body weight to about 50 mg/kg of body weight of the animal per day, and in another embodiment, about 0.025 mg/kg of body weight to about 20 mg/kg of body weight of the animal per day.

Administration can be as a single dose or as a divided dose. In one embodiment, an effective dosage amount is administered about every 24h until the Condition is abated. In another embodiment, an effective dosage amount is administered about every 12h until the Condition is abated. In another embodiment, an effective dosage amount is administered about every 8h until the Condition is abated. In another embodiment, an effective dosage amount is administered about every 6h until the Condition is abated. In another embodiment, an effective dosage amount is administered about every 4h until the Condition is abated. The effective dosage amounts described herein refer to total amounts administered; that is, if more than one Compound of the Invention is administered, the effective dosage amounts correspond to the total amount administered.

Where a cell capable of expressing the ORL-1 receptor is contacted with a Compound of the Invention *in vitro,* the amount effective for inhibiting or activating the ORL-1 receptor function in a cell will typically range from about $10^{-12}$ mol/L to about $10^{-4}$ mol/L, or from about $10^{-12}$ mol/L to about $10^{-5}$ mol/L, or from about $10^{-12}$ mol/L to about $10^{-6}$ mol/L, or from about $10^{-12}$ mol/L to about $10^{-9}$ mol/L of a solution or suspension of the compound in a pharmaceutically acceptable carrier or excipient. In one embodiment, the volume of solution or suspension comprising the Compound of the Invention will be from about 0.01$\mu$L to about 1mL. In another embodiment, the volume of solution or suspension will be about 200 $\mu$L.

Where a cell capable of expressing the $\mu$-opioid receptors is contacted with a Compound of the Invention in vitro, the amount effective for inhibiting or activating the $\mu$-opioid receptors function in a cell will typically range from about $10^{-12}$ mol/L to about $10^{-4}$ mol/L,or from about $10^{-12}$ mol/L to about $10^{-5}$ mol/L, or from about $10^{-12}$ mol/L to about $10^{-6}$ mol/L, or from about $10^{-12}$ mol/L to about $10^{-9}$ mol/L of a solution or suspension of the Compound of the Invention in a pharmaceutically acceptable carrier or excipient. In one embodiment, the volume of solution or suspension comprising the Compound of the Invention will be from about 0.01$\mu$L to about 1 mL. In another embodiment, the volume of solution or suspension will be about 200 $\mu$L.

Where a cell capable of expressing the $\delta$-opioid receptors is contacted with a Compound of the Invention in vitro, the amount effective for inhibiting or activating the $\delta$-opioid receptors function in a cell will typically range from about $10^{-12}$ mol/L to about $10^{-4}$ mol/L,or from about $10^{-12}$ mol/L to about $10^{-5}$ mol/L, or from about $10^{-12}$ mol/L to about $10^{-6}$ mol/L, or from about $10^{-12}$ mol/L to about $10^{-9}$ mol/L of a solution or suspension of the Compound of the Invention in a pharmaceutically acceptable carrier or excipient. In one embodiment, the volume of solution or suspension comprising the Compound of the Invention will be from about 0.01$\mu$L to about 1mL. In another embodiment, the volume of solution or suspension will be about 200 $\mu$L.

Where a cell capable of expressing the $\kappa$-opioid receptors is contacted with a Compound of the Invention in vitro, the amount effective for inhibiting or activating the $\kappa$-opioid receptors function in a cell will typically range from about $10^{-12}$ mol/L to about $10^{-4}$ mol/L,or from about $10^{-12}$ mol/L to about $10^{-5}$ mol/L, or from about $10^{-12}$ mol/L to about $10^{-6}$ mol/L, or from about $10^{-12}$ mol/L to about $10^{-9}$ mol/L of a solution or suspension of the Compound of the Invention in a pharmaceutically acceptable carrier or excipient. In one embodiment, the volume of solution or suspension comprising the Compound of the Invention will be from about 0.01$\mu$L to about 1 mL. In another embodiment, the volume of solution or suspension will be about 200 $\mu$L.

The Compounds of the Invention can be assayed *in vitro* or *in vivo* for the desired therapeutic or prophylactic activity prior to use in humans. Animal model systems can be used to demonstrate safety and efficacy. Certain Compounds of the Invention will have an $ED_{50}$ for treating pain ranging from about 0.5 mg/kg to about 20 mg/kg. Certain Compounds of the Invention will produce significant analgesia and/or anti-hyperalgesia at doses that do not induce respiratory depression. In contrast, oxygen tension, oxygen saturation and pH are significantly decreased, while carbon dioxide is significantly increased, in blood samples from rats given effective doses of conventional opioids, such as morphine. The use of the Compounds of the Invention for treating or preventing a Condition in an animal in need thereof can further comprise coadministering to the animal an effective amount of a second therapeutic agent in addition to a Compound of the Invention (*i.e.*, a first therapeutic agent). An effective amount of the second therapeutic agent will be known or determinable by a medical practitioner in view of this disclosure and published clinical studies. In one embodiment where a second therapeutic agent is administered to an animal for treatment of a Condition (*e.g.*, pain), the minimal effective amount of the Compound of the Invention (i.e., the first therapeutic agent) will be less than its minimal effective amount would be in circumstances where the second therapeutic agent is not administered. In this embodiment, the Compound of the Invention and the second therapeutic agent can act either additively or synergistically to treat or prevent a Condition.

Alternatively, the second therapeutic agent may be used to treat or prevent a disorder that is different from the Condition for which the first therapeutic agent is being administered, and which disorder may or may not be a Condition as defined hereinabove. In one embodiment, a Compound of the Invention is administered concurrently with a second therapeutic agent as a single composition comprising an effective amount of a Compound of the Invention and an effective amount of the second therapeutic agent. Alternatively, a composition comprising an effective amount of a Compound of the Invention and a second composition comprising an effective amount of the second therapeutic agent are concurrently administered. In another embodiment, an effective amount of a Compound of the Invention is administered prior or subsequent to administration of an effective amount of the second therapeutic agent. In this embodiment, the Compound of the Invention is administered while the second therapeutic agent exerts its therapeutic effect, or the second therapeutic agent is administered while the Compound of the Invention exerts its therapeutic effect for treating or preventing a Condition.

The second therapeutic agent can be, but is not limited to, an opioid agonist, a non-opioid analgesic, a non-steroidal anti-inflammatory agent, an antimigraine agent, a Cox-IA inhibitor, a 5-lipoxygenase inhibitor, an anti-emetic, a β-adrenergic blocker, an anticonvulsant, an antidepressant, a $Ca^{2+}$-channel blocker, an anti-cancer agent, an agent for treating or preventing UI, an agent for treating or preventing anxiety, an agent for treating or preventing a memory disorder, an agent for treating or preventing obesity, an agent for treating or preventing constipation, an agent for treating or preventing cough, an agent for treating or preventing diarrhea, an agent for treating or preventing high blood pressure, an agent for treating or preventing epilepsy, an agent for treating or preventing anorexia/cachexia, an agent for treating or preventing drug abuse, an agent for treating or preventing an ulcer, an agent for treating or preventing IBD, an agent for treating or preventing IBS, an agent for treating or preventing addictive disorder, an agent for treating or preventing Parkinson’s disease and parkinsonism, an agent for treating or preventing a stroke, an agent for treating or preventing a seizure, an agent for treating or preventing a pruritic condition, an agent for treating or preventing psychosis, an agent for treating or preventing Huntington’s chorea, an agent for treating or preventing ALS, an agent for treating or preventing a cognitive disorder, an agent for treating or preventing a migraine, an agent for treating, preventing or inhibiting vomiting, an agent for treating or preventing dyskinesia, an agent for treating or preventing depression, or any mixture thereof.

Examples of useful opioid agonists include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable derivatives thereof, or any mixture thereof.

In certain embodiments, the opioid agonist is selected from codeine, hydromorphone, hydrocodone, oxycodone, dihydrocodeine, dihydromorphine, morphine, tramadol, oxymorphone, pharmaceutically acceptable derivatives thereof, or any mixture thereof.

Examples of useful non-opioid analgesics include, but are not limited to, non-steroidal anti-inflammatory agents, such as aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, a pharmaceutically acceptable derivative thereof, or any mixture thereof. Other suitable non-opioid analgesics include the following, non-limiting, chemical classes of analgesic, antipyretic, nonsteroidal anti-inflammatory drugs: salicylic acid derivatives, including aspirin, sodium salicylate, choline magnesium trisalicylate, salsalate, diflunisal, salicylsalicylic acid, sulfasalazine, and olsalazin; para-aminophenol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid and meclofenamic acid; enolic acids, including oxicams (piroxicam, tenoxicam), and pyrazolidinediones (phenylbutazone, oxyphenthartazone); alkanones, including nabumetone; a pharmaceutically acceptable derivative thereof; or any mixture thereof. For a more detailed description of the NSAIDs, *see* Paul A. Insel, Analgesic-Antipyretic and Anti-inflammatory Agents and Drugs Employed in the Treatment of Gout, in Goodman & Gilman’s The Pharmacological Basis of Therapeutics 617-57 (Perry B. Molinhoff and Raymond W. Ruddon eds., 9th ed 1996); and Glen R. Hanson, Analgesic, Antipyretic and Anti-Inflammatory Drugs in Remington: The Science and Practice of Pharmacy Vol IA 1196-1221 (A.R. Gennaro ed. 19th ed. 1995).

Examples of useful Cox-II inhibitors and 5-lipoxygenase inhibitors, as well as combinations thereof, are described in U.S. Patent No. 6,136,839. Examples of useful Cox-II inhibitors include, but are not limited to, celecoxib, DUP-697, flosulide, meloxicam, 6-MNA, L-745337, rofecoxib, nabumetone, nimesulide, NS-398, SC-5766, T-614, L-768277, GR-253035, JTE-522, RS-57067-000, SC-58125, SC-078, PD-138387, NS-398, flosulide, D-1367, SC-5766, PD-164387, etoricoxib, valdecoxib, parecoxib, a pharmaceutically acceptable derivative thereof, or any mixture thereof.

Examples of useful antimigraine agents include, but are not limited to, alpiropride, bromocriptine, dihydroergotamine, dolasetron, ergocornine, ergocorninine, ergocryptine, ergonovine, ergot, ergotamine, flumedroxone acetate, fonazine, ketanserin, lisuride, lomerizine, methylergonovine, methysergide, metoprolol, naratriptan, oxetorone, pizotyline, propranolol, risperidone, rizatriptan, sumatriptan, timolol, trazodone, zolmitriptan, a pharmaceutically acceptable derivative thereof, or any mixture thereof.

Examples of useful anticonvulsants include, but are not limited to, acetylpheneturide, albutoin, aloxidone, aminoglutethimide, 4-amino-3-hydroxybutyric acid, atrolactamide, beclamide, buramate, calcium bromide, carbamazepine, cinromide, clomethiazole, clonazepam, decimemide, diethadione, dimethadione, doxenitroin, eterobarb, ethadione, ethosuximide, ethotoin, felbamate, fluoresone, gabapentin, 5-hydroxytryptophan, lamotrigine, magnesium bromide, magnesium sulfate, mephenytoin, mephobarbital, metharbital, methetoin, methsuximide, 5-methyl-5-(3-phenanthryl)-hydantoin, 3-methyl-5-phenylhydantoin, narcobarbital, nimetazepam, nitrazepam, oxcarbazepine, paramethadione, phenacemide, phenetharbital, pheneturide, phenobarbital, phensuximide, phenylmethylbarbituric acid, phenytoin, phethenylate sodium, potassium bromide, pregabaline, primidone, progabide, sodium bromide, solanum, strontium bromide, suclofenide, sulthiame, tetrantoin, tiagabine, topiramate, trimethadione, valproic acid, valpromide, vigabatrin, zonisamide, a pharmaceutically acceptable derivative thereof, or any mixture thereof.

Examples of useful $Ca^{2+}$-channel blockers include, but are not limited to, bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, fantofarone, perhexiline, a pharmaceutically acceptable derivative thereof, or any mixture thereof.

Examples of useful therapeutic agents for treating or preventing UI include, but are not limited to, propantheline, imipramine, hyoscyamine, oxybutynin, dicyclomine, a pharmaceutically acceptable derivative thereof, or any mixture thereof.

Examples of useful therapeutic agents for treating or preventing anxiety include, but are not limited to, benzodiazepines, such as alprazolam, brotizolam, chlordiazepoxide, clobazam, clonazepam, clorazepate, demoxepam, diazepam, estazolam, flumazenil, flurazepam, halazepam, lorazepam, midazolam, nitrazepam, nordazepam, oxazepam, prazepam, quazepam, temazepam, and triazolam; non-benzodiazepine agents, such as buspirone, gepirone, ipsapirone, tiospirone, zolpicone, zolpidem, and zaleplon; tranquilizers, such as barbituates, *e.g.*, amobarbital, aprobarbital, butabarbital, butalbital, mephobarbital, methohexital, pentobarbital, phenobarbital, secobarbital, and thiopental; propanediol carbamates, such as meprobamate and tybamate; a pharmaceutically acceptable derivative thereof; or any mixture thereof.

Examples of useful therapeutic agents for treating or preventing diarrhea include, but are not limited to, diphenoxylate, loperamide, a pharmaceutically acceptable derivative thereof, or any mixture thereof.

Examples of useful therapeutic agents for treating or preventing epilepsy include, but are not limited to, carbamazepine, ethosuximide, gabapentin, lamotrigine, phenobarbital, phenytoin, primidone, valproic acid, trimethadione, benzodiazepines, $\gamma$ vinyl GABA, acetazolamide, felbamate, a pharmaceutically acceptable derivative thereof, or any mixture thereof.

Examples of useful therapeutic agents for treating or preventing drug abuse include, but are not limited to, methadone, desipramine, amantadine, fluoxetine, buprenorphine, an opiate agonist, 3-phenoxypyridine, levomethadyl acetate hydrochloride, serotonin antagonists, a pharmaceutically acceptable derivative thereof, or any mixture thereof.

Examples of non-steroidal anti-inflammatory agents, 5-lipoxygenase inhibitors, anti-emetics, $\beta$ adrenergic blockers, antidepressants, and anti-cancer agents are known in the art and can be selected by those skilled in the art. Examples of useful therapeutic agents for treating or preventing memory disorder, obesity, constipation, cough, high blood pressure, anorexia/cachexia, an ulcer, IBD, IBS, addictive disorder, Parkinson's disease and parkinsonism, a stroke, a seizure, a pruritic condition, psychosis, Huntington's chorea, ALS, a cognitive disorder, a migraine, dyskinesia, depression, and/or treating, preventing or inhibiting vomiting include those that are known in the art and can be selected by those skilled in the art.

A composition of the invention is prepared by a method comprising admixing a Compound of the Invention (or a pharmaceutically acceptable salt, or solvate thereof) with a pharmaceutically acceptable carrier or excipient. Admixing can be accomplished using methods known for admixing a compound (or derivative) and a pharmaceutically acceptable carrier or excipient. In one embodiment, the Compound of the Invention (or pharmaceutically acceptable salt, or solvate thereof) is present in the composition in an effective amount.

## *Examples*

Examples which are not comprised in the appendant claims represent reference examples.

## EXAMPLE 1

**(6*S*,11*R*)-6-(4-(benzyloxy)butyl)-3-(cyclopropylmethyl)-8-methoxy-11-methyl-1,2,3,4,5,6-hexahydro-2,6-methanobenzo[*d*]azocine (Compound 12); and 4-((6*S*,11*R*)-3-(cyclopropylmethyl)-8-methoxy-11-methyl-1,2,3,4,5,6-hexahydro-2,6-methanobenzo[*d*]azocin-6-yl)butan-1-ol (Compound 13).**

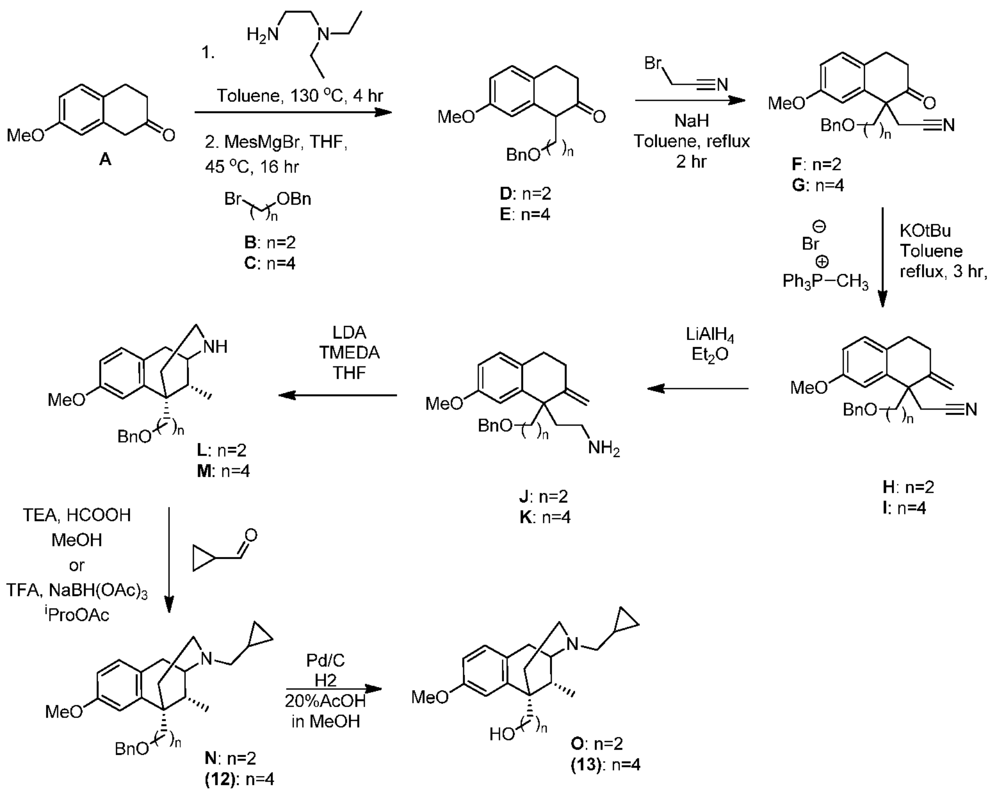


*N*,*N*-Diethylethylenediamine (122.80 mL, 874 mmol, 2.2 eq) [Sigma-Aldrich] was added to a solution of 7-methoxy-2-tetralone **A** (70 g, 397 mmol, 1.0 eq.) [Sigma-Aldrich] in toluene (2 L). The mixture was heated to reflux and water was trapped with Dean-Stark apparatus for 3 hr. The mixture was concentrated in vacuo. Imine was dissolved in THF (200 mL) and 2,4,6-trimethylphenylmagnesium bromide solution (1M in THF, 437 mL, 437 mmol, 1.1 eq.) [Sigma-Aldrich] was added slowly to the solution of imine in THF at 0 °C. After complete addition of the Grignard reagent, the mixture was stirred for 1 hr at 80 °C. The mixture was concentrated using a rotary evaporator and benzyl 2-bromoethyl ether **B** (75.4 mL, 477 mmol, 1.2 eq.) [Sigma-Aldrich] was added slowly 0 °C. The mixture was stirred overnight at 45 °C. 50 % aq. AcOH was added slowly at 0 °C then heated at 50 °C for 1 hr. The mixture was concentrated, adsorbed onto silica gel and purified by flash chromatography (silica gel, 0-100% EtOAc/Hex) to obtain a yellow oil. 94.7 g (77 % yield) of compound **D** was prepared. $^1H$ NMR: $\delta_H$ (400 MHz, $CDCl_3$): 7.45-7.25 (m, 5H), 7.14 (d, 1H), 6.77 (dd, 1H), 6.72 (d, 1H), 4.49 (d, 2H), 3.79 (s, 3H), 3.61 (t, 1H), 3.52 (t, 2H), 3.14-3.04 (m, 1H), 2.99-2.90 (m, 1H), 2.68-2.60 (m, 1H), 2.55-2.46 (m, 1H), 2.22 (qd, 2H).
LC/MS, m/z = 310.2 $[M + Na]^+$ (Calc: 310.39).

In a similar manner, compound **E** was prepared from compound **A** with benzyl 2-bromobutyl ether **C** [Sigma-Aldrich]. Yellow oil (56 % yield)
$^1H$ NMR: $\delta_H$ (400 MHz, $CDCl_3$): 7.40-7.26 (m, 5H), 7.14 (d, 1H), 6.77 (dd, 1H), 6.68 (d, 1H), 4.50 (s, 2H), 3.81 (s, 3H), 3.47 (t, 2H), 3.38 (t, 1H), 3.15-3.07 (m, 1H), 2.94 (dt, 1H), 2.65 (dt, 1H), 2.55-2.46 (m, 1H), 1.93-1.85 (m, 2H), 1.65 (quin, 2H), 1.48-1.38 (m, 2H).
LC/MS, *m/z* =338.2 $[M + Na]^+$ (Calc: 338.44).

A suspension of sodium hydride (60% dispersion in mineral oil, 12.3 g, 319 mmol, 1.1 eq.) in toluene (1 L) was heated to reflux. A solution of ketone **D** (90 g, 290 mmol, 1.0 eq.) in toluene (2.5 L) was added to the refluxing suspension and the mixture was heated to reflux for 1 hr. Bromoacetonitrile (26.3 mL, 377 mmol, 1.3 eq.) [Sigma-Aldrich] was added and refluxed 2 hr further. The reaction mixture was cooled to room temperature and quenched with water (2 L). The layers were separated and the aqueous layer was extracted with DCM. The organic layer was dried over $MgSO_4$ and concentrated. The crude oil was purified by flash chromatography (silica gel, 0-100% EtOAc/Hex) to obtain a white solid. 70.6 g (78.5 % yield) of compound **F** was obtained. $^1$H NMR: $\delta_H$ (400 MHz, $CDCl_3$): 7.26-7.17 (m, 3H), 7.12 (dd, 2H), 7.07 (d, 1H), 6.77-6.73 (m, 2H), 4.22 (q, 2H), 3.74 (s, 3H), 3.27-3.16 (m, 2H), 2.97 (s, 0.5H), 2.94 (td, 2.5H), 2.77 (s, 0.5H), 2.68 (t, 1.5H), 2.59-2.51 (m, 1H), 2.42-2.35 (m, 1H), 1.95 (dt, 1H).
LC/MS, *m*/*z* = 349.2 $[M + H]^+$ (Calc: 349.42).

In a similar manner, compound **G** was prepared from compound **E.** Yellow oil (62 % yield)
$^1$H NMR: $\delta_H$ (400 MHz, $CDCl_3$): 7.37-7.27 (m, 5H), 7.17 (d, 1H), 6.86-6.83 (m, 2H), 4.44 (s, 2H), 3.83 (s, 3H), 3.38 (t, 2H), 3.10-3.05 (m, 2H), 2.98 (d, 1H), 2.76-2.70 (m, 3H), 2.04-1.98 (m, 1H), 1.89-1.81 (m, 1H), 1.55-1.49 (m, 2H), 1.19-1.11 (m, 2H). LC/MS, *m*/*z* = 377.2 $[M + H]^+$ (Calc: 377.48).

Potassium *tert*-butoxide solution (1 M in THF, 389 mL, 389 mmol, 2.0 eq) was added slowly to a suspension of methyltriphenylphosphonium bromide (139.0 g, 389 mmol, 2.0 eq) [Sigma-Aldrich] in THF (2 L) at 0 °C. The yellow suspension was stirred for 20 min at 0 °C. A solution of ketone **F** (68 g, 195 mmol, 1.0 eq) in THF (1 L) was slowly added to the suspension at 0 °C. The ice bath was removed and the reaction mixture was stirred overnight. The reaction mixture was quenched with water. DCM/water was added and the layers were separated. The aqueous layer was extracted with DCM and the combined organic layers were dried over $MgSO_4$. The crude oil was purified by flash chromatography (silica gel, 0-100% EtOAc/Hex) to obtain a yellow oil. 60.8 g (90 % yield) of compound **H** was obtained.
$^1$H NMR: $\delta_H$ (400 MHz, $CDCl_3$): 7.36-7.24 (m, 5H), 7.04 (d, 1H), 6.87 (d, 1H), 6.76 (dd, 1H), 5.20 (d, 2H), 4.38 (d, 2H), 3.82 (s, 3H), 3.49-3.43 (m, 1H), 3.19-3.13 (m, 1H), 2.90 (dd, 2H,), 2.88-2.80 (m, 1H), 2.75-2.67 (m, 1H), 2.57-2.50 (m, 1H), 2.46-2.34 (m, 2H), 2.26-2.19 (m, 1H).
LC/MS, *m*/*z* = 347.2 $[M + H]^+$ (Calc: 347.45).

In a similar manner, compound **I** was prepared from compound **G.** Yellow oil (76 % yield)
$^1$H NMR: $\delta_H$ (400 MHz, $CDCl_3$): 7.32-7.23 (m, 5H), 7.01 (d, 1H), 6.77 (d, 1H), 6.71 (dd, 1H), 5.11 (d, 2H), 4.41 (s, 2H), 3.77 (s, 3H), 3.34 (t, 2H), 2.82-2.65 (m, 4H), 2.54-2.41 (m, 2H), 1.98 (td, 1H), 1.90 (td, 1H), 1.52-1.47 (m, 2H), 1.31-1.20 (m, 2H), 0.97-0.83 (m, 2H).
LC/MS, *m*/*z* = 375.2 $[M + H]^+$ (Calc: 375.50).

Lithium aluminum hydride solution (2 M in diethyl ether, 133.0 mL, 266 mmol, 2.1 eq) [Sigma-Aldrich] was added to a solution of nitrile **H** (44 g, 127 mmol, 1.0 eq) in diethylether (1.5 L) at 0 °C. The ice bath was removed and the mixture was stirred overnight. The reaction mixture was quenched with hydrated sodium sulfate (approximately 5 g) and $MgSO_4$ was added. The mixture was filtered through a pad of Celite and the filtrate was concentrated. The crude oil was purified by flash chromatography (silica gel, 0-50% DCM/10% $NH_4OH$ in MeOH) to obtain a yellow oil. 40.5 g (91 % yield) of compound **J** was obtained.
$^1$H NMR: $\delta_H$ (400 MHz, MeOH-$d_4$): 7.31-7.20 (m, 5H), 6.98-6.96 (m, 2H), 6.71 (dd, 1H), 5.10 (d, 2H), 4.34 (s, 2H), 3.79 (s, 3H), 3.43 (qd, 1H), 3.07 (qd, 1H), 2.64 (t, 2H), 2.58-2.51 (m, 1H), 2.43-2.36 (m, 2H), 2.23 (t, 2H), 2.17-2.01 (m, 3H).
LC/MS, m/z = 351.2 $[M + H]^+$ (Calc: 351.48).

In a similar manner, compound **K** was prepared from compound **I**. Yellow oil (80 % yield)
$^1$H NMR: $\delta_H$ (400 MHz, MeOH-$d_4$): 7.34-7.25 (m, 5H), 6.99 (d, 1H), 6.90 (d, 1H), 6.70 (dd, 1H), 5.05 (d, 2H), 4.42 (s, 2H), 3.77 (s, 3H), 3.40 (t, 2H), 2.66 (q, 2H), 2.56 (td, 1H), 2.43 (t, 2H), 2.15 (qd, 1H), 2.04 (td, 2H), 1.88 (dd, 2H), 1.53-1.43 (m, 2H), 1.36-1.26 (m, 1H), 0.99-0.90 (m, 1H).
LC/MS, *m*/*z* = 379.2 $[M + H]^+$ (Calc: 379.00).

TMEDA (2.88 mL, 19 mmol, 2.0 eq) was added to a solution of olelfin **J** (3.40 g, 10 mmol, 1.0 eq) in THF (50 mL) at RT. The reaction mixture was cooled to -78 °C using a dry ice/acetone bath. LDA solution (2 M in THF/heptane/ethylbenzene, 10 mmol, 1.0 eq) was added slowly and the mixture was stirred for 10 min at -78 °C. The cooling bath was removed and the reaction mixture was stirred overnight at RT. The reaction mixture was quenched with water. DCM/water was added and the layers were separated. The aqueous layer was extracted with DCM and the combined organic layers were dried over $MgSO_4$. The crude oil was purified by flash chromatography (silica gel, 0-20% DCM/MeOH then 0-50% DCM/10% $NH_4OH$ in MeOH) to obtain a yellow oil. 2.04 g (60 % yield) of compound **L** was obtained.
$^1$H NMR: $\delta_H$ (400 MHz, MeOH-$d_4$): 7.40-7.25 (m, 5H), 7.11 (d, 1H), 6.93 (d, 1H), 6.83 (dd, 1H), 4.58 (s, 2H), 3.81-3.65 (m, 6H), 3.27-3.22 (m, 1H), 3.10 (dd, 1H), 2.91 (d, 1H), 2.68 (td, 1H), 2.43-2.37 (m, 1H), 2.31 (td, 1H), 2.24-2.17 (m, 1H), 2.13-2.08 (m, 1H), 1.53 (t, 1H), 0.91 (d, 3H).
LC/MS, *m*/*z* = 351.2 $[M + H]^+$ (Calc: 351.48).

In a similar manner, compound **M** was prepared from compound **K**. Yellow oil (50 % yield). NMR data was collected on the TFA salt.
$^1$H NMR: $\delta_H$ (400 MHz, MeOH-$d_4$): 7.35-7.23 (m, 5H), 7.11 (d, 1H), 6.85 (d, 1H), 6.82 (dd, 1H), 4.65 (s, 2H), 3.77 (s,

3H), 3.67 (dd, 1H), 3.58 (t, 2H), 3.25 (dd, 1H), 3.11 (dd, 1H), 2.91 (d, 1H), 2.70 (td, 1H), 2.21-2.12 (m, 2H), 2.01 (td, 1H), 1.81-68 (m, 3H), 1.66-1.52 (m, 2H), 1.34 (dd, 1H), 0.84 (d, 3H).
LC/MS, *m/z* = 379.4 $[M + H]^+$ (Calc: 379.54).

Cyclopropanecarboxaldehyde (0.81 mL, 10.8 mmol, 2.0 eq.) [Sigma-Aldrich] was added to a solution of amine **L** (1.9 g, 5.4 mmol, 1.0 eq) in isopropyl acetate (70 mL) at 0 °C and stirred for 30 min. TFA (1.24 mL, 16 mmol, 3.0 eq) was then added and the mixture was stirred for 10 min at 0 °C. The cooling bath was removed and the reaction mixture was stirred another 30 min at RT. Sodium triacetoxyborohydride (2.29 g, 10.8 mmol, 2.0 eq) [Sigma-Aldrich] was added and the reaction mixture was stirred overnight. The reaction mixture was quenched with water. DCM/water was added and the layers were separated. The aqueous layer was extracted with DCM and organic layer was dried over $MgSO_4$. The crude oil was purified by flash chromatography (silica gel, 0-50% DCM/MeOH) to obtain a yellow oil. 1.8 g (82% yield) of compound **N** was obtained. NMR data was collected on the TFA salt.
$^1H$ NMR: $\delta_H$ (400 MHz, MeOH-$d_4$): 7.31-7.17 (m, 5H), 7.03 (d, 1H), 6.82 (d, 1H), 6.72 (dd, 1H), 4.50 (s, 2H), 3.81-3.65 (m, 6H), 3.27-3.23 (m, 1H), 3.14 (dd, 1.5H), 3.05 (s, 1.5H), 2.91 (dd, 1H), 2.53 (td, 1H), 2.39-2.22 (m, 3H), 2.06-1.98 (m, 1H), 1.45 (d, 1H), 1.06-0.95 (m, 1H), 0.87 (d, 3H), 0.69-0.64 (m, 2H), 0.36-0.30 (m, 2H).
LC/MS, *m/z* = 405.4 $[M + H]^+$ (Calc: 405.57).

A solution of TEA (2.57 mL, 18 mmol, 14 eq) in MeOH (10 mL) was cooled to 0 °C. Formic acid (1 mL, 26.35 mmol, 20 eq) was added dropwise to the solution at 0 °C and stirred for 10 min. Cyclopropanecarboxaldehyde (0.3 mL, 4.0 mmol, 3.0 eq) and a solution of amine **M** (0.5 g, 1.32 mmol, 1.0 eq) in MeOH (10 mL) were added to the mixture of TEA and formic acid. The mixture was and heated to 74 °C overnight. Water was added and pH was adjusted to 9-10 with concentrated $NH_4OH$. The aqueous was extracted with DCM and the organic layer was dried over $MgSO_4$. The crude oil was purified by flash chromatography (silica gel, 0-50% DCM/MeOH) to obtain a yellow oil. 1.8 g (82% yield) of **(6*S*,11*R*)-6-(4-(benzyloxy)butyl)-3-(cyclopropylmethyl)-8-methoxy-11-methyl-1,2,3,4,5,6-hexahydro-2,6-methanobenzo[*d*]azocine (Compound 12**) was obtained.
$^1H$ NMR: $\delta_H$ (400 MHz, DMSO-$d_6$): 7.31-7.8 (m, 5H), 6.92 (d, 1H), 6.68 (d, 1H), 6.61 (dd, 1H), 4.41 (s, 2H), 3.62 (s, 3H), 3.45 (t, 2H), 2.94 (broad, 1H), 2.73 (d, 1H), 2.55-2.47 (m, 1H), 2.40-2.27 (m, 2H), 2.17-2.09 (m, 1H), 1.92-1.75 (m, 3H), 1.63-1.50 (m, 3H), 1.50-1.30 (m, 2H), 0.93 (d, 1H), 0.75-0.66 (m, 1H), 0.63 (d, 3H), 0.36 (d, 2H), 0.07-0.01 (m, 2H).
LC/MS, *m/z* = 433.2 $[M + H]^+$ (Calc: 433.63).

10% Pd/C (0.18 g) was added to a solution of benzyl ether **N** (1.8 g, 4.4 mmol, 1.0 eq) in 20% AcOH in MeOH (100 mL). The mixture was stirred under $H_2$ at 50 psi overnight. The reaction mixture was filtered through a pad of Celite and concentrated. The crude oil was purified by flash chromatography (silica gel, 0-50% DCM/MeOH) to obtain a yellow oil. 1.05 g (75% yield) of compound **O** was obtained.
$^1H$ NMR: $\delta_H$ (400 MHz, MeOH-$d_4$): 7.04 (d, 1H), 6.82 (d, 1H), 6.72 (dd, 1H), 3.75 (s, 23H), 2.95 (d, 1H), 2.83-2.75 (m, 2H), 2.67 (dd, 1H), 2.43 (dd, 1H), 2.20 (d, 3H), 2.08 (sex, 1H), 1.81 (sex, 1H), 1.17 (t, 3H), 0.95 (quin, 1H), 0.84 (d, 3H), 0.60 (d, 2H), 0.25 (qd, 2H).
LC/MS, *m/z* = 315.5 $[M + H]^+$ (Calc: 315.45).

In a similar manner, **4-((6*S*,11*R*)-3-(cyclopropylmethyl)-8-methoxy-11-methyl-1,2,3,4,5,6-hexahydro-2,6-methanobenzo[*d*]azocin-6-yl)butan-1-ol (Compound 13)** was prepared from **Compound 12.** Yellow oil (77 % yield). NMR data was collected on the TFA salt.
$^1H$ NMR: $\delta_H$ (400 MHz, MeOH-$d_4$): 7.03 (d, 1H), 6.81 (d, 1H), 6.73 (dd, 1H), 3.84-3.79 (m, 1H), 3.68 (s, 3H), 3.56 (t, 2H), 3.27 (dd, 1H), 3.15 (dd, 1H), 3.05 (s, 2H), 2.93 (dd, 1H), 2.56 (td, 1H), 2.28-2.16 (m, 2H), 2.02-1.92 (m, 1H), 1.71 (td, 1H), 1.64-1.42 (m, 4H), 1.36 (d, 1H), 1.08-0.98 (m, 1H), 0.84 (d, 3H), 1.67 (d, 2H), 0.36 (d, 2H). LC/MS, *m/z* = 343.3 $[M + H]^+$ (Calc: 343.50).

## EXAMPLE 2

**(E)-methyl 4-((6*S*,11*R*)-3-(cyclopropylmethyl)-8-methoxy-11-methyl-1,2,3,4,5,6-hexahydro-2,6-methanobenzo[*d*]azocin-6-yl)but-2-enoate (Compound 8); 4-((6*S*,11*R*)-3-(cyclopropylmethyl)-8-methoxy-11-methyl-1,2,3,4,5,6-hexahydro-2,6-methanobenzo[*d*]azocin-6-yl)-N-isobutylbutan-1-amine (Compound 9); (2*S*)-3-((6*R*,11*R*)-3-(cyclopropylmethyl)-8-methoxy-11-methyl-1,2,3,4,5,6-hexahydro-2,6-methanobenzo[*d*]azocin-6-yl)propane-1,2-diol (Compound 6); (2*S*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol (Compound 11); (2*S*)-2-(2-((6*R*,11*R*)-3-(cyclopropylmethyl)-8-methoxy-11-methyl-1,2,3,4,5,6-hexahydro-2,6-methanobenzo[d]azocin-6-yl)acetamido)propanamide (Compound 7); N-((*S*)-1-amino-1-oxopropan-2-yl)-4-((6*S*,11*R*)-3-(cyclopropylmethyl)-8-methoxy-11-methyl-1,2,3,4,5,6-hexahydro-2,6-methanobenzo[*d*]azocin-6-yl)butanamide (Compound 14); 2-((6*R*,11*R*)-3-(cyclopropylmethyl)-8-methoxy-11-methyl-1,2,3,4,5,6-hexahydro-2,6-methanobenzo[*d*]azocin-6-yl)acetic acid (Compound 5); 4-((6*S*,11*R*)-3-(cyclopropylmethyl)-8-methoxy-11-methyl-1,2,3,4,5,6-hexahydro-2,6-methanobenzo[d]azocin-6-yl)butanoic acid (Compound 3); and (2*R*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol (Compound 10).**

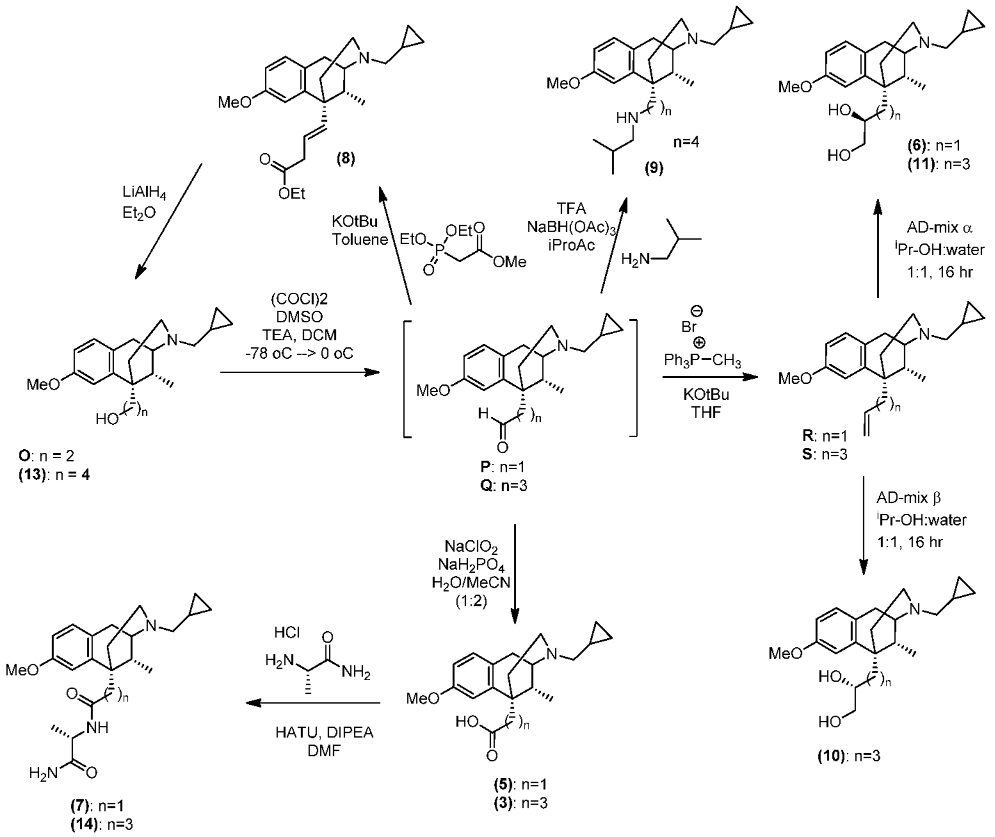


A solution of oxalyl chloride (50 μL, 0.6 mmol, 2.0 eq) in DCM (2 mL) was cooled to - 78 °C. DMSO (80 μL, 1.1 mmol, 4.0 eq) was added at -78 °C and stirred for 15 min. A solution of alcohol **O** (90 mg, 0.3 mmol, 1.0 eq) in DCM (2 mL) was added at the same temperature and stirred for 15 min. TEA (160 μL, 1.1 mmol, 4.0 eq) was added at the same temperature and stirred for 15 min. The reaction mixture was warmed to 0 °C and stirred an additional 10 min. Once the reaction was complete, DCM was removed under vacuum to obtain the crude aldehyde **P.** Aldehyde **P** was then dissolved in toluene (2 mL) and was added to a mixture of $KO^tBu$ (1 M in THF, 570 μL, 0.6 mmol, 2.0 eq) and methyltriphenylphosphonium bromide (210 mg, 0.6 mmol, 2.0 eq) in toluene (2 mL) at 0 °C. The mixture was heated to reflux for 2 hr. The mixture was cooled to room temperature and quenched with water. DCM/water was added and the layers were separated. The aqueous layer was extracted with DCM. The combined organic layers were dried over $MgSO_4$ and concentrated. The crude oil was purified by flash chromatography (silica gel, 0-50% DCM/MeOH) to obtain a yellow oil. 63 mg (70 % yield) of compound **R** was obtained.

$^1H$ NMR: $\delta_H$ (400 MHz, $CDCl_3$): 6.88 (d, 1H), 6.72 (d, 1H), 6.57 (dd, 1H), 5.99-5.89 (m, 1H), 5.06 (t, 2H), 3.67 (s, 3H),

3.06-3.01 (m, 1H), 2.76 (d, 1H), 2.65-2.47 (m, 3H), 2.46-2.33 (m, 2H), 2.22 (dd, 1H), 2.03 (td, 1H), 1.97-1.86 (m, 2H or 3H), 1.06 (dt, 1H), 0.74 (d, 3H), 0.39 (dd, 2H), 0.01 (d, 2H).
LC/MS, *m/z* = 311.4 $[M + H]^+$ (Calc: 311.46).

In a similar manner, compound **S** was prepared from crude aldehyde **Q** that was prepared from **Compound 12.** Crude **S** was purified by reverse-phase prep HPLC (C18, 0-100% 0.1 % TFA in water/0.1 % TFA in ACN) and obtained a white foam (10 % yield).
$^1$H NMR: $\delta_H$ (400 MHz, MeOH-$d_4$): 7.08 (d, 1H), 6.78 (d, 1H), 6.73 (dd, 1H), 5.86-5.75 (m, 1H), 5.00 (dq, 1H), 4.91 (d, 1H), 3.83-3.79 (m, 1H), 3.67 (s, 3H), 3.27 (dd, 1H), 3.15 (dd, 1H), 3.05 (s, 2H), 2.93 (dd, 1H), 2.54 (td, 1H), 2.29-2.05 (m, 4H), 1.95 (td, 1H), 1.67 (td, 1H), 1.56-1.40 (m, 2H), 1.33 (d, 1H), 1.07-0.97 (m, 2H), 0.83 (d, 3H), 0.66 (d, 2H), 0.35 (d, 2H).
LC/MS, *m/z* = 339.5 $[M + H]^+$ (Calc: 339.51).

A suspension of olefin **R** (17 mg, 54 μmol, 1.0 eq) and AD-mix α (74 mg, 1.36 g/mmol) [Sigma-Aldrich] in isopropyl alcohol/water (1:1, 1 mL) was stirred at RT overnight. The mixture was filtered through a pad of Celite and concentrated. The crude mixture was purified by reverse-phase prep HPLC (C18, 0-100% 0.1 % TFA in water/0.1 % TFA in ACN) to obtain a white foam. 15 mg (83 % yield) of **(2*S*)-3-((6*R*,11*R*)-3-(cyclopropylmethyl)-8-methoxy-11-methyl-1,2,3,4,5,6-hexahydro-2,6-methanobenzo[*d*]azocin-6-yl)propane-1,2-diol (Compound 6**) was prepared.
$^1$H NMR: $\delta_H$ (400 MHz, MeOH-$d_4$): 7.04 (d, 1H), 6.84 (dd, 1H), 6.72 (dd, 1H), 3.94-3.78 (2H), 3.68 (s, 3H), 3.52-3.32 (m, 2H), 3.18-3.10 (m, 1H), 3.08-2.74 (m, 3H), 2.62-2.42 (m, 2H), 2.34-2.14 (m, 1H), 2.08-1.86 (m, 1H), 1.72 (dd, 1H), 1.42 (t, 1H), 1.08-0.98 (m, 1H), 0.96 (d, 3H), 0.67 (d, 2H), 0.36 (d, 2H).
LC/MS, *m/z* = 345.2 $[M + H]^+$ (Calc: 345.48).

In a similar manner, **(2*S*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol (Compound 11)** was prepared from compound **S.** White foam (82 % yield)
$^1$H NMR: $\delta_H$ (400 MHz, MeOH-$d_4$): 7.03 (d, 1H), 6.82 (d, 1H), 6.73 (dd, 1H), 3.84-3.79 (m, 1H), 3.68 (s, 3H), 3.63-3.54 (m, 1H), 3.42-3.38 (m, 2H), 3.27 (dd, 1H), 3.15 (dd, 1H), 3.05 (s, 2H), 2.93 (dd, 1H), 2.56 (td, 1H), 2.30-2.15 (m, 2H), 2.05-1.85 (m, 1H), 1.80-1.32 (m, 6H), 1.08-0.97 (m, 1H), 0.85 (d, 3H), 0.67 (d, 2H), 0.35 (d, 2H). LC/MS, *m/z* = 373.4 $[M + H]^+$ (Calc: 373.53).

A suspension of olefin **S** (20 mg, 64 μmol, 1.0 eq) and AD-mix β (87 mg, 1.36 g/mmol) [Sigma-Aldrich] in isopropyl alcohol/water (1:1, 1 mL) was stirred overnight. The mixture was filtered through a pad of Celite and concentrated. The crude mixture was purified by reverse-phase prep HPLC (C18, 0-100% 0.1 % TFA in water/0.1 % TFA in ACN) to obtain a white foam. 18 mg (81 % yield) of **(2*R*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol (Compound 10)** was prepared.
$^1$H NMR: $\delta_H$ (400 MHz, MeOH-$d_4$): 7.03 (d, 1H), 6.82 (d, 1H), 6.73 (dd, 1H), 3.82-3.78 (m, 1H), 3.67 (s, 3H), 3.62-3.54 (m, 1H), 3.42-3.38 (m, 2H), 3.27 (dd, 1H), 3.15 (dd, 1H), 3.05 (s, 2H), 2.93 (dd, 1H), 2.56 (td, 1H), 2.30-2.16 (m, 2H), 2.04-1.86 (m, 1H), 1.78-1.32 (m, 6H), 1.08-0.97 (m, 1H), 0.85 (d, 3H), 0.67 (d, 2H), 0.35 (d, 2H). LC/MS, *m/z* = 373.2 $[M + H]^+$ (Calc: 373.53).

A solution of sodium dihydrogen phosphate (103 mg, 0.9 mmol, 3.0 eq) in water (1 mL) was added to a solution of crude aldehyde **P** in ACN (1 mL) at 0 °C. A solution of sodium chlorite (78 mg, 0.9 mmol, 3.0 eq) in water (1 mL) was added to the mixture at the same temperature and the cooling bath was removed. The mixture was stirred for 1 hr and concentrated under vacuum. The crude mixture was purified by reverse-phase prep HPLC (C18, 0-100% 0.1 % TFA in water/0.1 % TFA in ACN) to obtain a white foam. 73 mg (78 % yield) of **2-((6*R*,11*R*)-3-(cyclopropylmethyl)-8-methoxy-11-methyl-1,2,3,4,5,6-hexahydro-2,6-methanobenzo[*d*]azocin-6-yl)acetic acid (Compound 5**) was prepared.
$^1$H NMR: $\delta_H$ (400 MHz, MeOH-$d_4$): 7.05 (d, 1H), 6.69-6.72 (m, 2H), 3.84-3.80 (m, 1H), 3.68 (s, 3H), 3.31-3.25 (dd, 1H), 3.15 (dd, 1H), 3.10-3.04 (m, 3H), 2.91 (dd, 1H), 2.74-2.50 (m, 4H), 1.58 (d, 1H), 1.05-0.97 (m, 1H), 0.95 (d, 3H), 0.66 (d, 2H), 0.34 (d, 2H).
LC/MS, *m/z* = 329.1 $[M + H]^+$ (Calc: 329.43).

In a similar manner, **4-((6*S*,11*R*)-3-(cyclopropylmethyl)-8-methoxy-11-methyl-1,2,3,4,5,6-hexahydro-2,6-methanobenzo[*d*]azocin-6-yl)butanoic acid (Compound 3)** was prepared from crude compound **Q.** White foam (82 % yield)
$^1$H NMR: $\delta_H$ (400 MHz, MeOH-$d_4$): 7.03 (d, 1H), 6.79 (d, 1H), 6.73 (dd, 1H), 3.84-3.79 (m, 1H), 3.68 (s, 3H), 3.27 (dd, 1H), 3.15 (dd, 1H), 3.05 (s, 2H), 2.93 (dd, 1H), 2.55 (td, 1H), 2.41-2.16 (m, 4H), 2.00-1.92 (m, 1H), 1.76-1.63 (m, 3H), 1.36 (d, 1H), 1.07-0.97 (m, 1H), 0.86 (d, 3H), 0.67 (d, 2H), 0.35 (d, 2H).
LC/MS, *m/z* =357.2 $[M + H]^+$ (Calc: 357.49).

DIPEA (20 μL, 134 μmol, 2.2 eq) was added to the mixture of **Compound 5** (20 mg, 61 μmol, 1.0 eq) and HATU (30 mg, 91 μmol, 1.5 eq) in DMF (1 mL) and stirred for 5 min. A mixture of DIPEA (20 μL, 134 μmol, 2.2 eq) and Ala-$NH_2$.HCl (10 mg, 91 μmol, 1.5 eq) in DMF (1 mL) was added to the activated acid solution. The mixture was stirred overnight. DMF was removed under vacuum and the residue purified by reverse-phase prep HPLC (C18, 0-100% 0.1 % TFA in water/0.1 % TFA in ACN) to obtain a white foam. 15 mg (75 % yield) of compound **(2*S*)-2-(2-((6*R*,11*R*)-**

**3-(cyclopropylmethyl)-8-methoxy-11-methyl-1,2,3,4,5,6-hexahydro-2,6-methanobenzo[d]azocin-6-yl)acetamido)propanamide (Compound 7)** was prepared. $^1$H NMR: $\delta_H$ (400 MHz, MeOH-d$_4$): 7.04 (d, 1H), 6.82-6.72 (m, 2H), 4.28 (q, 1H), 3.82-3.76 (m, 1H), 3.68 (s, 3H), 3.32-3.22 (m, 1H), 3.18-3.10 (m, 1H), 3.08-3.02 (m, 2H), 3.01-2.84 (m, 3H), 2.78-2.66 (m, 1H), 2.62 (d, 1H), 2.58-2.48 (m, 2H), 1.54 (d, 1H), 1.30 (dd, 3H), 0.98 (dd, 3H), 0.66 (d, 2H), 0.34 (d, 2H). LC/MS, *m/z* = 399.4 [M + H]$^+$ (Calc: 399.53).

In a similar manner, compound **N-((*S*)-1-amino-1-oxopropan-2-yl)-4-((6*S*,11*R*)-3-(cyclopropylmethyl)-8-methoxy-11-methyl-1,2,3,4,5,6-hexahydro-2,6-methanobenzo[d]azocin-6-yl)butanamide (Compound 14)** was prepared from **Compound 3.** White foam (70 % yield)
$^1$H NMR: $\delta_H$ (400 MHz, MeOH-d$_4$): 7.14 (d, 1H), 6.93 (dd, 1H), 6.83 (ddd, 1H), 4.38 (dd, 1H), 3.95-3.90 (m, 1H), 3.79 (s, 3H), 3.39 (dd, 1H), 3.26 (dd, 1H), 3.17 (s, 2H), 3.04 (dd, 1H), 2.66 (td, 1H), 2.45-2.27 (m, 4H), 2.13-2.02 (m, 1H), 1.86-1.75 (m, 3H), 1.46 (dd, 1H), 1.37 (dd, 3H), 1.19-1.09 (m, 1H), 0.96 (d, 3H), 0.77 (d, 2H), 0.46 (d, 2H).
LC/MS, *m/z* = 427.3 [M + H]$^+$ (Calc: 427.58).

KOtBu solution (1 M in THF, 3.51 mL, 3.5 mmol, 2.0 eq) was added to a suspension of methyltriphenylphosphonium bromide (0.74 g, 3.5 mmol, 2.0 eq) in toluene (15 mL) at 0 °C and stirred for 15 min. A solution of crude aldehyde **P** (550 g, 1.7 mmol, 1.0 eq) in toluene (15 mL) was added to the mixture at 0 °C. The mixture was heated to reflux for 2 hr. The mixture was cooled to room temperature and quenched with water. DCM/water was added and the layers were separated. The aqueous layer was extracted with DCM. The combined organic layers were dried over $MgSO_4$ and concentrated. The crude oil was purified by flash chromatography (silica gel, 0-50% DCM/MeOH) to obtain a yellow oil. 0.54 g (83 % yield) of **(*E*)-methyl 4-((6*S*,11*R*)-3-(cyclopropylmethyl)-8-methoxy-11-methyl-1,2,3,4,5,6-hexahydro-2,6-methanobenzo[d]azocin-6-yl)but-2-enoate (Compound 8)** was obtained.
$^1$H NMR: $\delta_H$ (400 MHz, CDCl$_3$): 11.8 (broad, 1H), 7.03-6.93 (m, 2H), 6.74 (d, 1H), 6.72 (d, 1H), 5.92 (d, 1H), 3.89-3.2 (m, 1H), 3.72 (s, 3H), 3.68 (s, 3H), 3.43 (d, 1H), 3.09 (dd, 1H), 3.00-2.87 (m, 2H), 2.84 (d, 1H), 2.75 (dd, 1H), 2.62 (dd, 1H), 2.46-2.32 (m, 3H), 1.37 (d, 1H), m, 1.10-1.00 (m, 1H), 0.87 (d, 3H), 0.68 (d, 2H), 0.37-0.27 (m, 2H).
LC/MS, *m/z* = 369.2 [M + H]$^+$ (Calc: 369.5).

$LiAlH_4$ solution (2 M in diethyl ether, 2.03 mL, 4.0 mmol, 3.0 eq) was added to a solution of **Compound 8** (500 mg, 1.3 mmol, 1.0 eq) in ether (25 mL) at 0 °C. The ice bath was removed and the mixture was heated to reflux overnight. The mixture was cooled to room temperature and quenched with water. DCM/water was added and the layer was separated. The aquoes layer was extracted with DCM. The combined organic layers were dried over MgSO4 and concentrated. The crude oil was purified by flash chromatography (silica gel, 0-50% DCM/MeOH) to obtain a yellow oil. 300 mg (65% yield) of **Compound 13** was obtained.
NMR and LCMS data: Refer Example 1.

Isobutylamine (0.12 mL, 1.2 mmol, 2.0 eq) was added to a solution of crude aldehyde **Q** (0.2 g, 0.59 mmol, 1.0 eq) in isopropyl acetate (10 mL) at 0 °C and stirred for 30 min. TFA (0.13 mL, 1.8 mmol, 3.0 eq) was added to the mixture and stirred for 10 min at 0 °C and an additional 30 min at room temperature. Sodium triacetoxyborohyde (0.25 g, 1.2 mmol, 2.0 eq) was added and stirred for 2 hr. The reaction mixture was quenched with water and basified with sat. aq. sodium bicarbonate. The organic layers were separated and the aqueous layer was extracted with DCM. The combined organic layers were dried over $MgSO_4$ and concentrated. The crude oil was purified by reverse-phase prep HPLC (C18, 0-100% 0.1 % TFA in water/0.1 % TFA in ACN) to obtain a white foam. 0.19 g (83 % yield) of **4-((6*S*,11*R*)-3-(cyclopropylmethyl)-8-methoxy-11-methyl-1,2,3,4,5,6-hexahydro-2,6-methanobenzo[*d*]azocin-6-yl)-N-isobutylbutan-1-amine (Compound 9)** was prepared.
$^1$H NMR: $\delta_H$ (400 MHz, MeOH-d$_4$): 7.15 (d, 1H), 6.92 (d, 1H), 6.85 (dd, 1H), 3.96-3.91 (m, 1H), 3.79 (s, 3H), 3.40 (dd, 1H), 3.27 (dd, 1H), 3.17 (s, 2H), 3.15-3.00 (m, 3H), 2.90 (d, 2H), 2.67 (td, 1H), 2.44-2.29 (m, 2H), 2.16-1.99 (m, 2H), 1.91-1.79 (m, 3H), 1.65-1.51 (m, 2H), 1.46 (d, 1H), 1.20-1.10 (m, 1H), 1.07 (d, 6H), 0.95 (d, 3H), 0.78 (d, 2H), 0.47 (d, 2H).
LC/MS, *m/z* = 398.3 [M + H]$^+$ (Calc: 398.62).

## EXAMPLE 3

**4-((6*S*,11*R*)-3-(cyclopropylmethyl)-8-hydroxy-11-methyl-1,2,3,4,5,6-hexahydro-2,6-methanobenzo[d]azocin-6-yl)butanoic acid (Compound 4); 4-((6*S*,11*R*)-3-(cyclopropylmethyl)-8-hydroxy-11-methyl-1,2,3,4,5,6-hexahydro-2,6-methanobenzo[*d*]azocin-6-yl)butanamide (Compound 2); and (2*S*)-2-(2-((6*R*,11*R*)-3-(cyclopropylmethyl)-8-hydroxy-11-methyl-1,2,3,4,5,6-hexahydro-2,6-methanobenzo[*d*]azocin-6-yl)acetamido)propanamide (Compound 1).**

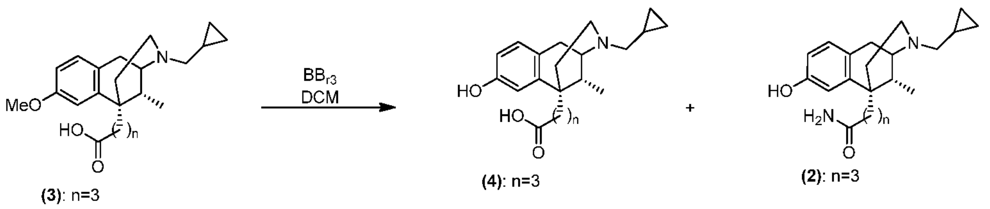


(3): n=3 (4): n=3 + (2): n=3

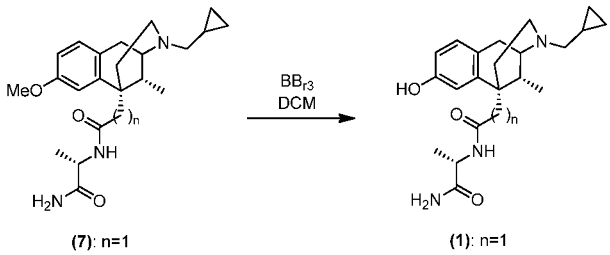


(7): n=1 (1): n=1

A solution of **Compound 3** (50 mg, 0.13 mmol, 1.0 eq) in DCM (1mL) was cooled to - 78 °C. $BBr_3$ solution (1 M in DCM, 250 μL, 0.25 mmol, 2.0 eq) was added dropwise at same temperature. The cooling bath was removed the reaction mixture was stirred for 1 hr. The reaction mixture was quenched with water and basified with $NH_4OH$. Crude **4-((6*S*,11*R*)-3-(cyclopropylmethyl)-8-hydroxy-11-methyl-1,2,3,4,5,6-hexahydro-2,6-methanobenzo[*d*]azocin-6-yl)butanoic acid (Compound 4)** and **4-((6*S*,11*R*)-3-(cyclopropylmethyl)-8-hydroxy-11-methyl-1,2,3,4,5,6-hexahydro-2,6-methanobenzo[*d*]azocin-6-yl)butanamide (Compound 2)** were concentrated and purified by reverse-phase prep HPLC (C18, 0-100% 0.1 % TFA in water/0.1 % TFA in ACN) to obtain a white foam. 16 mg (40 % yield) of **4-((6*S*,11*R*)-3-(cyclopropylmethyl)-8-hydroxy-11-methyl-1,2,3,4,5,6-hexahydro-2,6-methanobenzo[d]azocin-6-yl)butanoic acid (Compound 4)** and 15 mg (40 % yield) of **4-((6*S*,11*R*)-3-(cyclopropylmethyl)-8-hydroxy-11-methyl-1,2,3,4,5,6-hexahydro-2,6-methanobenzo[*d*]azocin-6-yl)butanamide (Compound 2)** were prepared.

$^1H$ NMR of **Compound 4:** $\delta_H$ (400 MHz, MeOH-$d_4$): 7.06 (d, 1H), 6.82 (d, 1H), 6.71 (dd, 1H), 3.97-3.91 (m, 1H), 3.71 (dd, 1H), 3.27 (dd, 1H), 3.16 (s, 2H), 3.07 (dd, 1H), 2.71 (td, 1H), 2.56-2.30 (m, 4H), 2.08-1.97 (m, 1H), 1.89-1.74 (m, 3H), 1.46 (d, 1H), 1.21-1.10 (m, 1H), 1.00 (d, 3H), 0.80 (d, 2H), 0.48 (d, 2H).

LC/MS, *m/z* = 343.2 $[M + H]^+$ (Calc: 343.46).

$^1H$ NMR of **Compound 2:** $\delta_H$ (400 MHz, MeOH-$d_4$): 7.04 (d, 1H), 6.80 (d, 1H), 6.69 (dd, 1H), 3.93-3.87 (m, 1H), 3.37 (dd, 1H), 3.27 (dd, 1H), 3.18-3.00 (m, 3H), 2.80-2.61 (m, 1H), 2.40-2.27 (m, 4H), 2.05-1.94 (m, 1H), 1.85-1.74 (m, 3H), 1.42 (d, 1H), 1.19-1.09 (m, 1H), 0.96 (d, 3H), 0.77 (d, 2H), 0.46 (d, 2H).

LC/MS, *m/z* = 342.2 $[M + H]^+$ (Calc: 342.48).

In a similar manner, **(2*S*)-2-(2-((6*R*,11*R*)-3-(cyclopropylmethyl)-8-hydroxy-11-methyl-1,2,3,4,5,6-hexahydro-2,6-methanobenzo[*d*]azocin-6-yl)acetamido)propanamide (Compound 1)** was prepared from **Compound 7.** White foam (75 % yield)

$^1H$ NMR: $\delta_H$ (400 MHz, MeOH-$d_4$): 7.06 (d, 1H), 6.77 (dd, 1H), 6.71 (dd, 1H), 4.38 (q, 1H), 3.91-3.86 (m, 1H), 3.42-3.33 (m, 1H), 3.30-3.20 (m, 1H), 3.15-2.92 (m, 4H), 2.84 (qd, 1H), 2.74-2.55 (m, 3H), 1.64 (t, 1H), 1.42 (dd, 3H), 1.15-0.97 (m, 4H), 0.78 (d, 2H), 0.45 (d, 2H).

LC/MS, *m/z* = 385.2 $[M + H]^+$ (Calc: 385.50).

## EXAMPLE 4

**(2*R*,6*S*,11*S*)-3-(cyclopropylmethyl)-6-(3-(furan-2-yl)propyl)-8-methoxy-11-methyl-1,2,3,4,5,6-hexahydro-2,6-methanobenzo[*d*]azocine (Compound 15).**

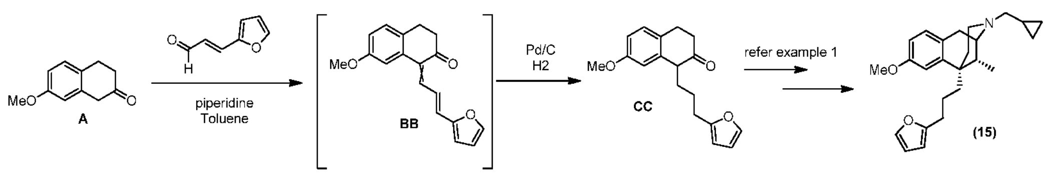


A BB CC (15)

To a mixture of 7-methoxy-2-tetralone **A** (1.828 g, 10.37 mmol, 1.0 eq) [Sigma-Aldrich] in toluene (40 mL) was added (*E*)-3-(furan-2-yl)acrylaldehyde (1.274 g, 10.43 mmol, 1.1 eq) [Sigma-Aldrich] and catalytic piperidine (0.025 mL, 0.25 mmol, 0.02 eq). A Dean-Starke condenser was installed and the reaction heated to reflux overnight. After cooling, the reaction mixture was transferred to a Parr hydrogenation bottle along with EtOH (25 mL), EtOAc (25 mL) and 10% Pd/C (0.213 g). The mixture was hydrogenated at 50psi for 2 hours. The catalyst was filtered off over a pad of celite, rinsed with additional EtOAc and the filtrate evaporated in vacuo to give the crude **CC.** LC/MS: *m*/*z*= 285.2 $[M+H]^+$. (Calc: 284.35).

In a similar manner to preparation of **Compound 12** (see example 1), compound **CC** was carried on to get **(2*R*,6*S*,11*S*)-3-(cyclopropylmethyl)-6-(3-(furan-2-yl)propyl)-8-methoxy-11-methyl-1,2,3,4,5,6-hexahydro-2,6-methanobenzo[*d*]azocine (Compound 15).**

$^1$H NMR: $\delta_H$ (400 MHz, MeOH-$d_4$): 7.27 (s, 1H), 7.01 (d, 1H), 6.79 (d, 1H), 6.72 (dd, 1H), 6.21 (d, 1H), 6.01 (d, 1H), 3.75 (br, 1H), 3.66 (s, 3H), 3.31-3.28 (m, 1H), 3.14 (dd, 1H), 3.09-2.88 (m, 3H), 2.72-2.48 (m, 3H), 2.25-2.13 (m, 2H), 1.95 (td, 1H), 1.79-1.60 (m, 3H), 1.35 (d, 1H), 1.09-0.93 (m, 1H), 0.74 (d, 3H), 0.63 (d, 2H), 0.31 (d, 2H). LC/MS, *m*/*z* =379.4 $[M + H]^+$ (Calc: 379.54).

## EXAMPLE 5

The following Tables provide results on the efficacy of binding and activity response of exemplified Compounds of the Invention at the ORL1, μ-, δ- and κ-opioid receptors.

In TABLE 1, binding affinity of certain Compounds of the Invention to the ORL-1, μ-, δ- and κ-opioid receptors was determined as described above.

In TABLE 2, activity response of certain Compounds of the Invention to the ORL-1, μ-, δ- and κ-opioid receptors was determined as described above for functional assays.

**TABLE 1: Binding Affinity of Benzomorphan Analog Compounds**

| Ref. No. | Compound | $K_i$ (nM) | | | |
|---|---|---|---|---|---|
| | | ORL-1 | Opioid Receptor | | |
| | | | μ | κ | δ |
| 1 | 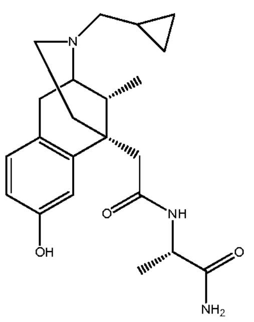 | | 31.65 ± 6.24 | 1.48 ± 0.27 | |
| 2 | 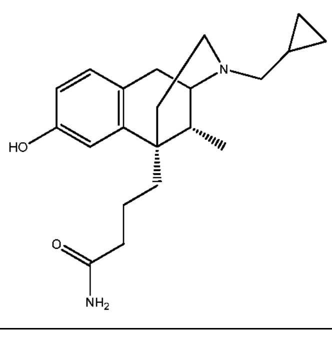 | | 10.72 ± 1.82 | 0.49 ± 0.11 | |

(continued)

| Ref. No. | Compound | Ki (nM) | | | |
|---|---|---|---|---|---|
| | | ORL-1 | Opioid Receptor | | |
| | | | μ | κ | δ |
| **3** | 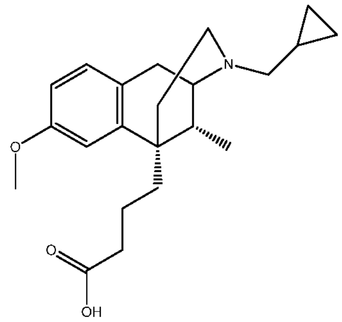 | | 1108.16 ± 32.59 | 137.02 ± 41.88 | |
| **4** | 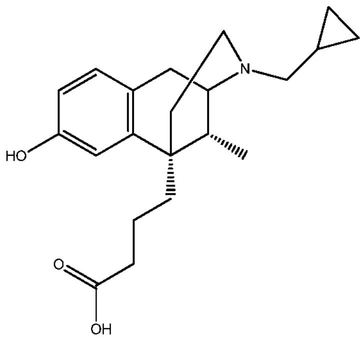 | | 7.61 ± 2.44 | 2.52 ± 0.23 | |
| **5** | 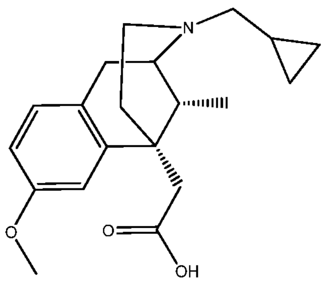 | | | 4459.72 ± 1847.96 | |
| **6** | 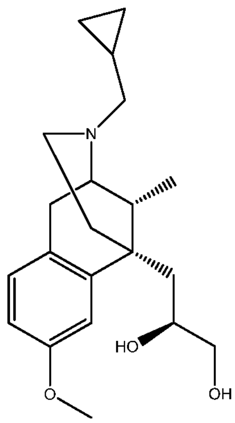 | | 3342.88 ± 895.27 | 46.42 ± 9.70 | |

(continued)

| Ref. No. | Compound | Ki (nM) | | | |
|---|---|---|---|---|---|
| | | ORL-1 | Opioid Receptor | | |
| | | | μ | κ | δ |
| 7 | 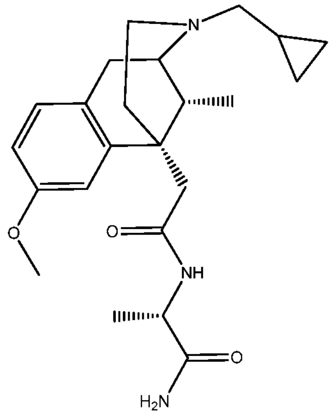 | | | 192.68 ± 68.71 | |
| 8 | 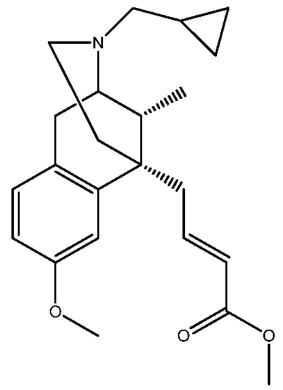 | | 1814.44 ± 552.58 | 3.21 ± 0.76 | |
| 9 | 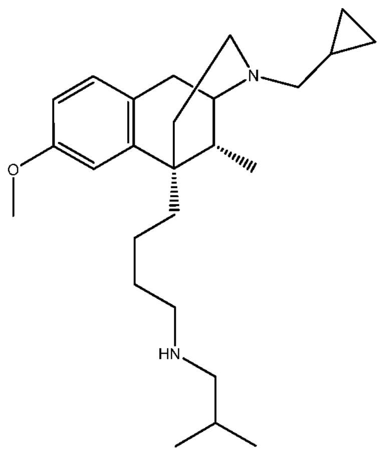 | | 447.87 ± 113.37 | 1.35 ± 0.57 | |

(continued)

| Ref. No. | Compound | Ki (nM) | | | |
|---|---|---|---|---|---|
| | | ORL-1 | Opioid Receptor | | |
| | | | μ | κ | δ |
| **10** | 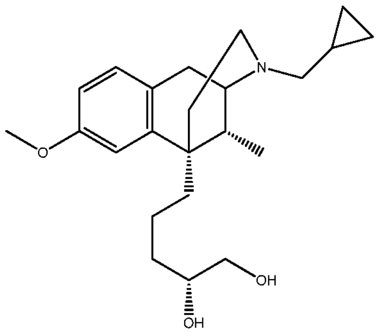 | | 3085.23 ± 922.48 | 6.68 ± 0.61 | |
| **11** | 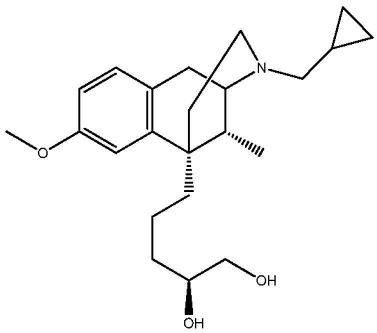 | 13112.60 ± 1061.73 | 234.97 ± 17.25 | 3.23 ± 0.50 | 4984.83 ± 456.22 |
| **12** | 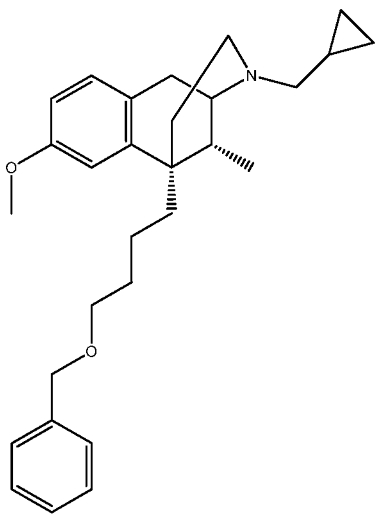 | | 165.52 ± 51.80 | 0.27 ± 0.01 | |

(continued)

| Ref. No. | Compound | $K_i$ (nM) | | | |
|---|---|---|---|---|---|
| | | ORL-1 | Opioid Receptor | | |
| | | | μ | κ | δ |
| **13** | 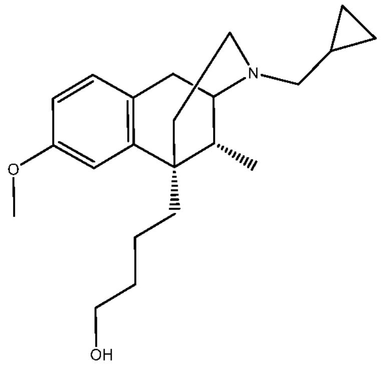 | | 3418.58 ± 763.07 | 7.15 ± 0.86 | 16203.00 ± 3047.33 |
| **14** | 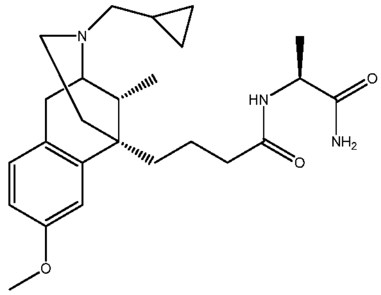 | | 4272.59 ± 1359.78 | 44.04 ± 17.70 | |
| **15** | 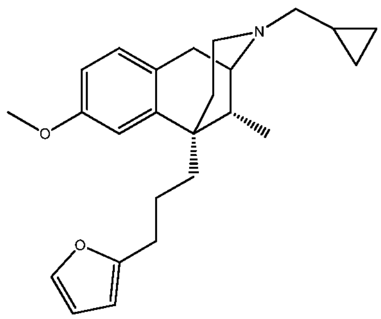 | | 388.91 ± 90.22 | 2.11 ± 0.22 | 735.70 ± 195.54 |
| **16** | 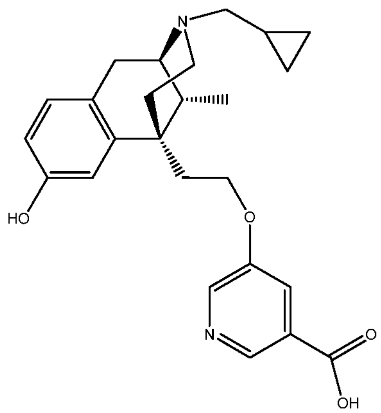 | | 6.85 ± 1.48 | 12.63 ± 2.11 | 62.48 ± 11.02 |

(continued)

| Ref. No. | Compound | $K_i$ (nM) | | | |
|---|---|---|---|---|---|
| | | ORL-1 | Opioid Receptor | | |
| | | | μ | κ | δ |
| **17** | 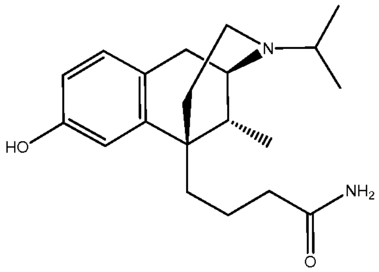 | | 165.47 ± 6.97 | 5.00 ± 0.53 | |
| **18** | 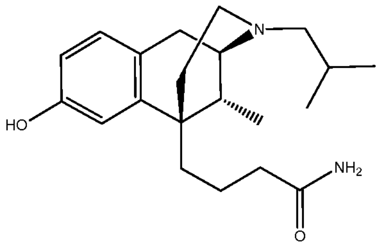 | | 19.84 ± 0.68 | 0.51 ± 0.05 | 155.39 ± 44.70 |
| **19** | 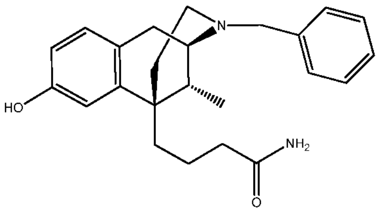 | | 2.65 ± 0.36 | | |
| **20** | 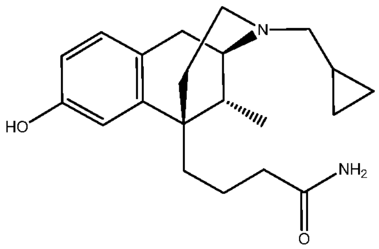 | | 5.38 ± 0.43 | 0.08 ± 0.01 | |
| **21** | 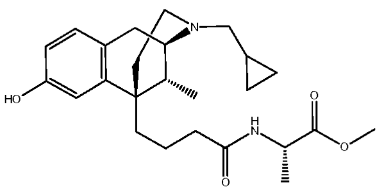 | 2431.45 ± 717.58 | 5.05 ± 1.34 | 0.13 ± 0.02 | 8.81 ± 0.71 |
| **22** | 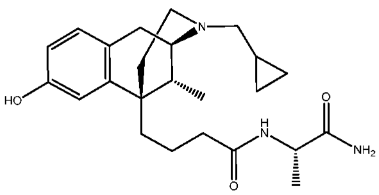 | | 6.33 ± 1.54 | 0.41 ± 0.08 | |

(continued)

| Ref. No. | Compound | $K_i$ (nM) | | | |
|---|---|---|---|---|---|
| | | ORL-1 | Opioid Receptor | | |
| | | | μ | κ | δ |
| 23 | 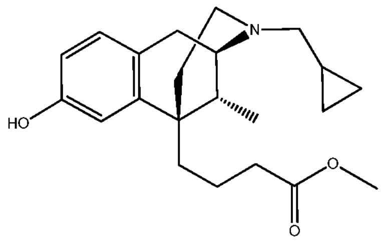 | 2240.44 ± 285.39 | 1.16 ± 0.36 | 0.07 ± 0.01 | 32.60 ± 2.97 |
| 24 | 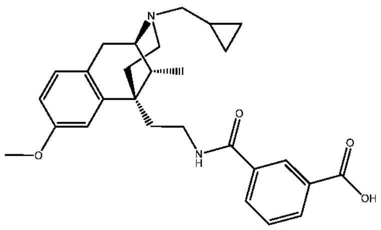 | | | 129.60 ± 26.47 | |
| 25 | 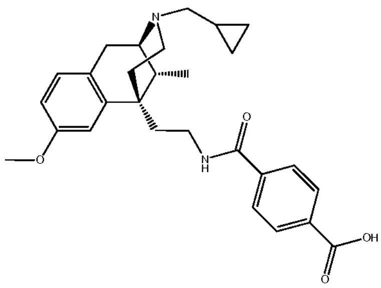 | | | 479.18 ± 36.31 | |
| 26 | 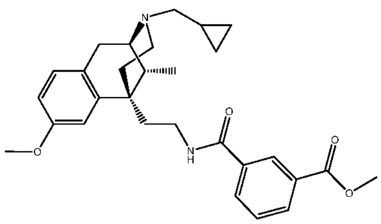 | | 157.80 ± 44.32 | 0.51 ± 0.07 | |
| 27 | 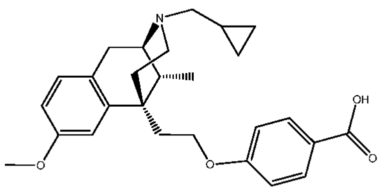 | | | 330.02 ± 70.12 | |
| 28 | 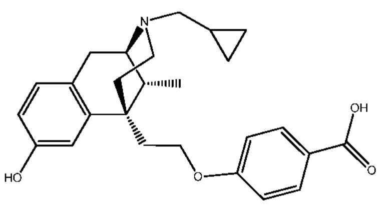 | | | 5.75 ± 1.04 | |

(continued)

| Ref. No. | Compound | Ki (nM) | | | |
|---|---|---|---|---|---|
| | | ORL-1 | Opioid Receptor | | |
| | | | μ | κ | δ |
| 29 | 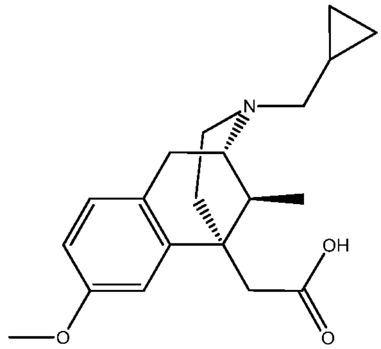 | | | > 20 μM | |
| 30 | 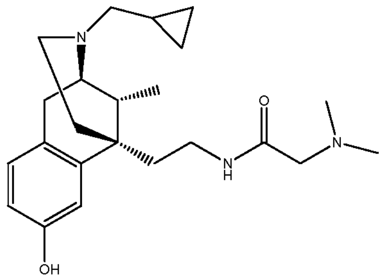 | | | 0.05 ± 0.02 | |
| 31 | 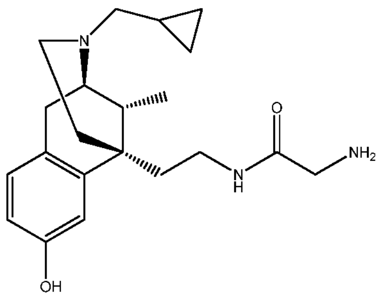 | | | 0.07 ± 0.01 | |

**TABLE 2: Activity Response of Benzomorphan Analog Compounds**

| Ref. No. | GTPγS ($EC_{50}$: nM, $E_{max}$: %) | | | | | | | |
|---|---|---|---|---|---|---|---|---|
| | ORL-1 | | Opioid Receptor | | | | | |
| | | | μ | | κ | | δ | |
| | $EC_{50}$ | $E_{max}$ | $EC_{50}$ | $E_{max}$ | $EC_{50}$ | $E_{max}$ | $EC_{50}$ | $E_{max}$ |
| 1 | | | > 20 μM | | 8.91 ±0.83 | 31.33 ± 0.67 | | |
| 2 | | | > 20 μM | | 8.98 ± 0.82 | 67.33 ± 4.10 | | |
| 3 | | | > 20 μM | | 3474.64 ± 361.44 | 80.67 ± 1.20 | | |
| 4 | | | > 20 μM | | 24.83 ± 2.84 | 67.33 ± 1.86 | | |
| 5 | | | > 20 μM | | | | | |

(continued)

| Ref. No. | GTPγS ($EC_{50}$: nM, $E_{max}$: %) | | | | | | | |
|---|---|---|---|---|---|---|---|---|
| | ORL-1 | | Opioid Receptor | | | | | |
| | | | μ | | κ | | δ | |
| | $EC_{50}$ | $E_{max}$ | $EC_{50}$ | $E_{max}$ | $EC_{50}$ | $E_{max}$ | $EC_{50}$ | $E_{max}$ |
| 6 | | | > 20 μM | | 3089.86 ± 691.66 | 69.33 ± 0.33 | | |
| 7 | | | > 20 μM | | 5004.64 ± 1174.09 | 29.67 ± 2.33 | | |
| 8 | | | 561.53 ± 69.64 | 46.33 ± 2.33 | 123.04 ± 14.76 | 99.33 ± 4.70 | | |
| 9 | | | 642.57 ± 200.09 | 60.00 ± 8.19 | 58.15 ± 4.07 | 102.67 ± 0.88 | | |
| 10 | | | | | 94.32 ± 15.67 | 95.00 ± 8.00 | | |
| 11 | | | > 20 μM | | 209.60 ± 51.12 | 81.00 ± 7.23 | | |
| 12 | | | 579.67 ± 210.31 | 30.75 ± 3.59 | 14.45 ± 1.31 | 97.67 ± 8.35 | | |
| 13 | | | | | 296.18 ± 26.71 | 71.33 ± 3.71 | | |
| 14 | | | 1303.40 ± 343.40 | 64.00 ± 5.31 | 1052.64 ± 199.11 | 63.00 ± 3.61 | | |
| 15 | | | 302.93 ± 47.70 | 34.60 ± 2.48 | 104.45 ± 24.91 | 82.33 ± 1.76 | 142.86 ± 19.97 | 67.00 ± 1.15 |
| 16 | | | 8.95 ± 0.55 | 26.00 ± 3.00 | 67.91 ± 11.18 | 60.67 ± 5.70 | 2.48 ± 0.48 | 37.75 ± 3.90 |
| 17 | | | 68.21 ± 17.83 | 38.00 ± 3.00 | 106.82 ± 35.84 | 60.67 ± 5.78 | | |
| 18 | | | 9.12 ± 2.96 | 25.33 ± 3.18 | 7.14 ± 1.42 | 43.00 ± 4.38 | 81.03 ± 5.45 | 74.33 ± 5.90 |
| 19 | | | 40.13 ± 8.94 | 13.67 ± 1.45 | > 20 μM | | | |
| 20 | | | 3.18 ± 1.32 | 19.00 ± 0.58 | 6.00 ± 1.38 | 58.25 ± 4.48 | | |
| 21 | | | 5.45 ± 1.25 | 36.67 ± 6.67 | 9.72 ± 2.77 | 67.50 ± 5.04 | | |
| 22 | | | 0.70 ± 0.10 | 22.33 ± 2.33 | 13.23 ± 3.65 | 53.33 ± 3.93 | | |
| 23 | | | 2.33 ± 0.74 | 18.00 ± 1.00 | 5.19 ± 0.43 | 57.00 ± 2.31 | | |
| 24 | | | 250.71 ± 54.10 | 76.33 ± 8.84 | 664.43 ± 238.45 | 88.67 ± 4.41 | | |
| 25 | | | 583.56 ± 221.90 | 103.67 ± 1.76 | 2414.28 ± 162.76 | 69.33 ± 3.18 | | |
| 26 | | | 46.10 ± 5.04 | 95.00 ± 6.43 | 15.31 ± 1.36 | 104.67 ± 2.03 | | |

(continued)

| Ref. No. | GTPγS ($EC_{50}$: nM, $E_{max}$: %) | | | | | | | |
|---|---|---|---|---|---|---|---|---|
| | ORL-1 | | Opioid Receptor | | | | | |
| | | | μ | | κ | | δ | |
| | $EC_{50}$ | $E_{max}$ | $EC_{50}$ | $E_{max}$ | $EC_{50}$ | $E_{max}$ | $EC_{50}$ | $E_{max}$ |
| **27** | | | 244.71 ± 22.97 | 94.67 ± 7.31 | 3690.06 ± 504.21 | 88.33 ± 0.67 | | |
| **28** | | | 5.92 ± 0.28 | 86.33 ± 6.44 | 39.09 ± 8.69 | 110.00 ± 7.37 | | |
| **29** | | | 345.74 ± 147.23 | 24.75 ± 1.93 | | | | |
| **30** | | | 4.85 ± 0.85 | 82.23 ± 12.99 | 1.77 ± 0.19 | 96.50 ± 6.95 | | |
| **31** | | | 2.87 ± 0.22 | 101.67 ± 12.39 | 1.20 ± 0.35 | 90.33 ± 11.68 | | |

## EXAMPLE 6

**Resolution of racemic intermediates by chiral column chromatography.**

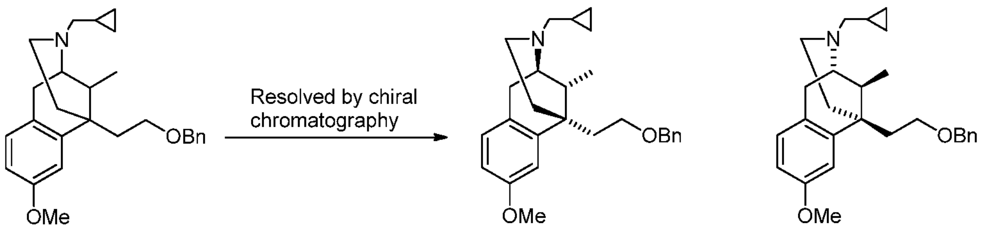


N N-1 N-2

Chiral chromatography was performed on racemic N using a RegisCell column (250mm x 50mm x5um) eluted with 0.5% diethylamine in methanol / CO2 at a ratio of 15/85 with a total flow rate of 80.0 grams/ minute to afford optically pure N-1 and N-2 after solvent removal under reduced pressure.

## EXAMPLE 7

**3-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoic acid (Compound 24) and methyl 3-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoate (Compound 26)**

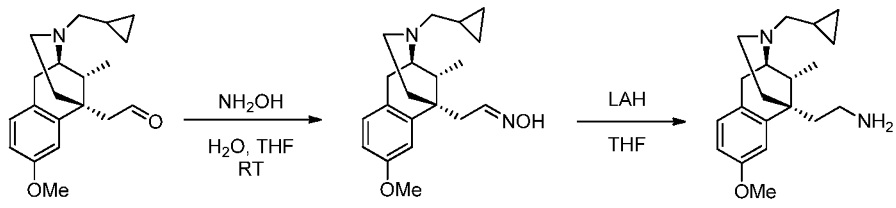


P W X

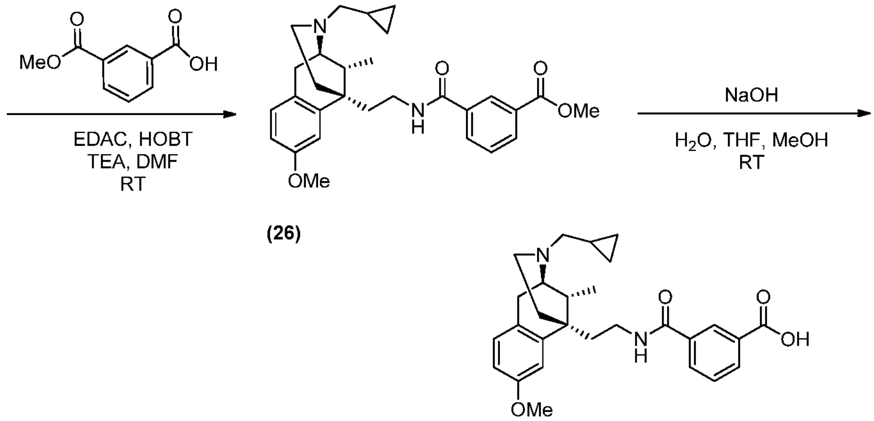


(26)

(24)

To a solution of **Compound P** (0.49 g, 1.56 mmol) in THF (3.3 mL) at RT was added 50% hydroxylamine in water (0.16 mL, 2.34 mmol). The reaction was complete in 20 min. Evaporation of the THF and water afforded a quantitative yield of **Compound W.** LC/MS, m/z = 329 [M + H]+ (Calc: 328).

To a solution of **Compound W** (0.43 g, 1.31 mmol) in THF (6.0 mL) at RT was added a 2M solution of LAH (1.96 mL, 3.92 mmol). The solution was heated to 55 °C for 1.2 h then cooled to RT and slowly added to hydrated $Na_2SO_4$. The solid was filtered and washed several times with THF. The filtrate was concentrated to afford **Compound X** as a resinous solid which was used as is in the next step.

LC/MS, m/z = 315 [M + H]+ (Calc: 314).

**Compound X** (0.100 g, 0.320 mmol) and 3-(methoxycarbonyl)benzoic acid (0.068 g, 0.382 mmol) were dissolved in DMF (1.6 mL) and TEA (0.080 mL, 0.572 mmol), EDCI hydrochloride (0.110 g, 0.572 mmol), and hydroxybenzotriazole (0.008 g, 0.064 mmol) were added and the mixture stirred at RT for 16 h. The reaction mixture was extracted with $CHCl_3$ and concentrated to give a residue which was purified by preparatory HPLC [0-60% ACN/$H_2O$ (0.01% TFA)] to afford 0.080 g of **Compound 26.**

$^1H$ NMR: $\delta_H$ (300 MHz, $CDCl_3$): 8.56 (s, 1H), 8.16 (t, J = 7.7 Hz, 2H), 7.91 (m, 1H), 7.53 (t, J = 7.7 Hz, 1H), 7.02 (d, J = 8.6 Hz, 1H), 6.89 (m, 1H), 6.77 (dd, J=2.8, 9.0 Hz, 1H), 3.94 (s, 3H), 3.89 (m, 2H), 3.80 (s, 3H), 3.60-3.48 (m, 1H), 3.33 (d, 1H), 3.17 (dd, J=5.9, 19.3 Hz, 1H), 3.08-2.97 (m, 2H), 2.93-2.83 (m, 3H), 2.57-2.44 (m, 2H), 2.21-2.12 (m, 1H), 1.41 (d, J = 14.7 Hz, 1H), 1.21-1.12 (m, 1H), 1.00 (d, J = 7.8 Hz, 3H), 0.78 (d, J = 7.9 Hz, 2H), 0.5-0.37 (m, 2H).

LC/MS, m/z = 477 [M + H]+ (Calc: 476).

To a solution of **Compound 26** in THF (0.65 mL) was added a solution of NaOH (0.022 g, 0.562 mmol) in $H_2O$ (0.65 mL) followed by MeOH (0.65 mL). The clear solution was stirred for 3 h at RT, the THF and MeOH were removed under reduced pressure and the aqueous portion was neutralized with dilute HCl and extracted with $CHCl_3$. The organic portion was concentrated to give 0.030 g of a semisolid. The aqueous portion was dried by lypholization and purified along with the organic portion above on $SiO_2$ with 0 to 20% (1N $NH_3$ in MeOH) in DCM then 0 to 20% (10% aqueous $NH_4OH$ in MeOH) in DCM to afford 0.010 g of **Compound 24** as the ammonium salt.

$^1H$ NMR: $\delta_H$ (300 MHz, $CD_3OD$): 8.50 (bs, 1H), 8.15 (d, J = 8.1 Hz, 1H), 7.95 (d, J = 7.5 Hz, 1H), 7.64 (s, 1H), 7.51 (t, J = 7.2 Hz, 1H), 7.12 (d, J = 8.6, 1H), 6.89 (m, 1H), 6.82 (dd, J = 2.4, 8.3 Hz, 1H), 3.91 (m, 1H), 3.79 (s, 3H), 3.71-3.51 (m, 2H), 3.42-3.35 (m, 1H), 3.27-3.15 (m, 2H), 3.12-3.01 (m, 2H), 2.71-2.53 (m, 3H), 2.36-2.26 (m, 1H), 2.21-2.11 (m, 1H), 1.51 (d, J = 11.6 Hz, 1H), 1.30-1.20 (m, 1H), 1.04 (d, J = 6.8 Hz, 1H), 0.81 (d, J = 8.1 Hz, 2H), 0.52 (t, J = 5.5 Hz, 2H).

LC/MS, m/z = 463 $[M + H]^+$ (Calc: 462).

In a similar manner, **4-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoic acid (Compound 25)** was prepared from **Compound X** by using 4-(methoxycarbonyl)benzoic acid rather than 3-(methoxycarbonyl)benzoic acid.

$^1$H NMR: $\delta_H$ (300 MHz, $CD_3OD$): 8.09 (d, J = 8.3 Hz, 2H), 7.91 (d, J = 8.6 Hz, 2H), 7.13 (d, J = 8.6 Hz, 1H), 6.91 (d, J = 2.6 Hz, 1H), 6.83 (dd, J = 2.4, 8.3 Hz, 1H), 3.93 (m, 1H), 3.80(s, 3H), 3.67-3.61 (m, 2H), 3.34 (m, 1H), 3.27-3.19 (m, 2H), 3.13 (s, 1H), 3.10-3.00 (m, 1H), 2.71 (m, 1H), 2.63-2.48 (m, 2H), 2.40-2.29 (m, 1H), 2.21-2.11 (m, 1H), 1.55 (d, J = 11.6 Hz, 1H), 1.25-1.16 (m, 1H), 1.08 (d, J = 7.0 Hz, 3H), 0.81 (d, J = 7.9 Hz, 2H), 0.55-0.47 (m, 2H).
LC/MS, m/z = 463 [M + H]+ (Calc: 462).

## EXAMPLE 8

**4-(2-((2*R*,6*S*,11*R*)-3-(cyclopropylmethyl)-8-methoxy-11-methyl-1,2,3,4,5,6-hexahydro-2,6-methanobenzo[*d*]azocin-6-yl)ethoxy)benzoic acid (Compound 27) and 4-(2-((2*R*,6*S*,11*R*)-3-(cyclopropylmethyl)-8-hydroxy-11-methyl-1,2,3,4,5,6-hexahydro-2,6-methanobenzo[*d*]azocin-6-yl)ethoxy)benzoic acid (Compound 28)**

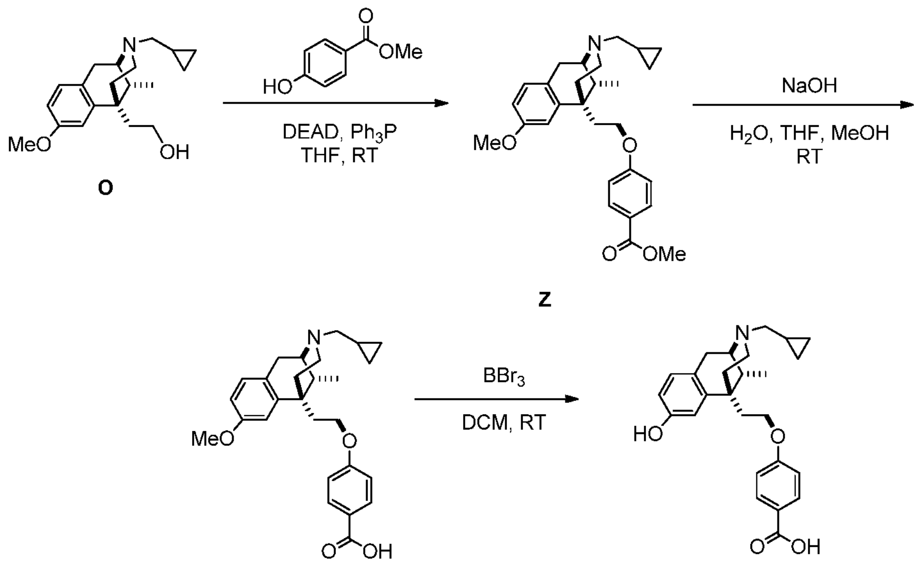


**O** **Z** **(27)** **(28)**

**Compound O** (0.150 g, 0.476 mmol), methyl 4-hydroxybenzoate (0.181 g, 1.189 mmol) and triphenylphosphine (0.311 g, 1.189 mmol) were dissolved in THF (2.4 mL), cooled with an ice bath and DEAD (0.54 mL of a 40 wt % solution in toluene, 1.189 mmol) was added and the ice bath was removed. The mixture was stirred for 16 h at RT, $H_2O$ was added and the product was extracted with EtOAc, dried over $Na_2SO_4$ and concentrated to give a semisolid which was purified on $SiO_2$ with 0 to 90% EtOAc in hexanes to afford 0.100 g of **Compound Z** containing 20% of triphenyl phosphine. This crude product was carried on as is to the next step.
LC/MS, m/z = 450 [M + H]+ (Calc: 449).

To a solution of **Compound Z** in THF (1.0 mL) was added a solution of NaOH (0.036 g, 0.890 mmol) in $H_2O$ (1.0 mL) followed by MeOH (1.0 mL). The clear solution was stirred for 16 h at RT and the THF and MeOH were evaporated and the aqueous suspension was extracted twice with ether to remove the triphenylphosphine carried over from the previous step. The aqueous portion was neutralized with dilute HCl and filtered to collect the solid which was dissolved in DCM and purified on $SiO_2$ with 0 to 15% MeOH in DCM to afford 0.043 g of **Compound 27** as a white solid.
$^1$H NMR: $\delta_H$ (300 MHz, $CDCl_3$): 8.07 (d, J = 6.6 Hz, 2H), 7.07 (d, J = 8.6 Hz, 1H), 6.96 (d, J = 8.8 Hz, 2H), 6.89 (d, J = 2.4 Hz, 1H), 6.79 (dd, J = 2.6, 8.6 Hz, 1H), 4.32-4.24 (m, 2H), 3.98 (m, 1H), 3.81 (s, 3H), 3.36 (d, J = 12.7 Hz, 1H), 3.13 (dd, J = 5.3, 19.0 Hz, 2H), 2.99-2.87 (m, 3H), 2.83-2.67 (bs, 1H), 2.53-2.42 (m, 2H), 2.42-2.32 (m, 2H), 1.43-1.36 (m, 1H), 1.02 (d, J = 7.0 Hz, 3H), 0.75 (d, J = 7.7 Hz, 2H), 0.63-0.55 (m, 1H), 0.44-0.38 (m, 1H).
LC/MS, m/z = 436 [M + H]+ (Calc: 435).

To a solution of **Compound 27** (0.031 g, 0.069 mmol) in DCM (0.4 mL) at 0 °C was added $BBr_3$ (0.070 g, 0.279 mmol). The ice bath was removed and the mixture was stirred at room temperature for 1 h. The reaction was quenched with $H_2O$ and neutralized with $NaHCO_3$. The layers were adsorbed onto $SiO_2$ and purified on $SiO_2$ with 0 to 20% MeOH in DCM to afford 0.030 g of **Compound 28** as a white solid.
$^1$H NMR: $\delta_H$ (300 MHz, $CD_3OD$): 8.00 (d, J = 8.6 Hz, 2H), 7.07-7.00 (m, 3H), 6.85 (m, 1H), 6.75 (d, J = 8.3 Hz, 1H), 4.44-4.29 (m, 2H), 3.96 (bs, 1H), 3.42-3.37 (m, 1H), 3.28-3.16 (m, 2H), 3.13-2.97 (m, 2H), 2.81-2.65 (m, 3H), 2.61-2.51

(m, 1H), 2.40-2.30 (m, 1H), 1.98 (s, 1H), 1.56 (d, J = 13.1 Hz, 1H), 1.30-1.21 (m, 1H), 1.05 (d, J = 6.8 Hz, 3H), 0.80 (d, J = 8.1 Hz, 2H), 0.54 (d, J = 25.0 Hz, 2H).
LC/MS, m/z = 422 [M + H]+ (Calc: 421).

In a similar manner **5-(2-((2*R*,6*S*,11*R*)-3-(cyclopropylmethyl)-8-hydroxy-11-methyl-1,2,3,4,5,6-hexahydro-2,6-methanobenzo[*d*]azocin-6-yl)ethoxy)nicotinic acid (Compound 16)** was prepared as the ammonium salt from **Compound O** using methyl 5-hydroxynicotinate rather than methyl 4-hydroxybenzoate.
$^1$H NMR: $\delta_H$ (400 MHz, $CDCl_3$): 8.69 (s, 1H), 8.35 (d, J = 2.8 Hz, 1H), 7.89 (s, 1H), 7.05 (d, J = 8.6 Hz, 1H), 6.86 (s, 1H), 6.75 (dd, J = 2.4, 8.3 Hz, 1H), 4.42-4.34 (m, 2H), 3.96-3.91 (m, 1H), 3.42-3.32 (m, 3H), 3.22-3.15 (m, 2H), 3.11-2.98 (m, 2H), 2.73-2.66 (m, 3H), 2.63-2.54 (m, 1H), 1.61-1.54 (m, 1H), 1.24-1.17 (m, 1H), 1.07 (d, J = 6.80 Hz, 3H), 0.79 (d, J = 7.9 Hz, 2H), 0.52-0.45 (m, 2H).
LC/MS, m/z = 423 [M + H]+ (Calc: 422).

## EXAMPLE 9

**N-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)-2-(dimethylamino)acetamide (Compound 30)**

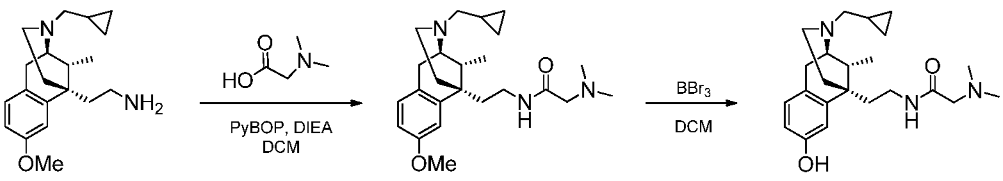


X AA (30)

Into a vial containing a solution of **Compound X** (0.12 g, 0.383 mmol) in DCM (1.8 mL) was added 2-(dimethylamino)acetic acid (0.047 g, 0.458 mmol), diisopropylethyl amine (0.148 g, 1.145 mmol) and PyBOP (0.238 g, 0.458 mmol). The reaction was stirred for 10 min at RT, reduced in volume and purified on $SiO_2$ with 15 to 30% EtOAc in hexanes followed by 0 to 30% MeOH in EtOAc to afford 0.050 g of **Compound AA.** LC/MS, m/z = 400 [M + H]+ (Calc: 399).

Into a sealed tube containing a solution of **Compound AA** (0.050 g, 0.125 mmol) in DCM (0.5 mL) at 0 °C was added 0.5 mL of a 1M solution of boron tribromide in DCM (0.5 mmol). The ice bath was removed and the suspension was stirred for 1 h at RT. It was cooled again with an ice bath and quenched with saturated aqueous $NaHCO_3$. The aqueous and organic phases were concentrated together and adsorbed onto $SiO_2$ and purified on $SiO_2$ with 0 to 30% MeOH in EtOAc and then with 0 to 10% (1N $NH_3$ in MeOH) in DCM to afford 0.014 g of **Compound 30** as a white solid.
$^1$H NMR: $\delta_H$ (300 MHz, $CD_3OD$): 6.91 (d, J = 8.3 Hz, 1H), 6.74 (d, J = 2.6 Hz, 1H), 6.62 (dd, J = 2.4, 8.1 Hz, 1H), 3.57-3.43 (m, 2H), 3.19 (m, 1H), 2.99 (s, 2H), 2.85 (d, J = 18.2 Hz, 1H), 2.74-2.64 (m, 2H), 2.54-2.47 (m, 1H), 2.39-2.32 (m, 7H), 2.15-2.05 (m, 4H), 2.01-1.91 (m, 1H), 1.26 (d, J = 9.6 Hz, 1H), 0.94 (d, J = 7.0 Hz, 3H), 0.88 (m, 1H), 0.55 (d, J = 7.9 Hz, 2H), 0.21-0.11 (m, 2H).
LC/MS, m/z = 386 [M + H]+ (Calc: 385).

In a similar manner, **2-amino-*N*-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)acetamide (Compound 31)** was prepared from **Compound X** using N-Boc-glycine rather than 2-(dimethylamino)acetic acid.
$^1$H NMR: $\delta_H$ (300 MHz, $CD_3OD$): 6.91 (d, J = 8.3 Hz, 1H), 6.72 (d, J = 2.6 Hz, 1H), 6.61 (dd, J = 2.6, 8.3 Hz, 1H), 3.51-3.43 (m, 2H), 3.35-3.33 (m, 4H), 3.30 (s, 2H), 3.21-3.18 (m, 1H), 2.85 (d, J = 18.6 Hz, 1H), 2.74-2.64 (m, 2H), 2.54-2.48 (m, 1H), 2.39-2.32(m, 1H), 2.13-2.04 (m, 4H), 2.00-1.91 (m, 1H), 1.25 (d, J = 9.6 Hz, 1H), 0.94 (d, J = 7.0 Hz, 3H), 0.88 (m, 1H), 0.55 (d, J = 7.9 Hz, 2H), 0.20-0.11 (m, 2H).
LC/MS, m/z = 358 [M + H]+ (Calc: 357).

## EXAMPLE 10

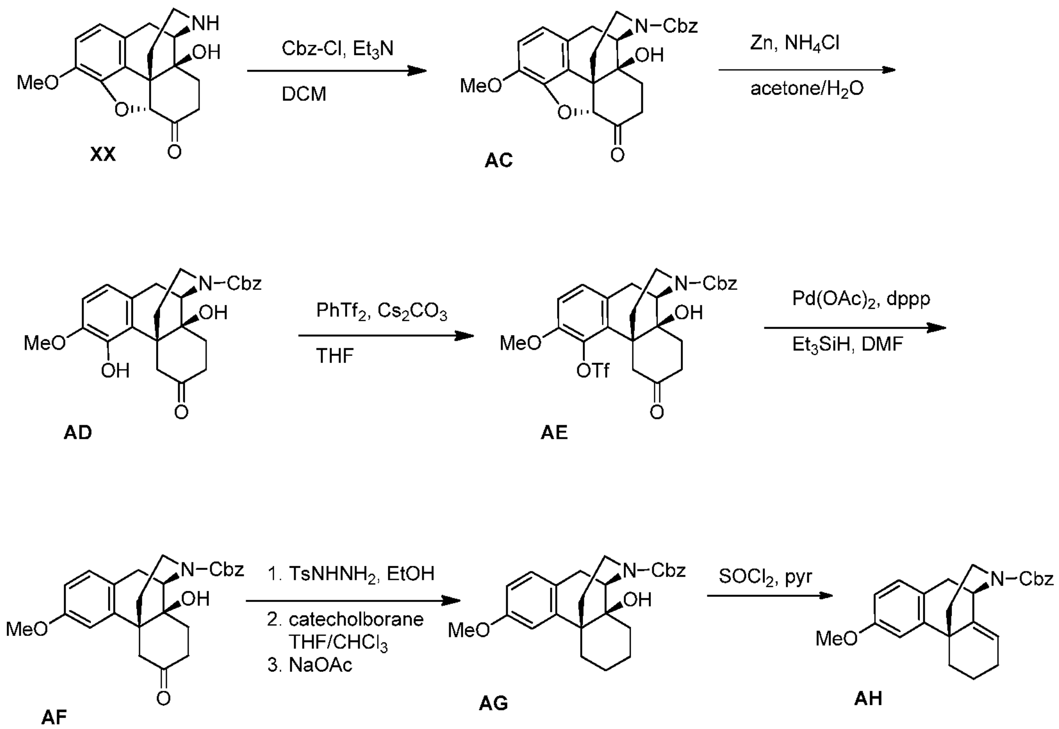


XX AC AD AE AF AG AH

Benzyl chloroformate (Cbz-Cl) (6.96 mL, 48.8 mmol) was added slowly to noroxycodone (**Compound XX**, 12.25 g, 40.7 mmol) [J. Org. Chem. 1984, 49, 2081] and TEA (17.00 mL, 122 mmol) in DCM (200 mL) at 0 °C. The solution was stirred at 0 °C to RT for 2.3 h, after which an additional aliquot of Cbz-Cl (3 mL, 0.5 equiv) was added. The reaction was stopped after 3.5 h. DCM was added and the solution washed with 10% HCl. The aqueous layer was washed with DCM, the combined organic layers dried with $Na_2SO_4$ and conconcentrated. **Compound AC** was isolated as a pale yellow oil, 21.37 g (>100%). The material was carried on without further purification.

$^1H$ NMR: $\delta_H$ (400 MHz, DMSO-d6): 7.46-7.29 (m, 5 H), 6.79 (d, *J*=8.2 Hz, 1H), 6.78 (d, *J*=8.2 Hz, 1H), 5.44 (d, *J*=24.1 Hz, 1H), 5.21-5.05 (m, 2H), 4.84 (d, *J*=2.2 Hz, 1H), 4.77 (s, 2H), 4.38 (dd, *J*=21.4, 5.1 Hz, 1H), 3.90 (dd, *J*=12.7, 4.2 Hz, 1H), 3.12-2.73 (m, 3H), 2.61 (dt, *J*=13.0, 3.7 Hz, 1H), 2.38-2.40 (m, 1H), 2.06 (d, *J*=14.0 Hz, 1H), 1.88 (bt, *J*=11.4 Hz, 1H), 1.46 (tt, *J*=14.2, 3.5 Hz, 1H), 1.29 (td, *J*=12.7, 3.3 Hz, 1H).

LC/MS, *m/z* = 458 $[M + Na]^+$ (Calc: 435).

Zinc (10.64 g, 163 mmol) was added to **Compound AC** (17.72 g, 40.7 mmol) and saturated aqueous ammonium chloride (64.0 mL, 448 mmol) in acetone (136 mL). The solution was heated at 60 °C for 16.5 h. The reaction mixture was cooled to RT and filtered, washing the solid with more acetone. The filtrate was concentrated to remove most of the acetone, basified with conc. $NH_4OH$ to pH 10-11, and washed with two portions of DCM. The combined organic layers were dried with $Na_2SO_4$, concentrated, and purified by MPLC (0-100% EtOAc/hexanes, 220 g). **Compound AD** was isolated as a white foam (13.22 g, 74%).

$^1H$ NMR: $\delta_H$ (400 MHz, DMSO-d6): 8.48 (bs, 1H), 7.48-7.11 (m, 5H), 6.79 (d, *J*=8.1 Hz, 1H), 6.53 (d, *J*=7.7 Hz, 1H), 5.21-4.99 (m, 2H), 4.28 (dd, *J*=18.5, 5.9 Hz, 1H), 3.89 (d, *J*=11.4 Hz, 1H), 3.80 (d, *J*=8.0 Hz, 1H), 3.74 (s, 3H), 2.71-2.57 (m, 3H), 2.38-2.23 (m, 1H), 1.97-1.74 (m, 3H), 1.52-1.35 (m, 1H).

LC/MS, *m/z* = 460 $[M + Na]^+$ (Calc: 437).

*N*-Phenyl-bis(triftuoromethanesulfonimide) (124 g, 348 mmol) was added to **Compound AD** (139 g, 317 mmol) and cesium carbonate (134 g, 412 mmol) in THF (1000 mL). The reaction was heated to 70 °C for 20.5 h. The reaction mixture was concentrated, DCM and water were added. The aqueous later was washed with DCM, and the combined organic layers dried with $Na_2SO_4$ and concentrated. **Compound AE** was isolated as a pale brown foam, (157 g, 87%) and carried on without further purification.

LC/MS, *m/z* = 470 $[M + H]^+$ (Calc: 569).

Triethylsilane (2.194 mL, 13.74 mmol) was added slowly to **Compound AE** (6.52 g, 11.45 mmol), palladium(II) acetate (0.257 g, 1.145 mmol) and 1,3-bis(diphenylphosphino)propane (0.472 g, 1.145 mmol) in DMF (50 mL) and the solution was stirred at 70 °C. After 1 h, additional triethylsilane (2.194 mL, 13.74 mmol) was added. The reaction was stopped after 90 min. EtOAc was added, the organic layer washed with 10% HCl, dried with $Na_2SO_4$, and concentrated. The resulting material was purified by MPLC (0-60% acetone/hexanes, 120 g). **Compound AF** was isolated as a clear oil (3.51 g, 73%).

$^1$H NMR: $\delta_H$ (400 MHz, DMSO-d6): 7.44-7.27 (m, 5H), 7.05 (d, *J*-8.3 Hz, 1H), 6.81-6.73 (m, 2H), 5.26-5.00 (m, 3H), 4.35 (dd, *J*=19.5, 5.7 Hz, 1H), 3.85-3.74 (m, 2H), 3.70 (s, 3H), 3.23-3.11 (m, 1H), 2.86-2.76 (m, 1H), 2.70-2.58 (m, 2H), 2.27-2.15 (m, 1H), 1.95-1.77 (m, 3H), 1.76-1.61 (m, 1H), 1.03 (t, *J*=12.0 Hz, 1H).
LC/MS, *m/z* = 444 [M + Na]$^+$ (Calc: 421).

EtOH (250 mL) was added to **Compound AF** (24.15 g, 57.3 mmol) and *p*-toluenesulfonhydrazide (12.80 g, 68.8 mmol) and the solution heated at reflux for 3.25 h. and then concentrated. Chloroform (400 mL) was added, followed by the slow addition of catecholborane (1*M* in THF, 143 mL, 143 mmol) at 0 °C. The reaction mixture was stirred at 0 °C for 45 min, sodium acetate (14.10 g, 172 mmol) was added and the solution heated at reflux for 90 min. The mixture was cooled and concentrated. EtOAc was added, washed with two portions of 1 *M* NaOH, dried with $Na_2SO_4$, and concentrated. **Compound AG** was isolated as an orange oil (27.4 g, >100%) and carried on immediately as is.
LC/MS, *m/z* = 430 [M + Na]$^+$ (Calc: 407).

Thionyl chloride (1.186 mL, 16.26 mmol) was added dropwise to **Compound AG** in pyridine (65 mL) at 0 °C. The reaction was stirred at 0 °C to RT for 17 h. The solution was concentrated and purified by MPLC (0-5% DCM/MeOH, 80 g) to yield **Compound AH** as a yellow oil (3.27 g, 62%).
$^1$H NMR: $\delta_H$ (400 MHz, DMSO-d6): 7.43-7.26 (m, 5H), 7.00 (d, *J*=8.3 Hz, 1H), 6.82 (s, 1H), 6.75 (dd, *J*=8.3, 2.6 Hz, 1H), 5.72 (s, 1H), 5.11 (s, 1H), 5.06 (d, *J*=8.3 Hz, 1H), 4.77 (d, *J*=5.5 Hz, 1H), 3.81 (dd, *J*=13.4, 4.8 Hz, 1H), 3.73 (s, 1H), 3.15-3.03 (m, 1H), 2.85 (dd, *J*=17.3, 3.1 Hz, 1H), 2.75-2.54 (m, 1H), 2.12 (t, *J*=12.7 Hz, 1H), 2.06-1.77 (m, 4H), 1.69-1.54 (m, 1H), 1.52-1.40 (m, 2H).
LC/MS, *m/z* = 390 [M + H]$^+$ (Calc: 389).

## EXAMPLE 11

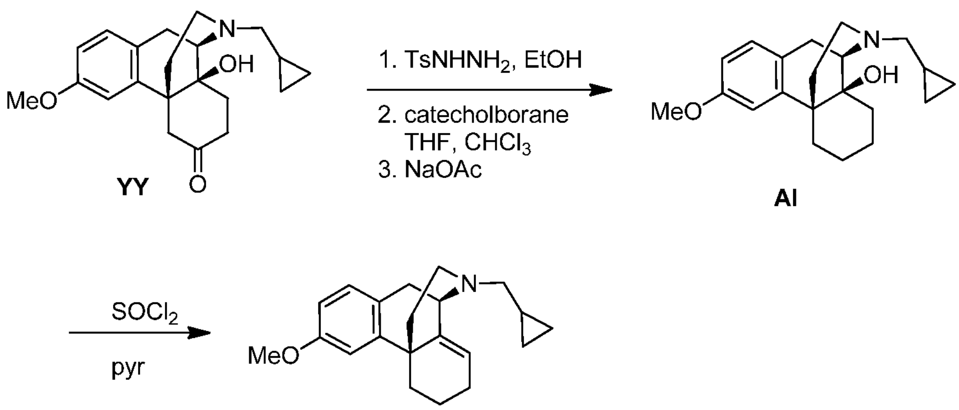

YY
AI

AJ

**Compound AI** was synthesized from **Compound YY** (Tetrahedron Lett., 2010, 51, 2359) in an analogous manner to the synthesis of **Compound AG** from **Compound AF.**
$^1$H NMR: $\delta_H$ (400 MHz, DMSO-d6): 6.94 (d, *J*=8.7 Hz, 1H), 6.66 (d, *J*=2.6 Hz, 1H), 6.61 (dd, *J*=8.3, 2.6 Hz, 1H), 4.27 (bs, 1H), 3.61 (s, 3H), 2.89 (d, *J*=18.4 Hz, 1H), 2.72 (d, *J*=6.1 Hz, 1H), 2.58 (dd, *J*=18.4, 6.1 Hz, 1H), 2.29-2.11 (m, 4H), 1.83 (d, *J*=7.9 Hz, 2H), 1.80-1.55 (m, 3H), 1.33-1.30 (m, 3H), 1.29-1.15 (m, 4H), 0.84 (d, *J*=8.1 Hz, 2H), 0.77-0.66 (m, 1H), 0.41-0.31 (m, 2H), 0.07-0.00 (m, 2H).
LC/MS, *m/z* = 328 [M + H]$^+$ (Calc: 327).

**Compound AJ** was synthesized from **Compound AI** in a analogous manner to the synthesis of **Compound AH** from **Compound AG,** and isolated as the HCl salt.
$^1$H NMR: $\delta_H$ (400 MHz, DMSO-d6): 10.15 (bs, 0.5H), 9.84 (bs, 0.5H), 7.09 (t, *J*=8.1 Hz, 1H), 6.91-6.78 (m, 2H), 6.00 (d, *J*=18.6 Hz, 1H), 4.37 (d, *J*=6.8 Hz, 0.5H), 4.24 (d, *J*=6.8 Hz, 0.5H), 3.74 (s, 3H), 3.50 (d, *J*=18.8 Hz, 1H), 3.30-3.19 (m, 2H), 3.18-3.04 (m, 1H), 3.03-2.83 (m, 2H), 2.37-2.18 (m, 1H), 2.17-2.07 (m, 2H), 2.03-1.87 (m, 2H), 1.75-1.56 (m, 1.5H), 1.48 (d, *J*=16.2 Hz, 1H), 1.43-1.30 (m, 0.5 H), 1.21-1.02 (m, 1H), 0.72-0.56 (m, 2H), 0.50-0.33 (m, 2H).
LC/MS, *m/z* = 310 [M + H]$^+$ (Calc: 309).

## EXAMPLE 12

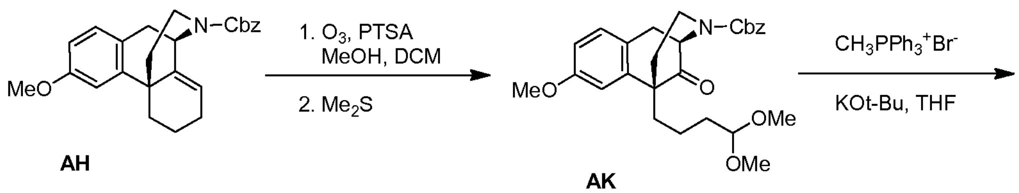


AH AK

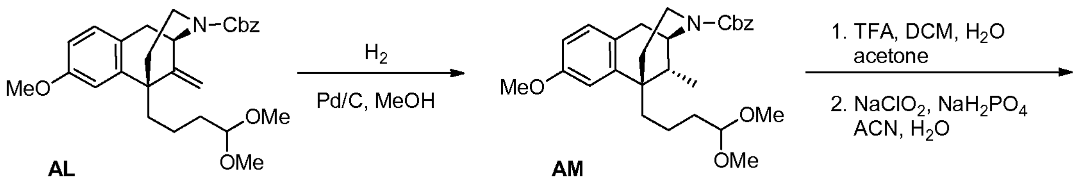


AL AM

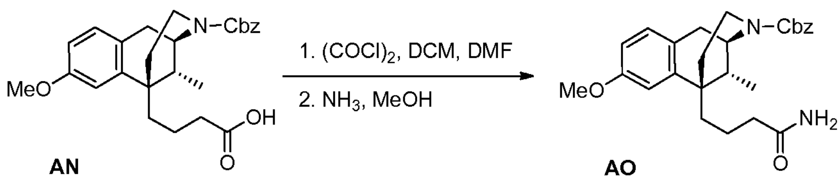


AN AO

A Pacific Ozone Technology L11 ozone generator was used to bubble $O_3$ through **Compound AH** (3.27 g, 8.40 mmol) in 4:1 DCM/MeOH (40 mL) at -78 °C for 12 min. After ~ 10 min, the color turns yellow to green-gray. Nitrogen was bubbled through the solution for 3 min, PTSA (0.319 g, 1.679 mmol) was added and the solution stirred at RT for 2 h. An additional aliquot of PTSA (0.319 g, 1.679 mmol) was added and the solution stirred for an additional 1 h. Dimethyl sulfide (1.242 mL, 16.79 mmol) was added and the solution stirred at RT for 17 h. DCM was added, washed with sat. $NaHCO_3$, dried with $Na_2SO_4$, and concentrated. **Compound AK** was purified by MPLC (0-60% EtOAc/hexanes, 40 g followed by 0-3% MeOH/DCM, 40 g) and isolated as a clear oil (630 mg, 16%).
$^1$H NMR: $\delta_H$ (400 MHz, DMSO-d6): 7.46-7.23 (m, 5H), 7.12 (d, *J*=8.3 Hz, 1H), 6.85 (dd, *J*=8.3, 2.6 Hz, 1H), 6.80 (d, *J*=2.6 Hz, 1H), 5.66-5.13 (m, 2H), 4.58-4.50 (m, 1H), 4.29 (t, *J*=5.6 Hz, 1H), 3.75 (s, 3H), 3.46-3.34 (m, 1H), 3.32-3.26 (m, 1H), 3.21 (s, 1H), 3.17 (s, 5H), 3.12-2.87 (m, 1H), 2.85-2.55 (m, 1H), 1.98-1.77 (m, 4H), 1.68-1.47 (m, 2H), 1.29-1.13 (m, 1H), 1.11-0.95 (m, 1H).
LC/MS, *m/z* = 490 $[M + Na]^+$ (Calc: 467).

Potassium tert-butoxide (1 M in THF, 2.021 mL, 2.021 mmol) was added slowly to methyltriphenylphosphonium bromide (0.578 g, 1.617 mmol) in THF (3 mL) at 0 °C. The solution was stirred at 0 °C for 5 min, after which **Compound AK** (0.630 g, 1.347 mmol) in THF (5 mL) was added dropwise at 0 °C. The reaction as stirred at 0 °C to RT for 3.5 h and heated at reflux for 2 h. EtOAc was added, the organic layer washed with sat.$NaHCO_3$, dried with $Na_2SO_4$, and concentrated. MPLC (0-60% EtOAc/hexanes, 40 g) led to the isolation of **Compound AL** as a clear oil (360 mg, 57%).
$^1$H NMR: $\delta_H$ (400 MHz, DMSO-d6): 7.43-7.23 (m, 5H), 7.02 (dd, *J*=8.1, 5.3 Hz, 1H), 6.81 (bs, 1H), 6.76 (dd, *J*=8.3, 2.4 Hz, 1H), 5.11 (s, 2H), 5.06 (d, *J*=9.4 Hz, 1H), 4.87-4.81 (m, 2H), 4.33 (t, *J*=5.6 Hz, 1H), 3.78-3.69 (m, 1H), 3.74 (s, 3H), 3.17 (d, *J*=5.0 Hz, 6H), 3.10-2.98 (m, 1H), 2.86 (d, *J*=16.8 Hz, 1H), 2.18-2.06 (m, 1H), 1.95-1.83 (m, 1H), 1.65-1.49 (m, 4H), 1.42 (d, *J*=3.5 Hz, 1H), 1.27-1.11 (m, 2H).
LC/MS, *m/z* = 488 $[M + Na]^+$ (Calc: 465).

MeOH (15 mL) was added to **Compound AL** (0.310 g, 0.666 mmol) and the solution run with the Pd/C cartridge on the H-Cube [ThalesNano, model HC-2.SS] at 50 °C in full $H_2$ mode in a recirculating fashion at 0.5 mL/min for 21 h. The solution was concentrated to yield **Compound AM** (290 mg, 93%), which was carried on without further purification.
$^1$H NMR: $\delta_H$ (400 MHz, DMSO-d6): 7.35-7.15 (m, 5H), 6.94 (d, *J*=8.1 Hz, 1H), 6.75 (bs, 1H), 6.67 (dd, *J*=8.1, 2.6 Hz, 1H), 5.05-4.95 (m, 2H), 4.34 (t, *J*=5.7 Hz, 1H), 4.29-4.15 (m, 1H), 3.77-3.63 (m, 2H), 3.64 (s, 3H), 3.16 (s, 6H), 3.09 (d, *J*=6.6 Hz, 2H), 3.00 (d, *J*=16.4, 1H), 1.93-1.80 (m, 1H), 1.80-1.64 (m, 2H), 1.61-1.42 (m, 3H), 1.38-1.24 (m, 2H), 1.11-1.01 (m, 1H), 0.96-0.87 (m, 1H), 0.73-0.62 (m, 2H).
LC/MS, *m/z* = 490 $[M + Na]^+$ (Calc: 467).

DCM (5 mL) and TFA (2 mL) were added to **Compound AM** (250 mg, 0.54 mmol) and the solution was stirred at RT. Additional TFA (2 mL) was added after 1 h, $H_2O$ (1 mL) added after 90 min, and acetone (5 mL) added after 3 h. After a total of 3.75 h. the reaction was concentrated. ACN (2 mL) was added, followed by the dropwise addition of a chilled, premixed solution of sodium chlorite (0.145 g, 1.604 mmol) and monobasic sodium phosphate (0.221 g, 1.604 mmol) in water (2 mL). The reaction mixture was stirred at 0 °C to RT for 1.75 h. EtOAc was added, and the organic layer washed 1 M NaOH. The aqueous layer was acidified with 10% HCl and back-extracted with EtOAc. The combined

organic layers were dried with $Na_2SO_4$ and concentrated to yield **Compound AN** (280 mg, >100%), which was carried on without further purification. A small portion of **Compound AN** was purified by preparatory HPLC [0-60% ACN/$H_2O$ (0.01% TFA)].

$^1H$ NMR: $\delta_H$ (400 MHz, $CD_3OD$): 7.30-7.17 (m, 5H), 6.90 (dd, *J*=8.3, 4.8 Hz, 1H), 6.75 (d, *J*=1.8 Hz, 1H), 6.64 (dd, *J*=8.8, 2.2 Hz, 1H), 5.07-4.99 (m, 2H), 4.38-4.24 (m, 1H), 3.84-3.71 (m, 1H), 3.69 (s, 2H), 3.66 (s, 1H), 3.17-2.99 (m, 1H), 2.70-2.41 (m, 2H), 2.40-2.17 (m, 2H), 1.95-1.78 (m, 3H), 1.70-1.58 (m, 2H), 1.11 (t, *J*=13.1 Hz, 1H), 0.99 (t, *J*=6.7 Hz, 1H), 0.81-0.68 (m, 2H).

LC/MS, *m/z* = 438 $[M + H]^+$ (Calc: 437).

One drop DMF was added to a solution of **Compound AN** (0.280 g, 0.640 mmol) and oxalyl chloride (0.224 mL, 2.56 mmol) in DCM (5 mL) and the reaction was stirred at RT for 30 min. The reaction mixture was concentrated. DCM (3 mL) and ammonia (7 M in MeOH, 1.828 mL, 12.80 mmol) were added and the solution stirred at RT for 2 h. DCM was added, washed with 10% HCl, dried with $Na_2SO_4$, and concentrated to yield **Compound AO** as a yellow oil (250 mg. 89%). A small portion was purified by preparatory HPLC [0-60% ACN/$H_2O$ (0.01% TFA)], and the remainder carried on without further purification.

$^1H$ NMR: $\delta_H$ (400 MHz, $CD_3OD$): 7.30-7.15 (m, 5H), 6.91 (dd, *J*=8.1, 5.1 Hz, 1H), 6.75 (d, *J*=2.4 Hz, 1H), 6.64 (dd, *J*=8.3, 2.4 Hz, 1H), 5.08-4.95 (m, 2H), 4.39-4.25 (m, 1H), 3.84-3.70 (m, 1H), 3.69 (s, 3H), 3.67 (s, 1H), 3.66 (s, 2H), 3.06 (dt, *J*=17.8, 5.8 Hz, 1H), 2.67-2.42 (m, 2H), 2.21 (q, *J*=6.9 Hz, 1.5H), 2.12 (t, *J*=7.2 Hz, 0.5 Hz), 1.91-1.80 (m, 3H), 1.70-1.60 (m, 2H), 1.10 (t, *J*=13.3, 1H), 0.98 (t, *J*=7.1, 1H), 0.76 (dd, *J*=10.6, 6.9 Hz, 2H).

LC/MS, *m/z* = 437 $[M + H]^+$ (Calc: 436).

## EXAMPLE 13

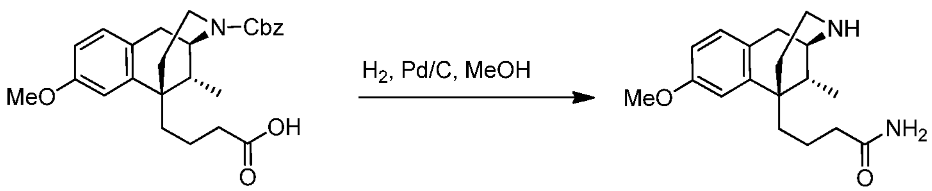


AO AP

**Compound AO** (0.250 g, 0.573 mmol) in MeOH (20 mL) was added slowly to Pd/C (0.050 g, 0.470 mmol) under Ar. The reaction mixture was shaken on a Parr shaker at 50 PSI for 16 h. The mixture was filtered over celite and concentrated to yield **Compound AP** as a white solid that was used as is (180 mg, 100%).

LC/MS, *m/z* = 303 $[M + H]^+$ (Calc: 302).

## EXAMPLE 14

**4-((2*R*,6*R*,11*R*)-8-hydroxy-3-isopropyl-11-methyl-1,2,3,4,5,6-hexahydro-2,6-methanobenzo[*d*]azocin-6-yl)butanamide (Compound 17)**

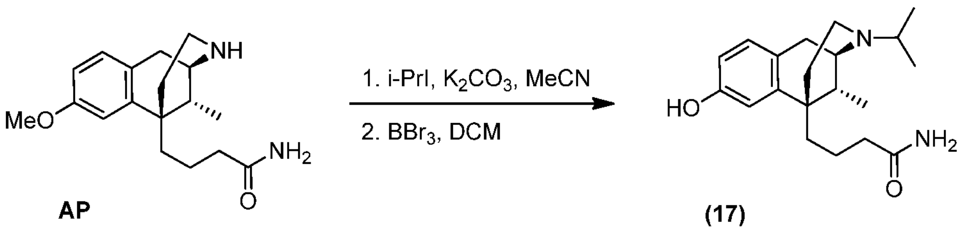


AP (17)

2-Iodopropane (0.016 mL, 0.164 mmol) was added to **Compound AP** (0.033 g, 0.109 mmol) and potassium carbonate (0.030 g, 0.218 mmol) in ACN (1 mL). The reaction was stirred at RT for 17 h and heated at 60 °C for 6.5 h. The reaction mixture was passed through a syringe filter and concentrated. DCM (1 mL) was added followed by boron tribromide (0.031 mL, 0.327 mmol) dropwise at 0 °C. The solution was stirred at 0 °C to RT for 90 min, slowly quenched

with 10 drops of MeOH, and concentrated. The resulting oil was purify by preparatory HPLC [0-60% ACN/$H_2O$ (0.01 % TFA)] to yield **Compound 17** as its TFA salt.

$^1H$ NMR: $\delta_H$ (400 MHz, $CD_3OD$): 6.93 (d, *J*=8.3 Hz, 1H), 6.67 (d, *J*=2.6 Hz, 1H), 6.58 (dd, *J*=8.3, 2.6 Hz, 1H), 3.83 (bs, 1H), 3.49 (q, *J*=6.4 Hz, 1H), 3.42 (dd, *J*=13.1, 4.6 Hz, 1H), 3.10-2.92 (m, 2H), 2.52 (dd, *J*=13.0, 3.6, 1H), 2.31-2.21 (m, 2H), 2.17-2.06 (m, 2H), 1.96-1.82 (m, 1H), 1.73-1.60 (m, 3H), 1.41-1.35 (m, 1H), 1.32 (dd, *J*=6.5, 3.4 Hz, 6H), 0.85 (d, *J*=6.8 Hz, 2.5H), 0.79 (d, *J*=6.8 Hz, 0.5H).

LC/MS, *m/z* = 331 $[M + H]^+$ (Calc: 330).

In a similar manner **4-((2*R*,6*R*,11*R*)-8-hydroxy-3-isobutyl-11-methyl-1,2,3,4,5,6-hexahydro-2,6-methanobenzo[*d*]azocin-6-yl)butanamide (Compound 18)** was synthesized from **Compound AP** using 1-iodo-2-methylpropane rather than 2-iodopropane. **Compound 18** was isolated as its TFA salt.

$^1H$ NMR: $\delta_H$ (400 MHz, $CD_3OD$): 6.94 (d, *J*=8.3 Hz, 1H), 6.67 (d, *J*=2.4 Hz, 1H), 6.58 (dd, *J*=8.3, 2.4 Hz, 1H), 3.65 (bs, 0.8H), 3.59 (bs, 0.2H), 3.14 (d, *J*=12.9 Hz, 1H), 3.10-2.93 (m, 3H), 2.86 (dd, *J*=13.1, 8.8 Hz, 1H), 2.64 (td, *J*=13.2, 3.5, 1H), 2.33-2.16 (m, 4H), 2.03 (q, *J*=6.8 Hz, 1H), 1.94-1.81 (m, 1H), 1.77-1.60 (m, 4H), 1.31 (d, *J*=14.3 Hz, 1H), 0.98 (dd, *J*=6.6, 3.3 Hz, 6H), 0.85 (d, *J*=6.8 Hz, 2.5 H), 0.81 (d, *J*=6.8 Hz, 0.5H). LC/MS, *m/z* = 345 $[M + H]^+$ (Calc: 344).

In a similar manner **4-((2*R*,6*R*,11*R*)-3-benzyl-8-hydroxy-11-methyl-1,2,3,4,5,6-hexahydro-2,6-methanobenzo[*d*]azocin-6-yl)butanamide (Compound 19)** was synthesized from **Compound AP** using benzyl bromide rather than 2-iodopropane. **Compound 19** was isolated as its TFA salt.

$^1H$ NMR: $\delta_H$ (400 MHz, $CD_3OD$): 7.50-7.35 (m, 5H), 7.00 (d, *J*=8.1 Hz, 0.8H), 6.88 (d, *J*=8.1 Hz, 0.2H), 6.70 (d, *J*=2.4 Hz, 0.2H), 6.69 (d, *J*=2.4 Hz, 0.8H), 6.61 (dd, *J*=8.1, 2.4 Hz, 0.8H), 6.58 (dd, *J*=8.1, 2.4 Hz, 0.2H), 4.50 (s, 0.4H), 4.34 (s, 1.6H), 3.59 (bs, 0.2H), 3.50 (bs, 0.8H), 3.28 (d, *J*=19.7 Hz, 1H), 3.16 (d, *J*=9.6 Hz, 1H), 3.03 (dd, *J*=19.7, 6.3 Hz, 0.8H), 2.97-2.83 (m, 0.2H), 2.74 (td, *J*=12.9, 3.2 Hz, 1H), 2.50-2.27 (m, 0.8H), 2.25-2.07 (m, 4H), 1.96-1.82 (m, 1H), 1.70-1.56 (m, 3H), 1.32 (d, *J*=13.2 Hz, 0.8H), 1.23 (d, *J*=13.2 Hz, 0.2H), 0.82 (d, *J*=6.8 Hz, 0.5H), 0.78 (d, *J*=6.8 Hz, 2.5H). LC/MS, *m/z* = 379 $[M + H]^+$ (Calc: 378).

In a similar manner **4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamide (Compound 20)** was synthesized from **Compound AP** using (bromomethyl)cyclopropane rather than 2-iodopropane. **Compound 20** was isolated as its TFA salt.

$^1H$ NMR: $\delta_H$ (400 MHz, $CD_3OD$): 6.93 (d, *J*=8.3 Hz, 1H), 6.69 (d, *J*=2.6 Hz, 1H), 6.58 (dd, *J*=8.1, 2.4 Hz, 1H), 3.80 (s, 1H), 3.32-3.09 (m, 2H), 3.02 (s, 2H), 2.98-2.86 (m, 1H), 2.58 (td, *J*=13.5, 3.4 Hz, 1H), 2.31-2.05 (m, 4H), 1.96-1.80 (m, 1H), 1.76-1.59 (m, 3H), 1.33 (d, *J*=14.7 Hz, 1H), 1.07-0.94 (m, 1H), 0.86 (d, *J*=6.8 Hz, 2.5H), 0.80 (d, *J*=6.8 Hz, 0.5H), 0.73-0.62 (m, 2H), 0.42-0.31 (m, 2H).

LC/MS, *m/z* = 343 $[M + H]^+$ (Calc: 342).

## EXAMPLE 15

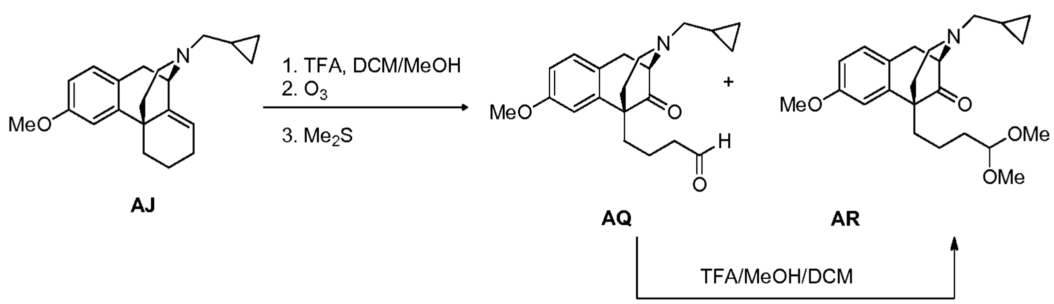


TFA (6.70 mL, 87 mmol) was added to **Compound AJ** (3.01 g, 8.70 mmol) in 4:1 DCM/MeOH (45 mL) and the solution was stirred at RT for 30 min. The solution was cooled to -78 °C and $O_3$ was bubbled through the solution for 12 min. Nitrogen was bubbled through the solution for 3 min and dimethyl sulfide (1.287 mL, 17.40 mmol) was added. The solution was stirred at -78 °C to RT for 22 h. Concentration and MPLC (0-100% acetone/hexanes followed by 0-15% MeOH/DCM, 80 g) led to the isolation of 1.31 g of **Compound AJ** and 1.14 g of **Compound AQ.**

TFA (3 mL) was added to the recovered **Compound AJ** in 4:1 DCM/MeOH (20 mL) and and the solution was stirred at RT for 30 min. The solution was cooled to -78 °C and $O_3$ was bubbled through the solution for 12 min. Nitrogen was bubbled through the solution for 3 min and dimethyl sulfide (0.6 mL, 2 equiv) was added. The solution was stirred at -78 °C to RT for 16.5 h.

TFA (3 mL) was added to **Compound AQ** in 4:1 DCM/MeOH (20 mL) and the reaction stirred at RT for 16.5 h. The reaction mixtures of **Compound AQ** and the second ozonolysis of **Compound AJ** were then combined. DCM

was added, washed with 1 M NaOH, dried with $Na_2SO_4$, and concentrated. Purification by MPLC (0-100% acetone/hexanes, 40 g) led to the isolation of **Compound AR** as an orange oil (740 mg, 22%).

$^1$H NMR: $\delta_H$ (400 MHz, DMSO-d6): 7.02 (d, *J*=8.6 Hz, 1H), 6.72 (dd, *J*=8.4, 2.6 Hz, 1H), 6.62 (d, *J*=2.6 Hz, 1H), 4.22 (t, *J*=5.7 Hz, 1H), 3.66 (s, 3H), 3.39 (d, *J*=5.3 Hz, 1H), 3.10 (s, 6H), 2.87 (dd, *J*=18.2, 5.9Hz, 1H), 2.60 (dd, *J*=12.3, 3.1 Hz, 1H), 2.39-2.22 (m, 4H), 1.96-1.65 (m, 3H), 1.56 (d, *J*=12.3 Hz, 1H), 1.51-1.37 (m, 2H), 1.25-1.12 (m, 1H), 1.09-0.94 (m, 1H), 0.74-0.63 (m, 1H), 0.41-0.32 (m, 2H), 0.04-0.00 (m, 2H). LC/MS, *m/z* = 388 [M + H]$^+$ (Calc: 387).

## EXAMPLE 16

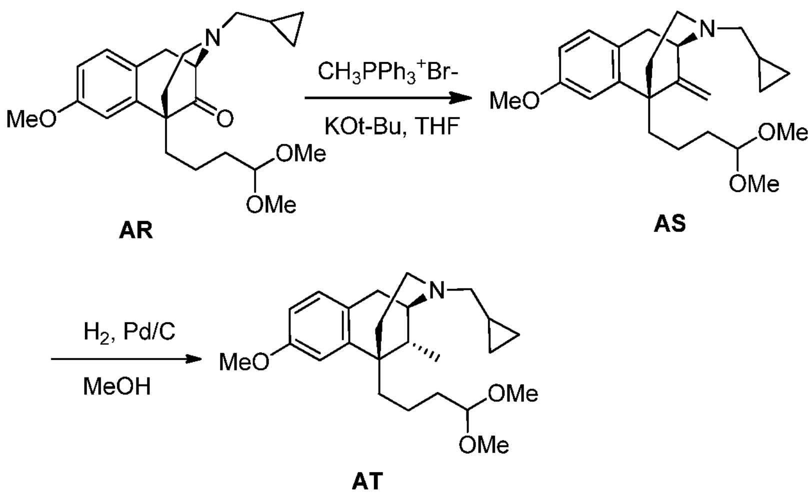


Potassium tert-butoxide (1 M in THF, 2.86 ml, 2.86 mmol) was added slowly to methyltriphenylphosphonium bromide (0.819 g, 2.292 mmol) in THF (3 ml) at 0 °C. The mixture was stirred at 0 °C for 5 min and **Compound AR** (0.740 g, 1.910 mmol) in THF (5 mL) was added. The reaction was heated at reflux for 2.5 h, concentrated, and purified by MPLC (0-50% acetone/hexanes, 40 g). **Compound AS** was isolated as a clear oil (440 mg). MeOH (20 mL) was added to **Compound AS** and and the solution run with the Pd/C cartridge on the H-Cube [ThalesNano, model HC-2.SS] at 60 °C at 60 bar $H_2$ in a recirculating fashion at 1 mL/min for 9 h followed by one pass-through. The solution was concentrated to yield **Compound AT** (420 mg, 57%).

LC/MS, *m/z* = 388 [M + H]$^+$ (Calc: 387).

## EXAMPLE 17

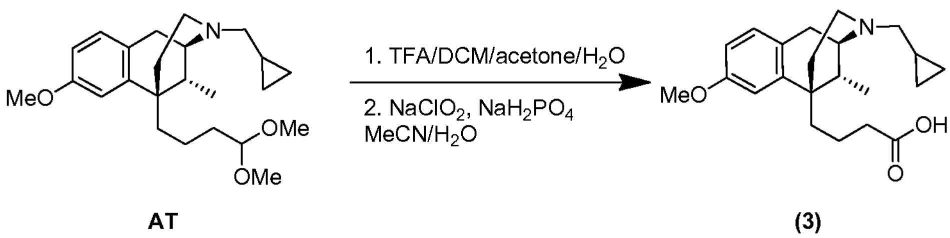


TFA (2 mL) was added to **Compound AT** (0.430 g, 1.110 mmol) in DCM (5 mL)/water (2 mL)/acetone (2 mL) and the solution was stirred at RT for 2 h and concentrated. The resulting material was taken up in ACN (5 mL) and a premixed solution of sodium chlorite (0.301 g, 3.33 mmol) and sodium phosphate monobasic monohydrate (0.459 g, 3.33 mmol) in water (5 mL) was added dropwise at 0 °C. The solution was stirred at 0 °C for 35 min and dilluted with DCM. The organic layer was dried with $Na_2SO_4$, concentrated, and purified by MPLC (0-100% acetone/hexanes, 12 g Gold). The TFA salt of **Compound 3** was isolated as a yellow oil (420 mg, 80%).

$^1$H NMR: $\delta_H$ (400 MHz, $CD_3OD$): 7.03 (d, *J*=8.6 Hz, 1H), 6.80 (d, *J*=2.4 Hz, 1H), 6.73 (dd, *J*=8.4, 2.2 Hz, 1H), 3.82 (s, 1H), 3.68 (s, 3H), 3.35-3.23 (m, 2H), 3.19-3.12 (m, 1H), 3.06 (s, 2H), 3.00-2.87 (m, 1H), 2.65-2.50 (m, 1H), 2.44-2.30

(m, 2H), 2.29-2.18 (m, 3H), 2.20-2.10 (m, 1H), 2.00-1.90 (m, 1H), 1.77-1.57 (m, 4H), 1.37 (d, *J*=14.5 Hz, 1H), 1.09-0.97 (m, 2H), 0.92-0.77 (m, 3H), 0.75-0.64 (m, 2H), 0.42-0.32 (m, 2H). LC/MS, *m/z* = 358 [M + H]$^+$ (Calc: 357).

**EXAMPLE 18**

**(*S*)-methyl 2-(4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamido)propanoate (Compound 21)**

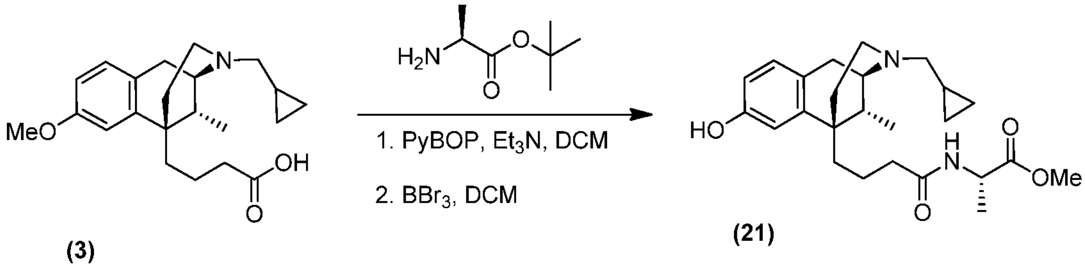


(3) (21)

TEA (0.027 mL, 0.191 mmol) was added to **Compound 3** (0.030 g, 0.064 mmol), H-Ala-OTBU, HCl (0.014 g, 0.076 mmol), and PyBOP (0.040 g, 0.076 mmol) in DCM (1 mL). The reaction was stirred at RT for 16.5 h and concentrated. The material was dissolved in DCM (1 mL) and boron tribromide (0.024 mL, 0.255 mmol) was added dropwise at 0 °C. The reaction was stirred at 0 °C to RT for 90 min. An additional aliquot of boron tribromide (0.024 mL, 0.255 mmol) was added. The reaction was stopped after 3 h, slowly quenched with 10 drops MeOH and concentrated. The resulting oil was purifed by preparatory HPLC [0-60% ACN/$H_2O$ (0.01% TFA)] to yield **Compound 21** as its TFA salt.
$^1$H NMR: $\delta_H$ (400 MHz, $CD_3OD$): 6.92 (d, *J*=8.3 Hz, 1H), 6.70 (d, *J*=2.6 Hz, 1H), 6.58 (dd, *J*=8.3, 2.6 Hz, 1H), 4.32 (q, *J*=7.4 Hz, 1H), 3.80 (s, 1H), 3.56 (s, 3H), 3.32-3.23 (m, 1H), 3.19-3.10 (m, 1H), 3.01 (s, 2H), 2.97-2.86 (m, 1H), 2.58 (td, *J*=13.0, 3.5, 1H), 2.32-2.25 (m, 3H), 2.25-2.11 (m, 2H), 1.98-1.83 (m, 1H), 1.76-1.64 (m, 4H), 1.37-1.25 (m, 2H), 1.30 (d, *J*=7.4 Hz, 3H), 1.05-0.99 (m, 2H), 0.85 (d, *J*=6.8 Hz, 2.5H), 0.79 (d, *J*=6.8 Hz, 0.5H), 0.72-0.65 (m, 2H), 0.43-0.31 (m, 2H).
LC/MS, *m/z* = 429 [M + H]$^+$ (Calc: 428).

In a similar manner ***N*-((*S*)-1-amino-1-oxopropan-2-yl)-4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamide (Compound 22)** was synthesized from **Compound 3** using H-Ala-$NH_2$, HCl rather than H-Ala-OTBU, HCl. **Compound 22** was isolated as its TFA salt.
$^1$H NMR: $\delta_H$ (400 MHz, $CD_3OD$): 6.92 (d, *J*=8.3 Hz, 1H), 6.68 (d, *J*=2.6 Hz, 1H), 6.57 (dd, *J*=8.3, 2.6 Hz, 1H), 4.27 (q, *J*=7.2 Hz, 1H), 3.79 (s, 1H), 3.32-3.25 (m, 1H), 3.18-3.08 (m, 1H), 3.02 (s, 2H), 2.98-2.90 (m, 1H), 2.59 (td, *J*=13.1, 3.0, 1H), 2.34-2.25 (m, 3H), 2.23-2.16 (m, 2H), 2.16-2.08 (m, 1H), 1.97-1.82 (m, 1H), 1.77-1.63 (m, 3H), 1.37-1.25 (m, 2H), 1.29 (d, *J*=7.2 Hz, 3H), 1.09-0.95 (m, 2H), 0.85 (d, *J*=6.8 Hz, 2.5H), 0.80 (d, *J*=6.8 Hz, 0.5H), 0.75-0.64 (m, 2H), 0.43-0.28 (m, 2H).
LC/MS, *m/z* = 414 [M + H]$^+$ (Calc: 413).

**EXAMPLE 19**

**methyl 4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanoate (Compound 23)**

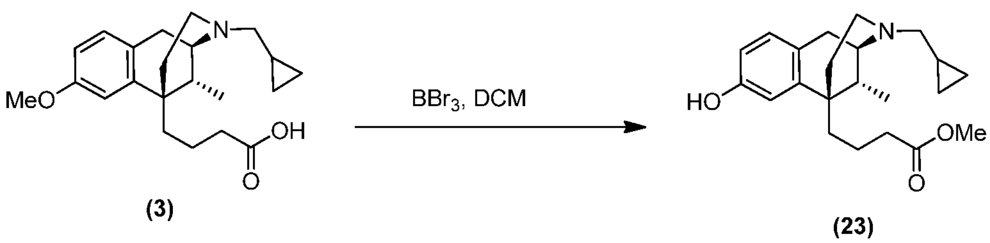


(3) (23)

Boron tribromide (0.018 mL, 0.191 mmol) was added dropwise to **Compound 3** (0.030 g, 0.064 mmol) in DCM (1 mL) at 0 °C. The reaction was quenched by slow addition of 10 drops of MeOH and concentrated. The resulting oil was purifed by preparatory HPLC [0-60% ACN/$H_2O$ (0.01% TFA)] to yield **Compound 23** as its TFA salt.
$^1H$ NMR: $\delta_H$ (400 MHz, $CD_3OD$): 6.93 (d, *J*=8.3 Hz, 1H), 6.67 (d, *J*=2.6 Hz, 1H), 6.58 (dd, *J*=8.3, 2.6 Hz, 1H), 3.80 (s, 1H), 3.33-3.24 (m, 1H), 3.19-3.10 (m, 1H), 3.02 (s, 2H), 2.98-2.90 (m, 1H), 2.58 (td, *J*=13.1, 3.3, 1H), 2.47-2.32 (m, 2H), 2.27-2.15 (m, 2H), 1.94-1.83 (m, 1H), 1.76-1.62 (m, 3H), 1.34 (d, *J*=14.3 Hz, 1H), 1.08-0.94 (m, 1H), 0.86 (d, *J*=6.8 Hz, 2.5H), 0.81 (d, *J*=6.8 Hz, 0.5H), 0.73-0.62 (m, 2H), 0.41-0.31 (m, 2H).
LC/MS, *m/z* = 358 $[M + H]^+$ (Calc: 357).

Other embodiments of the invention will be apparent to those skilled in the art from consideration of the specification and practice of the invention disclosed herein.

## Claims

1. A compound of Formula I:

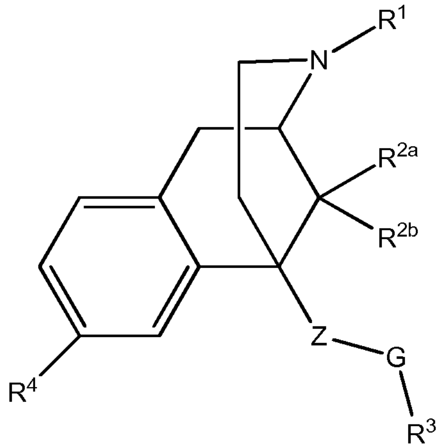

wherein

$R^1$ is selected from the group consisting of -($C_3$-$C_{12}$)cycloalkyl, ($C_3$-$C_{12}$)cycloalkyl-($C_1$-$C_6$)alkyl-, and -(6- to 14-membered)aryl, each of which is optionally substituted by 1, 2 or 3 independently selected $R^9$ groups;
$R^{2a}$ and $R^{2b}$ are each independently selected from:

(a) -H; or
(b) -($C_1$-$C_5$)alkyl, -($C_2$-$C_5$)alkenyl, or -($C_2$-$C_5$)alkynyl;

Z is absent or -$(CH_2)_m$-, optionally substituted with 1 or 2 independently selected -($C_1$-$C_6$)alkyl;
G is selected from the group consisting of:

a) -($C_1$-$C_6$)alkylene, -($C_2$-$C_6$)alkenylene; or
b) -C(=O); or
c) $NR^8$;

$R^3$ is selected from the group consisting of hydrogen, -($C_1$-$C_{10}$)alkyl, -($C_2$-$C_{12}$)alkenyl, -C(=O)-($C_1$-$C_6$)alkyl, -C(=O)-(6- to 14-membered)aryl, -C(=O)-(5- to 12-membered)heteroaryl, -($C_2$-$C_{12}$)alkynyl, -($C_1$-$C_{10}$)alkoxy, -$(OCH_2CH_2)_s$-O($C_1$-$C_6$)alkyl, -$(CH_2CH_2O)_s$-($C_1$-$C_6$)alkyl, -$NH_2$, -NH($C_1$-$C_6$)alkyl, CN, -$CONR^5R^6$, -($C_1$-$C_6$)alkyl-$CONR^5R^6$, -$COOR^7$, -($C_1$-$C_6$)alkyl-$COOR^7$, -($C_1$-$C_6$)alkoxy-$COOR^7$, -C(=O)-$(CH_2)_n$-$COOR^7$, -C(=O)-$(CH_2)_n$-$CONR^5R^6$, -($C_3$-$C_{12}$)cycloalkyl, (($C_3$-$C_{12}$)cycloalkyl)-($C_1$-$C_6$)alkyl-, -($C_4$-$C_{12}$)cycloalkenyl, (($C_4$-$C_{12}$)cycloalkenyl)-($C_1$-$C_6$)alkyl-, -($C_6$-$C_{14}$)bicycloalkyl, (($C_6$-$C_{14}$)bicycloalkyl)-($C_1$-$C_6$)alkyl-, -($C_8$-$C_{20}$)tricycloalkyl, (($C_8$-$C_{20}$)tricycloalkyl)-($C_1$-$C_6$)alkyl-, -($C_7$-$C_{14}$)bicycloalkenyl, (($C_7$-$C_{14}$)bicycloalkenyl)-($C_1$-$C_6$)alkyl-, -($C_8$-$C_{20}$)tricycloalkenyl, (($C_8$-$C_{20}$)tricycloalkenyl)-($C_1$-$C_6$)alkyl-, -(6- to 14-membered)aryl, ((6-to 14-membered)aryl)-($C_1$-$C_6$)alkyl-, -(7- to 12-membered)bicyclic ring system, ((7- to 12-membered)bicyclic ring system)-($C_1$-$C_6$)alkyl-, -(7- to 12-membered)bicyclic aryl, ((7- to 12-membered)bicyclic aryl)-($C_1$-$C_6$)alkyl-, -(5- to

12-membered)heteroaryl, ((5- to 12-membered)heteroaryl)-($C_1$-$C_6$)alkyl-, -(3- to 12-membered)heterocycle, ((3- to 12 membered)heterocycle)-($C_1$-$C_6$)alkyl-, -(7- to 12-membered)bicycloheterocycle, and ((7- to 12-membered)bicycloheterocycle)-($C_1$-$C_6$)alkyl-; each of which is optionally substituted with one, two, or three substituents independently selected from the group consisting of -OH, (=O),
halo, -C(halo)$_3$, -CH(halo)$_2$, -$CH_2$(halo), -($C_1$-$C_6$)alkyl, halo($C_1$-$C_6$)alkyl-, -($C_2$-$C_6$)alkenyl, -($C_2$-$C_6$)alkynyl, hydroxy($C_1$-$C_6$)alkyl-, dihydroxy($C_1$-$C_6$)alkyl-, -($C_1$-$C_6$)alkoxy, (($C_1$-$C_6$)alkoxy)-C(=O)-($C_1$-$C_6$)alkoxy-, -$NH_2$, -$NR^5R^6$, -NH($C_1$-$C_6$)alkyl, -($C_1$-$C_6$)alkyl-NH($C_1$-$C_6$)alkyl-$R^{14}$, -CN, -SH, -$OR^4$, -$CONR^5R^6$, -($C_1$-$C_6$alkyl)-C(=O)-$NR^5R^6$, -$COOR^7$, -($C_1$-$C_6$)alkyl-$COOR^7$, -($C_1$-$C_6$)alkoxy-$COOR^7$, -($OCH_2CH_2$)$_s$-O($C_1$-$C_6$)alkyl, -($CH_2CH_2O$)$_s$-($C_1$-$C_6$)alkyl, (($C_1$-$C_6$)alkyl)sulfonyl($C_1$-$C_6$)alkyl-, -NH-$SO_2$($C_1$-$C_6$)alkyl, -N-($SO_2$-($C_1$-$C_6$)alkyl)$_2$, -C(=NH)-$NH_2$, -NH-C(=O)-($C_1$-$C_6$)alkyl, -NH-C(=O)-$NH_2$, -NH-C(=O)-NH-($C_1$-$C_6$)alkyl, -NH-C(=O)-(6-to 14-membered)aryl, -NH-C(=O)-($C_1$-$C_6$)alkyl-(6-to 14- membered)aryl, -NH-($C_1$-$C_6$)alkyl-$COOR^7$, -NH-C(=O)-($C_1$-$C_6$)alkyl-$COOR^7$, -NH-C(=O)-CH($NH_2$)-($C_1$-$C_6$)alkyl-C(=O)-$OR^7$, -($C_3$-$C_{12}$)cycloalkyl, (($C_3$-$C_{12}$)cycloalkyl)-($C_1$-$C_6$)alkyl-, -(6- to 14-membered)aryl, -(6- to 14-membered)aryloxy, -($C_1$-$C_6$)alkoxyC(O)$NR^5R^6$, -NH-($C_1$-$C_6$)alkylC(O)-$NR^5R^6$, -C(O)NH-($C_1$-$C_6$)alkyl-$COOR^7$, ((6- to 14-membered)aryl)-($C_1$-$C_6$)alkyl-, -(5-to 12-membered)heteroaryl, ((5- to 12-membered)heteroaryl)-($C_1$-$C_6$)alkyl-, -(3- to 12-membered)heterocycle, ((3- to 12-membered)heterocycle)-($C_1$-$C_6$)alkyl-, -(7- to 12-membered)bicycloheterocycle, and ((7- to 12-membered)bicycloheterocycle)-($C_1$-$C_6$)alkyl-;
$R^4$ is selected from

(a) -H, -OH, halo, -C(halo)$_3$, -CH(halo)$_2$, -$CH_2$(halo), COOH, or $CONH_2$; or
(b) -($C_1$-$C_5$)alkyl, -($C_2$-$C_5$)alkenyl, -($C_2$-$C_5$)alkynyl, -($CH_2$)$_n$-O-($CH_2$)$_n$-$CH_3$, or -($C_1$-$C_5$)alkoxy, each of which is optionally substituted with 1, 2, or 3 independently selected $R^9$ groups;

$R^5$ and $R^6$ are each independently selected from

(a) hydrogen, -OH, halo, -C(halo)$_3$, -CH(halo)$_2$, -$CH_2$(halo); or
(b) -($C_1$-$C_6$)alkyl, -($C_2$-$C_5$)alkenyl, -($C_2$-$C_5$)alkynyl, -($CH_2$)$_n$-O-($CH_2$)$_n$-$CH_3$, -($C_1$-$C_6$)alkoxy, each of which is optionally substituted with 1, 2, or 3 independently selected $R^9$ groups; or
(c) -($C_3$-$C_8$)cycloalkyl, (($C_3$-$C_8$)cycloalkyl)-($C_1$-$C_6$)alkyl-, -$COOR^7$, -($C_1$-$C_6$)alkyl-$COOR^7$, -$CONH_2$, or ($C_1$-$C_6$)alkyl-CONH-; or
(d) $R^5$ and $R^6$ together with the nitrogen atom to which they are attached form a (4-to 8-membered)heterocycle;

$R^7$ is selected from the group consisting of hydrogen, -($C_1$-$C_6$)alkyl, -($C_2$-$C_6$)alkenyl, -($C_2$-$C_6$)alkynyl, -($C_3$-$C_{12}$)cycloalkyl, -($C_4$-$C_{12}$)cycloalkenyl, (($C_3$-$C_{12}$)cycloalkyl)-($C_1$-$C_6$)alkyl-, and (($C_4$-$C_{12}$)cycloalkenyl)-($C_1$-$C_6$)alkyl-;
$R^8$ is selected from H, -($C_1$-$C_6$)alkyl, -($C_2$-$C_6$)alkenyl, -($C_2$-$C_6$)alkynyl, -($C_1$-$C_{10}$)alkoxy, -($C_3$-$C_{12}$)cycloalkyl, -($C_3$-$C_{12}$)cycloalkenyl, (($C_3$-$C_{12}$)cycloalkyl)-($C_1$-$C_6$)alkyl-, (($C_3$-$C_{12}$)cycloalkenyl)-($C_1$-$C_6$)alkyl-, -C(=O)($C_1$-$C_6$)alkyl or $SO_2$($C_1$-$C_6$)alkyl;
each $R^9$ is independently selected from -OH, halo, -($C_1$-$C_{10}$)alkyl, -($C_2$-$C_{10}$)alkenyl, -($C_2$-$C_{10}$)alkynyl, -($C_1$-$C_{10}$)alkoxy, -($C_3$-$C_{12}$)cycloalkyl, -CHO, -C(O)OH, -C(halo)$_3$, - CH(halo)$_2$, $CH_2$(halo), or -($CH_2$)$_n$-O-($CH_2$)$_n$-$CH_3$;
each $R^{14}$ is independently selected from the group consisting of -$COOR^7$, -($C_1$-$C_6$)alkyl-$COOR^7$, -C(=O)-($C_1$-$C_6$)alkyl-$COOR^7$, -($C_1$-$C_6$)alkyl-C(=O)-($C_1$-$C_6$)alkyl-$COOR^7$, $CONH_2$, and -($C_1$-$C_6$)alkyl-CONH;
m is an integer 1, 2, 3, 4, 5, or 6;
n is an integer 0, 1, 2, 3, 4, 5, or 6;
s in an integer 1, 2, 3, 4, 5, or 6;
and the pharmaceutically acceptable salts and solvates thereof;
provided that Z-G-$R^3$ is not unsubstituted ($C_1$-$C_6$)alkyl.

**2.** The compound of claim 1, and the pharmaceutically acceptable salts and solvates thereof, wherein
$R^3$ is selected from the group consisting of hydrogen, -($C_1$-$C_{10}$)alkyl, -($C_2$-$C_{12}$)alkenyl, -($C_2$-$C_{12}$)alkynyl, -($C_1$-$C_{10}$)alkoxy, -($OCH_2CH_2$)$_s$-O($C_1$-$C_6$)alkyl, - ($CH_2CH_2O$)$_s$-($C_1$-$C_6$)alkyl, -$NH_2$, -NH($C_1$-$C_6$)alkyl, CN, -$CONR^5R^6$, -($C_1$-$C_6$)alkyl-CO-$NR^5R^6$, -$COOR^7$, -($C_1$-$C_6$)alkyl-CO-$OR^7$, -($C_1$-$C_6$)alkoxy-$COOR^7$, -CO-($CH_2$)$_n$-$COOR^7$, -CO-($CH_2$)$_n$-CO-$NR^5R^6$, -($C_3$-$C_{12}$)cycloalkyl, (($C_3$-$C_{12}$)cycloalkyl)-($C_1$-$C_6$)alkyl-, -($C_4$-$C_{12}$)cycloalkenyl, (($C_4$-$C_{12}$)cycloalkenyl)-($C_1$-$C_6$)alkyl-, -($C_6$-$C_{14}$)bicycloalkyl, (($C_6$-$C_{14}$)bicycloalkyl)-($C_1$-$C_6$)alkyl-, -($C_8$-$C_{20}$)tricycloalkyl, (($C_8$-$C_{20}$)tricycloalkyl)-($C_1$-$C_6$)alkyl-, -($C_7$-$C_{14}$)bicycloalkenyl, (($C_7$-$C_{14}$)bicycloalkenyl)-($C_1$-$C_6$)alkyl-, -($C_8$-$C_{20}$)tricycloalkenyl, (($C_8$-$C_{20}$)tricycloalkenyl)-($C_1$-$C_6$)alkyl-, -(6- to 14-membered)aryl, ((6- to 14-membered)ar-

yl)-($C_1$-$C_6$)alkyl-, -(7- to 12-membered)bicyclic ring system, ((7- to 12-membered)bicyclic ring system)-($C_1$-$C_6$)alkyl-, -(7- to 12-membered)bicyclic aryl, ((7- to 12-membered)bicyclic aryl)-($C_1$-$C_6$)alkyl-, -(5- to 12-membered)heteroaryl, ((5- to 12-membered)heteroaryl)-($C_1$-$C_6$)alkyl-, -(3- to 12-membered)heterocycle, ((3- to 12 membered)heterocycle)-($C_1$-$C_6$)alkyl-, -(7- to 12-membered)bicycloheterocycle, and ((7- to 12-membered)bicycloheterocycle)-($C_1$-$C_6$)alkyl-; each of which is optionally substituted with one, two, or three substituents independently selected from the group consisting of -OH, (=O), halo, -C(halo)$_3$, -CH(halo)$_2$, -$CH_2$(halo), -($C_1$-$C_6$)alkyl, halo($C_1$-$C_6$)alkyl-, -($C_2$-$C_6$)alkenyl, -($C_2$-$C_6$)alkynyl, hydroxy($C_1$-$C_6$)alkyl-, dihydroxy($C_1$-$C_6$)alkyl-, -($C_1$-$C_6$)alkoxy, (($C_1$-$C_6$)alkoxy)CO($C_1$-$C_6$)alkoxy-, -$NH_2$, -NH($C_1$-$C_6$)alkyl, -($C_1$-$C_6$)alkyl-NH($C_1$-$C_6$)alkyl-$R^{14}$, -CN, -SH, -$OR^4$, -$CONR^5R^6$, -($C_1$-$C_6$alkyl)-CO-$NR^5R^6$, -$COOR^7$, -($C_1$-$C_6$)alkyl-CO-$OR^7$, -($C_1$-$C_6$)alkoxy-$COOR^7$, -($OCH_2CH_2$)$_s$-O($C_1$-$C_6$)alkyl, -($CH_2CH_2O$)$_s$-($C_1$-$C_6$)alkyl, (($C_1$-$C_6$)alkyl)sulfonyl($C_1$-$C_6$)alkyl-, -NH-$SO_2$($C_1$-$C_6$)alkyl, -N($SO_2$($C_1$-$C_6$)alkyl)$_2$, -C(=NH)$NH_2$, -NH-CO-($C_1$-$C_6$)alkyl, -NH-CO-$NH_2$, -NH-C(=O)-NH-($C_1$-$C_6$)alkyl, -NH-C(=O)-(6- to 14-membered)aryl, -NH-C(=O)-($C_1$-$C_6$)alkyl-(6- to 14- membered)aryl, -NH-($C_1$-$C_6$)alkyl-CO-$OR^7$, -NH-C(=O)-($C_1$-$C_6$)alkyl-CO-$OR^7$, -NH-C(=O)-CH($NH_2$)-($C_1$-$C_6$)alkyl-CO-$OR^7$, -($C_3$-$C_{12}$)cycloalkyl, (($C_3$-$C_{12}$)cycloalkyl)-($C_1$-$C_6$)alkyl-, -(6- to 14-membered)aryl, -(6- to 14-membered)aryloxy, -($C_1$-$C_6$)alkoxyC(O)$NR^5R^6$, -NH-($C_1$-$C_6$)alkylC(O)-$NR^5R^6$, -C(O)NH-($C_1$-$C_6$)alkyl-$COOR^7$, ((6- to 14-membered)aryl)-($C_1$-$C_6$)alkyl-, -(5-to 12-membered)heteroaryl, ((5- to 12-membered)heteroaryl)-($C_1$-$C_6$)alkyl-, -(3- to 12-membered)heterocycle, ((3- to 12-membered)heterocycle)-($C_1$-$C_6$)alkyl-, -(7- to 12-membered)bicycloheterocycle, and ((7- to 12-membered)bicycloheterocycle)-($C_1$-$C_6$)alkyl-.

**3.** The compound of claim 1, and the pharmaceutically acceptable salts and solvates thereof, wherein $R^{2a}$ and $R^{2b}$ are each independently selected from:

(a) -H; or
(b) -($C_1$-$C_5$)alkyl;

$R^4$ is selected from

(a) -H, or
(b) ($C_1$-$C_5$)alkoxy, which is optionally substituted with 1, 2, or 3 independently selected $R^9$ groups.

**4.** The compound of claim 1, and the pharmaceutically acceptable salts and solvates thereof, wherein
$R^1$ is ($C_3$-$C_{12}$)cycloalkyl-($C_1$-$C_6$)alkyl-, which is optionally substituted by 1, 2 or 3 independently selected $R^9$ groups;
$R^{2a}$ and $R^{2b}$ are each independently -($C_1$-$C_5$)alkyl;
$R^4$ is selected from -OH or -$OCH_3$.

**5.** A compound of any one of claims 1 to 4 having the Formula IA, IB, IC or ID:

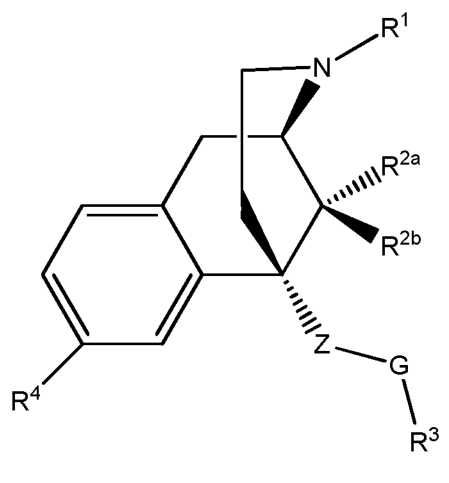

IA

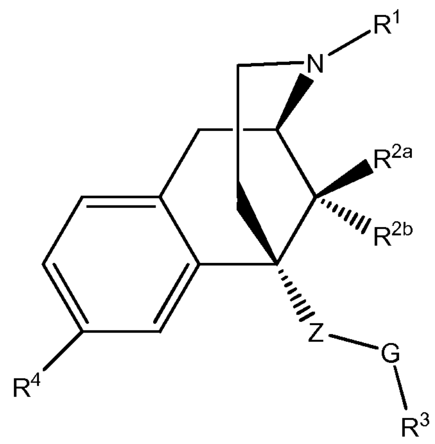

IB

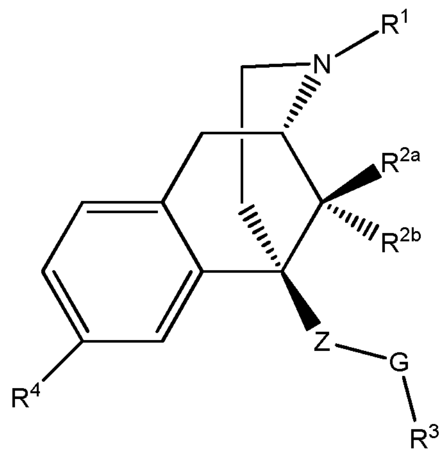

IC

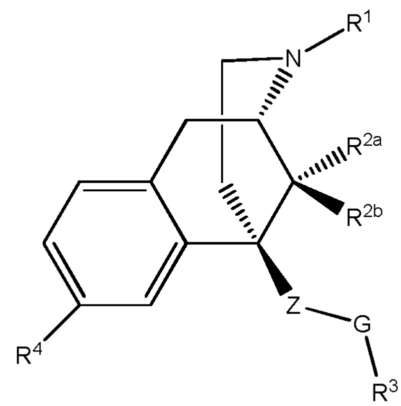

ID

**6.** A compound of any one of claims 1 to 5, wherein Z is absent, methylene, ethylene, propylene or butylene.

**7.** A compound of any one of claims 1 to 6, wherein G is selected from

(i) -C(=O);
(ii) -($C_1$-$C_6$)alkylene, and preferably methylene, ethylene, and propylene;
(iii) -($C_2$-$C_6$)alkenylene and preferably ethenylene and propenylene; or
(iv) G is $NR^8$, wherein preferably $R^8$ is selected from the group consisting of -H, -($C_1$-$C_6$)alkyl, -($C_1$-$C_{10}$)alkoxy, -($C_3$-$C_{12}$)cycloalkyl, and (($C_3$-$C_{12}$)cycloalkyl)-($C_1$-$C_6$)alkyl-.

**8.** A compound of any one of claims 1 and 3 to 7, wherein $R^3$ is selected from

(i) H, ($C_1$-$C_6$)alkyl, $NH_2$, NH($C_1$-$C_6$)alkyl, or -C(=O)-($C_1$-$C_6$)alkyl;
(ii) -$CONR^5R^6$ and -($C_1$-$C_6$)alkyl-$CONR^5R^6$, wherein preferably $R^5$ or $R^6$ are each independently selected from -H or -($C_1$-$C_6$)alkyl;
(iii) $COOR^7$, wherein preferably $R^7$ is H or -($C_1$-$C_6$)alkyl;
(iv) -(6- to 14-membered)aryl or ((6- to 14-membered)aryl)-($C_1$-$C_6$)alkyl-;
and preferably phenyl or benzyl;
(v) -(3- to 12-membered)heterocycle, ((3- to 12 membered)heterocycle)-($C_1$-$C_6$)alkyl-, -(5- to 12-membered)heteroaryl, or ((5- to 12-membered)heteroaryl)-($C_1$-$C_6$)alkyl-;
(vi) -($C_1$-$C_{10}$)alkoxy; or
(vii) -C(=O)-(5- to 12-membered)heteroaryl or -C(=O)-(6- to 14-membered)aryl;

and wherein preferably $R^3$ is substituted with one, two or three substituents independently selected from the group consisting of -$COOR^7$, -$NR^5R^6$, -$CONR^5R^6$, phenyl, benzyl, -NH-C(=O)-(6- to 14-membered)aryl, with -NH-C(=O)-($C_1$-$C_6$)alkyl-(6- to 14-membered)aryl, -OH, hydroxy($C_1$-$C_6$)alkyl-, and dihydroxy($C_1$-$C_6$)alkyl.

**9.** A compound of any one of claims claim 1 and 3 to 5, wherein Z-G-$R^3$ is selected from

(i) -$CH_2$-COOH, -$(CH_2)_3$-COOH, $(CH_2)_3$-$COOCH_3$, -$(CH_2)_3$-$CONH_2$; -$(CH_2)_2$-O-(5-to 12-membered)heteroaryl-$COOR^7$ or -$(CH_2)_3$-O-(5-to 12-membered)heteroaryl-$COOR^7$, -$(CH_2)_3$-$CONR^5R^6$, -$(CH_2)_3$-C(=O)-NH-($C_1$-$C_6$)alkyl-$COOR^7$, dihydroxy($C_1$-$C_6$)alkyl, -($C_1$-$C_6$)alkenyl-$COOR^7$, -($C_1$-$C_6$)alkyl-NH-($C_1$-$C_6$)alkyl, -$(CH_2)_3$-C(=O)-NH-($C_1$-$C_6$)alkyl-C(=O)-$NH_2$, or -($C_1$-$C_6$)alkyl-C(=O)-NH-CH($CH_3$)-$CONH_2$; or
(ii) -$(CH_2)_2$-((6- to 14-membered)aryl)-$COOR^7$, -$(CH_2)_2$-NH-C(=O)-((6- to 14-membered)aryl)-$COOR^7$, -$(CH_2)_2$-NH-C(=O)-((5- to 12-membered)heteroaryl), -$(CH_2)_2$-NH-C(=O)-((6- to 14-membered)aryl)-$COOR^7$, -$(CH_2)_2$-NH-C(=O)-((5- to 12-membered)heteroaryl)-$COOR^7$, or -$(CH_2)_2$-O-((6- to 14-membered)aryl)-$COOR^7$; or
(iii) -$(CH_2)_2$-NH-CO-$CH_2$-$NH_2$, -($C_1$-$C_6$)alkyl-O-$CH_2$-phenyl, -($C_1$-$C_6$)alkyl-OH, -($C_1$-$C_6$)alkyl-furanyl, or -($C_1$-$C_6$)alkyl-C(=O)H or -($C_1$-$C_6$)alkenyl; or
(iv) -$(CH_2)_2$-NH-CO-$CH_2$-$NR^5R^6$.

**10.** A compound of claim 1 selected from the group consisting of:

(2*S*)-2-(2-((6*R*,11*R*)-3-(cyclopropylmethyl)-8-hydroxy-11-methyl-1,2,3,4,5,6-hexahydro-2,6-methanobenzo[*d*]azocin-6-yl)acetamido)propanamide;
4-((6*S*,11*R*)-3-(cyclopropylmethyl)-8-hydroxy-11-methyl-1,2,3,4,5,6-hexahydro-2,6-methanobenzo[*d*]azocin-6-yl)butanamide;
4-((6*S*,11*R*)-3-(cyclopropylmethyl)-8-methoxy-11-methyl-1,2,3,4,5,6-hexahydro-2,6-methanobenzo[*d*]azocin-6-yl)butanoic acid;
4-((6*S*,11*R*)-3-(cyclopropylmethyl)-8-hydroxy-11-methyl-1,2,3,4,5,6-hexahydro-2,6-methanobenzo[*d*]azocin-6-yl)butanoic acid;
2-((6*R*,11*R*)-3-(cyclopropylmethyl)-8-methoxy-11-methyl-1,2,3,4,5,6-hexahydro-2,6-methanobenzo[*d*]azocin-6-yl)acetic acid;
(2*S*)-3-((6*R*,11*R*)-3-(cyclopropylmethyl)-8-methoxy-11-methyl-1,2,3,4,5,6-hexahydro-2,6-methanobenzo[*d*]azocin-6-yl)propane-1,2-diol;
(2*S*)-2-(2-((6*R*,11*R*)-3-(cyclopropylmethyl)-8-methoxy-11-methyl-1,2,3,4,5,6-hexahydro-2,6-methanobenzo[*d*]azocin-6-yl)acetamido)propanamide;
(*E*)-methyl 4-((6*S*,11*R*)-3-(cyclopropylmethyl)-8-methoxy-11-methyl-1,2,3,4,5,6-hexahydro-2,6-methanobenzo[*d*]azocin-6-yl)but-2-enoate;
4-((6*S*,11*R*)-3-(cyclopropylmethyl)-8-methoxy-11-methyl-1,2,3,4,5,6-hexahydro-2,6-methanobenzo[*d*]azocin-6-yl)-N-isobutylbutan-1-amine;
(2*R*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol;
(2*S*)-5-((6*S*,11*R*)-3-(cyclopropylmethyl)-8-methoxy-11-methyl-1,2,3,4,5,6-hexahydro-2,6-methanobenzo[*d*]azocin-6-yl)pentane-1,2-diol;
(6*S*,11*R*)-6-(4-(benzyloxy)butyl)-3-(cyclopropylmethyl)-8-methoxy-11-methyl-1,2,3,4,5,6-hexahydro-2,6-methanobenzo[*d*]azocine;
4-((6*S*,11*R*)-3-(cyclopropylmethyl)-8-methoxy-11-methyl-1,2,3,4,5,6-hexahydro-2,6-methanobenzo[*d*]azocin-6-yl)butan-1-ol;
N-((*S*)-1-amino-1-oxopropan-2-yl)-4-((6*S*,11*R*)-3-(cyclopropylmethyl)-8-methoxy-11-methyl-1,2,3,4,5,6-hexahydro-2,6-methanobenzo[*d*]azocin-6-yl)butanamide;
(2*R*,6*S*,11*S*)-3-(cyclopropylmethyl)-6-(3-(furan-2-yl)propyl)-8-methoxy-11-methyl-1,2,3,4,5,6-hexahydro-2,6-methanobenzo[*d*]azocine;
5-(2-((2*R*,6*S*,11*R*)-3-(cyclopropylmethyl)-8-hydroxy-11-methyl-1,2,3,4,5,6-hexahydro-2,6-methanobenzo[*d*]azocin-6-yl)ethoxy)nicotinic acid;
4-((2*R*,6*R*,11*R*)-3-benzyl-8-hydroxy-11-methyl-1,2,3,4,5,6-hexahydro-2,6-methanobenzo[*d*]azocin-6-yl)butanamide;
4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamide;
(*S*)-methyl 2-(4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamido)propanoate;
*N*-((*S*)-1-amino-1-oxopropan-2-yl)-4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamide;
methyl 4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanoate;
3-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoic acid;
4-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoic acid;
methyl 3-((2-((2*R*,6*R*,11*R*)-3-(cyclopropylmethyl)-8-methoxy-11-methyl-1,2,3,4,5,6-hexahydro-2,6-methanobenzo[*d*]azocin-6-yl)ethyl)carbamoyl)benzoate;
4-(2-((2*R*,6*S*,11*R*)-3-(cyclopropylmethyl)-8-methoxy-11-methyl-1,2,3,4,5,6-hexahydro-2,6-methanobenzo[*d*]azocin-6-yl)ethoxy)benzoic acid;
4-(2-((2*R*,6*S*,11*R*)-3-(cyclopropylmethyl)-8-hydroxy-11-methyl-1,2,3,4,5,6-hexahydro-2,6-methanobenzo[*d*]azocin-6-yl)ethoxy)benzoic acid;
2-((2*S*,6*R*,11*S*)-3-(cyclopropylmethyl)-8-methoxy-11-methyl-1,2,3,4,5,6-hexahydro-2,6-methanobenzo[*d*]azocin-6-yl)acetic acid;
*N*-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)-2-(dimethylamino)acetamide;
2-amino-*N*-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)acetamide;

and the pharmaceutically acceptable salts and solvates thereof.

**11.** A pharmaceutical composition comprising an effective amount of a compound of any one of claims 1 to 10, or a pharmaceutically salt or solvate thereof, and a pharmaceutically acceptable carrier or excipient.

**12.** An *in-vitro* method for modulating opioid receptor function in a cell, comprising contacting a cell capable of expressing an opioid receptor with an effective amount of a compound of any one of claims 1 to 10, or a pharmaceutically acceptable salt or solvate thereof,
wherein preferably:

(i) the compound modulates μ-opioid receptor function, and
wherein more preferably the compound acts as an agonist or as an antagonist at the μ-opioid receptor; or
(ii) the compound acts as an agonist at the κ-opioid receptor; or
(iii) the compound modulates ORL-1 receptor function, and wherein more preferably the compound acts as an antagonist at the ORL-1 receptor.

**13.** A method for preparing a composition, comprising the step of admixing a compound according to any one of claims 1 to 10, or a pharmaceutically acceptable salt or solvate thereof, with a pharmaceutically acceptable carrier or

excipient.

**14.** A compound of any one of claims 1 to 10 or a pharmaceutically acceptable salt or solvate thereof, for use in the treatment, prevention, or amelioration of a Condition, wherein preferably the Condition is pain or constipation.

**15.** Use of a compound according to any one of claims 1 to 10 or a pharmaceutically acceptable salt or solvate thereof in the manufacture of a medicament useful for treating or preventing a Condition, wherein preferably the Condition is pain or constipation.

**Patentansprüche**

**1.** Verbindung der Formel I:

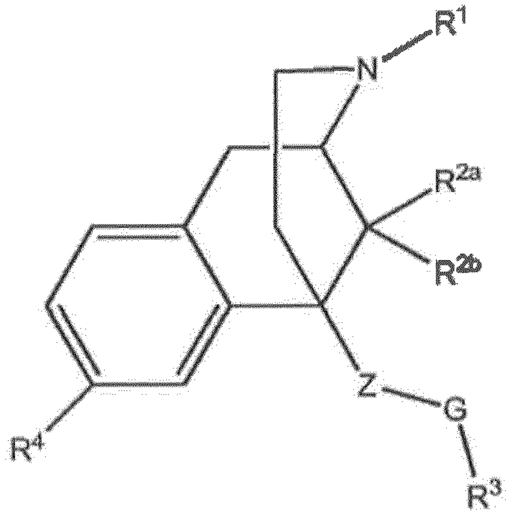

wobei

$R^1$ ausgewählt ist aus der Gruppe bestehend aus -($C_3$-$C_{12}$)-Cycloalkyl, ($C_3$-$C_{12}$)-Cycloalkyl-($C_1$-$C_6$)-Alkyl-, und -(6- bis 14-gliedriges)-Aryl, wovon jedes optional durch 1, 2 oder 3 unabhängig ausgewählte $R^9$-Gruppen substituiert ist;
$R^{2a}$ und $R^{2b}$ jeweils unabhängig ausgewählt sind aus:

(a) -H; oder
(b) -($C_1$-$C_5$)-Alkyl, -($C_2$-$C_5$)-Alkenyl, oder -($C_2$-$C_5$)-Alkynyl;

Z abwesend oder -$(CH_2)_m$- ist, optional substituiert mit 1 oder 2 unabhängig ausgewählten -($C_1$-$C_6$)-Alkyl;
G ausgewählt ist aus der Gruppe bestehend aus:

a) -($C_1$-$C_6$)-Alkylen, -($C_2$-$C_6$)-Alkenylen; oder
b) -C(=O); oder
c) $NR^8$;

$R^3$ ausgewählt ist aus der Gruppe bestehend aus Wasserstoff,-($C_1$-$C_{10}$)-Alkyl, -($C_2$-$C_{12}$)-Alkenyl, -C(=O)-($C_1$-$C_6$)-Alkyl, -C(=O)-(6- bis 14-gliedriges)-Aryl, -C(=O)-(5- bis 12-gliedriges)-Heteroaryl, -($C_2$-$C_{12}$)-Alkynyl, -($C_1$-$C_{10}$)-Alkoxy, -$(OCH_2CH_2)_s$-O($C_1$-$C_6$)-Alkyl, -$(CH_2CH_2O)_s$-($C_1$-$C_6$)-Alkyl, -$NH_2$, -NH($C_1$-$C_6$)-Alkyl, CN, -$CONR^5R^6$, -($C_1$-$C_6$)-Alkyl-$CONR^5R^6$, -$COOR^7$, -($C_1$-$C_6$)-Alkyl-$COOR^7$, - ($C_1$-$C_6$)-Alkoxy, -$COOR^7$, -C(=O)-$(CH_2)_n$-$COOR^7$, -C(=O)-$(CH_2)_n$-$CONR^5R^6$, -($C_3$-$C_{12}$)-Cycloalkyl, (($C_3$-$C_{12}$)-Cycloalkyl)-($C_1$-$C_6$)-Alkyl, -($C_4$-$C_{12}$)-Cycloalkenyl, (($C_4$-$C_{12}$)-Cycloalkenyl)-($C_1$-$C_6$)-Alkyl-, -($C_6$-$C_{14}$)-Bicycloalkyl, (($C_6$-$C_{14}$)bicycloalkyl)-($C_1$-$C_6$)-Alkyl-, -($C_8$-$C_{20}$)-Tricycloalkyl, (($C_8$-$C_{20}$)-Tricycloalkyl)-($C_1$-$C_6$)-Alkyl-,-($C_7$-$C_{14}$)-Bicycloalkenyl, (($C_7$-$C_{14}$)-Bicycloalkenyl)-($C_1$-$C_6$)-Alkyl-, -($C_8$-$C_{20}$)-Tricycloalkenyl, (($C_8$-$C_{20}$)-Tricycloalkenyl)-($C_1$-$C_6$)-Alkyl-, -(6-bis14-gliedriges)-Aryl, ((6-bis14-gliedriges)-Aryl)-($C_1$-$C_6$)-Alkyl-, -(7-bis12-gliedriges)-bicyclisches Ringsystem, ((7-bis12-gliedriges)-bicyclisches Ringsystem)-($C_1$-$C_6$)-Alkyl, -(7-bis12)-gliedriges)-bicyclisches Aryl, ((7-bis12-gliedriges)-bicyclisches Aryl)-($C_1$-$C_6$)-Alkyl-, -(5-bis 12-gliedriges)-Heteroaryl, ((5-bis12-gliedriges)-Heteroaryl)-($C_1$-$C_6$)-Alkyl-, -(3-bis12-gliedriger)-Heterocyclus, ((3-bis 12-gliedriger)-Heterocyclus)-($C_1$-$C_6$)-Alkyl-, -(7-bis12-gliedriger)-Bicycloheterocyclus, und ((7-bis12-gliedriger)-Bicycloheterocyclus)-($C_1$-$C_6$)-Alkyl-; wovon jedes optional substituiert ist mit einem, zwei oder drei Substituenten, unabhängig ausgewählt aus der Gruppe bestehend aus -OH, (=O), Halo, -C(Halo)$_3$, -CH(Halo)$_2$, -$CH_2$(Halo), -($C_1$-$C_6$)-Alkyl, Halo($C_1$-$C_6$)-Alkyl-, -($C_2$-$C_6$)-Alkenyl, -($C_2$-$C_6$)-Alkynyl, Hydroxy-($C_1$-$C_6$)-Alkyl-, Dihydroxy-($C_1$-$C_6$)-Alkyl-, -($C_1$-$C_6$)-Alkoxy, (($C_1$-$C_6$)-Alkoxy,)-C(=O)-($C_1$-$C_6$)-Alkoxy, -, -$NH_2$, -$NR^5R^6$, -NH($C_1$-$C_6$)-Alkyl, -($C_1$-$C_6$)-Alkyl-NH($C_1$-$C_6$)-Alkyl-$R^{14}$, -CN, -SH, -$OR^4$, -$CONR^5R^6$,-($C^1$-$C^6$-Alkyl)-C(=O)-$NR^5R^6$, -$COOR^7$, -($C_1$-$C_6$)-Alkyl-$COOR^7$, -($C_1$-$C_6$)-Alkoxy, -$COOR^7$, -($OCH_2CH_2$)$_s$-O($C_1$-$C_6$)-Alkyl, -($CH_2CH_2O$)$_s$-($C_1$-$C_6$)-Alkyl, (($C_1$-$C_6$)-Alkyl)-Sulfonyl-($C_1$-$C_6$)-Alkyl-, -NH-$SO_2$($C_1$-$C_6$)-Alkyl, -N-($SO_2$-($C_1$-$C_6$)-Alkyl)$_2$, -C(=NH)-$NH_2$, -NH-C(=O)-($C_1$-$C_6$)-Alkyl, -NH-C(=O)-$NH_2$, -NH-C(=O)-NH-($C_1$-$C_6$)-Alkyl, -NH-C(=O)-(6-bis 14-gliedriges)-Aryl, -NH-C(=O)-($C_1$-$C_6$)-Alkyl-(6-bis14-gliedriges)-Aryl, -NH-($C_1$-$C_6$)-Alkyl-$COOR^7$, -NH-C(=O)-($C_1$-$C_6$)-Alkyl-$COOR^7$, -NH-C(=O)-CH($NH_2$)-($C_1$-$C_6$)-Alkyl-C(=O)-$OR^7$, -($C_3$-$C_{12}$)-Cycloalkyl, (($C_3$-$C_{12}$)-Cycloalkyl)-($C_1$-$C_6$)-Alkyl-, -(6- bis 14-gliedriges)-Aryl,-(6-bis14-gliedriges)-Aryloxy, -($C_1$-$C_6$)-Alkoxy-C(O)$NR^5R^6$, -NH-($C_1$-$C_6$)-Alkyl-C(O)-$NR^5R^6$, -C(O)NH-($C_1$-$C_6$)-Alkyl-$COOR^7$, ((6-bis14-gliedriges)-Aryl)-($C_1$-$C_6$)-Alkyl-, -(5-bis 12-gliedriges)-Heteroaryl, ((5-bis12-gliedriges)-Heteroaryl)-($C_1$-$C_6$)-Alkyl-, -(3-bis12-gliedriger)-Heterocyclus, ((3-bis12-gliedriger)-Heterocyclus)-($C_1$-$C_6$)-Alkyl-, -(7-bis12-gliedriger)-Bicycloheterocyclus, und ((7-bis12-gliedriger)-Bicycloheterocyclus)-($C_1$-$C_6$)-Alkyl-;
$R^4$ ausgewählt ist aus

**(a)** -H, -OH, Halo, -C(Halo)$_3$, -CH(Halo)$_2$, -CH2(Halo), COOH oder $CONH_2$; oder
**(b)** -($C_1$-$C_5$)-Alkyl, -($C_2$-$C_5$)-Alkenyl, -($C_2$-$C_5$)-Alkynyl, -($CH_2$)$_n$-O-($CH_2$)$_n$-$CH_3$ oder -($C_1$-$C_5$)-Alkoxy, wovon jedes optional mit 1, 2, oder 3 unabhängig ausgewählten $R^9$-Gruppen substituiert ist;

$R^5$ und $R^6$ jeweils unabhängig ausgewählt sind aus

**(a)** Wasserstoff, -OH, Halo, -C(Halo)$_3$, -CH(Halo)$_2$, -$CH_2$(Halo); oder
**(b)** -($C_1$-$C_6$)-Alkyl, -($C_2$-$C_5$)-Alkenyl, -($C_2$-$C_5$)-Alkynyl, -($CH_2$)$_n$-O-($CH_2$)$_n$-$CH_3$, -($C_1$-$C_6$)-Alkoxy, wovon jedes optional mit 1, 2, oder 3 unabhängig ausgewählten $R^9$-Gruppen substituiert ist; oder
**(c)** -($C_3$-$C_8$)-Cycloalkyl, (($C_3$-$C_8$)-Cycloalkyl)-($C_1$-$C_6$)-Alkyl-, - $COOR^7$, -($C_1$-$C_6$)-Alkyl-$COOR^7$, -$CONH_2$ oder ($C_1$-$C_6$)-Alkyl-CONH-; oder
**(d)** $R^5$ und $R^6$ zusammen mit dem Stickstoffatom, an das sie angelagert sind, einen (4- bis 8-gliedrigen)-Heterocyclus bilden;

$R^7$ ausgewählt ist aus der Gruppe bestehend aus Wasserstoff,-($C_1$-$C_6$)-Alkyl, -($C_2$-$C_6$)-Alkenyl, -($C_2$-$C_6$)-Alkynyl, -($C_3$-$C_{12}$)-Cycloalkyl, -($C_4$-$C_{12}$)-Cycloalkenyl, (($C_3$-$C_{12}$)-Cycloalkyl)-($C_1$-$C_6$)-Alkyl- und (($C_4$-$C_{12}$)-Cycloalkenyl)-($C_1$-$C_6$)-Alkyl-;
$R^8$ ausgewählt ist aus H, -($C_1$-$C_6$)-Alkyl, -($C_2$-$C_6$)-Alkenyl, -($C_2$-$C_6$)-Alkynyl, -($C_1$-$C_{10}$)-Alkoxy, -($C_3$-$C_{12}$)-Cycloalkyl, -($C_3$-$C_{12}$)-Cycloalkenyl, (($C_3$-$C_{12}$)-Cycloalkyl)-($C_1$-$C_6$)-Alkyl-, (($C_3$-$C_{12}$)-Cycloalkenyl)-($C_1$-$C_6$)-Alkyl, -C(=O)($C_1$-$C_6$)-Alkyl oder $SO_2$($C_1$-$C_6$)-Alkyl;
jedes $R^9$ unabhängig ausgewählt ist aus -OH, Halo, -($C_1$-$C_{10}$)-Alkyl, -($C_2$-$C_{10}$)-Alkenyl, -($C_2$-$C_{10}$)-Alkynyl, -($C_1$-$C_{10}$)-Alkoxy, -($C_3$-$C_{12}$)-Cycloalkyl, -CHO, -C(O)OH, -C(Halo)$_3$, -CH(Halo)$_2$, $CH_2$(Halo) oder -($CH_2$)$_n$-O-($CH_2$)$_n$-$CH_3$;
jedes $R^{14}$ unabhängig ausgewählt ist aus der Gruppe bestehend aus -$COOR^7$, -($C_1$-$C_6$)-Alkyl-$COOR^7$, -C(=O)-($C_1$-$C_6$)-Alkyl-$COOR^7$, -($C_1$-$C_6$)-Alkyl-C(=O)-($C_1$-$C_6$)-Alkyl-$COOR^7$, $CONH_2$ und-($C_1$-$C_6$)-Alkyl-CONH;
m eine Ganzzahl 1, 2, 3, 4, 5 oder 6 ist;
n eine Ganzzahl 0, 1, 2, 3, 4, 5 oder 6 ist;
s eine Ganzzahl 1, 2, 3, 4, 5 oder 6 ist;
und die pharmazeutisch annehmbaren Salze und Solvate davon; vorausgesetzt, Z-G-$R^3$ ist nicht unsubstituiertes ($C_1$-$C_6$)-Alkyl.

**2.** Verbindung nach Anspruch 1 und die pharmazeutisch annehmbaren Salze und Solvate davon, wobei $R^3$ ausgewählt ist aus der Gruppe bestehend aus Wasserstoff, - ($C_1$-$C_{10}$)-Alkyl, -($C_2C_{12}$)-Alkenyl, -($C_2C_{12}$)-Alkynyl, -($C_1$-$C_{10}$)-Alkoxy, -($OCH_2CH_2$)$_s$-O($C_1$-$C_6$)-Alkyl, -($CH_2CH_2O$)$_s$-($C_1$-$C_6$)-Alkyl, - $NH_2$, -NH($C_1$-$C_6$)-Alkyl, CN, -$CONR^5R^6$, -($C_1$-$C_6$)-Alkyl-CO-$NR^5R^6$,-$COOR^7$, -(C1-$C_6$)-Alkyl-CO-$OR^7$, -($C_1$-$C_6$)-Alkoxy, -$COOR^7$, -CO-($CH_2$)$_n$-$COOR^7$, -CO-($CH_2$)$_n$-CO-$NR^5R^6$, -($C_3$-$C_{12}$)-Cycloalkyl, (($C_3$-$C_{12}$)-Cycloalkyl)-($C_1$-$C_6$)-Alkyl-, -($C_4$-$C_{12}$)-Cycloalkenyl, (($C_4$-$C_{12}$)-Cycloalkenyl)-($C_1$-$C_6$)-Alkyl-, ($C_6$-$C_{14}$)-Bicycloalkyl, (($C_6$-$C_{14}$)-Bicycloalkyl)-($C_1$-$C_6$)-Alkyl-, ($C_8$-$C_{20}$)-Tricycloalkyl, (($C_8$-$C_{20}$)-Tricycloalkyl)-($C_1$-$C_6$)-Alkyl-, -($C_7$-$C_{14}$)-Bicycloalkenyl,

(($C_7$-$C_{14}$)-Bicycloalkenyl)-($C_1$-$C_6$)-Alkyl-, -($C_8$-$C_{20}$)-Tricycloalkenyl, (($C_8$-$C_{20}$)-Tricycloalkenyl)-($C_1$-$C_6$)-Alkyl-, -(6-bis14-gliedriges)-Aryl, ((6-bis14-gliedriges)-Aryl)-($C_1$-$C_6$)-Alkyl-,-(7-bis12-gliedriges)-bicyclisches Ringsystem, ((7-bis 12-gliedriges)-bicyclisches Ringsystem)-($C_1$-$C_6$)-Alkyl-, -(7-bis 12-gliedriges)-bicyclisches Aryl, ((7-bis12-gliedriges)-bicyclisches Aryl)-($C_1$-$C_6$)-Alkyl-, -(5-bis12-gliedriges)-Heteroaryl, ((5-bis12-gliedriges)-Heteroaryl)-($C_1$-$C_6$)-Alkyl-, -(3-bis12-gliedriger)-Heterocyclus, ((3-bis12gliedriger)-Heterocyclus)-($C_1$-$C_6$)-Alkyl-, -(7-bis12-gliedriger)-Bicycloheterocyclus, und ((7-bis12-gliedriger)-Bicycloheterocyclus)-($C_1$-$C_6$)-Alkyl-; wovon jedes optional substituiert ist mit einem, zwei oder drei Substituenten, unabhängig ausgewählt aus der Gruppe bestehend aus -OH, (=O), Halo, -C(Halo)$_3$, -CH(Halo)$_2$, -$CH_2$(Halo), -($C_1$-$C_6$)-Alkyl, Halo($C_1$-$C_6$)-Alkyl-, -($C_2$-$C_6$)-Alkenyl, -($C_2$-$C_6$)-Alkynyl, Hydroxy-($C_1$-$C_6$)-Alkyl-, Dihydroxy-($C_1$-$C_6$)-Alkyl-, -($C_1$-$C_6$)-Alkoxy, (($C_1$-$C_6$)-Alkoxy,)CO($C_1$-$C_6$)-Alkoxy, -, -$NH_2$, -NH($C_1$-$C_6$)-Alkyl, -($C_1$-$C_6$)-Alkyl-NH($C_1$-$C_6$)-Alkyl-$R^{14}$, -CN, -SH, -$OR^4$, -$CONR^5R^6$, -($C_1$-$C_6$alkyl)-CO-$NR^5R^6$, -$COOR^7$, -($C_1$-$C_6$)-Alkyl-CO-$OR^7$, -($C_1$-$C_6$)-Alkoxy, -$COOR^7$, -($OCH_2CH_2$)$_s$-O($C_1$-$C_6$)-Alkyl, -($CH_2CH_2O$)$_s$-($C_1$-$C_6$)-Alkyl, (($C_1$-$C_6$)-Alkyl)-Sulfonyl-($C_1$-$C_6$)-Alkyl-, -NH-$SO_2$($C_1$-$C_6$)-Alkyl, -N($SO_2$($C_1$-$C_6$)-Alkyl)$_2$, -C(=NH)$NH_2$, -NH-CO-($C_1$-$C_6$)-Alkyl, - NH-CO-$NH_2$, -NH-C(=O)-NH-($C_1$-$C_6$)-Alkyl, -NH-C(=O)-(6-bis14-gliedriges)-Aryl, -NH-C(=O)-($C_1$-$C_6$)-Alkyl-(6-bis14-gliedriges)-Aryl, -NH-($C_1$-$C_6$)-Alkyl-CO-$OR^7$, -NH-C(=O)-($C_1$-$C_6$)-Alkyl-CO-$OR^7$, -NH-C(=O)-CH($NH_2$)-($C_1$-$C_6$)-Alkyl-CO-$OR^7$, -($C_3$-$C_{12}$)-Cycloalkyl, (($C_3$-$C_{12}$)-Cycloalkyl)-($C_1$-$C_6$)-Alkyl-, -(6-bis14-gliedriges)-Aryl, -(6-bis14-gliedriges)-Aryloxy, -($C_1$-$C_6$-Alkoxy-C(O)$NR^5R^6$, -NH-($C_1$-$C_6$)-Alkyl-C(O)-$NR^5R^6$, -C(O)NH-($C_1$-$C_6$)-Alkyl-$COOR^7$, ((6-bis14-gliedriges)-Aryl)-($C_1$-$C_6$-Alkyl-, -(5-bis 12-gliedriges)-Heteroaryl, ((5-bis12-gliedriges)-Heteroaryl)-($C_1$-$C_6$)-Alkyl-, -(3-bis12-gliedriger)-Heterocyclus, ((3-bis12-gliedriger)-Heterocyclus)-($C_1$-$C_6$)-Alkyl-, -(7-bis12-gliedriger)-Bicycloheterocyclus, und ((7-bis12-gliedriger)-Bicycloheterocyclus)-($C_1$-$C_6$)-Alkyl-.

**3.** Verbindung nach Anspruch 1 und die pharmazeutisch annehmbaren Salze und Solvate davon, wobei
$R^{2a}$ und $R^{2b}$ jeweils unabhängig ausgewählt sind aus:

**(a)** -H; oder
**(b)** -($C_1$-$C_5$)-Alkyl;

$R^4$ ausgewählt ist aus

**(a)** -H, order
**(b)** ($C_1$-$C_5$)-Alkoxy, das optional substituiert ist mit 1, 2, oder 3 unabhängig ausgewählten $R^9$-Gruppen.

**4.** Verbindung nach Anspruch 1 und die pharmazeurisch annehmbaren Salze und Solvate davon, wobei
$R^1$ ($C_3$-$C_{12}$)-Cycloalkyl-($C_1$-$C_6$)-Alkyl- ist, das optional substituiert ist durch 1, 2 oder 3 unabhängig ausgewählte $R^9$-Gruppen;
$R^{2a}$ und $R^{2b}$ jeweils unabhängig-($C_1$-$C_5$)-Alkyl sind;
$R^4$ ausgewählt ist aus -OH oder -$OCH_3$.

**5.** Verbindung nach einem der Ansprüche 1 bis 4 mit der Formel IA, IB, IC oder ID:

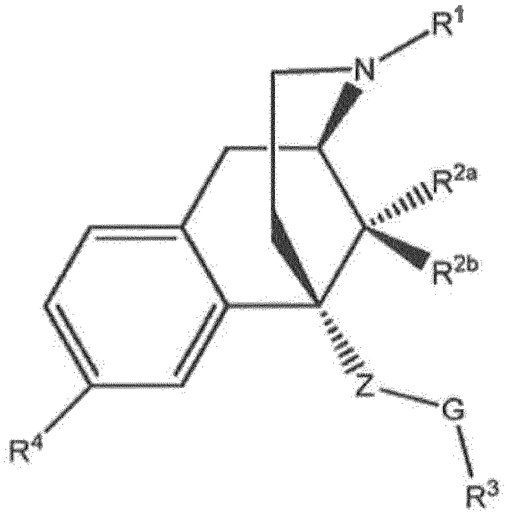

IA

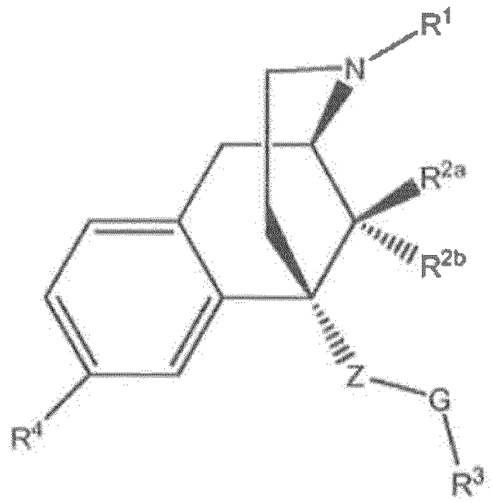

IB

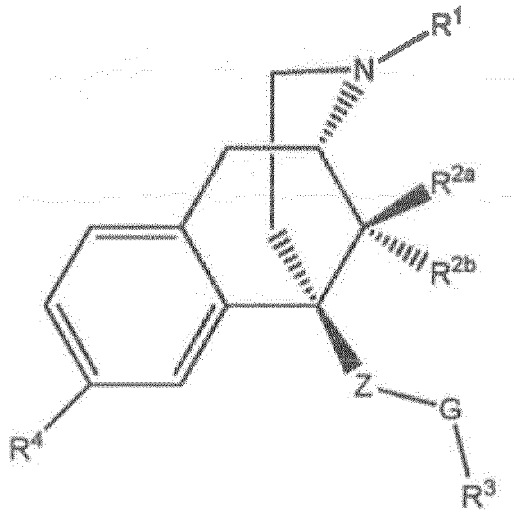

IC

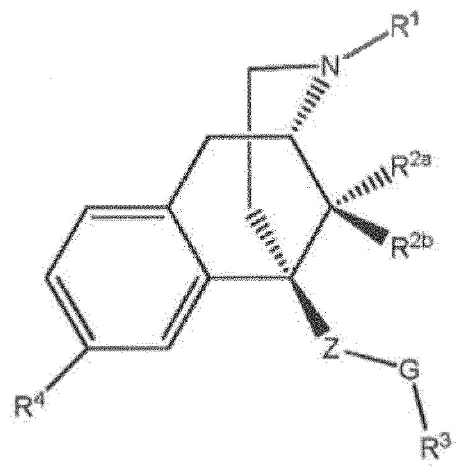

ID

**6.** Verbindung nach einem der Ansprüche 1 bis 5, wobei Z nicht vorhanden, Methylen, Ethylen, Propylen oder Butylen ist.

**7.** Verbindung nach einem der Ansprüche 1 bis 6, wobei G ausgewählt ist aus

**(i)** -C(=O);
**(ii)** -($C_1$-$C_6$)-Alkylen und vorzugsweise Methylen, Ethylen und Propylen;
**(iii)** -($C_2$-$C_6$)-Alkenylen und vorzugsweise Ethenylen und Propenylen; oder
**(iv)** G $NR^8$ ist, wobei vorzugsweise $R^8$ ausgewählt ist aus der Gruppe bestehend aus -H, -($C_1$-$C_6$)-Alkyl, -($C_1$-$C_{10}$)-Alkoxy, -($C_3$-$C_{12}$)-Cycloalkyl, und (($C_3$-$C_{12}$)-Cycloalkyl)-($C_1$-$C_6$)-Alkyl-.

**8.** Verbindung nach einem der Ansprüche 1 und 3 bis 7, wobei $R^3$ ausgewählt ist aus

**(i)** H, ($C_1$-$C_6$)-Alkyl, $NH_2$, NH($C_1$-$C_6$)-Alkyl oder -C(=O)-($C_1$-$C_6$)-Alkyl;
**(ii)** -$CONR^5R^6$ und -($C_1$-$C_6$) alkyl-$CONR^5R^6$, wobei vorzugsweise $R^5$ oder $R^6$ jeweils unabhängig ausgewählt sind aus -H oder -($C_1$-$C_6$)-Alkyl;
**(iii)** $COOR^7$, wobei vorzugsweise $R^7$ H oder -($C_1$-$C_6$)-Alkyl ist;
**(iv)** -(6-bis14-gliedriges)-Aryl oder ((6-bis14-gliedriges)-Aryl)-($C_1$-$C_6$)-Alkyl-; und vorzugsweise Phenyl oder Benzyl;

**(v)** -(3-bis12-gliedriger)-Heterocyclus, ((3-bis12 gliedriger)-Heterocyclus)-($C_1$-$C_6$)-Alkyl-, -(5-bis12-gliedriges)-Heteroaryl oder ((5-bis12-gliedriges)-Heteroaryl)-($C_1$-$C_6$)-Alkyl-;
**(vi)** -($C_1$-$C_{10}$)-Alkoxy; oder
**(vii)** -C(=O)-(5-bis12-gliedriges)-Heteroaryl oder -C(=O)-(6-bis14-gliedriges)-Aryl;

und wobei vorzugsweise $R^3$ substituiert ist mit einem, zwei oder drei Substituenten, unabhängig ausgewählt aus der Gruppe bestehend aus -$COOR^7$, -$NR^5R^6$, -$CONR^5R^6$, Phenyl, Benzyl, -NH-C(=O)-(6-bis14-gliedriges)-Aryl, mit-NH-C(=O)-($C_1$-$C_6$)-Alkyl- (6-bis 14-gliedriges)-Aryl, -OH, Hydroxy-($C_1$-$C_6$)-Alkyl- und Dihydroxy-($C_1$-$C_6$)-Alkyl.

**9.** Verbindung nach einem der Ansprüche 1 und 3 bis 5, wobei Z-G-$R^3$ ausgewählt ist aus

**(i)** -$CH_2COOH$, -$(CH_2)_3$-COOH, $(CH_2)_3$-$COOCH_3$, -$(CH_2)_3$-$CONH_2$; - $(CH_2)_2$-O-(5- bis 12-gliedriges)-Heteroaryl-$COOR^7$ oder -$(CH_2)_3$-O-(5-bis12-gliedriges)-Heteroaryl-$COOR^7$, -$(CH_2)_3$-$CONR^5R^6$,-$(CH_2)_3$-C(=O)-NH-($C_1$-$C_6$)-Alkyl- $COOR^7$, Dihydroxy-($C_1$-$C_6$)-Alkyl,-($C_1$-$C_6$)-Alkenyl-$COOR^7$, ($C_1$-$C_6$)-Alkyl-NH-($C_1$-$C_6$)-Alkyl, -$(CH_2)_3$-C(=O)-NH-($C_1$-$C_6$)-Alkyl-C(=O)-$NH_2$, oder -($C_1$-$C_6$)-Alkyl-C(=O)-NH-CH($CH_3$)-$CONH_2$; oder
**(ii)** -$(CH_2)_2$-((6-bis14-gliedriges)-Aryl)-$COOR^7$, -$(CH_2)_2$-NH-C(=O)-((6- bis 14-gliedriges)-Aryl)-$COOR^7$, -$(CH_2)_2$-NH-C(=O)-((5-bis 12-gliedriges)-Heteroaryl), -$(CH_2)_2$-NH-C(=O)-((6-bis14-gliedriges)-Aryl)-$COOR^7$, -$(CH_2)_2$-NH-C(=O)-((5-bis12-gliedriges)-Heteroaryl)-$COOR^7$ oder -$(CH_2)_2$-O-((6-bis14-gliedriges)-Aryl)-$COOR^7$; oder
**(iii)** -$(CH_2)_2$-NH-CO-$CH_2$-$NH_2$, -($C_1$-$C_6$)-Alkyl-O-CH2-phenyl, -($C_1$-$C_6$)-Alkyl-OH, -($C_1$-$C_6$)-Alkyl-Furanyl oder -($C_1$-$C_6$)-Alkyl-C(=O)H oder -($C_1$-$C_6$)-Alkenyl; oder
**(iv)** -$(CH_2)_2$-NH-CO-$CH_2$-$NR^5R^6$.

**10.** Verbindung nach Anspruch 1, ausgewählt aus der Gruppe bestehend aus:

(2*S*)-2-(2-((6*R*,11*R*)-3-(Cyclopropylmethyl)-8-hydroxy-11-methyl-1,2,3,4,5,6-hexahydro-2,6-methanobenzo[*d*]azocin-6-yl)acetamido)propanamid;
4-((6*S*,11*R*)-3-(Cyclopropylmethyl)-8-hydroxy-11-methyl-1,2,3,4,5,6-hexahydro-2,6-methanobenzo[*d*]azocin-6-yl)butanamid;
4-((6*S*,11*R*)-3-(Cyclopropylmethyl)-8-methoxy-11-methyl-1,2,3,4,5,6-hexahydro-2,6-methanobenzo[*d*]azocin-6-yl)butansäure;
4-((6*S*,11*R*)-3-(cyclopropylmethyl)-8-hydroxy-11-methyl-1,2,3,4,5,6-hexahydro-2,6-methanobenzo[*d*]azocin-6-yl)butansäure;
2-((6*R*,11*R*)-3-(Cyclopropylmethyl)-8-methoxy-11-methyl-1,2,3,4,5,6-hexahydro-2,6-methanobenzo[*d*]azocin-6-yl)essigsäure;
(2*S*)-3-((6*R*,11*R*)-3-(Cyclopropylmethyl)-8-methoxy-11-methyl-1,2,3,4,5,6-hexahydro-2,6-methanobenzo[*d*]azocin-6-yl)propane-1,2-diol;
(2*S*)-2-(2-((6*R*,11*R*)-3-(Cyclopropylmethyl)-8-methoxy-11-methyl-1,2,3,4,5,6-hexahydro-2,6-methanobenzo[*d*]azocin-6-yl)acetamido)propanamid;
(*E*)-Methyl 4-((6*S*,11*R*)-3-(Cyclopropylmethyl)-8-methoxy-11-methyl-1,2,3,4,5,6-hexahydro-2,6-methanobenzo[*d*]azocin-6-yl)but-2-enoate;
4-((6*S*,11*R*)-3-(Cyclopropylmethyl)-8-methoxy-11-methyl-1,2,3,4,5,6-hexahydro-2,6-methanobenzo[*d*]azocin-6-yl)-N-isobutylbutan-1-amine;
(2*R*)-5-((6*S*,11*R*)-3-(Cyclopropylmethyl)-8-methoxy-11-methyl-1,2,3,4,5,6-hexahydro-2,6-methanobenzo[*d*]azocin-6-yl)pentan-1,2'-diol;
(2*S*)-5-((6*S*,11*R*)-3-(Cyclopropylmethyl)-8-methoxy-11-methyl-1,2,3,4,5,6-hexahydro-2,6-methanobenzo[*d*]azocin-6-yl)pentan-1,2-diol;
(6*S*,11*R*)-6-(4-(Benzyloxy)butyl)-3-(cyclopropylmethyl)-8-methoxy-11-methyl-1,2,3,4,5,6-hexahydro-2,6-methanobenzo[*d*]azocin;
4-((6*S*,11*R*)-3-(Cyclopropylmethyl)-8-methoxy-11-methyl-1,2,3,4,5,6-hexahydro-2,6-methanobenzo[*d*]azocin-6-yl)butan-1-ol;
N-((*S*)-1-Amino-1-oxopropan-2-yl)-4-((6*S*,11*R*)-3-(cyclopropylmethyl)-8-methoxy-11-methyl-1,2,3,4,5,6-hexahydro-2,6-methanobenzo[*d*]azocin-6-yl)butanamid;
(2*R*,6*S*,11*S*)-3-(Cyclopropylmethyl)-6-(3-(furan-2-yl)propyl)-8-methoxy-11-methyl-1,2,3,4,5,6-hexahydro-2,6-methanobenzo[*d*]azocin;
5-(2-((2*R*,6*S*,11*R*)-3-(Cyclopropylmethyl)-8-hydroxy-11-methyl-1,2,3,4,5,6-hexahydro-2,6-methanobenzo[*d*]azocin-6-yl)ethoxy)nikotinsäure;

4-((2*R*,6*R*,11*R*)-3-Benzyl-8-hydroxy-11-methyl-1,2,3,4,5,6-hexahydro-2,6-methanobenzo[*d*]azocin-6-yl)butanamid;
4-((2*R*,6*R*,11*R*)-3-(Cyclopropylmethyl)-8-hydroxy-11-methyl-1,2,3,4,5,6-hexahydro-2,6-methanobenzo[*d*]azocin-6-yl)butanamid;
(*S*)-Methyl 2-(4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamido)propanoate;
*N*-((*S*)-1-Amino-1-oxopropan-2-yl)-4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanamid;
Methyl 4-((2*R*,6*R*,11*R*)-3-(cyclopropylmethyl)-8-hydroxy-11-methyl-1,2,3,4,5,6-hexahydro-2,6-methanobenzo[*d*]azocin-6-yl)butanoate;
3-((2-((2*R*,6*R*,11*R*)-3-(Cyclopropylmethyl)-8-methoxy-11-methyl-1,2,3,4,5,6-hexahydro-2,6-methanobenzo[*d*]azocin-6-yl)ethyl)carbamoyl)benzoesäure;
4-((2-((2*R*,6*R*,11*R*)-3-(Cyclopropylmethyl)-8-methoxy-11-methyl-1,2,3,4,5,6-hexahydro-2,6-methanobenzo[*d*]azocin-6-yl)ethyl)carbamoyl)benzoesäure;
Methyl 3-((2-((2*R*,6*R*,11*R*)-3-(Cyclopropylmethyl)-8-methoxy-11-methyl-1,2,3,4,5,6-hexahydro-2,6-methanobenzo[*d*]azocin-6-yl)ethyl)carbamoyl)benzoat;
4-(2-((2*R*,6*S*,11*R*)-3-(Cyclopropylmethyl)-8-methoxy-11-methyl-1,2,3,4,5,6-hexahydro-2,6-methanobenzo[*d*]azocin-6-yl)ethoxy)benzoesäure;
4-(2-((2*R*,6*S*,11*R*)-3-(Cyclopropylmethyl)-8-hydroxy-11-methyl-1,2,3,4,5,6-hexahydro-2,6-methanobenzo[*d*]azocin-6-yl)ethoxy)benzoesäure;
2-((2*S*, 6*R*,11*S*)-3-(Cyclopropylmethyl)-8-methoxy-11-methyl-1,2,3,4,5,6-hexahydro-2,6-methanobenzo[*d*]azocin-6-yl)essigsäure;
*N*-(2-((2*R*,6*R*,11*R*)-3-(Cyclopropylmethyl)-8-hydroxy-11-methyl-1,2,3,4,5,6-hexahydro-2,6-methanobenzo[*d*]azocin-6-yl)ethyl)-2-(dimethylamino)acetamid;
2-Amino-*N*-(2-((2*R*,6*R*,11*R*)-3-(cyclopropylmethyl)-8-hydroxy-11-methyl-1,2,3,4,5,6-hexahydro-2,6-methanobenzo[*d*]azocin-6-yl)ethyl)acetamid;

und die pharmazeutisch annehmbaren Salze und Solvate davon.

**11.** Pharmazeutische Zusammensetzung umfassend eine wirksame Menge einer Verbindung nach einem der Ansprüche 1. bis 10, oder ein pharmazeutisches Salz oder Solvat davon, und einen pharmazeutisch annehmbaren Träger oder Exzipienten.

**12.** *In-vitro*-Verfahren zum Modulieren einer Opioid-Rezeptor-Funktion in einer Zelle, umfassend ein Inkontaktbringen einer Zelle, die in der Lage ist, einen Opioid-Rezeptor zu exprimieren, mit einer wirksamen Menge einer Verbindung nach einem der Ansprüche 1 bis 10, oder einem pharmazeutisch annehmbaren Salz oder Solvat davon,
wobei vorzugsweise:

**(i)** die Verbindung eine μ-Opioid-Rezeptor-Funktion moduliert,
und
wobei bevorzugter die Verbindung als ein Agonist oder als ein Antagonist an dem μ-Opioid-Rezeptor wirkt; oder
**(ii)** die Verbindung als ein Agonist an dem K-Opioid-Rezeptor wirkt; oder
**(iii)** die Verbindung eine ORL-1-Rezeptor-Funktion moduliert, und wobei bevorzugter die Verbindung als ein Antagonist an dem ORL-1-Rezeptor wirkt.

**13.** Erfahren zum Zubereiten einer Zusammensetzung, umfassend den Schritt des Mischens einer Verbindung nach einem der Ansprüche 1 bis 10, oder eines pharmazeutisch annehmbaren Salzes oder Solvats davon, mit einem pharmazeutisch annehmbaren Träger- oder Exzipienten.

**14.** Verbindung nach einem der Ansprüche 1 bis 10 oder ein pharmazeutisch annehmbares Salz oder Solvat davon zur Verwendung in der Behandlung, Prävention oder Besserung eines Zustands, wobei vorzugsweise der Zustand Schmerz oder Darmträgheit ist.

**15.** Verwendung einer Verbindung nach einem der Ansprüche 1 bis 10 oder eines pharmazeutisch annehmbaren Salzes oder Solvats davon bei der Herstellung eines Medikaments, das zur Behandlung oder Prävention eines Zustands nützlich ist, wobei vorzugsweise der Zustand Schmerz oder Darmträgheit ist.

## Revendications

1. Composé de formule I :

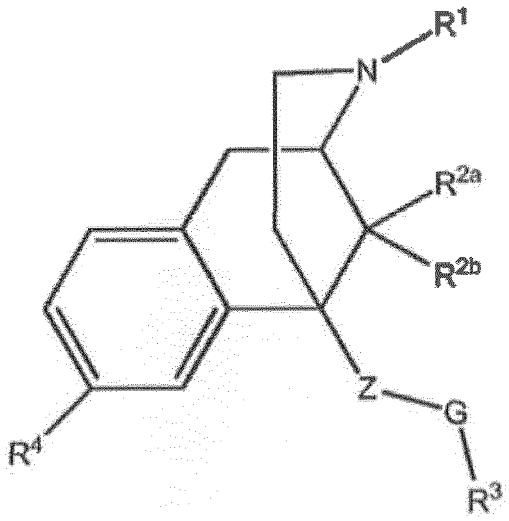

dans lequel

$R^1$ est choisi dans le groupe constitué des groupes -cycloalkyle (en $C_3$ à $C_{12}$), cycloalkyl (en $C_3$ à $C_{12}$)-alkyl (en $C_1$ à $C_6$)-, et - aryle (de 6 à 14 chaînons), chacun d'entre eux étant éventuellement substitué par 1, 2 ou 3 groupes $R^9$ choisis indépendamment ;
$R^{2a}$ et $R^{2b}$ sont choisis chacun indépendamment parmi des groupes :

(a) -H ; ou
(b) -alkyle (en $C_1$ à $C_5$), -alcényle (en $C_2$ à $C_5$), ou alcynyle (en $C_2$ à $C_5$) ;

Z est absent ou représente $-(CH_2)_m-$, éventuellement substitué par 1 ou 2 groupes -alkyle (en $C_1$ à $C_6$) choisis indépendamment ; G est choisi dans le groupe constitué des groupes :

a) -alkylène (en $C_1$ à $C_6$), -alcénylène (en $C_2$ à $C_6$) ; ou
b) -C(=O) ; ou
c) $NR^8$ ;

$R^3$ est choisi dans le groupe constitué d'un atome d'hydrogène, des groupes -alkyle (en $C_1$ à $C_{10}$), -alcényle (en $C_2$ à $C_{12}$),-C(=O)-alkyle (en $C_1$ à $C_6$), -C(=O)-aryle (de 6 à 14 chaînons), - C(=O)-hétéroaryle (de 5 à 12 chaînons), -alcynyle (en $C_2$ à $C_{12}$), -alcoxy (en $C_1$ à $C_{10}$), $-(OCH_2CH_2)_s$-O-alkyle (en $C_1$ à $C_6$), - $(CH_2CH_2O)_s$-alkyle (en $C_1$ à $C_6$), $-NH_2$, -NH-alkyle (en $C_1$ à $C_6$), CN, $-CONR^5R^6$, -alkyl (en $C_1$ à $C_6$) $-CONR^5R^6$, $-COOR^7$, -alkyl (en $C_1$ à $C_6$)-$COOR^7$, -alcoxy (en $C_1$ à $C_6$)-$COOR^7$, -C(=O)-$(CH_2)_n$-$COOR^7$, -C(=O)-$(CH_2)_n$-$CONR^5R^6$, -cycloalkyle (en $C_3$ à $C_{12}$), (cycloalkyl (en $C_3$ à $C_{12}$))-alkyl (en $C_1$ à $C_6$)-, -cycloalcényle (en $C_4$ à $C_{12}$), (cycloalcényl (en $C_4$ à $C_{12}$))-alkyl (en $C_1$ à $C_6$)-, -bicycloalkyle (en $C_6$ à $C_{14}$), (bicycloalkyl (en $C_6$ à $C_{14}$))-alkyl (en $C_1$ à $C_6$)-,-tricycloalkyle (en $C_8$ à $C_{20}$), (tricycloalkyl (en $C_8$ à $C_{20}$))-alkyl (en $C_1$ à $C_6$)-, -bicycloalcényle (en $C_7$ à $C_{14}$), (bicycloalcényl (en $C_7$ à $C_{14}$))-alkyl (en $C_1$ à $C_6$)-, -tricycloalcényle (en $C_8$ à $C_{20}$), (tricycloalcényl (en $C_8$ à $C_{20}$))-alkyl (en $C_1$ à $C_6$)-, -aryle (de 6 à 14 chaînons), (aryl (de 6 à 14 chaînons))-alkyl (en $C_1$ à $C_6$)-, -système de cycle bicyclique (de 7 à 12 chaînons), (système de cycle bicyclique (de 7 à 12 chaînons))-alkyl (en $C_1$ à $C_6$)-, -aryle bicyclique (de 7 à 12 chaînons), (aryl bicyclique (de 7 à 12 chaînons))-alkyl (en $C_1$ à $C_6$)-, -hétéroaryle (de 5 à 12 chaînons), (hétéroaryl (de 5 à 12 chaînons))-alkyl (en $C_1$ à $C_6$)-, -hétérocycle (de 3 à 12 chaînons), (hétérocycle (de 3 à 12 chaînons))-alkyl (en $C_1$ à $C_6$)-, -bicyclohétérocycle (de 7 à 12 chaînons), et (bicyclohétérocycle (de 7 à 12 chaînons))-alkyl (en $C_1$ à $C_6$)- ; chacun d'entre eux étant éventuellement substitué par un, deux, ou trois substituants choisis indépendamment dans le groupe constitué des groupes -OH, (=O), halogéno,-C(halogéno)$_3$, -CH(halogéno)$_2$, $-CH_2$(halogéno), -alkyle (en $C_1$ à $C_6$), halogénoalkyl (en $C_1$ à $C_6$)-, -alcényle (en $C_2$ à $C_6$), -alcynyle (en $C_2$ à $C_6$), hydroxyalkyl (en $C_1$ à $C_6$)-, dihydroxyalkyl (en $C_1$ à $C_6$)-, -alcoxy (en $C_1$ à $C_6$), (alcoxy (en $C_1$ à $C_6$))-C(=O)-alcoxy (en $C_1$ à $C_6$)-, $-NH_2$, $-NR^5R^6$, -NH-alkyle (en $C_1$ à $C_6$), -alkyl (en $C_1$ à

$C_6$)-NH-alkyl (en $C_1$ à $C_6$)-$R^{14}$, -CN, -SH, -$OR^4$, -$CONR^5R^6$,-alkyl (en $C_1$ à $C_6$)-C(=O)-$NR^5R^6$, -$COOR^7$, -alkyl (en $C_1$ à $C_6$)-$COOR^7$, -alcoxy (en $C_1$ à $C_6$)-$COOR^7$, -$(OCH_2CH_2)_s$-O-alkyle (en $C_1$ à $C_6$),-$(CH_2CH_2O)_s$-alkyle (en $C_1$ à $C_6$), (alkyl (en $C_1$ à $C_6$))sulfonyl-alkyl (en $C_1$ à $C_6$)-, -NH-$SO_2$-alkyle (en $C_1$ à $C_6$), -N-($SO_2$-alkyle (en $C_1$ à $C_6$))$_2$, -C(=NH)-$NH_2$, -NH-C(=O)-alkyle (en $C_1$ à $C_6$), -NH-C(=O)-$NH_2$, -NH-C(=O)-NH-alkyle (en $C_1$ à $C_6$), -NH-C(=O)-aryle (de 6 à 14 chaînons), -NH-C(=O)-alkyl (en $C_1$ à $C_6$)-aryle (de 6 à 14 chaînons), -NH-alkyl (en $C_1$ à $C_6$)-$COOR^7$, -NH-C(=O)-alkyl (en $C_1$ à $C_6$)-$COOR^7$, -NH-C(=O)-CH($NH_2$)-alkyl (en $C_1$ à $C_6$)-C(=O)-$OR^7$,-cycloalkyle (en $C_3$ à $C_{12}$), (cycloalkyl (en $C_3$ à $C_{12}$))-alkyl (en $C_1$ à $C_6$)-, -aryle (de 6 à 14 chaînons), -aryloxy (de 6 à 14 chaînons), -alcoxy (en $C_1$ à $C_6$)-C(O)$NR^5R^6$, -NH-alkyl (en $C_1$ à $C_6$)-C(O)-$NR^5R^6$, -C(O)NH-alkyl (en $C_1$ à $C_6$)-$COOR^7$, (aryl (de 6 à 14 chaînons))-alkyl (en $C_1$ à $C_6$)-, -hétéroaryle (de 5 à 12 chaînons), (hétéroaryl (de 5 à 12 chaînons))-alkyl (en $C_1$ à $C_6$)-, -hétérocycle (de 3 à 12 chaînons), (hétérocycle (de 3 à 12 chaînons))-alkyl (en $C_1$ à $C_6$)-, -bicyclohétérocycle (de 7 à 12 chaînons), et (bicyclo-hétérocycle (de 7 à 12 chaînons))-alkyl (en $C_1$ à $C_6$)- ;
$R^4$ est choisi parmi des groupes

(a) -H, -OH, halogéno, -C(halogéno)$_3$, -CH(halogéno)$_2$,-$CH_2$(halogéno), COOH, ou $CONH_2$ ; ou
(b) -alkyle (en $C_1$ à $C_5$), -alcényle (en $C_2$ à $C_5$), -alcynyle (en $C_2$ à $C_5$), -$(CH_2)_n$-O-$(CH_2)_n$-$CH_3$, ou -alcoxy (en $C_1$ à $C_5$), chacun d'entre étant éventuellement substitué par 1, 2, ou 3 groupes $R^9$ choisis indépendamment ;

$R^5$ et $R^6$ sont choisis chacun indépendamment parmi des groupes

(a) hydrogène, -OH, halogéno, -C(halogéno)$_3$, -CH(halogéno)$_2$,-$CH_2$(halogéno) ; ou
(b) -alkyle (en $C_1$ à $C_6$), -alcényle (en $C_2$ à $C_5$), -alcynyle (en $C_2$ à $C_5$), -$(CH_2)_n$-O-$(CH_2)_n$-$CH_3$, ou -alcoxy (en $C_1$ à $C_6$), chacun d'entre étant éventuellement substitué par 1, 2, ou 3 groupes $R^9$ choisis indépendamment ; ou
(c) -cycloalkyle (en $C_3$ à $C_8$), (cycloalkyl (en $C_3$ à $C_8$))-alkyl (en $C_1$ à $C_6$)-, -$COOR^7$, -alkyl (en $C_1$ à $C_6$)-$COOR^7$, -$CONH_2$, ou alkyl (en $C_1$ à $C_6$)-CONH- ; ou
(d) $R^5$ et $R^6$ conjointement avec l'atome d'azote auquel ils sont fixés forment un hétérocycle (de 4 à 8 chaînons) ;

$R^7$ est choisi dans le groupe constitué d'un atome d'hydrogène, des groupes -alkyle (en $C_1$ à $C_6$), -alcényle (en $C_2$ à $C_6$),-alcynyle (en $C_2$ à $C_6$), -cycloalkyle (en $C_3$ à $C_{12}$), -cycloalcényle (en $C_9$ à $C_{12}$), (cycloalkyl (en $C_3$ à $C_{12}$))-alkyl (en $C_1$ à $C_6$)-, et (cycloalcényl (en $C_4$ à $C_{12}$))-alkyl (en $C_1$ à $C_6$)- ;
$R^8$ est choisi parmi H, des groupes -alkyle (en $C_1$ à $C_6$), -alcényle (en $C_2$ à $C_6$), -alcynyle (en $C_2$ à $C_6$), -alcoxy (en $C_1$ à $C_{10}$),-cycloalkyle (en $C_3$ à $C_{12}$), -cycloalcényle (en $C_3$ à $C_{12}$), (cycloalkyl (en $C_3$ à $C_{12}$))-alkyl (en $C_1$ à $C_6$)-, (cycloalcényl (en $C_3$ à $C_{12}$))-alkyl (en $C_1$ à $C_6$)-, -C(=O)-alkyle (en $C_1$ à $C_6$) ou $SO_2$-alkyle (en $C_1$ à $C_6$) ;
chaque $R^9$ est choisi indépendamment parmi des groupes -OH, halogéno, -alkyle (en $C_1$ à $C_{10}$), -alcényle (en $C_2$ à $C_{10}$), -alcynyle (en $C_2$ à $C_{10}$), -alcoxy (en $C_1$ à $C_{10}$), -cycloalkyle (en $C_3$ à $C_{12}$), -CHO, -C(O)OH, -C(halogéno)$_3$, CH(halogéno)$_2$, $CH_2$(halogéno), ou-$(CH_2)_n$-O-$(CH_2)_n$-$CH_3$ ;
chaque $R^{14}$ est choisi indépendamment dans le groupe constitué des groupes -$COOR^7$, -alkyl (en $C_1$ à $C_6$)-$COOR^7$, -C(=O)-alkyl (en $C_1$ à $C_6$)-$COOR^7$, -alkyl (en $C_1$ à $C_6$)-C(=O)-alkyl (en $C_1$ à $C_6$)-$COOR^7$, $CONH_2$, et -alkyl (en $C_1$ à $C_6$)-CONH ;
m est un nombre entier de 1, 2, 3, 4, 5, ou 6 ;
n est un nombre entier de 0, 1, 2, 3, 4, 5, ou 6 ;
s est un nombre entier de 1, 2, 3, 4, 5, ou 6 ;
et ses sels pharmaceutiquement acceptables et solvates ;
à condition que Z-G-$R^3$ ne représente pas un groupe alkyl (en $C_1$ à $C_6$) non substitué.

**2.** Composé selon la revendication 1, et ses sels pharmaceutiquement acceptables et solvates, dans lequel $R^3$ est choisi dans le groupe constitué d'un atome d'hydrogène, des groupes -alkyle (en $C_1$ à $C_{10}$), -alcényle (en $C_2$ à $C_{12}$),-alcynyle (en $C_2$ à $C_{12}$), -alcoxy (en $C_1$ à $C_{10}$), -$(OCH_2CH_2)_s$-O-alkyle (en $C_1$ à $C_6$), -$(CH_2CH_2O)_s$-alkyle (en $C_1$ à $C_6$), -$NH_2$, -NH-alkyle (en $C_1$ à $C_6$), CN, -$CONR^5R^6$, -alkyl (en $C_1$ à $C_6$)-$CONR^5R^6$, -$COOR^7$,-alkyl (en $C_1$ à $C_6$)-CO-$OR^7$, -alcoxy (en $C_1$ à $C_6$)-$COOR^7$, -CO-$(CH_2)_n$-$COOR^7$, -CO-$(CH_2)_n$-CO-$NR^5R^6$, -cycloalkyle (en $C_3$ à $C_{12}$), (cycloalkyl (en $C_3$ à $C_{12}$))-alkyl (en $C_1$ à $C_6$)-, -cycloalcényle (en $C_4$ à $C_{12}$), (cycloalcényl (en $C_4$ à $C_{12}$))-alkyl (en $C_1$ à $C_6$)-, -bicycloalkyle (en $C_6$ à $C_{14}$), (bicycloalkyl (en $C_6$ à $C_{14}$))-alkyl (en $C_1$ à $C_6$)-, - tricycloalkyle (en $C_8$ à $C_{20}$), (tricycloalkyl (en $C_8$ à $C_{20}$))-alkyl (en $C_1$ à $C_6$)-, -bicycloalcényle (en $C_7$ à $C_{14}$), (bicycloalcényl (en $C_7$ à $C_{14}$))-alkyl (en $C_1$ à $C_6$)-, -tricycloalcényle (en $C_8$ à $C_{20}$), (tricycloalcényl (en $C_8$ à $C_{20}$))-alkyl (en $C_1$ à $C_6$)-, -aryle (de 6 à 14 chaînons), (aryl (de 6 à 14 chaînons))-alkyl (en $C_1$ à $C_6$)-, -système de cycle bicyclique (de 7 à 12 chaînons),

(système de cycle bicyclique (de 7 à 12 chaînons))-alkyl (en $C_1$ à $C_6$)-, -aryle bicyclique (de 7 à 12 chaînons), (aryl bicyclique (de 7 à 12 chaînons))-alkyl (en $C_1$ à $C_6$)-, -hétéroaryle (de 5 à 12 chaînons), (hétéroaryl (de 5 à 12 chaînons))-alkyl (en $C_1$ à $C_6$)-, -hétérocycle (de 3 à 12 chaînons), (hétérocycle (de 3 à 12 chaînons))-alkyl (en $C_1$ à $C_6$)-, -bicyclohétérocycle (de 7 à 12 chaînons), et (bicyclohétérocycle (de 7 à 12 chaînons))-alkyl (en $C_1$ à $C_6$)- ; chacun d'entre eux étant éventuellement substitué par un, deux, ou trois substituants choisis indépendamment dans le groupe constitué des groupes -OH, (=O), halogéno,-C(halogéno)$_3$, -CH(halogéno)$_2$, -$CH_2$(halogéno), -alkyle (en $C_1$ à $C_6$), halogénoalkyl (en $C_1$ à $C_6$)-, -alcényle (en $C_2$ à $C_6$), -alcynyle (en $C_2$ à $C_6$), hydroxyalkyl (en $C_1$ à $C_6$)-, dihydroxyalkyl (en $C_1$ à $C_6$)-, -alcoxy (en $C_1$ à $C_6$), (alcoxy (en $C_1$ à $C_6$))-CO-alcoxy (en $C_1$ à $C_6$)-, -$NH_2$, -NH-alkyle (en $C_1$ à $C_6$), -alkyl (en $C_1$ à $C_6$)-NH-alkyl (en $C_1$ à $C_6$)-$R^{19}$, -CN, -SH, -$OR^4$, -$CONR^5R^6$, -alkyl (en $C_1$ à $C_6$)-CO-$NR^5R^6$, -$COOR^7$, -alkyl (en $C_1$ à $C_6$)-CO-$OR^7$, -alcoxy (en $C_1$ à $C_6$)-$COOR^7$, -$(OCH_2CH_2)_s$-O-alkyle (en $C_1$ à $C_6$), -$(CH_2CH_2O)_s$-alkyle (en $C_1$ à $C_6$), (alkyl (en $C_1$ à $C_6$)) sulfonyl-alkyl (en $C_1$ à $C_6$)-,-NH-$SO_2$-alkyle (en $C_1$ à $C_6$), -N-($SO_2$-alkyle (en $C_1$ à $C_6$))$_2$,-C(=NH)$NH_2$, -NH-CO-alkyle (en $C_1$ à $C_6$), -NH-CO-$NH_2$, -NH-C(=O)-NH-alkyl (en $C_1$ à $C_6$), -NH-C(=O)-aryle (de 6 à 14 chaînons), -NH-C(=O)-alkyl (en $C_1$ à $C_6$)-aryle (de 6 à 14 chaînons), -NH-alkyl (en $C_1$ à $C_6$)-CO-$OR^7$, -NH-C(=O)-alkyl (en $C_1$ à $C_6$)-CO-$OR^7$, -NH-C(=O)-CH($NH_2$)-alkyl (en $C_1$ à $C_6$)-CO-$OR^7$, -cycloalkyle (en $C_3$ à $C_{12}$), (cycloalkyl (en $C_3$ à $C_{12}$))-alkyl (en $C_1$ à $C_6$)-, -aryle (de 6 à 14 chaînons), -aryloxy (de 6 à 14 chaînons), -alcoxy (en $C_1$ à $C_6$)-C(O)$NR^5R^6$, -NH-alkyl (en $C_1$ à $C_6$)-C(O)-$NR^5R^6$, -C(O)NH-alkyl (en $C_1$ à $C_6$)-$COOR^7$, (aryl (de 6 à 14 chaînons))-alkyl (en $C_1$ à $C_6$)-, -hétéroaryle (de 5 à 12 chaînons), (hétéroaryl (de 5 à 12 chaînons))-alkyl, (en $C_1$ à $C_6$)-, -hétérocycle (de 3 à 12 chaînons), (hétérocycle (de 3 à 12 chaînons))-alkyl (en $C_1$ à $C_6$)-, -bicyclohétérocycle (de 7 à 12 chaînons), et (bicyclo-hétérocycle (de 7 à 12 chaînons))-alkyl (en $C_1$ à $C_6$)-.

**3.** Composé selon la revendications 1, et ses sels pharmaceutiquement acceptables et solvates, dans lequel $R^{2a}$ et $R^{2b}$ sont choisis chacun indépendamment parmi des groupes :

(a) -H ; ou
(b) -alkyle (en $C_1$ à $C_5$) ;

$R^4$ est choisi parmi des groupes

(a) -H, ou
(b) -alcoxy (en $C_1$ à $C_5$), qui est éventuellement substitué par 1, 2, ou 3 groupes $R^9$ choisis indépendamment.

**4.** Composé selon la revendication 1, et ses sels pharmaceutiquement acceptables et solvates, dans lequel $R^1$ représente un groupe -cycloalkyl (en $C_3$ à $C_{12}$)-alkyl (en $C_1$ à $C_6$)-, qui est éventuellement substitué par 1, 2 ou 3 groupes $R^9$ choisis indépendamment ;
$R^{2a}$ et $R^{2b}$ représentent chacun indépendamment un groupe -alkyl (en $C_1$ à $C_5$) ;
$R^4$ est choisi parmi -OH ou -$OCH_3$.

**5.** Composé selon l'une quelconque des revendications 1 à 4 ayant la formule IA, IB, IC ou ID :

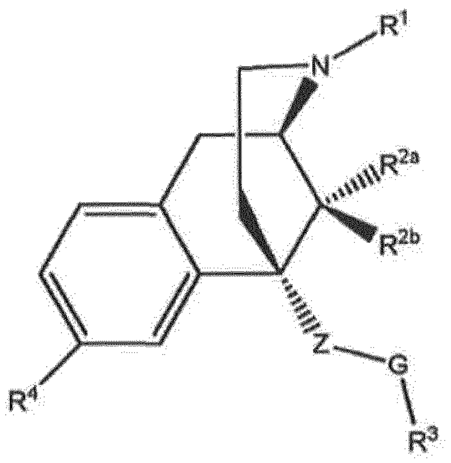

**IA**

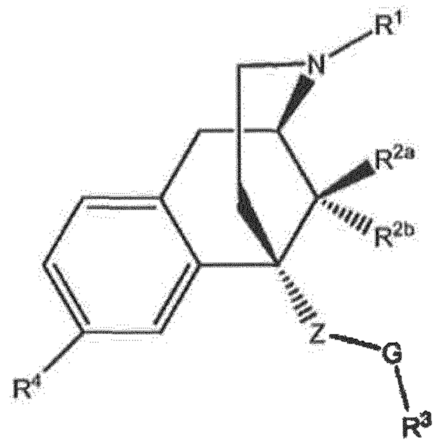

IB

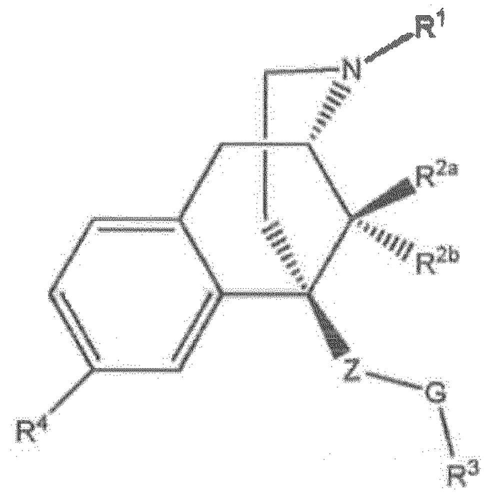

IC

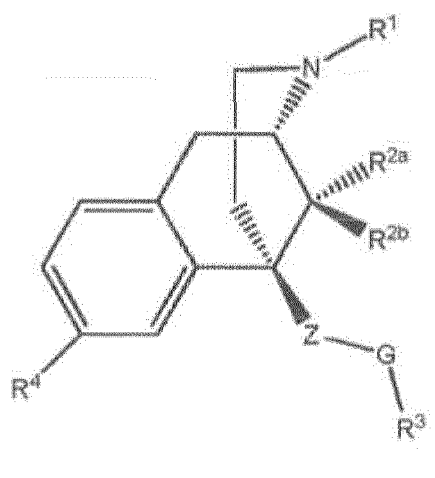

ID

**6.** Composé selon l'une quelconque des revendications 1 à 5, dans lequel Z est absent, représente un groupe méthylène, éthylène, propylène ou butylène.

**7.** Composé selon l'une quelconque des revendications 1 à 6, dans lequel G est choisi parmi des groupes

(i) -C(=O) ;
(ii) -alkylène (en $C_1$ à $C_6$), et de préférence méthylène, éthylène, et propylène ;
(iii) -alcénylène (en $C_2$ à $C_6$) et de préférence éthénylène et propénylène ; ou
(iv) G représente $NR^8$, dans lequel de préférence $R^8$ est choisi dans le groupe constitué de -H, des groupes -alkyle (en $C_1$ à $C_6$), -alcoxy (en $C_1$ à $C_{10}$), -cycloalkyle (en $C_3$ à $C_{12}$), et (cycloalkyl (en $C_3$ à $C_{12}$))-alkyl (en $C_1$ à $C_6$)-.

**8.** Composé selon l'une quelconque des revendications 1 et 3 à 7, dans lequel $R^3$ est choisi parmi des groupes

(i) H, alkyle (en $C_1$ à $C_6$), $NH_2$, NH-alkyle (en $C_1$ à $C_6$), ou-C(=O)-alkyle (en $C_1$ à $C_6$) ;
(ii) $-CONR^5R^6$ et -alkyl (en $C_1$ à $C_6$)-$CONR^5R^6$, dans lequel de préférence
$R^5$ et $R^6$ sont choisis chacun indépendamment parmi -H ou un groupe -alkyle (en $C_1$ à $C_6$) ;
(iii) $COOR^7$, dans lequel de préférence $R^7$ représente H ou un groupe -alkyle (en $C_1$ à $C_6$) ;
(iv) -aryle (de 6 à 14 chaînons) ou (aryl (de 6 à 14 chaînons)-alkyl (en $C_1$ à $C_6$)- ;
et de préférence phényle ou benzyle ;
(v) -hétérocycle (de 3 à 12 chaînons), (hétérocycle (de 3 à 12 chaînons))-alkyl (en $C_1$ à $C_6$)-, -hétéroaryle (de 5 à 12 chaînons), ou (hétéroaryle (de 5 à 12 chaînons))-alkyl (en $C_1$ à $C_6$)- ;
(vi) -alcoxy (en $C_1$ à $C_{10}$) ; ou
(vii) -C(=O)-hétéroaryle (de 5 à 12 chaînons) ou -C(=O)-aryle (de 6 à 14 chaînons) ;
et dans lequel de préférence $R^3$ est substitué par un, deux ou trois substituants choisis indépendamment dans le groupe constitué des groupes $-COOR^7$, $-NR^5R^6$, $-CONR^5R^6$, phényle, benzyle, -NH-C(=O)-aryle (de 6 à 14 chaînons), avec -NH-C(=O)-alkyl (en $C_1$ à $C_6$)-aryle (de 6 à 14 chaînons), -OH, hydroxyalkyl (en $C_1$ à $C_6$)-, et dihydroxyalkyle (en $C_1$ à $C_6$).

**9.** Composé selon l'une quelconque des revendications 1 et 3 à 5, dans lequel Z-G-$R^3$ est choisi parmi

(i) $-CH_2$-COOH, $-(CH_2)_3$-COOH, $(CH_2)_3$-$COOCH_3$, $-(CH_2)_3$-$CONH_2$ ;$-(CH_2)_2$-O-hétéroaryl (de 5 à 12 chaînons)-$COOR^7$ ou $-(CH_2)_3$-O-hétéroaryl (de 5 à 12 chaînons)-$COOR^7$, $-(CH_2)_3$-$CONR^5R^6$, $-(CH_2)_3$-C(=O)-NH-alkyl (en $C_1$ à $C_6$)-$COOR^7$, dihydroxyalkyle (en $C_1$ à $C_6$), -alcényl (en $C_1$ à $C_6$)-$COOR^7$, -alkyl (en $C_1$ à $C_6$)-NH-alkyle (en $C_1$ à $C_6$), $-(CH_2)_3$-C(=O)-NH-alkyl (en $C_1$ à $C_6$)-C(=O)-$NH_2$, ou -)alkyl (en $C_1$ à $C_6$)-C(=O)-NH-$CH(CH_3)$-$CONH_2$ ; ou
(ii) $-(CH_2)_2$-aryl (de 6 à 14 chaînons)-$COOR^7$, $-(CH_2)_2$-NH-C(=O)-(aryl (de 6 à 14 chaînons))-$COOR^7$, $-(CH_2)_2$-NH-C(=O)-(hétéroaryle (de 5 à 12 chaînons)), - $(CH_2)_2$-NH-C(=O)-(aryl (de 6 à 14 chaînons))-$COOR^7$, $-(CH_2)_2$-NH-C(=O)-(hétéroaryl (de 5 à 12 chaînons))-$COOR^7$, ou $-(CH_2)_2$-O-(aryl (de 6 à 14 chaînons))-$COOR^7$ ; ou
(iii) $-(CH_2)_2$-NH-CO-$CH_2$-$NH_2$, -alkyl (en $C_1$ à $C_6$-O-$CH_2$-phényle,-alkyl (en $C_1$ à $C_6$)-OH, -alkyl (en $C_1$ à $C_6$) -furanyle, ou -alkyl (en $C_1$ à $C_6$)-C(=O)H ou -alcényle (en $C_1$ à $C_6$) ; ou
(iv) $-(CH_2)_2$-NH-CO-$CH_2$-$NR^5R^6$.

**10.** Composé selon la revendication 1 choisi dans le groupe constitué de :

le (2*S*')-2-(2-((6*R*,11*R*)-3-(cyclopropylméthyl)-8-hydroxy-11-méthyl-1,2,3,4,5,6-hexahydro-2,6-méthanobenzo*[d]*-azocin-6-yl)acétamido)propanamide;
le 4-((6*S*,11*R*)-3-(cyclopropylméthyl)-8-hydroxy-11-méthyl-1,2,3,4,5,6-hexahydro-2,6-méthanobenzo*[d]*azocin-6-yl)-butanamide ;
l'acide 4-((6*S*,11*R*)-3-(cyclopropylméthyl)-8-méthoxy-11-méthyl-1,2,3,4,5,6-hexahydro-2,6-méthanobenzo*[d]*azocin-6-yl)butanoïque ;
l'acide 4-((6*S*,11*R*)-3-(cyclopropylméthyl)-8-hydroxy-11-méthyl-1,2,3,4,5,6-hexahydro-2,6-méthanobenzo*[d]*azocin-6-yl)butanoïque ;
l'acide 2-((6*R*,11*R*)-3-(cyclopropylméthyl)-8-méthoxy-11-méthyl-1,2,3,4,5,6-hexahydro-2,6-méthanobenzo*[d]*azocin-6-yl)acétique ;
le (2*S*)-3-((6*R*,11*R*)-3-(cyclopropylméthyl)-8-méthoxy-11-méthyl-1,2,3,4,5,6-hexahydro-2,6-méthanobenzo*[d]*azocin-6-yl)propane-1,2-diol ;
le (2*S*)-2-(2-((6*R*,11*R*)-3-(cyclopropylméthyl)-8-méthoxy-11-méthyl-1,2,3,4,5,6-hexahydro-2,6-méthanobenzo*[d]*-azocin-6-yl)acétamido)propanamide ;
le (*E*)-4-((6*S*,11*R*)-3-(cyclopropylméthyl)-8-méthoxy-11-méthyl-1,2,3,4,5,6-hexahydro-2,6-méthanobenzo*[d]*azocin-6-yl)but-2-énoate de méthyle ;
la 4-((6*S*,11*R*)-3-(cyclopropylméthyl)-8-méthoxy-11-méthyl-1,2,3,4,5,6-hexahydro-2,6-méthanobenzo*[d]*azocin-6-yl)-N-isobutylbutan-1-amine ;
le (2*R*)-5-((6*S*,11*R*)-3-(cyclopropylméthyl)-8-méthoxy-11-méthyl-1,2,3,4,5,6-hexahydro-2,6-méthanobenzo*[d]*azocin-6-yl)pentane-1,2-diol ;
le (2*S*)-5-((6*S*,11*R*)-3-(cyclopropylméthyl)-8-méthoxy-11-méthyl-1,2,3,4,5,6-hexahydro-2,6-méthanobenzo*[d]*azocin-6-yl)pentane-1,2-diol ;
la (6*S*,11*R*)-6-(4-(benzyloxy)butyl)-3-(cyclopropylméthyl)-8-méthoxy-11-méthyl-1,2,3,4,5,6-hexahydro-2,6-méthano-benzo*[d]*azocine ;
le 4-((6*S*,11*R*)-3-(cyclopropylméthyl)-8-méthoxy-11-méthyl-1,2,3,4,5,6-hexahydro-2,6-méthanobenzo*[d]*azocin-6-yl)-butan-1-ol ;
le N-((*S*)-1-amino-1-oxopropan-2-yl)-4-((6*S*,11*R*)-3-(cyclopropylméthyl)-8-méthoxy-11-méthyl-1,2,3,4,5,6-hexahydro-2,6-méthanobenzo*[d]*azocin-6-yl)butanamide ;
la (2*R*,6*S*,11*S*)-3-(cyclopropylméthyl)-6-(3-(furan-2-yl)-propyl)-8-méthoxy-11-méthyl-1,2,3,4,5,6-hexahydro-2,6-méthanobenzo*[d]*azocine ;
l'acide 5-(2-((2*R*,6*S*,11*R*)-3-(cyclopropylméthyl)-8-hydroxy-11-méthyl-1,2,3,4,5,6-hexahydro-2,6-méthano-benzo-*[d]*azocin-6-yl)éthoxy)nicotinique ;
le 4-((2*R*,6*R*,11*R*)-3-benzyl-8-hydroxy-11-méthyl-1,2,3,4,5,6-hexahydro-2,6-méthanobenzo*[d]*azocin-6-yl)-butanamide ;
le 4-((2*R*,6*R*,11*R*)-3-(cyclopropylméthyl)-8-hydroxy-11-méthyl-1,2,3,4,5,6-hexahydro-2,6-méthanobenzo*[d]*azocin-6-yl)butanamide ;
le (*S*)-2-(4-((2*R*,6*R*,11*R*)-3-(cyclopropylméthyl)-8-hydroxy-11-méthyl-1,2,3,4,5,6-hexahydro-2,6-méthanobenzo*[d]*-azocin-6-yl)butanamido)propanoate de méthyle ;
le *N-((S)*-1-amino-1-oxopropan-2-yl)-4-((2*R*,*6R*,11*R*)-3-(cyclopropylméthyl)-8-hydroxy-11-méthyl-1,2,3,4,5,6-hexahydro-2,6-méthanobenzo*[d]*azocin-6-yl)butanamide ;
le 4-((2*R*,6*R*,11*R*)-3-(cyclopropylméthyl)-8-hydroxy-11-méthyl-1,2,3,4,5,6-hexahydro-2,6-méthanobenzo*[d]*azocin-6-yl)butanoate de méthyle ;
l'acide 3-((2-((2*R*,6*R*,11*R*)-3-(cyclopropylméthyl)-8-méthoxy-11-méthyl-1,2,3,4,5,6-hexahydro-2,6-méthano-benzo-*[d]*azocin-6-yl)éthyl)carbamoyl)benzoïque ;
l'acide 4-((2-((2*R*,6*R*,11*R*)-3-(cyclopropylméthyl)-8-méthoxy-11-méthyl-1,2,3,4,5,6-hexahydro-2,6-méthano-benzo-*[d]*azocin-6-yl)éthyl)carbamoyl)benzoïque ;
le 3-((2-((2*R*,6*R*,11*R*)-3-(cyclopropylméthyl)-8-méthoxy-11-méthyl-1,2,3,4,5,6-hexahydro-2,6-méthanobenzo*[d]*azocin-6-yl)éthyl)carbamoyl)benzoate de méthyle ;
l'acide 4-(2-((2*R*,6*S*,11*R*)-3-(cyclopropylméthyl)-8-méthoxy-11-méthyl-1,2,3,4,5,6-hexahydro-2,6-méthano-benzo-*[d]*azocin-6-yl)éthoxy)benzoïque ;
l'acide 4-(2-((2*R*,6*S*,11*R*)-3-(cyclopropylméthyl)-8-hydroxy-11-méthyl-1,2,3,4,5,6-hexahydro-2,6-méthano-benzo-*[d]*-azocin-6-yl)éthoxy)benzoïque ;
l'acide 2-((2*S*,6*R*,11*S*)-3-(cyclopropylméthyl)-8-méthoxy-11-méthyl-1,2,3,4,5,6-hexahydro-2,6-méthanobenzo*[d]*-azocin-6-yl)acétique ;
le *N*-(2-((2*R*,6*R*,11*R*)-3-(cyclopropylméthyl)-8-hydroxy-11-méthyl-1,2,3,4,5,6-hexahydro-2,6-méthanobenzo*[d]*azocin-6-yl)éthyl)-2-(diméthylamino)acétamide ;
le 2-amino-*N*-(2-((2*R*,6*R*,11*R*)-3-(cyclopropylméthyl)-8-hydroxy-11-méthyl-1,2,3,4,5,6-hexahydro-2,6-méthanobenzo-*[d]*azocin-6-yl)éthyl)acétamide ; et leurs sels pharmaceutiquement acceptables et solvates.

**11.** Composition pharmaceutique comprenant une quantité efficace d'un composé selon l'une quelconque des revendications 1 à 10, ou de l'un de ses sels pharmaceutiquement acceptables ou solvates, et un support ou un excipient pharmaceutiquement acceptable.

**12.** Procédé *in* vitro pour la modulation de la fonction des récepteurs aux opiacés avec une quantité efficace d'un composé selon l'une quelconque des revendications 1 à 10, ou de l'un de ses sels pharmaceutiquement acceptables ou solvates,
dans lequel de préférence :

(i) le composé module la fonction des récepteurs aux opiacés $\mu$,
et
dans lequel de façon davantage préférée, le composé agit en tant qu'agoniste ou en tant qu'antagoniste au

niveau du récepteur aux opiacés μ ; ou
(ii) le composé agit en tant qu'agoniste au niveau du récepteur aux opiacés κ ; ou
(iii) le composé module la fonction du récepteur ORL-1, et dans lequel de façon davantage préférée, le composé agit en tant qu'antagoniste au niveau du récepteur ORL-1.

**13.** Procédé pour la préparation d'une composition, comprenant l'étape de mélange d'un composé selon l'une quelconque des revendications 1 à 10, ou de l'un de ses sels pharmaceutiquement acceptables ou solvates, avec un support ou un excipient pharmaceutiquement acceptable.

**14.** Composé selon l'une quelconque des revendications 1 à 10 ou l'un de ses sels pharmaceutiquement acceptables ou solvates, pour une utilisation dans le traitement, la prévention, ou l'amélioration d'une affection, dans lequel de préférence l'affection est la douleur ou la constipation.

**15.** Utilisation d'un composé selon l'une quelconque des revendications 1 à 10 ou de l'un de ses sels pharmaceutiquement acceptables ou solvates dans la fabrication d'un médicament utile pour le traitement ou la prévention d'une affection, dans laquelle de préférence l'affection est la douleur ou la constipation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4425353 A
- US 4406904 A
- US 4366325 A
- WO 9965485 A1
- WO 2009068989 A1
- WO 9948492 A
- WO 9854168 A
- US 5698155 A
- US 3845770 A
- US 3916899 A
- US 3536809 A
- US 3598123 A
- US 4008719 A
- US 5674533 A
- US 5059595 A
- US 5591767 A
- US 5120548 A
- US 5073543 A
- US 5639476 A
- US 5354556 A
- US 5733566 A
- US 6136839 A


### Non-patent literature cited in the description


- **K.M. FOLEY ; PAIN.** Cecil Textbook of Medicine. 1996, 100-107
- Opioid Analgesics. **GUTSTEIN ; HOWARD B. ; AKIL, HUDA.** Goodman & Gilman's The Pharmacological Basis of Therapeutics
- Opioid Analgesics. **GUTSTEIN ; HOWARD B. ; AKIL ; HUDA.** Goodman & Gilman's The Pharmacological Basis of Therapeutics
- **C. ALTIER et al.** ORL-1 receptor-mediated internalization of N-type calcium channels. *Nature Neuroscience,* 2005, vol. 9, 31
- **D. BARLOCCO et al.** The opioid-receptor-like 1 (ORL-1) as a potential target for new analgesics. *Eur. J. Med. Chem.,* 2000, vol. 35, 275
- **J.S. MOGIL et al.** Orphanin FQ is a functional anti-opioid peptide. *Neurosci.,* 1996, vol. 75, 333
- **K. LUTFY et al.** Tolerance develops to the inhibitory effect of orphanin FQ on morphine-induced antinociception in the rat. *NeuroReport,* 1999, vol. 10, 103
- **M.M. MORGAN et al.** Antinociception mediated by the periaqueductal gray is attenuated by orphanin FQ. *NeuroReport,* 1997, vol. 8, 3431
- **J. TIAN et al.** Involvement of endogenous Orphanin FQ in electroacupuncture-induced analgesia. *NeuroReport,* 1997, vol. 8, 497
- **J. TIAN et al.** Functional studies using antibodies against orphanin FQ/nociceptin. *Peptides,* 2000, vol. 21, 1047
- **H. UEDA et al.** Enhanced Spinal Nociceptin Receptor Expression Develops Morphine Tolerance and Dependence. *J. Neurosci.,* 2000, vol. 20, 7640
- **WOOD ; GALLIGAN.** Function of opioids in the enteric nervous system. *Neurogastroenterology & Motility,* 2004, vol. 16 (2), 17-28
- **M. FILIZOLA et al.** *Bioorganic and Medicinal Chemistry,* 2001, vol. 9 (1), 69-76
- **SHINKAI et al.** 4-aminoquinolines: Novel nociceptin antagonists with analgesic activity. *J. Med. Chem.,* 2000, vol. 43, 4667-4677
- **KAWAMOTO et al.** Discovery of the first potent and selective small molecule opioid receptor-like (ORL-1) antagonist: 1-[(3R,4R)-1-cyclooctylmethyl-3-hydroxymethyl-4-piperidyl]-3-ethyl-1,3-dihydro-2H-benzimidazol-2-one (J-113397). *J. Med. Chem.,* 1999, vol. 42, 5061-6063
- Pharmacodynamics: Mechanisms of Drug Action and the Relationship Between Drug Concentration and Effect. **ROSS ; KENAKIN.** Goodman & Gilman's the Pharmacological Basis of Therapeutics, 10th Ed. 2001, 31-32
- Isotopes in the Physical and Biomedical Sciences. **FILER.** Labeled Compounds. 1987, vol. 1
- **M. CAIRA et al.** *J. Pharmaceut. Sci.,* 2004, vol. 93 (3), 601-611
- **E.C. VAN TONDER et al.** *AAPS Pharm. Sci. Tech.,* 2004, vol. 5 (1
- **A.L. BINGHAM et al.** *Chem. Commun.,* 2001, 603-604
- **HATAKEYAMA et al.** *J. Am. Chem. Soc.,* 2005, vol. 127, 14192-14193
- **TROST et al.** *J. Am. Chem. Soc.,* 2003, vol. 125, 8744-8745
- **F.E. D'AMOUR et al.** A Method for Determining Loss of Pain Sensation. *J. Pharmacol. Exp. Ther.,* 1941, vol. 72, 74-79

- **G. WOOLFE ; A.D. MACDONALD.** *J. Pharmacol. Exp. Ther.,* 1944, vol. 80, 300-307
- **L. BARTHO et al.** Involvement of Capsaicin-sensitive Neurones in Hyperalgesia and Enhanced Opioid Antinociception in Inflammation. *Naunyn-Schmiedeberg's Archives of Pharmacol,* 1990, vol. 342, 666-670
- **Z. SELTZER et al.** A Novel Behavioral Model of Neuropathic Pain Disorders Produced in Rats by Partial Sciatic Nerve Injury. *Pain,* 1990, vol. 43, 205-218
- **S.H. KIM.** An Experimental Model for Peripheral Neuropathy Produced by Segmental Spinal Nerve Ligation in the Rat. *Pain,* 1992, vol. 50 (3), 355-363
- **C. STEIN.** Unilateral Inflammation of the Hindpaw in Rats as a Model of Prolonged Noxious Stimulation: Alterations in Behavior and Nociceptive Thresholds. *Pharmacol. Biochem. and Behavior,* 1988, vol. 31, 451-455
- **K. HARGREAVES et al.** A New and Sensitive Method for Measuring Thermal Nociception in Cutaneous Hyperalgesia. *Pain,* 1988, vol. 32 (1), 77-88
- **D. TORBATI et al.** *Intensive Care Med.,* 2000, vol. 26, 585-591
- Remington's Pharmaceutical Sciences. 1995, 1447-1676
- Handbook of Pharmaceutical Excipients. American Pharmaceutical Association, 1986
- Pharmaceutical Dosage Forms: Tablets. Marcel Dekker, Inc,
- Remington's Pharmaceutical Sciences. Mack Publishing, 1980, 1553-1593
- Pharmaceutical Dosage Forms: Disperse Systems,. Marcel Dekker, Inc,
- **LANGER.** *Science,* 1990, vol. 249, 1527-1533
- **TREAT et al.** *Liposomes in the Therapy of Infectious Disease and Cancer,* 1989, 317-327, 353-365
- **GOODSON.** Dental Applications. *Medical Applications of Controlled Release,* vol. 2, 115-138
- Applications and Evaluation. CRC Press, 1984
- **SEFTON.** *CRC Crit. Ref. Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574
- Medical Applications of Controlled Release. 1974
- Controlled Drug Bioavailability. Drug Product Design and Performance. 1984
- **RANGER ; PEPPAS.** *J. Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 71, 105
- Insel, Analgesic-Antipyretic and Anti-inflammatory Agents and Drugs Employed in the Treatment of Gout. **PAUL A.** Goodman & Gilman's The Pharmacological Basis of Therapeutics. 1996, 617-57
- Analgesic, Antipyretic and Anti-Inflammatory Drugs in Remington. **GLEN R. HANSON.** The Science and Practice of Pharmacy Vol IA. 1995, 1196-1221
- *J. Org. Chem.,* 1984, vol. 49, 2081
- *Tetrahedron Lett.,* 2010, vol. 51, 2359